(19) Europäisches Patentamt
European Patent Office
Office européen des brevets

(11) EP 1 187 842 B1

(12) EUROPEAN PATENT SPECIFICATION

(45) Date of publication and mention of the grant of the patent:
**26.04.2006 Bulletin 2006/17**

(21) Application number: **00936337.5**

(22) Date of filing: **26.05.2000**

(51) Int Cl.:
***C07H 21/04*** *(2006.01)* ***C12P 21/06*** *(2006.01)*
***A61K 38/00*** *(2006.01)*

(86) International application number:
**PCT/US2000/014554**

(87) International publication number:
**WO 2000/073321 (07.12.2000 Gazette 2000/49)**

(54) **Fc fusion proteins of the HUMAN TUMOR NECROSIS FACTOR RECEPTOR TR10**

Fc-Fusionsproteine des MENSCHLICHEN TUMOR-NEKROSE-FAKTOR-Rezeptors TR10

Protéine de fusion entre Fc et TR10, RECEPTEUR DE FACTEUR DE NECROSE TUMORALE HUMAIN

(84) Designated Contracting States:
**AT BE CH CY DE DK ES FI FR GB GR IE IT LI LU MC NL PT SE**

(30) Priority: **28.05.1999 US 136786 P**
**07.07.1999 US 142563 P**
**15.07.1999 US 144023 P**

(43) Date of publication of application:
**20.03.2002 Bulletin 2002/12**

(73) Proprietor: **HUMAN GENOME SCIENCES, INC.**
**Rockville, MD 20850 (US)**

(72) Inventors:
- **ROSEN, Craig, A.**
  **Laytonsville, MD 20882 (US)**
- **NI, Jian**
  **Rockville, MD 20853 (US)**

(74) Representative: **Vossius & Partner**
**Siebertstrasse 4**
**81675 München (DE)**

(56) References cited:
**WO-A-98/54202**
**WO-A-99/03992**
**WO-A-99/10484**
**WO-A1-99/03922**
**WO-A1-99/07850**
**WO-A1-99/10484**

- **DEGLI ESPOSITI M A ET AL: "The novel receptor TRAIL-R4 induces NF-kappaB and protects against TRAIL-mediated apoptosis, yet retains an incomplete death domain" IMMUNITY, CELL PRESS, US, vol. 7, no. 6, 1 December 1997 (1997-12-01), pages 813-820, XP002087442 ISSN: 1074-7613**
- **KORNBLAU S M ET AL: "Apoptosis regulating proteins as targets of therapy for haematological malignancies" EXPERT OPINION ON INVESTIGATIONAL DRUGS 1999 UNITED KINGDOM, vol. 8, no. 12, 1999, pages 2027-2057, XP009014371 ISSN: 1354-3784**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

## FIELD OF THE INVENTION

The present invention relates to a novel member of the tumor necrosis factor family of receptors. More specifically, isolated nucleic acid molecules are provided encoding a novel human tumor necrosis factor receptor, TR10. TR10 polypeptides are also provided, as are vectors, host cells, and recombinant methods for producing the same, and antibodies that bind to TR10 polypeptides. The invention further relates to screening methods for identifying agonists and antagonists of TR10 activity.

## BACKGROUND OF THE INVENTION

Many biological actions, for instance, response to certain stimuli and natural biological processes, are controlled by factors, such as cytokines. Many cytokines act through receptors by engaging the receptor and producing an intracellular response.

For example, tumor necrosis factors (TNF) alpha and beta are cytokines, which act through TNF receptors to regulate numerous biological processes, including protection against infection and induction of shock and inflammatory disease. The TNF molecules belong to the "TNF-ligand" superfamily, and act together with their receptors or counter-ligands, the "TNF-receptor" superfamily. So far, nine members of the TNF ligand superfamily have been identified and ten members of the TNF-receptor superfamily have been characterized.

Among the ligands there are included TNF-, lymphotoxin- (LT- , also known as TNF-β), LT-β (found in complex heterotrimer LT-2-β), FasL, CD40L, CD27L, CD30L, 4-IBBL, OX40L and nerve growth factor (NGF). The superfamily of TNF receptors includes the p55TNF receptor, p75TNF receptor, TNF receptor-related protein, FAS antigen or APO-1, CD40, CD27, CD30, 4-1BB, OX40, low affinity p75 and NGF-receptor (A. Meager, *Biologicals 22*:291-295 (1994)).

Many members of the TNF-ligand superfamily are expressed by activated T-cells, implying that they are necessary for T-cell interactions with other cell types which underlie cell ontogeny and functions. (A. Meager, *supra*).

Considerable insight into the essential functions of several members of the TNF receptor family has been gained from the identification and creation of mutants that abolish the expression of these proteins. For example, naturally occurring mutations in the FAS antigen and its ligand cause lymphoproliferative disease (R. Watanabe-Fukunaga *et al., Nature 356*:314 (1992)), perhaps reflecting a failure of programmed cell death. Mutations of the CD40 ligand cause an X-linked immunodeficiency state characterized by high levels of immunoglobulin M and low levels of immunoglobulin G in plasma, indicating faulty T-cell-dependent B-cell activation (R.C. Allen *et al., Science 259*:990 (1993)). Targeted mutations of the low affinity nerve growth factor receptor cause a disorder characterized by faulty sensory innovation of peripheral structures (K.F. *Lee et al., Cell 69*:737 (1992)).

TNF and LT- are capable of binding to two TNF receptors (the 55- and 75-kd TNF receptors). A large number of biological effects elicited by TNF and LT-, acting through their receptors, include hemorrhagic necrosis of transplanted tumors, cytotoxicity, a role in endotoxic shock, inflammation, immunoregulation, proliferation and anti-viral responses, as well as protection against the deleterious effects of ionizing radiation. TNF and LT- are involved in the pathogenesis of a wide range of diseases, including endotoxic shock, cerebral malaria, tumors, autoimmune disease, AIDS and graft-host rejection (B. Beutler and C. Von Huffel, *Science 264*:667-668 (1994)). Mutations in the p55 receptor cause increased susceptibility to microbial infection.

Moreover, an about 80 amino acid domain near the C-terminus of TNFR1 (p55) and Fas was reported as the "death domain," which is responsible for transducing signals for programmed cell death (Tartaglia *et al., Cell 74*:845 (1993)).

Apoptosis, or programmed cell death, is a physiologic process essential to the normal development and homeostasis of multicellular organisms (H. Steller, *Science* 267:1445-1449 (1995)). Derangements of apoptosis contribute to the pathogenesis of several human diseases including cancer, neurodegenerative disorders, and acquired immune deficiency syndrome (C.B. Thompson, *Science* 267:1456-1462 (1995)). Recently, much attention has focused on the signal transduction and biological function of two cell surface death receptors, Fas/APO-1 and TNFR-1 (J.L. Cleveland *et al., Cell* 81:479-482 (1995); A. Fraser *et al., Cell* 85:781-784 (1996); S. Nagata *et al., Science* 267:1449-56 (1995)). Both are members of the TNF receptor family, which also include TNFR-2, low affinity NGFR, CD40, and CD30, among others (C.A. Smith *et al., Science 248*: 1019-23 (1990); M. Tewari *et al.,* in *Modular Texts in Molecular and Cell Biology* M. Purton, Heldin, Carl, Ed. (Chapman and Hall, London, 1995). While family members are defined by the presence of cysteine-rich repeats in their extracellular domains, Fas/APO-1 and TNFR-1 also share a region of intracellular homology, appropriately designated the "death domain," which is distantly related to the Drosophila suicide gene, reaper (P. Golstein *et al., Cell 81*:185-6 (1995); K. White *et al., Science* 264:677-83 (1994)). This shared death domain suggests that both receptors interact with a related set of signal transducing molecules that, until recently, remained unidentified. Activation of Fas/APO-1 recruits the death domain-containing adapter molecule FADD/MORT1 (A.M. Chinnaiyan *et al., Cell 81*:

505-512 (1995); M. P. Boldin *et al., J. Biol. Chem. 270*:7795-8 (1995); F.C. Kischkel *et al., EMBO 14*:5579-5588 (1995)), which in turn binds and presumably activates FLICE/MACH 1, a member of the ICE/CED-3 family of pro-apoptotic proteases (M. Muzio *et al., Cell 85*: 817-827 (1996): M.P. Boldin *et al., Cell 85*:803-815 (1996)). While the central role of Fas/APO-1 is to trigger cell death. TNFR-1 can signal an array of diverse biological activities-many of which stem from its ability to activate NF-kB (L-A. Tartaglia *et al., Immunol Today 13*:151-153 (1992)). Accordingly. TNFR-1 recruits the multivalent adapter molecule TRADD, which like FADD, also contains a death domain (H. Hsu *et al., Cell 81*:495-504 (1995); H. *Hsu et al., Cell* 84:299-308 (1996)). Through its associations with a number of signaling molecules including FADD, TRAF2, and RIP, TRADD can signal both apoptosis and NF-kB activation(H. Hsu *et al., Cell 84*:299-308 (1996): H. *Hsu et al.. Immunity* 4:387-396 (1996)).

Recently, a new apoptosis inducing TNF ligand has been discovered. S.R. Wiley *et al., Immunity* 3:673-682 (1995), named the new molecule, "TNF-related apoptosis-inducing ligand" or "TRAIL." R.M. Pitti *et al., J. Biol. Chem. 271:*12687-12690 (1996), named the molecule "Apo-2 ligand" or "Apo-2L." This molecule was also disclosed in co-pending U.S. provisional patent application no. 60/013405. For convenience, this molecule will be referred to herein as TRAIL.

Unlike FAS ligand, whose transcripts appear to be largely restricted to stimulated T-cells, significant levels of TRAIL are detected in many human tissues (e.g., spleen, lung, prostate, thymus, ovary, small intestine, colon, peripheral blood lymphocytes, placenta, kidney), and it is constitutively transcribed by some cell lines. It has been shown that TRAIL acts independently from the FAS ligand (S.R. *Wiley et al., supra*). It has also been shown that TRAIL activates apoptosis rapidly, within a time frame that is similar to death signaling by Fas/Apo-1L, but much faster than TNF-induced apoptosis. S.A. Marsters *et al., Current Biology 6*:750-752 (1996). The inability of TRAIL to bind TNFR-1, Fas, or the recently identified DR3, suggests that TRAIL may interact with a unique receptor(s).

Work to date suggests that there are several unique TNF receptors for TRAIL. In co-pending U.S. provisional patent application no. 60/035,722, one novel death domain containing receptor for TRAIL, DR4, was disclosed. See, Pan *et al., Science* 276:111-113 (April 1997). In co-pending U.S. provisional patent application no. 60/040,846, a novel death domain containing receptor, DR5 (TR7), was disclosed. This receptor has now been shown to bind TRAIL. In co-pending U.S. provisional patent application no. 60/035,496, another receptor, TR5, was disclosed. This receptor has also now been shown to bind TRAIL, however, TR5 has been shown to be a non-signaling decoy receptor which antagonizes apoptosis.

The effects of TNF family ligands and receptors are varied and influence numerous functions, both normal and abnormal, in the biological processes of the mammalian system. There is a clear need, therefore, for identification and characterization of such receptors and ligands that influence biological activity, both normally and in disease states. In particular, there is a need to isolate and characterize additional novel receptors that bind TRAIL.

## SUMMARY OF THE INVENTION

The present invention provides isolated nucleic acid molecules comprising or, alternatively, consisting of a polynucleotide encoding the TR 10 receptor having the amino acid sequence shown in SEQ ID NO:2 or the amino acid sequence encoded by the cDNA clone deposited as American Type Culture Collection ("ATCC") Deposit No. 209040 on May 15, 1997. The ATCC is located at 10801 University Boulevard, Manassas, Virginia 20110-2209.

The present invention also relates to recombinant vectors, which include the isolated nucleic acid molecules of the present invention, and to host cells containing the recombinant vectors, as well as to methods of making such vectors and host cells and for using them for production of TR10 polypeptides or peptides by recombinant techniques.

The invention further provides an isolated TR10 polypeptide having an amino acid sequence encoded by a polynucleotide described herein.

The present invention also provides diagnostic assays such as quantitative and diagnostic assays for detecting levels of TRIO protein. Thus, for instance, a diagnostic assay in accordance with the invention for detecting over-expression of TRIO, or soluble form thereof, compared to normal control tissue samples may be used to detect the presence of tumors.

Tumor Necrosis Factor (TNF) family ligands are known to be among the most pleiotropic cytokines, inducing a large number of cellular responses, including cytotoxicity, anti-viral activity, immunoregulatory activities, and the transcriptional regulation of several genes. Cellular response to TNF-family ligands include not only normal physiological responses, but also diseases associated with increased apoptosis or the inhibition of apoptosis. Apoptosis-programmed cell death is a physiological mechanism involved in the deletion of peripheral T lymphocytes of the immune system, and its dysregulation can lead to a number of different pathogenic processes. Diseases associated with increased cell survival, or the inhibition of apoptosis, include cancers, autoimmune disorders, viral infections, inflammation, graft vs. host disease, acute graft rejection, and chronic graft rejection. Diseases associated with increased apoptosis include AIDS, neurodegenerative disorders, myelodysplastic syndromes, ischemic injury, toxin-induced liver disease, septic shock, cachexia, and anorexia.

Thus, the invention further provides a method for inhibiting apoptosis induced by a TNF-family ligand, which involves administering to a cell which expresses the TR10 polypeptide an effective amount of an agonist capable of increasing TR10 mediated signaling. Preferably, TR10 mediated signaling is increased to treat a disease wherein increased apoptosis is exhibited.

In a further aspect; the present invention is directed to a method for enhancing apoptosis induced by a TNF-family ligand, which involves administering to a cell which expresses the TR10 polypeptide an effective amount of an antagonist capable of decreasing TR10 mediated activity. Preferably, TR10 mediated activity is decreased to treat a disease wherein decreased apoptosis is exhibited.

Whether any candidate "agonist" or "antagonist" of the present invention can enhance or inhibit apoptosis can be determined using art-known TNF-family ligand/receptor cellular response assays, including those described in more detail below. Thus, in a further aspect, a screening method is provided for determining whether a candidate agonist or antagonist is capable of enhancing or inhibiting a cellular response to a TNF-family ligand. The method involves contacting cells which express the TR10 polypeptide with a candidate compound and a TNF-family ligand, assaying a cellular response, and comparing the cellular response to a standard cellular response, the standard being assayed when contact is made with the ligand in absence of the candidate compound, whereby an increased cellular response over the standard indicates that the candidate compound is an agonist of the ligand/receptor signaling pathway and a decreased cellular response compared to the standard indicates that the candidate compound is an antagonist of the ligand/receptor signaling pathway. By the invention, a cell expressing the TR10 polypeptide can be contacted with either an endogenous or exogenously administered TNF-family ligand.

## BRIEF DESCRIPTION OF THE FIGURES

Figures 1A-D shows the nucleotide (SEQ ID NO:1) and deduced amino acid sequence (SEQ ID NO:2) of the TR10 receptor. Predicted amino acids 155 constitute the signal peptide (amino acid residues from about -55 to about -1 in SEQ ID NO:2); amino acids 56 212 constitute the extracellular domain (amino acid residues from about 1 to about 157 in SEQ ID NO:2); amino acids 213 230 constitute the transmembrane domain (amino acid residues from about 158 to about 175 in SEQ ID NO:2); and amino acids 231 386 constitute the intracellular domain (amino acid residues from about 176 to about 331 in SEQ ID NO:2), of which amino acids 353 363 constitute the partial death domain (amino acid residues from about 298 to about 308 in SEQ ID NO:2).

Figure 2 shows the regions of similarity between the amino acid sequences of the TR10 receptor protein (SEQ ID NO:2), and the Fas receptor (SEQ ID NO:3), NGFR p75 (SEQ ID NO:4), human TNFR 1 (SEQ ID NO:5), and DR4 (SEQ ID NO:6).

Figure 3 shows an analysis of the TR10 amino acid sequence. Alpha, beta, turn and coil regions; hydrophilicity and hydrophobicity; amphipathic regions; flexible regions; antigenic index and surface probability are shown. In the "Antigenic Index - Jameson-Wolf" graph, amino acid residues about 57 to about 113. about 130 to about 197, and about 250 to about 283 in Figures 1A-D correspond to the shown highly antigenic regions of the TR10 protein. These highly antigenic fragments in Figures 1A-D correspond to the following fragments, respectively, in SEQ ID NO:2: amino acid residues from about 2 to about 58. from about 75 to about 142, and from about 195 to about 228.

Figure 4 shows the effect of TR10 on the cytokine stimulated proliferation and differentiation of mouse bone marrow cells expressed as a measure of tritiated thymidine incorporation (See, e.g.. Example 16). Mouse bone marrow cells are grown in the presence or absence of cytokines (stem cell factor (SCF) and IL-3), and TR10. Tritiated thymidine is added and the level of radioactivity incorporated into cellular DNA (a measure of cell proliferation) is determined by scintillation spectrometry. Addition of TR10 to cells in the presence of SCF plus IL-3 resulted in a dose-dependant decrease in the cell proliferation response.

## DETAILED DESCRIPTION OF THE INVENTION

The present invention provides isolated nucleic acid molecules comprising a polynucleotide encoding a TR10 polypeptide having the amino acid sequence shown in Figures 1A-D (SEQ ID NO:2), which was determined by sequencing a cloned cDNA. The TR10 polypeptide of the present invention shares sequence homology with human NGFR, TNFRI, DR4, and Fas (Figure 2). The nucleotide sequence shown in Figures 1A-D (SEQ ID NO:1) was obtained by sequencing a cDNA clone, which was deposited on May 15, 1997 at the American Type Culture Collection, , and given Accession Number 209040. The deposited clone is inserted in the pCMVSport 2.0 plasmid (Life Technologies, Rockville, MD) using the Sal I/Not I restriction endonuclease cleavage sites.

## ***Nucleic Acid Molecules***

Unless otherwise indicated, all nucleotide sequences determined by sequencing a DNA molecule herein were determined using an automated DNA sequencer (such as the Model 373 from Applied Biosystems, Inc.), and all amino acid sequences of polypeptides encoded by DNA molecules determined herein were predicted by translation of a DNA sequence determined as above. Therefore, as is known in the art for any DNA sequence determined by this automated approach, any nucleotide sequence determined herein may contain some errors. Nucleotide sequences determined by automation are typically at least about 90% identical, more typically at least about 95% to at least about 99.9% identical to the actual nucleotide sequence of the sequenced DNA molecule. The actual sequence can be more precisely determined by other approaches including manual DNA sequencing methods well known in the art. As is also known in the art, a single insertion or deletion in a determined nucleotide sequence compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion.

Using the information provided herein, such as the nucleic acid sequence set out in SEQ ID NO:1, a nucleic acid molecule of the present invention encoding a TR10 polypeptide may be obtained using standard cloning and screening procedures, such as those for cloning cDNAs using mRNA as starting material. Illustrative of the invention, the nucleic acid molecule described in SEQ ID NO: 1 was discovered in a cDNA library derived from keratinocytes. The gene of the present invention has also been identified in cDNA libraries from the following tissues: fetal liver, peripheral blood lymphocytes (PBL), lung, kidney, small intestine, colon, endothelial cells, and monocyte activated tissue. Furthermore, the following cancer cell lines express TR10: Hela cell S3, SW480 (colorectal adenocarcinoma), and A549 (lung carcinoma).

The determined nucleotide sequence of the TR10 cDNA of SEQ ID NO: 1 contains an open reading frame encoding a protein of about 331 amino acid residues, with a predicted leader sequence of about 55 amino acid residues, and a deduced molecular weight of about 42 kDa. The amino acid sequence of the predicted mature TR10 receptor is shown in SEQ ID NO:2 from amino acid residue about 1 to residue about 331. Of known members of the TNF receptor family, the TR10 polypeptide of the invention shares the greatest degree of homology with human DR4 (See Figure 2), including significant sequence homology over multiple cysteine rich domains.

Owing to the sequence homology exhibited between TR10 and DR4 (and other death domain containing receptors), it was immediately recognized that TR10 would likely also bind to TRAIL The cytoplasmic domain, interestingly, contains only a partial (or truncated) death domain. As described in Example 5, below, TR10 binds TRAIL but does not appear to cause cell death. TR10 binding of TRAIL, to the contrary, antagonizes apoptosis. Such antagonistic effect on TRAIL induced apoptosis can be achieved both through ectopic expression of TR10 and through exogenous administration of soluble TR10.

To examine the tissue distribution of TR10, Northern blot analysis was performed. A single transcript was detected in multiple human tissues at varying levels of expression, including, heart, lung, brain, placenta, liver, skeletal muscle, kidney, pancreas, spleen, thymus, prostate, testis, ovary, small intestine, colon, PBLs, lymph node, bone marrow and fetal liver. TR10 expression was not observed in most cancer cell lines tested. See Example 7, below.

As indicated, the present invention also provides the mature form(s) of the TR10 receptor of the present invention. According to the signal hypothesis, proteins secreted by mammalian cells have a signal or secretory leader sequence which is cleaved from the mature protein once export of the growing protein chain across the rough endoplasmic reticulum has been initiated. Most mammalian cells and even insect cells cleave secreted proteins with the same specificity. However, in some cases, cleavage of a secreted protein is not entirely uniform, which results in two or more mature species on the protein. Further, it has long been known that the cleavage specificity of a secreted protein is ultimately determined by the primary structure of the complete protein, that is, it is inherent in the amino acid sequence of the polypeptide.

Therefore, the present invention provides a nucleotide sequence encoding the mature TR10 polypeptide having the amino acid sequence encoded by the cDNA clone contained in the host identified as ATCC Deposit No. 209040, and as shown in Figures 1A-D (SEQ ID NO:2). By the mature TR10 protein having the amino acid sequence encoded by the cDNA clones contained in the host identified as ATCC Deposit No. 209040 is meant the mature form(s) of the TR10 receptor produced by expression in a mammalian cell (e.g., COS cells, as described below) of the complete open reading frame encoded by the human DNA sequence of the clone contained in the vector in the deposited host. As indicated below, the mature TR10 receptor having the amino acid sequence encoded by the cDNA clone contained in ATCC Deposit No. 209040, may or may not differ from the predicted mature TR10 protein shown in SEQ ID NO:2 (amino acids from about 1 to about 331) depending on the accuracy of the predicted cleavage site based on computer analysis.

Methods for predicting whether a protein has a secretory leader as well as the cleavage point for that leader sequence are available. For instance, the method of McGeoch *(Virus Res. 3*:271-286 (1985)) and von Heinje *(Nucleic Acids Res. 14*:4683-4690 (1986)) can be used. The accuracy of predicting the cleavage points of known mammalian secretory proteins for each of these methods is in the range of 75-80%, von Heinje, *supra.* However, the two methods do not always produce the same predicted cleavage point(s) for a given protein.

In the present case, the predicted amino acid sequence of the complete TR10 polypeptide of the present invention was analyzed by a computer program ("PSORT"). *See* K. Nakai and M. Kanehisa, *Genomics 14*:897-911 (1992). PSORT is an expert system for predicting the cellular location of a protein based on the amino acid sequence. As part of this computational prediction of localization, the methods of McGeoch and von Heinje are incorporated. The analysis by the PSORT program predicted the cleavage site between amino acids -1 and 1 in SEQ ID NO:2. Thereafter, the complete amino acid sequences were further analyzed by visual inspection, applying a simple form of the (-1,-3) rule of von Heinje, von Heinje, *supra.* Thus, the leader sequence for the TR10 protein is predicted to consist of amino acid residues from about -55 to about -1 in SEQ ID NO:2, while the mature TRIO protein is predicted to consist of residues from about 1-331 in SEQ ID NO:2.

As one of ordinary skill would appreciate, due to the possibilities of sequencing errors, as well as the variability of cleavage sites for leaders in different known proteins, the predicted TR10 polypeptide encoded by the deposited cDNA comprises about 386 amino acids, but may be anywhere in the range of 376-396 amino acids: and the predicted leader sequence of this protein is about 55 amino acids, but may be anywhere in the range of about 45 to about 65 amino acids. It will further be appreciated that, the domains described herein have been predicted by computer analysis, and accordingly, that depending on the analytical criteria used for identifying various functional domains, the exact "address" of, for example, the extracellular domain, intracellular domain, partial death domain, cysteine-rich motifs, and transmembrane domain of TR10 may differ slightly. For example, the exact location of the TR10 extracellular domain in Figures 1A-D (SEQ ID NO:2) may vary slightly (e.g., the address may "shift" by about I to about 20 residues, more likely about I to about 5 residues) depending on the criteria used to define the domain. In any event, as discussed further below, the invention further provides polypeptides having various residues deleted from the N-terminus and/or C-terminus of the complete TR10, including polypeptides lacking one or more amino acids from the N-termini of the extracellular domain described herein, which constitute soluble forms of the extracellular domain of the TR10 polypeptides.

As indicated, nucleic acid molecules of the present invention may be in the form of RNA, such as mRNA, or in the form of DNA, including, for instance, cDNA and genomic DNA obtained by cloning or produced synthetically. The DNA may be double-stranded or single-stranded. Single-stranded DNA may be the coding strand, also known as the sense strand, or it may be the non-coding strand, also referred to as the anti-sense strand.

By "isolated" nucleic acid molecule(s) is intended a nucleic acid molecule, DNA or RNA, which has been removed from its native environment For example, recombinant DNA molecules contained in a vector are considered isolated for the purposes of the present invention. Further examples of isolated DNA molecules include recombinant DNA molecules maintained in heterologous host cells or purified (partially or substantially) DNA molecules in solution. Isolated RNA molecules include *in vivo* or *in vitro* RNA transcripts of the DNA molecules of the present invention. Isolated nucleic acid molecules according to the present invention further include such molecules produced synthetically. However, a nucleic acid molecule contained in a clone that is a member of a mixed clone library (e.g., a genomic or cDNA library) and that has not been isolated from other clones of the library (e.g., in the form of a homogeneous solution containing the clone without other members of the library) or a chromosome isolated or removed from a cell or a cell lysate (e.g., a "chromosome spread", as in a karyotype), is not "isolated" for the purposes of this invention.

Isolated nucleic acid molecules of the present invention include DNA molecules comprising or, alternatively, consisting of an open reading frame (ORF) shown in SEQ ID NO:1; DNA molecules comprising or, alternatively, consisting of the coding sequence for the mature TR10 protein; and DNA molecules comprising or, alternatively, consisting of a sequence substantially different from those described above, but which, due to the degeneracy of the genetic code, still encode the TR10 protein. Of course, the genetic code is well known in the art. Thus, it would be routine for one skilled in the art to generate such degenerate variants.

In addition, the invention provides nucleic acid molecules having nucleotide sequences related to extensive portions of SEQ ID NO: I which have been determined from the following related cDNA clones: HSABD50R (SEQ ID NO:7), HGBDL20R (SEQ ID NO:8), and HELDL61 R (SEQ ID NO:9), and AA150849 (SEQ ID NO: 15).

In another aspect, the invention provides isolated nucleic acid molecules encoding the TR10 polypeptide having an amino acid sequence as encoded by the cDNA clone contained in the plasmid deposited as ATCC Deposit No. 209040 on May 15, 1997. In a further embodiment, nucleic acid molecules are provided that encode the mature TR10 polypeptide or the full length TR10 polypeptide lacking the N-terminal methionine. The invention further provides an isolated nucleic acid molecule having the nucleotide sequence shown in SEQ ID NO: 1 or the nucleotide sequence of the TR10 cDNA contained in the above-described deposited clone, or a nucleic acid molecule having a sequence complementary to one of the above sequences. Such isolated molecules, particularly DNA molecules, are useful as probes for gene mapping by *in situ* hybridization with chromosomes, and for detecting expression of the TR10 gene in human tissue, for instance, by Northern blot analysis.

The present invention is further directed to fragments of the isolated nucleic acid molecules described herein. By a fragment of an isolated DNA molecule having the nucleotide sequence of the deposited cDNA or the nucleotide sequence shown in SEQ ID NO:1 is intended DNA fragments at least about 15nt, and more preferably at least about 20 nt, still more preferably at least about 30 nt, and even more preferably, at least about 40 nt in length which are useful as diagnostic probes and primers as discussed herein. Of course, larger fragments 50-1500 nt in length are also useful according to the present invention, as are fragments corresponding to most, if not all, of the nucleotide sequence of the deposited cDNA or as shown in SEQ ID NO:1. By a fragment at least 20 nt in length, for example, is intended fragments which include 20 or more contiguous bases from the nucleotide sequence of the deposited cDNA or the nucleotide sequence as shown in SEQ ID NO:1. In this context "about" includes the particularly recited size, larger or smaller by several (5,4,3,2, or 1) nucleotides, at either terminus or at both termini.

Representative examples of TR10 polynucleotide fragments of the invention include, for example, fragments that comprise, or alternatively, consist of, a sequence from about nucleotide I to 50, 51 to 108, 109 to 159, 160 to 210, 211 to 261, 262 to 273, 274 to 324, 325 to 375, 376 to 426, 427 to 477, 478 to 528, 529 to 579, 580 to 630, 631 to 681, 682 to 732, 733 to 744, 745 to 798. 799 to 849. 850 to 900, 901 to 951, 952 to 1002, 1003 to 1053, 1054 to 1104, 1105 to 1155, 1156 to 1164, 1165 to 1197, 1198 to 1248, 1249 to 1266, 1267 to 1317, 1318 to 1368, 1369 to 1419, 1420 to 1470, 1471 to 1521, 1522 to 1572, 1573 to 1623, 1624 to 1674, 1675 to 1725, 1726 to 1776, 1777 to 1827, 1828 to 1878, 1879 to 1929, 1930 to 1980, 1981 to 2031. 2032 to 2082, 2083 to 2133, 2134 to 2184, 2185 to 2235, 2236 to 2286, 2287 to 2337, 2338 to 2388, 2389 to 2489, 2490 to 2540, 2451 to 2501, 2502 to 2552, 2553 to 2603, 2604 to 2654, 2655 to 2705, 2706 to 2756, 2806 to 2856, 2857 to 2907, 2908 to 2958, 2959 to 3009, 3010 to 3060, 3061 to 3111, 3112 to 3162, 3163 to 3213, 3214 to 3264, 3265 to 3315, 3316 to 3366, 3367 to 3417, 3418 to 3468, 3469 to 3519, and/or 3520 to 3566 of SEQ ID NO:1, or the complementary strand thereto, or the cDNA contained in the deposited clone. In this context "about" includes the particularly recited ranges, larger or smaller by several (5,4,3,2, or 1) nucleotides, at either terminus or at both termini. In specific embodiments, the polynucleotide fragments of the invention comprise, or alternatively, consist of, a sequence from nucleotide 109 to 744, 274 to 744, 745 to 798, and 799 to 1266 of SEQ ID NO:1, or the complementary strand thereto. Polynucleotides that hybridize to these polynucleotide fragments are also encompassed by the invention.

Preferably, the polynucleotide fragments of the invention encode a polypeptide which demonstrates a TR10 functional activity. By a polypeptide demonstrating a TR10 "functional activity" is meant, a polypeptide capable of displaying one or more known functional activities associated with a full-length (complete) TR10 protein. Such functional activities include, but are not limited to, biological activity (e.g., to inhibit TRAIL induced apoptosis *in vitro or in vivo,* to regulate (e.g., inhibit) B cell proliferation (see, e.g,. Example 38), and/or to regulate (e.g., inhibit) hematopoiesis), antigenicity (ability to bind (or compete with a TR10 polypeptide for binding) to an anti-TR10 antibody), immunogenicity (ability to generate antibody which binds to a TR10 polypeptide), ability to form multimers with TR10 polypeptides of the invention, and ability to bind to a receptor or ligand for a TR10 polypeptide (e.g., TRAIL, and/or receptors located on the surface of NK cells, and/or endothelial cells).

The functional activity of TR10 polypeptides, and fragments, variants derivatives, and analogs thereof, can be assayed by various methods.

For example, in one embodiment where one is assaying for the ability to bind or compete with full-length TR10 polypeptide for binding to anti-TR10 antibody, various immunoassays known in the art can be used, including but not limited to, competitive and non-competitive assay systems using techniques such as radioimmunoassays, ELISA (enzyme linked immunosorbent assay), "sandwich" immunoassays, immunoradiometric assays, gel diffusion precipitation reactions, immunodiffusion assays, *in situ* immunoassays (using colloidal gold, enzyme or radioisotope labels, for example), western blots, precipitation reactions, agglutination assays (e.g., gel agglutination assays, hemagglutination assays), complement fixation assays, immunofluorescence assays, protein A assays, and immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the present invention.

In another embodiment, where a TR 10 ligand is identified (e.g., TRAIL), or the ability of a polypeptide fragment, variant or derivative of the invention to multimerize is being evaluated, binding can be assayed, e.g., by means well-known in the art, such as, for example, reducing and non-reducing gel chromatography, protein affinity chromatography, and affinity blotting. See generally, Phizicky, E., *et al., Microbiol. Rev. 59*:94-123 (1995). In another embodiment, physiological correlates of TR10 binding to its substrates (signal transduction) can be assayed.

In addition, assays described herein (see Example 5 and otherwise known in the art may routinely be applied to measure the ability of TR10 polypeptides and fragments, variants derivatives and analogs thereof to elicit TR10 related biological activity (e.g., to inhibit TRAIL induced apoptosis, to regulate (e.g., inhibit) B cell proliferation (see, e.g,. Example 38) and/or to regulate (e.g., inhibit) hematopoiesis *in vitro or in vivo).* For example, techniques known in the art (such as for example assaying for thymidine incorporation), may be applied or routinely modified to assay for the ability of the compositions of the invention to inhibit proliferation of hematopoietic cells. Additionally, assays desribed herein (see e.g., Example 20 and Example 38) and otherwise known in the art may be applied or routinely modified to assay for the ability of the compositions of the invention to inhibit or stimulate B cell proliferation.

Other methods will be known to the skilled artisan and are within the scope of the invention.

Preferred nucleic acid fragments of the present invention include nucleic acid molecules encoding a member selected from the group: a polypeptide comprising or, alternatively, consisting of the TR10 receptor extracellular domain (amino acid residues from about 56 to about 212 in Figures 1A-D or from about 1 to about 157 in SEQ ID NO:2); a polypeptide comprising or, alternatively, consisting of the TR10 cysteine rich domain (amino acid residues from about 81 to about 182 in Figures 1A-D or from about 26 to about 127 in SEQ ID NO:2); a polypeptide comprising or, alternatively, consisting of the TR10 transmembrane domain (amino acid residues from about 213 to about 230 in Figures 1A-D or from about 158 to about 175 in SEQ ID NO:2); a polypeptide comprising or, alternatively, consisting of the TR10 intracellular domain (amino acid residues from about 231 to about 386 in Figures 1A-D or from about 176 to about 331 in SEQ ID NO:2); and a polypeptide comprising or, alternatively, consisting of the incomplete TR10 death domain (amino acid residues from about 353 to about 363 in Figures 1A-D or from about 298 to about 308 in SEQ ID NO:2). Since the location of these domains have been predicted by computer analysis, one of ordinary skill would appreciate that the amino acid residues constituting these domains may vary slightly (e.g., by about 1 to 15 amino acid residues) depending on the criteria used to define each domain.

Preferred nucleic acid fragments of the invention encode a full-length TR10 polypeptide lacking the nucleotides encoding the amino terminal methionine (nucleotides 109-111 in SEQ ID NO:1), as it is known that the methionine is cleaved naturally and such sequences may be useful in genetically engineering TR 10 expression vectors. Polypeptides encoded by such polynucleotides are also contemplated by the invention.

Preferred nucleic acid fragments of the present invention further include nucleic acid molecules encoding epitope-bearing portions of the TR10 receptor protein. In particular, such nucleic acid fragments of the present invention include nucleic acid molecules encoding: a polypeptide comprising or, alternatively, consisting of amino acid residues from about 57 to about 113 in Figures 1A-D (corresponding to about amino acid 2 to about 58 in SEQ ID NO:2); a polypeptide comprising or, alternatively, consisting of amino acid residues from about 130 to about 197 in Figures 1A-D (corresponding to about amino acid 75 to about 142 in SEQ ID NO:2); and a polypeptide comprising or, alternatively, consisting of amino acid residues from about 250 to about 283 in Figures 1A-D (corresponding to about amino acid 195 to about 228 in SEQ ID NO:2). The inventors have determined that the above polypeptide fragments are antigenic regions of the TR10 protein. Methods for determining other such epitope-bearing portions of the TR10 protein are described in detail below.

It is believed one or both of the extracellular cysteine rich motifs of TR10 disclosed in Figures 1A-D is important for interactions between TR10 and its ligands (e.g., TRAIL). Accordingly, specific embodiments of the invention are directed to polynucleotides encoding polypeptides which comprise, or alternatively consist of, the amino acid sequence of amino acid residues 26 to 80, and/or 81 to 127 of SEQ ID NO:2 (corresponding to amino acid residues 81 to 135, and/or 136 to 182 of Figures 1A-D). In a specific embodiment the polynucleotides encoding TR10 polypeptides of the invention comprise, or alternatively consist of both of the extracellular cysteine rich motifs disclosed in Figures 1A-D. Polypeptides encoded by these polynucleotides are also encompassed by the invention.

In additional embodiments, the polynucleotides of the invention encode functional attributes of TR10. Preferred embodiments of the invention in this regard include fragments that comprise alpha-helix and alpha-helix forming regions ("alpha-regions"), beta-sheet and beta-sheet forming regions ("beta-regions"), turn and turn-forming regions ("turn-regions"), coil and coil-forming regions ("coil-regions"), hydrophilic regions, hydrophobic regions, alpha amphipathic regions, beta amphipathic regions, flexible regions, surface-forming regions and high antigenic index regions of TR10.

The data representing the structural or functional attributes of TR10 set forth in Figure 3 and/or Table I, as described above, was generated using the various modules and algorithms of the DNA*STAR set on default parameters. In a preferred embodiment, the data presented in columns VIII, IX, XIII, and XIV of Table I can be used to determine regions of TR10 which exhibit a high degree of potential for antigenicity. Regions of high antigenicity are determined from the data presented in columns VIII, IX, XIII, and/or XIV by choosing values which represent regions of the polypeptide which are likely to be exposed on the surface of the polypeptide in an environment in which antigen recognition may occur in the process of initiation of an immune response.

Certain preferred regions in these regards are set out in Figure 3, but may, as shown in Table I, be represented or identified by using tabular representations of the data presented in Figure 3. The DNA*STAR computer algorithm used to generate Figure 3 (set on the original default parameters) was used to present the data in Figure 3 in a tabular format (See Table I). The tabular format of the data in Figure 3 may be used to easily determine specific boundaries of a preferred region.

The above-mentioned preferred regions set out in Figure 3 and in Table 1 include, but are not limited to, regions of the aforementioned types identified by analysis of the amino acid sequence set out in Figure 1. As set out in Figure 3 and in Table I, such preferred regions include Gamier-Robson alpha-regions, beta-regions, turn-regions, and coil-regions, Chou-Fasman alpha-regions, beta-regions, and turn-regions, Kyte-Doolittle hydrophilic regions and Hopp-Woods hydrophobic regions, Eisenberg alpha- and beta-amphipathic regions, Karplus-Schulz flexible regions, Jameson-Wolf regions of high antigenic index and Emini surface-forming regions.

**Table I**

| Res | Position | I | II | III | IV | V | VI | VII | VIII | IX | X | XI | XII | XIII | XIV |
|---|---|---|---|---|---|---|---|---|---|---|---|---|---|---|---|
| Met | 1 | . | . | B | . | . | . | . | -0.44 | 0.93 | . | . | . | -0.40 | 0.32 |
| Gly | 2 | . | . | B | . | . | . | . | -0.06 | 0.93 | . | . | . | -0.40 | 0.25 |
| Leu | 3 | . | . | . | . | T | . | . | 0.03 | 0.90 | . | . | . | 0.00 | 0.33 |
| Trp | 4 | . | . | . | . | T | . | . | -0.43 | 0.86 | . | . | . | 0.00 | 0.45 |
| Gly | 5 | . | . | . | . | . | . | C | -0.26 | 0.89 | . | . | F | -0.05 | 0.34 |
| Gln | 6 | . | . | B | . | . | . | . | 0.03 | 0.89 | * | . | F | -0.25 | 0.64 |
| Ser | 7 | . | . | B | . | . | . | . | -0.21 | 0.69 | . | . | F | -0.25 | 0.87 |
| Val | 8 | . | . | B | . | . | . | . | 0.30 | 0.27 | * | . | F | 0.05 | 0.89 |
| Pro | 9 | . | . | B | . | . | . | . | 0.29 | 0.23 | * | . | F | 0.05 | 0.69 |
| Thr | 10 | . | . | . | . | . | T | C | 0.04 | 0.21 | . | * | F | 0.45 | 0.69 |
| Ala | 11 | . | . | B | . | . | T | . | 0.16 | 0.33 | . | * | F | 0.25 | 0.94 |
| Ser | 12 | . | . | B | . | . | T | . | -0.13 | -0.31 | . | * | F | 1.00 | 1.19 |
| Ser | 13 | . | . | B | . | . | T | . | 0.38 | -0.24 | * | . | F | 1.13 | 0.83 |
| Ala | 14 | . | . | B | . | . | . | . | 0.70 | -0.30 | * | . | F | 1.21 | 0.81 |
| Arg | 15 | . | . | B | . | . | T | . | 0.77 | -0.80 | * | . | F | 2.14 | 1.19 |
| Ala | 16 | . | . | B | . | . | T | . | 1.14 | -0.43 | * | . | F | 2.12 | 1.39 |
| Gly | 17 | . | . | . | . | T | T | . | 1.10 | -0.39 | * | . | F | 2.80 | 2.13 |
| Arg | 18 | . | . | B | . | . | T | . | 0.81 | -0.46 | * | . | F | 2.12 | 1.08 |
| Tyr | 19 | . | . | B | . | . | T | . | 1.51 | 0.04 | * | . | F | 1.24 | 1.08 |
| Pro | 20 | . | . | B | . | . | T | . | 1.09 | -0.46 | * | . | F | 1.56 | 2.13 |
| Gly | 21 | . | . | B | . | . | T | . | 1.09 | -0.40 | . | . | F | 1.28 | 1.57 |
| Ala | 22 | . | . | B | . | . | T | . | 1.13 | 0.10 | . | . | F | 0.70 | 1.01 |
| Arg | 23 | . | . | B | . | . | . | . | 0.68 | -0.27 | . | . | F | 1.25 | 0.88 |
| Thr | 24 | . | . | B | . | . | . | . | 0.61 | -0.27 . | . | * | F | 1.55 | 0.88 |
| Ala | 25 | . | . | B | . | . | T | . | 0.93 | -0.21 | . | * | F | 2.20 | 1.25 |
| Ser | 26 | . | . | . | . | . | T | C | 1.07 | -0.71 | * | * | F | 3.00 | 1.25 |
| Gly | 27 | . | . | . | . | T | T | . | 1.37 | -0.29 | * | * | F | 2.60 | 1.34 |
| Thr | 28 | . | . | . | . | . | T | C | 0.44 | 0.14 | * | * | F | 1.50 | 1.40 |
| Arg | 29 | . | . | B | . | . | T | . | -0.06 | 0.33 | . | . | F | 0.85 | 0.86 |
| Pro | 30 | . | . | B | . | . | T | . | 0.53 | 0.63 | . | . | F | 0.25 | 0.72 |
| Trp | 31 | . | . | B | . | . | T | . | 0.62 | 0.20 | * | . | . | 0.10 | 0.83 |
| Leu | 32 | . | . | B | . | . | T | . | 1.01 | 0.14 | * | * | . | 0.10 | 0.65 |
| Leu | 33 | . | . | B | . | . | . | . | 0.43 | 0.14 | * | * | . | -0.10 | 0.85 |
| Asp | 34 | . | . | B | . | . | T | . | -0.49 | 0.40 | * | . | F | 0.25 | 0.56 |
| Pro | 35 | A | . | . | . | . | T | . | -0.23 | 0.17 | . | * | F | 0.25 | 0.56 |
| Lys | 36 | A | . | . | . | . | T | . | -0.64 | -0.51 | * | * | F | 1.30 | 1.37 |
| Ile | 37 | A | . | . | . | . | T | . | -0.69 | -0.41 | * | . | . | 0.70 | 0.71 |
| Leu | 38 | A | . | . | B | . | . | . | -0.73 | 0.23 | * | * | . | -0.30 | 0.34 |

Table continued

| Res | Position | I | II | III | IV | V | VI | VII | VIII | IX | X | XI | XII | XIII | XIV |
|---|---|---|---|---|---|---|---|---|---|---|---|---|---|---|---|
| Lys | 39 | . | . | B | B | . | . | . | -1.43 | 0.44 | * | * | . | -0.60 | 0.13 |
| Phe | 40 | . | . | B | B | . | . | . | -2.11 | 1.23 | * | . | . | -0.60 | 0.16 |
| Val | 41 | . | . | B | B | . | . | . | -3.01 | 1.23 | * | . | . | -0.60 | 0.13 |
| Val | 42 | . | . | B | B | . | . | . | -2.71 | 1.19 | * | . | . | -0.60 | 0.05 |
| Phe | 43 | . | . | B | B | . | . | . | -2.76 | 1.69 | * | * | . | -0.60 | 0.06 |
| Ile | 44 | . | . | B | B | . | . | . | -3.61 | 1.54 | * | . | . | -0.60 | 0.06 |
| Val | 45 | . | . | B | B | . | . | . | -3.72 | 1.59 | . | . | . | -0.60 | 0.06 |
| Ala | 46 | . | . | B | B | . | . | . | -3.08 | 1.63 | . | . | . | -0.60 | 0.06 |
| Val | 47 | . | . | B | B | . | . | . | -3.08 | 1.27 | . | * | . | -0.60 | 0.13 |
| Leu | 48 | . | . | B | B | . | . | . | -2.27 | 1.23 | * | * | . | -0.60 | 0.13 |
| Leu | 49 | . | . | B | B | . | . | . | -2.23 | 0.59 | . | * | . | -0.60 | 0.26 |
| Pro | 50 | . | . | B | B | . | . | . | 1.38 | 0.73 | . | * | . | -0.60 | 0.26 |
| Val | 51 | . | . | B | B | . | . | . | -1.09 | 0.09 | . | * | . | -0.30 | 0.53 |
| Arg | 52 | . | . | B | B | . | . | . | 0.82 | -0.21 | . | * | . | 0.30 | 0.86 |
| Val | 53 | . | . | B | B | . | . | . | 0.32 | -0.40 | . | * | . | 0.30 | 0.56 |
| Asp | 54 | . | . | B | B | . | . | . | 0.40 | -0.34 | . | * | F | 0.60 | 1.09 |
| Ser | 55 | . | . | B | B | . | . | . | -0.40 | -0.30 | * | * | F | 0.45 | 0.39 |
| Ala | 56 | . | . | B | B | . | . | . | 0.57 | 0.13 | * | * | F | -0.15 | 0.81 |
| Thr | 57 | . | . | B | B | . | . | . | 0.46 | -0.51 | . | * | F | 0.75 | 0.95 |
| Ile | 58 | . | . | B | B | . | . | . | 1.31 | -0.11 | . | . | F | 0.60 | 1.23 |
| Pro | 59 | . | . | . | . | . | . | C | 1.31 | -0.50 | . | . | F | 1.30 | 2.03 |
| Arg | 60 | . | A | . | . | T | . | . | 0.76 | -1.00 | . | . | F | 1.30 | 2.44 |
| Gln | 61 | . | A | . | . | T | . | . | 1.13 | -0.84 | . | . | F | 1.30 | 2.58 |
| Asp | 62 | . | A | . | . | T | . | . | 1.44 | -1.10 | * | . | F | 1.30 | 2.58 |
| Glu | 63 | . | A | B | . | . | . | . | 2.33 | -1.13 | * | . | F | 0.90 | 2.28 |
| Val | 64 | . | A | B | . | . | . | . | 2.23 | -0.73 | * | . | F | 0.90 | 2.28 |
| Pro | 65 | . | . | B | . | . | . | . | 1.27 | -0.64 | * | . | F | 1.10 | 1.97 |
| Gln | 66 | . | . | . | B | T | . | . | 0.68 | -0.00 | . | . | F | 0.85 | 0.85 |
| Gln | 67 | . | . | B | B | . | . | . | 0.47 | 0.50 | . | . | F | -0.30 | 1.15 |
| Thr | 68 | . | . | B | B | . | . | . | 0.47 | 0.29 | . | . | F | 0.00 | 1.15 |
| Val | 69 | . | . | B | B | . | . | . | 1.32 | 0.26 | . | . | F | 0.22 | 1.15 |
| Ala | 70 | . | . | B | B | . | . | . | 1.53 | 0.26 | . | . | F | 0.44 | 1.15 |
| Pro | 71 | . | . | B | B | . | . | . | 1.64 | 0.26 | . | . | F | 0.66 | 1.38 |
| Gln | 72 | . | . | B | . | . | . | . | 1.76 | -0.23 | * | . | F | 1.68 | 3.64 |
| Gln | 73 | . | . | B | . | . | . | . | 1.77 | -0.87 | * | . | F | 2.20 | 7.06 |
| Gln | 74 | . | . | B | . | . | T | . | 1.81 | -0.99 | * | . | F | 2.18 | 6.12 |

Table continued

| Res | Position | I | II | III | IV | V | VI | VII | VIII | IX | X | XI | XII | XIII | XIV |
|---|---|---|---|---|---|---|---|---|---|---|---|---|---|---|---|
| Arg | 75 | . | . | B | . | . | T | . | 2.44 | 0.73 | * | . | F | 1.96 | 2.91 |
| Arg | 76 | . | . | . | . | T | T | . | 2.66 | 1.13 | * | . | F | 2.14 | 3.37 |
| Ser | 77 | . | . | . | . | . | T | C | 2.66 | -1.53 | * | . | F | 1.72 | 3.37 |
| Leu | 78 | . | A | . | . | . | . | C | 2.66 | -1.93 | * | . | F | 1.10 | 2.98 |
| Lys | 79 | . | A | | . | | | C | 1.99 | -1.93 | * | . | F | 1.10 | 2.63 |
| Glu | 80 | A | A | . | . | . | . | . | 1.67 | -1.36 | * | . | F | 0.90 | 1.05 |
| Glu | 81 | A | A | . | . | . | . | . | 0.97 | -1.31 | * | . | F | 0.90 | 1.97 |
| Glu | 82 | A | A | . | . | . | . | . | 0.92 | -1.50 | . | . | F | 0.75 | 1.00 |
| Cys | 83 | A | . | . | . | . | T | . | 1.43 | -1.07 | . | . | F | 1.15 | 0.57 |
| Pro | 84 | A | . | . | . | . | T | . | 1.36 | -0.69 | . | . | F | 1.15 | 0.44 |
| Ala | 85 | A | . | . | . | . | T | . | 1.47 | -0.19 | . | . | F | 0.85 | 0.35 |
| Gly | 86 | A | . | . | . | . | T | . | 1.17 | -0.19 | . | * | F | 1.00 | 1.27 |
| Ser | 87 | A | . | . | . | . | . | . | 1.17 | -0.37 | . | * | F | 0.80 | 1.10 |
| His | 88 | . | . | . | . | . | . | C | 1.59 | -0.80 | . | * | F | 1.58 | 1.88 |
| Arg | 89 | . | . | B | . | . | . | . | 1.49 | -0.54 | . | * | F | 1.66 | 2.98 |
| Ser | 90 | . | . | B | . | . | . | . | 1.73 | -0.49 | . | * | F | 1.64 | 3.21 |
| Glu | 91 | . | . | . | . | T | . | . | 1.49 | -0.44 | . | * | F | 2.32 | 2.33 |
| Tyr | 92 | . | . | . | . | T | T | . | 1.12 | -0.44 | . | * | F | 2.80 | 1.20 |
| Thr | 93 | . | . | . | . | T | T | . | 1.16 | 0.13 | . | * | F | 1.77 | 0.48 |
| Gly | 94 | . | . | . | . | T | T | . | 0.83 | 0.14 | * | * | . | 1.34 | 0.45 |
| Ala | 95 | . | . | . | . | T | T | . | 0.47 | 0.57 | . | . | . | 1.01 | 0.44 |
| Cys | 96 | . | . | . | . | T | . | . | 0.16 | 0.39 | . | . | . | 1.08 | 0.16 |
| Asn | 97 | . | . | . | . | . | T | C | 0.40 | 0.39 | . | . | . | 1.05 | 0.24 |
| Pro | 98 | . | . | . | . | T | T | . | 0.37 | -0.04 | * | . | F | 2.25 | 0.41 |
| Cys | 99 | . | . | . | . | T | T | . | -0.14 | -0.11 | * | . | F | 2.50 | 0.76 |
| Thr | 100 | . | . | B | . | . | T | . | 0.44 | -0.04 | * | . | F | 1.85 | 0.35 |
| Glu | 101 | . | . | B | . | . | . | . | 0.87 | -0.44 | * | . | F | 1.40 | 0.38 |
| Gly | 102 | . | . | B | . | . | T | . | 0.56 | -0.11 | * | * | F | 1.50 | 1.10 |
| Val | 103 | . | . | B | . | . | T | . | -0.12 | -0.20 | . | . | . | 1.10 | 1.10 |
| Asp | 104 | . | . | B | . | . | T | . | -0.04 | -0.00 | . | . | . | 0.70 | 0.45 |
| Tyr | 105 | . | . | B | . | . | T | . | -0.03 | 0.50 | . | * | . | - 0.20 | 0.46 |
| Thr | 106 | . | . | B | . | . | . | . | -0.03 | 0.46 | . | . | . | - 0.40 | 0.82 |
| Ile | 107 | . | . | B | . | . | . | . | 0.31 | 0.21 | . | * | . | - 0.10 | 0.79 |
| Ala | 108 | . | . | B | . | . | T | . | 0.36 | 0.61 | . | * | . | - 0.20 | 0.81 |
| Ser | 109 | . | . | . | . | T | T | . | 0.14 | 0.54 | . | . | F | 0.35 | 0.36 |
| Asn | 110 | . | . | . | . | T | T | . | 0.09 | 0.49 | . | . | F | 0.50 | 1.02 |
| Asn | 111 | . | . | . | . | T | T | . | -0.27 | 0.19 | . | . | F | 0.80 | 1.36 |
| Leu | 112 | . | . | . | . | . | T | C | -0.19 | 0.26 | . | . | F | 0.45 | 0.54 |
| Pro | 113 | . | . | . | . | T | T | . | -0.41 | 0.56 | . | . | F | 0.35 | 0.28 |
| Ser | 114 | . | . | . | . | T | T | . | -0.78 | 0.84 | . | . | . | 0.20 | 0.14 |
| Cys | 115 | . | . | B | . | . | T | . | -1.09 | 1.01 | * | . | . | - 0.20 | 0.09 |
| Leu | 116 | . | . | B | B | . | . | . | -1.94 | 0.81 | * | . | . | -0. 60 | 0. 09 |
| Leu | 117 | . | . | B | B | . | . | . | -1.80 | 1.03 | * | . | . | - 0.60 | 0.05 |

Table continued

| Res | Position | I | II | III | IV | V | VI | VII | VIII | IX | X | XI | XII | XIII | XIV |
|---|---|---|---|---|---|---|---|---|---|---|---|---|---|---|---|
| Cys | 118 | . | . | B | B | . | . | . | -1.54 | 1.21 | * | . | . | - 0.60 | 0.05 |
| Thr | 119 | . | . | B | B | . | . | . | -1.54 | 0.64 | * | . | . | - 0.60 | 0.12 |
| Val | 120 | . | . | B | B | . | . | . | -1.22 | 0.34 | * | . | . | - 0.30 | 0.19 |
| Cys | 121 | . | . | B | . | . | T | . | -0.41 | 0.09 | * | . | . | 0.10 | 0.35 |
| Lys | 122 | . | . | B | . | . | T | . | 0.09 | -0.09 | * | . | F | 0.85 | 0.42 |
| Ser | 123 | . | . | . | . | T | T | . | 0.76 | -0.09 | . | . | F | 1.59 | 0.81 |
| Gly | 124 | . | . | . | . | T | T | . | 1.11 | -0.33 | . | . | F | 2.08 | 2.44 |
| Gln | 125 | . | . | . | . | T | . | . | 1.67 | -0.90 | . | . | F | 2.52 | 2.44 |
| Thr | 126 | . | . | . | . | T | . | . | 2.03 | -0.51 | . | . | F | 2.86 | 2.44 |
| Asn | 127 | . | . | . | . | T | T | . | 1.32 | -0.51 | . | . | F | 3.40 | 3.31 |
| Lys | 128 | . | . | . | . | T | T | . | 1.31 | -0.37 | . | . | F | 2.76 | 1.02 |
| Ser | 129 | . | . | . | . | T | T | . | 1.34 | -0.29 | . | . | F | 2.42 | 1.02 |
| Ser | 130 | . | . | . | . | T | T | . | 1.03 | -0.29 | * | . | F | 1.93 | 0.92 |
| Cys | 131 | . | . | B | B | . | . | . | 1.46 | -0.20 | * | . | F | 0.79 | 0.66 |
| Thr | 132 | . | . | B | B | . | . | . | 1.46 | -0.20 | * | . | F | 0.45 | 0.97 |
| Thr | 133 | . | . | B | B | . | . | . | 1.10 | -0.59 | * | * | F | 0.90 | 1.21 |
| Thr | 134 | . | . | . | . | T | T | . | 0.54 | -0.49 | . | . | F | 1.40 | 3.25 |
| Arg | 135 | . | . | . | . | T | T | . | 0.18 | -0.41 | . | . | F | 1.40 | 1.67 |
| Asp | 136 | . | . | . | . | T | T | . | 0.84 | -0.33 | * | . | F | 1.25 | 0.62 |
| Thr | 137 | . | . | B | . | . | T | . | 0.49 | -0.41 | * | . | . | 0.70 | 0.75 |
| Val | 138 | . | . | B | B | . | . | . | 0.80 | -0.33 | * | . | . | 0.61 | 0.20 |
| Cys | 139 | . | . | B | B | . | . | . | 1.16 | -0.33 | * | . | . | 0.92 | 0.21 |
| Gin | 140 | . | . | B | B | . | . | . | 0.70 | -0.33 * | . | . | . | 1.23 | 0.29 |
| Cys | 141 | . | . | B | B | . | . | . | 0.40 | -0.39 | * | . | . | 1.54 | 0.39 |
| Glu | 142 | . | . | . | . | T | T | . | 0.01 | -0.64 | * | * | F | 3.10 | 0.98 |
| Lys | 143 | . | . | . | . | T | T | . | 0.87 | -0.43 | * | . | F | 2.49 | 0.49 |
| Gly | 144 | . | . | . | . | T | T | . | 1.53 | -0.43 | . | * | F | 2.67 | 1.58 |
| Ser | 145 | . | . | . | . | T | T | . | 1.58 | -1.00 | . | * | F | 3.00 | 1.52 |
| Phe | 146 | A | . | . | . | . | . | . | 2.24 | -1.00 | . | * | F | 2.43 | 1.52 |
| Gln | 147 | . | . | . | . | T | . | . | 1.94 | -0.60 | . | * | F | 2.86 | 2.47 |
| Asp | 148 | . | . | . | . | T | T | . | 1.69 | -0.64 | . | * | F | 3.40 | 2.47 |
| Lys | 149 | . | . | . | . | T | T | . | 2.03 | -0.60 | . | * | F | 3.06 | 4.42 |
| Asn | 150 | . | . | . | . | . | T | C | 1.73 | -1.39 | . | * | F | 2.52 | 4.42 |
| Ser | 151 | . | . | . | . | . | T | C | 1.77 | -1.17 | * | * | F | 2.18 | 2.62 |
| Pro | 152 | . | . | . | . | T | . | . | 1-88 | -0.60 | * | . | F | 1.69 | 0.70 |
| Glu | 153 | . | . | . | . | T | . | . | 1.57 | -0.60 | * | . | F | 1.35 | 0.85 |
| Met | 154 | . | . | B | B | . | . | . | 0.86 | -0.51 | * | . | . | 0.60 | 0.92 |
| Cys | 155 | . | . | B | B | . | . | . | 0.97 | -0.33 | * | . | . | 0.30 | 0.32 |
| Arg | 156 | . | . | B | B | . | . | . | 0.96 | -0.76 | * | . | . | 0.60 | 0.36 |
| Thr | 157 | . | . | B | B | . | . | . | 0.82 | -0.27 | * | . | . | 0.64 | 0.53 |
| Cys | 158 | . | . | . | . | T | T | . | 0.16 | -0.46 | * | . | F | 1.93 | 0.97 |
| Arg | 159 | . | . | . | . | T | T | . | 0.54 | -0.46 | * | . | F | 2.27 | 0.27 |
| Thr | 160 | . | . | . | . | T | T | . | 1.32 | -0.03 | * | . | F | 2.61 | 0.28 |
| Gly | 161 | . | . | . | . | T | T | . | 0.87 | -0-51 | * | . | F | 3.40 | 1.04 |
| Cys | 162 | . | . | . | . | . | T | C | 0.58 | -0.66 | . | * | F | 2.71 | 0.53 |
| Pro | 163 | . | . | . | . | T | T | . | 0.39 | -0.04 | . | * | F | 2.27 | 0.36 |

Table continued

| Res | Position | I | II | III | IV | V | VI | VII | VIII | IX | X | XI | XII | XIII | XIV |
|---|---|---|---|---|---|---|---|---|---|---|---|---|---|---|---|
| Arg | 164 | . | . | . | . | T | T | . | 0.32 | 0.11 | * | * | F | 1.33 | 0.27 |
| Gly | 165 | . | . | B | . | . | T | . | -0.22 | -0.31 | * | * | . | 1.19 | 1.01 |
| Met | 166 | . | . | B | . | . | . | . | -0.18 | -0.24 | * | * | . | 0.50 | 0.48 |
| Val | 167 | . | . | B | . | . | . | . | 0.49 | -0.29 | * | * | . | 0.50 | 0.33 |
| Lys | 168 | . | . | B | . | . | . | . | 0.03 | 0.11 | * | * | . | -0.10 | 0.54 |
| Val | 169 | . | . | B | . | . | T | . | -0.39 | 0.26 | * | * | . | 0.10 | 0.29 |
| Ser | 170 | . | . | B | . | . | T | . | -0.26 | 0.13 | * | * | F | 0.59 | 0.57 |
| Asn | 171 | . | . | B | . | . | T | . | 0.46 | -0.09 | * | * | F | 1.53 | 0.44 |
| Cys | 172 | . | . | B | . | . | T | . | 1.01 | -0.09 | * | * | F | 2.02 | 1.16 |
| Thr | 173 | . | . | B | . | . | T | . | 0.97 | -0.34 | * | * | F | 2.36 | 1.16 |
| Pro | 174 | . | . | . | . | T | T | . | 0.93 | -0.73 | * | * | F | 3.40 | 1.20 |
| Arg | 175 | . | . | . | . | T | T | . | 1.28 | -0.44 | * | * | F | 2.76 | 1.57 |
| Ser | 176 | . | . | B | . | T | T | . | 0.61 | -1.01 | . | * | F | 2.72 | 2.18 |
| Asp | 177 | . | . | . | . | T | . | . | 1.32 | -0.93 | . | * | F | 2.03 | 0.76 |
| Ile | 178 | . | . | B | . | . | . | . | 1.63 | -1.36 | . | * | F | 1.29 | 0.77 |
| Lys | 179 | . | . | B | . | . | . | . | 1.84 | -0.96 | . | * | F | 0.95 | 0.93 |
| Cys | 180 | . | . | B | . | . | T | . | 1.43 | -1.34 | . | * | F | 1.15 | 0.96 |
| Lys | 181 | . | . | B | . | . | T | . | 1.14 | -0.96 | . | * | F | 1.30 | 1.83 |
| Asn | 182 | A | . | . | . | . | T | . | 0.56 | -1.14 | . | * | F | 1.15 | 0.93 |
| Glu | 183 | A | . | . | . | . | T | . | 1.14 | -0.64 | . | * | F | 1.30 | 1.73 |
| Ser | 184 | A | A | . | . | . | . | . | 0.80 | -0.83 | . | * | F | 1.18 | 1.17 |
| Ala | 185 | A | A | . | . | . | . | . | 1.16 | -0.44 | . | . | F | 1.01 | 0.98 |
| Ala | 186 | A | A | . | . | . | . | . | 0.77 | -0.36 | . | . | F | 1.29 | 0.81 |
| Ser | 187 | A | A | . | . | . | . | . | 0.81 | 0.07 | . | . | F | 0.97 | 0.60 |
| Ser | 188 | . | . | . | . | T | T | . | 0.50 | -0.31 | * | . | F | 2.80 | 1.19 |
| Thr | 189 | . | . | . | . | T | T | . | 0.59 | -0.33 | * | * | F | 2.52 | 1.70 |
| Gly | 190 | . | . | . | . | T | T | . | 0.59 | -0.40 | . | . | F | 2.24 | 1.96 |
| Lys | 191 | . | . | . | . | . | T | C | 0.59 | -0.29 | . | . | F | 1.76 | 1.48 |
| Thr | 192 | . | A | . | . | . | . | C | 0.89 | -0.17 | . | . | F | 1.08 | 1.03 |
| Pro | 193 | . | A | . | . | . | . | C | 1.19 | -0.66 | . | . | F | 1.10 | 1.81 |
| Ala | 194 | A | A | . | . | . | . | . | 1.19 | -1.09 | . | . | F | 0.90 | 1.57 |
| Ala | 195 | A | A | . | . | . | . | . | 0.68 | -0.60 | . | . | F | 0.90 | 1.57 |
| Glu | 196 | A | A | . | . | . | . | . | 0.32 | -0.44 | . | . | F | 0.45 | 0.75 |
| Glu | 197 | A | A | . | B | . | . | . | 0.32 | -0.69 | * | . | F | 0.60 | 1.07 |
| Thr | 198 | A | A | . | B | . | . | . | -0. 36 | -0.40 | * | . | F | 0.60 | 1.53 |
| Val | 199 | A | . | . | B | . | . | . | -0.58 | -0.21 | * | . | F | 0.45 | 0.62 |
| Thr | 200 | A | . | . | B | . | . | . | -0.33 | 0.47 | * | . | F | -0.45 | 0.30 |
| Thr | 201 | A | . | . | B | . | . | . | -0.93 | 0.90 | * | . | . | -0.60 | 0.20 |
| Ile | 202 | A | A | . | B | . | . | . | -1.74 | 1.03 | * | . | . | -0.60 | 0.27 |
| Leu | 203 | . | A | B | B | . | . | . | -2.02 | 1.07 | * | . | . | -0.60 | 0.15 |
| Gly | 204 | . | A | B | . | . | . | . | -1.47 | 1.09 | * | . | . | -0.60 | 0.11 |
| Met | 205 | . | A | B | . | . | . | . | -1.37 | 0.99 | . | . | . | -0.60 | 0.21 |

Table continued

| Res | Position | I | II | III | IV | V | VI | VII | VIII | IX | X | XI | XII | XIII | XIV |
|---|---|---|---|---|---|---|---|---|---|---|---|---|---|---|---|
| Leu | 206 | . | A | B | . | . | . | . | -1.30 | 0.73 | * | . | . | -0.60 | 0.39 |
| Ala | 207 | . | A | B | . | . | . | . | -0.44 | 0.80 | . | . | . | -0.60 | 0.61 |
| Ser | 208 | . | . | . | . | . | T | C | 0.12 | 0.87 | . | . | . | 0.00 | 0.84 |
| Pro | 209 | A | . | . | . | . | T | . | -0.34 | 1.01 | . | . | . | -0.05 | 1.60 |
| Tyr | 210 | A | . | . | . | . | T | . | -0.63 | 1.01 | . | . | . | -0.05 | 1.31 |
| His | 211 | . | . | B | . | . | T | . | -0.71 | 1.20 | . | . | . | -0.20 | 0.68 |
| Tyr | 212 | . | . | B | B | . | . | . | -1.01 | 1.50 | . | . | . | -0.60 | 0.31 |
| Leu | 213 | . | . | B | B | . | . | . | -1.57 | 1.76 | . | . | . | -0.60 | 0.14 |
| Ile | 214 | . | . | B | B | . | . | . | -2.21 | 1.64 | . | . | . | -0.60 | 0.08 |
| Ile | 215 | . | . | B | B | . | . | . | -2.78 | 1.79 | . | . | . | -0.60 | 0.04 |
| Ile | 216 | . | . | B | B | . | . | . | -3.60 | 1.71 | . | . | . | -0.60 | 0.04 |
| Val | 217 | . | . | B | B | . | . | . | -4.24 | 1.67 | . | . | . | -0.60 | 0.04 |
| Val | 218 | . | . | B | B | . | . | . | -4.32 | 1.67 | . | . | . | -0.60 | 0.04 |
| Leu | 219 | . | . | B | B | . | . | . | -4.24 | 1.67 | . | . | . | -0.60 | 0.04 |
| Val | 220 | . | . | B | B | . | . | . | -3.94 | 1.67 | . | . | . | -0.60 | 0.04 |
| Ile | 221 | . | . | B | B | . | . | . | -3.91 | 1.53 | . | . | . | -0.60 | 0.06 |
| Ile | 222 | . | . | B | B | . | . | . | -3.91 | 1.53 | . | . | . | -0.60 | 0.05 |
| Leu | 223 | . | . | B | B | . | . | . | -3.91 | 1.49 | . | . | . | -0.60 | 0.05 |
| Ala | 224 | . | . | B | B | . | . | . | -3.96 | 1.49 | . | . | . | -0.60 | 0.05 |
| Val | 225 | . | . | B | B | . | . | . | -3.44 | 1.44 | . | . | . | -0.60 | 0.06 |
| Val | 226 | . | . | B | B | . | . | . | -3.26 | 1.19 | . | . | . | -0.60 | 0.07 |
| Val | 227 | . | . | B | B | . | . | . | -2.67 | 1.29 | . | . | . | -0.60 | 0.06 |
| Val | 228 | . | . | B | B | . | . | . | -2.52 | 1.17 | . | * | . | -0.60 | 0.11 |
| Gly | 229 | . | . | B | B | . | . | . | -1.82 | 1.10 | . | * | . | -0.60 | 0.08 |
| Phe | 230 | A | . | . | . | . | T | . | -0.92 | 0.46 | . | * | . | -0.20 | 0.21 |
| Ser | 231 | A | . | . | . | . | T | . | -0.02 | -0.19 | . | * | . | 0.70 | 0.55 |
| Cys | 232 | A | . | . | . | . | T | . | 0.13 | -0.83 | . | * | . | 1.15 | 1.12 |
| Arg | 233 | A | . | . | . | . | T | . | 0.10 | -0.47 | . | * | F | 1.00 | 1.12 |

Table continued

| Res | Position | I | II | III | IV | V | VI | VII | VIII | IX | X | XI | XII | XIII | XIV |
|---|---|---|---|---|---|---|---|---|---|---|---|---|---|---|---|
| Lys | 234 | . | A | . | B | T | . | . | 0.14 | -0.57 | . | * | F | 1.15 | 0.58 |
| Lys | 235 | A | A | . | B | . | . | . | 0.60 | -0.57 | * | * | F | 0.90 | 1.46 |
| Phe | 236 | . | A | B | B | . | . | . | 0.09 | -0.39 | * | * | . | 0.45 | 1.17 |
| Ile | 237 | . | A | B | B | . | . | . | 0.80 | 0.30 | * | * | . | -0.30 | 0.48 |
| Ser | 238 | . | . | B | B | . | . | . | 0.34 | 0.30 | * | * | . | -0.30 | 0.48 |
| Tyr | 239 | . | . | B | B | . | . | . | -0.59 | 0.73 | * | . | . | -0.60 | 0.55 |
| Leu | 240 | . | . | B | B | . | . | . | -1.30 | 0.63 | * | * | . | -0.60 | 0.55 |
| Lys | 241 | . | . | B | B | . | . | . | -0.90 | 0.51 | * | . | . | -0.60 | 0.22 |
| Gly | 242 | . | . | B | B | . | . | . | -0.36 | 0.51 | * | . | . | -0.60 | 0.19 |
| Ile | 243 | . | . | B | B | . | . | . | -0.40 | 0.19 | * | . | . | -0.30 | 0.23 |
| Cys | 244 | . | . | B | . | . | T | . | -0.50 | -0.07 | * | . | . | 0.70 | 0.11 |
| Ser | 245 | . | . | B | . | . | T | . | -0.03 | 0.36 | * | . | F | 0.25 | 0.11 |
| Gly | 246 | . | . | . | . | T | T | . | -0.42 | 0.36 | * | . | F | 0.65 | 0.16 |
| Gly | 247 | . | . | . | . | T | T | . | -0.29 | 0.10 | . | . | F | 0.92 | 0.29 |
| Gly | 248 | . | . | . | . | . | . | C | 0.60 | -0.04 | * | . | F | 1.39 | 0.34 |
| Gly | 249 | . | . | . | . | . | . | C | 1.38 | -0.43 | * | * | F | 1.66 | 0.59 |
| Gly | 250 | . | . | . | . | . | T | C | 0.82 | -0.86 | * | * | F | 2.58 | 1.17 |
| Pro | 251 | . | . | . | . | . | T | C | 1.13 | -0.64 | * | . | F | 2.70 | 0.87 |
| Glu | 252 | . | . | B | . | . | T | . | 1.59 | -0.57 | * | . | F | 2.38 | 1.20 |
| Arg | 253 | . | . | B | . | . | T | . | 1.08 | -1.00 | * | . | F | 2.11 | 2.38 |
| Val | 254 | . | . | B | B | . | . | . | 0.61 | -0.79 | * | . | . | 1.29 | 1.14 |
| His | 255 | . | . | B | B | . | . | . | 0.26 | -0.53 | * | * | . | 0.87 | 0.54 |
| Arg | 256 | . | . | B | B | . | . | . | 0.58 | 0.26 | * | . | . | -0.30 | 0.24 |
| Val | 257 | . | . | B | B | . | . | . | 0.69 | 0.26 | * | * | . | -0.30 | 0.63 |
| Leu | 258 | . | . | B | B | . | . | . | 0.69 | -0.39 | * | * | . | 0.64 | 0.91 |
| Phe | 259 | . | . | B | B | . | . | . | 1.2-4 | -0.89 | * | * | . | 1.28 | 0.91 |
| Arg | 260 | . | . | B | . | . | T | . | 0.61 | -0.50 | . | * | . | 1.87 | 1.65 |
| Arg | 261 | . | . | . | . | T | T | . | 0.29 | -0.57 | . | * | F | 3.06 | 1-07 |
| Arg | 262 | . | . | . | . | T | T | . | 0.84 | -0.83 | . | * | F | 3.40 | 1.91 |
| Ser | 263 | . | . | . | . | T | T | . | 1.77 | -1.23 | * | * | F | 3.06 | 1.31 |
| Cys | 264 | . | . | . | . | . | T | C | 1.61 | -1.23 | . | * | F | 2.52 | 1.31 |
| Pro | 265 | . | . | . | . | T | T | . | 1.29 | -0.59 | . | * | F | 2.23 | 0.50 |
| Ser | 266 | . | . | . | . | T | T | . | 0.83 | -0.16 | . | * | F | 1.86 | 0.57 |
| Arg | 267 | . | . | B | . | . | T | . | 0.13 | -0.11 | * | . | F | 1.54 | 1.06 |
| Val | 268 | . | . | B | . | . | T | . | 0.43 | -0.19 | . | * | F | 1.66 | 0.69 |
| Pro | 269 | . | . | B | . | . | T | . | 1.10 | -0.61 | . | * | F | 2.23 | 0.89 |
| Gly | 270 | . | . | . | . | . | T | C | 1.31 | -1.00 | * | * | F | 2.70 | 0.76 |
| Ala | 271 | A | . | B | . | . | T | . | 1.02 | -0.60 | . | * | F | 2.38 | 1.65 |
| Glu | 272 | A | . | . | . | . | . | . | 1.02 | -0.74 | * | * | F | 1.91 | 1.08 |
| Asp | 273 | A | . | . | . | . | . | . | 1.88 | -1.17 | * | * | F | 1.64 | 2.13 |
| Asn | 274 | A | . | . | . | . | T | . | 2.09 | -1.20 | * | * | F | 1.57 | 3.39 |

Table continued

| Res | Position | I | II | III | IV | V | VI | VII | VIII | IX | X | XI | XII | XIII | XIV |
|---|---|---|---|---|---|---|---|---|---|---|---|---|---|---|---|
| Ala | 275 | A | . | . | . | . | T | . | 2.12 | -1.70 | . | * | F | 1.30 | 3.39 |
| Arg | 276 | A | . | . | . | . | T | . | 1.90 | -1.21 | . | * | F | 1.64 | 2.93 |
| Asn | 277 | A | . | . | . | . | T | . | 1.60 | -0.53 | . | * | F | 1.98 | 1.50 |
| Glu | 278 | A | . | . | . | . | . | . | 1.60 | -0.54 | . | * | F | 2.12 | 1.99 |
| Thr | 279 | A | . | . | . | . | . | . | 1.71 | -0.64 | . | * | F | 2.46 | 1.64 |
| Leu | 280 | . | . | . | . | T | T | . | 2.06 | -0.64 | * | . | F | 3.40 | 1.99 |
| Ser | 281 | . | . | . | . | T | T | . | 1.13 | -0.29 | * | . | F | 2.76 | 1.80 |
| Asn | 282 | . | . | . | . | T | T | . | 1.13 | 0.40 | * | . | F | 1.52 | 1.03 |
| Arg | 283 | . | . | . | . | T | T | . | 0.92 | 0.31 | * | . | F | 1.48 | 2.17 |
| Tyr | 284 | . | . | . | . | T | . | . | 0.92 | 0.06 | * | . | F | 0.94 | 2.50 |
| Leu | 285 | . | . | B | . | . | . | . | 1.73 | 0.16 | * | . | F | 0.20 | 2.24 |
| Gin | 286 | . | . | B | . | . | T | . | 1.18 | 0.16 | . | . | F | 0.40 | 1.98 |
| Pro | 287 | . | . | . | . | . | T | C | 0.88 | 0.80 | . | . | F | 0.15 | 0.94 |
| Thr | 288 | . | . | . | . | . | T | C | 0.77 | 0.43 | . | . | F | 0.30 | 1.53 |
| Gin | 289 | . | . | B | . | . | T | . | 1.01 | -0.26 | . | . | F | 1.00 | 1.53 |
| Val | 290 | . | A | B | . | . | . | . | 1.82 | -0.26 | . | . | F | 0.60 | 1.71 |
| Ser | 291 | . | A | B | . | . | . | . | 0.93 | -0.69 | * | . | F | 0.90 | 2.05 |
| Glu | 292 | . | A | B | . | . | . | . | 1.14 | -0.49 | * | * | F | 0.45 | 0.83 |
| Gin | 293 | . | A | B | . | . | . | . | 1.11 | -0.49 | . | * | F | 0.60 | 1.94 |
| Glu | 294 | A | A | . | . | . | . | . | 1.11 | -0.70 | . | * | F | 0.90 | 1.43 |
| Ile | 295 | A | A | . | . | . | . | . | 1.97 | -0.69 | . | . | F | 0.90 | 1.43 |
| Gin | 296 | A | A | . | . | . | . | . | 1.46 | -0.69 | . | * | F | 0.90 | 1.43 |
| Gly | 297 | A | A | . | . | . | . | . | 0.87 | -0.40 | . | * | F | 0.45 | 0.68 |
| Gin | 298 | A | A | . | . | . | . | . | 0.87 | 0.10 | . | * | F | - 0.15 | 0.98 |
| Glu | 299 | A | A | . | . | . | . | . | 0.06 | -0.59 | . | * | F | 0.75 | 0.98 |
| Leu | 300 | A | A | . | . | . | . | . | 0.63 | -0.30 | . | * | F | 0.45 | 0.82 |
| Ala | 301 | A | A | . | . | . | . | . | 0.29 | -0.24 | . | . | . | 0.30 | 0.68 |
| Glu | 302 | A | A | . | . | . | . | . | -0.22 | -0.21 | . | . | . | 0.30 | 0.39 |
| Leu | 303 | A | A | . | B | . | . | . | -0.53 | 0.43 | . | . | . | - 0.60 | 0.35 |
| Thr | 304 | A | A | . | B | . | . | . | -1.39 | 0.23 | . | . | . | - 0.30 | 0.50 |
| Gly | 305 | A | A | . | B | . | . | . | -0.58 | 0.37 | . | . | . | - 0.30 | 0.21 |
| Val | 306 | . | . | B | B | . | . | . | -0.29 | 0.37 | . | . | . | - 0.30 | 0.45 |
| Thr | 307 | . | . | B | B | . | . | . | -0.50 | 0.07 | . | . | F | 0.15 | 0.42 |
| Val | 308 | . | . | . | B | . | . | C | 0.31 | 0.01 | . | . | F | 0.65 | 0.65 |
| Glu | 309 | . | . | . | B | . | . | C | 0.62 | -0.41 | . | . | F | 1.70 | 1.53 |
| Ser | 310 | . | . | . | . | . | T | C | 0.76 | -1.06 | . | * | F | 2.70 | 1.83 |
| Pro | 311 | . | . | . | . | . | T | C | 1.61 | -1.11 | * | * | F | 3.00 | 3.82 |
| Glu | 312 | A | . | . | . | . | T | . | 2.03 | -1.36 | * | * | F | 2.50 | 3.82 |
| Glu | 313 | A | . | . | . | . | T | . | 2.08 | -1.36 | * | . | F | 2.20 | 5.58 |
| Pro | 314 | A | . | . | . | . | . | . | 1.27 | -1.06 | * | . | F | 1.70 | 2.97 |
| Gln | 315 | A | A | . | . | . | . | . | 1.57 | -0.80 | * | . | F | 1.20 | 1.42 |
| Arg | 316 | A | A | . | . | . | . | . | 1.78 | -0.80 | * | . | F | 0.90 | 1.42 |
| Leu | 317 | A | A | . | . | . | . | . | 1.19 | -0.40 | * | * | F | 0.60 | 1.59 |
| Leu | 318 | A | A | . | . | . | . | . | 1.19 | -0.33 | * | * | F | 0.45 | 0.93 |
| Glu | 319 | A | A | . | . | . | . | . | 0.81 | -0.73 | * | * | F | 0.75 | 0.82 |

Table continued

| Res | Position | I | II | III | IV | V | VI | VII | VIII | IX | X | XI | XII | XIII | XIV |
|---|---|---|---|---|---|---|---|---|---|---|---|---|---|---|---|
| Gln | 320 | A | A | . | . | . | . | . | 0.81 | -0.23 | * | * | F | 0.60 | 1.00 |
| Ala | 321 | A | A | . | . | . | . | . | 0.36 | -0.91 | * | * | F | 0.90 | 2.10 |
| Glu | 322 | A | A | . | . | . | . | . | 0.50 | -1.17 | * | . | F | 0.90 | 1.20 |
| Ala | 323 | A | A | . | . | . | . | . | 1.31 | -0.60 | * | * | F | 0.75 | 0.37 |
| Glu | 324 | A | A | . | . | . | . | . | 1.42 | -0.60 | . | * | F | 0.75 | 0.64 |
| Gly | 325 | A | A | . | . | . | . | . | 1.53 | -1.10 | . | * | F | 0.75 | 0.72 |
| Cys | 326 | A | A | . | . | . | . | . | 2.23 | -1.10 | . | * | F | 0.90 | 1.40 |
| Gln | 327 | A | A | . | . | . | . | . | 1.42 | -1.60 | . | * | F | 0.90 | 1.58 |
| Arg | 328 | A | A | . | . | . | . | . | 1.20 | -0.91 | . | * | F | 0.90 | 1-32 |
| Arg | 329 | . | A | B | B | . | . | . | 0.34 | -0.66 | . | * | F | 0.90 | 2.03 |
| Arg | 330 | . | A | B | B | . | . | . | 0.48 | -0.59 | . | * | . | 0.60 | 0.87 |
| Leu | 331 | . | A | B | B | . | . | . | 0.29 | -0.56 | . | * | . | 0.60 | 0.69 |
| Leu | 332 | . | A | B | B | . | . | . | 0.29 | 0.09 | * | * | . | -0.30 | 0.26 |
| Val | 333 | . | A | B | B | . | . | . | 0.18 | 0.49 | * | * | . | -0.60 | 0.21 |
| Pro | 334 | . | A | B | . | . | . | . | -0.52 | 0.49 | * | . | . | -0.60 | 0.43 |
| Val | 335 | . | . | B | . | . | . | . | -0.63 | 0-30 | * | * | . | -0.10 | 0.53 |
| Asn | 336 | . | . | B | . | . | . | . | -0.12 | -0.39 | . | . | F | 0.80 | 1.19 |
| Asp | 337 | A | . | . | . | . | T | . | 0.10 | -0.64 | . | . | F | 1.30 | 1.03 |
| Ala | 338 | A | . | . | . | . | T | . | 0.96 | -0.57 | . | . | F | 1.30 | 1.41 |
| Asp | 339 | A | . | . | . | . | T | . | 0.28 | -1.21 | . | . | F | 1.30 | 1.46 |
| Ser | 340 | A | . | . | . | . | T | . | 0.83 | -0.93 | . | . | F | 1.15 | 0.61 |
| Ala | 341 | A | . | . | . | . | . | . | 0.52 | -0.54 | . | . | F | 0.95 | 0.81 |
| Asp | 342 | A | . | . | B | . | . | . | -0.29 | -0.56 | . | . | F | 0.75 | 0.70 |
| Ile | 343 | A | . | . | B | . | . | . | -0.51 | 0.13 | . | * | F | -0.15 | 0.43 |
| Ser | 344 | . | A | B | B | . | . | . | -0.51 | 0.43 | . | . | F | -0.45 | 0.35 |
| Thr | 345 | . | A | B | B | . | . | . | -0.80 | -0.07 | . | . | F | 0.45 | 0.35 |
| Leu | 346 | A | A | . | B | . | . | . | -0.51 | 0.43 | * | * | . | -0.60 | 0.51 |
| Leu | 347 | A | A | . | B | . | . | . | -1.10 | 0.13 | * | * | . | -0.30 | 0.51 |
| Asp | 348 | A | A | . | B | . | . | . | -0.52 | 0.24 | * | * | . | -0.30 | 0.36 |
| Ala | 349 | A | A | . | . | . | . | . | -1.03 | 0.24 | . | * | . | -0.30 | 0.62 |
| Ser | 350 | A | A | . | . | . | . | . | -0.72 | 0.24 | * | * | . | -0.30 | 0.62 |
| Ala | 351 | A | A | . | . | . | . | . | 0.09 | -0.44 | * | * | . | 0.30 | 0.65 |
| Thr | 352 | A | A | . | . | . | . | . | 0.56 | -0.44 | . | * | . | 0.45 | 1.11 |
| Leu | 353 | A | A | . | . | . | . | . | 0.52 | -0.51 | . | * | F | 0.75 | 0.82 |
| Glu | 354 | A | A | . | . | . | . | . | 0.52 | -0.40 | . | * | F | 0.60 | 1.10 |
| Glu | 355 | A | A | . | . | . | . | . | 0.87 | -0.40 | . | . | F | 0.45 | 0.77 |
| Gly | 356 | A | A | . | . | . | . | . | 1.46 | -0.89 | . | . | F | 0.90 | 1.87 |
| His | 357 | A | A | . | . | . | . | . | 1.46 | -1.57 | . | * | F | 0.90 | 1.87 |
| Ala | 358 | A | A | . | . | . | . | . | 1.38 | -1.09 | . | * | F | 0.90 | 1.56 |
| Lys | 359 | A | A | . | . | . | . | . | 1.38 | -0.40 | . | * | F | 0.60 | 1.10 |

Table continued

| Res | Position | I | II | III | IV | V | VI | VII | VIII | IX | X | XI | XII | XIII | XIV |
|---|---|---|---|---|---|---|---|---|---|---|---|---|---|---|---|
| Glu | 360 | A | A | . | . | . | . | . | 1.38 | -0.43 | . | * | F | 0.60 | 1.41 |
| Thr | 361 | A | A | . | . | . | . | . | 1.72 | -0.93 | * | * | F | 0.90 | 2.32 |
| Ile | 362 | A | A | . | . | . | . | . | 0.94 | -1.03 | . | * | F | 0.90 | 2.01 |
| Gln | 363 | A | A | . | . | . | . | . | 0.68 | -0.34 | . | * | F | 0.45 | 0.96 |
| Asp | 364 | A | A | . | . | . | . | . | 0.29 | 0.30 | . | * | F | -0.15 | 0.49 |
| Gln | 365 | A | A | . | . | . | . | . | -0.01 | 0.24 | . | . | F | 0.06 | 0.70 |
| Leu | 366 | . | A | B | . | . | . | . | 0.30 | -0.06 | * | * | F | 0.87 | 0.54 |
| Val | 367 | . | A | B | . | . | . | . | 1.23 | -0.46 | * | * | F | 1.08 | 0.56 |
| Gly | 368 | . | . | . | . | . | T | C | 0.42 | -0.46 | * | . | F | 1.89 | 0.64 |
| Ser | 369 | . | . | . | . | . | T | C | -0.28 | -0.17 | * | . | F | 2.10 | 0.64 |
| Glu | 370 | A | . | . | . | . | T | . | -0.52 | -0.07 | . | * | F | 1.69 | 0.75 |
| Lys | 371 | A | . | . | . | . | T | . | 0.29 | 0.04 | . | . | F | 1.03 | 1.19 |
| Leu | 372 | A | A | . | . | . | . | . | 1.14 | -0.39 | . | . | F | 1.02 | 1.54 |
| Phe | 373 | A | A | . | . | . | . | . | 1.49 | -0.77 | * | . | . | 0.96 | 1.54 |
| Tyr | 374 | A | A | . | . | . | . | . | 1.79 | -0.77 | * | . | . | 0.75 | 1.28 |
| Glu | 375 | A | A | . | . | . | . | . | 1.20 | -0.77 | * | . | F | 0.90 | 2.70 |
| Glu | 376 | A | A | . | . | . | . | . | 0.81 | -0.96 | * | . | F | 0.90 | 3.15 |
| Asp | 377 | A | A | . | . | . | . | . | 1.32 | -1.31 | . | . | F | 0.90 | 1.99 |
| Glu | 378 | A | . | . | . | . | T | . | 1.43 | -1.69 | . | . | F | 1.30 | 1.54 |
| Ala | 379 | A | . | . | . | . | T | . | 1.37 | -1.19 | . | . | F | 1.15 | 0.90 |
| Gly | 380 | A | . | . | . | . | T | . | 1.07 | -0.70 | . | . | F | 1.15 | 0.78 |
| Ser | 381 | A | . | . | . | . | T | . | 0.40 | -0.31 | * | . | F | 0.85 | 0.60 |
| Ala | 382 | A | . | . | . | . | T | . | -0.41 | 0.26 | * | . | F | 0.25 | 0.32 |
| Thr | 383 | A | . | . | . | . | T | . | -0.80 | 0.44 | . | . | F | -0.05 | 0.27 |
| Ser | 384 | A | . | . | . | . | T | . | -0.60 | 0.44 | . | . | F | -0.05 | 0.25 |
| Cys | 385 | . | . | B | . | . | T | . | -0.64 | 0.49 | . | . | . | -0.20 | 0.32 |
| Leu | 386 | . | . | B | . | . | . | . | -0.73 | 0.41 | . | . | . | -0.40 | 0.28 |

In another aspect, the invention provides an isolated nucleic acid molecule comprising a polynucleotide which hybridizes under stringent hybridization conditions to a portion of the polynucleotide in a nucleic acid molecule of the invention described above, for instance, the complement of the TR10 coding polynucleotide sequence disclosed herein or the cDNA clone contained in ATCC Deposit No. 209040. By "stringent hybridization conditions" is intended overnight incubation at 42°C in a solution comprising: 50% formamide, 5x SSC (750 mM NaCl, 75mM trisodium citrate), 50 mM sodium phosphate (pH 7.6), 5x Denhardt's solution, 10% dextran sulfate, and 20 g/ml denatured, sheared salmon sperm DNA, followed by washing the filters in 0.1x SSC at about 65°C.

By a polynucleotide which hybridizes to a "portion" of a polynucleotide is intended a polynucleotide (either DNA or RNA) hybridizing to at least about 15 nucleotides (nt), and more preferably at least about 20 nt, still more preferably at least about 30 nt, and even more preferably about 30-70 nt of the reference polynucleotide. These are useful as diagnostic probes and primers as discussed above and in more detail below. In this context "about" includes the particularly recited size, larger or smaller by several (5, 4, 3, 2, or 1) nucleotides, at either terminus or at both termini.

By a portion of a polynucleotide of "at least 20 nt in length," for example, is intended 20 or more contiguous nucleotides from the nucleotide sequence of the reference polynucleotide (e.g., the deposited cDNA or the nucleotide sequence as shown in SEQ ID NO:1).

Of course, a polynucleotide which hybridizes only to a poly A sequence (such as the 3' terminal poly(A) tract of the TR10 cDNA shown in SEQ ID NO:1), or to a complementary stretch of T (or U) resides, would not be included in a polynucleotide of the invention used to hybridize to a portion of a nucleic acid of the invention, since such a polynucleotide would hybridize to any nucleic acid molecule containing a poly (A) stretch or the complement thereof (e.g., practically any double-stranded cDNA clone).

In specific embodiments, the polynucleotides of the invention are less than 100000 kb, 50000 kb, 10000 kb, 1000 kb, 500 kb, 400 kb, 350 kb, 300 kb, 250 kb, 200 kb, 175 kb, 150 kb, 125 kb, 100 kb, 75 kb, 50 kb, 40 kb, 30 kb, 25 kb, 20 kb, 15 kb, 10 kb, 7.5 kb, or 5 kb in length.

In further embodiments, polynucleotides of the invention comprise at least 15, at least 30, at least 50, at least 100, or at least 250, at least 500, or at least 1000 contiguous nucleotides of TR10 coding sequence, but consist of less than or equal to 1000 kb, 500 kb, 250 kb, 200 kb, 150 kb, 100 kb, 75 kb, 50 kb, 30 kb, 25 kb, 20 kb, 15 kb, 10 kb, or 5 kb of genomic DNA that flanks the 5' or 3' coding nucleotide set forth in Figures 1A-D (SEQ ID NO:1). In further embodiments, polynucleotides of the invention comprise at least 15, at least 30, at least 50, at least 100, or at least 250, at least 500, or at least 1000 contiguous nucleotides of TR10 coding sequence, but do not comprise all or a portion of any TR10 intron. In another embodiment, the nucleic acid comprising TR10 coding sequence does not contain coding sequences of a genomic flanking gene (i.e., 5' or 3' to the TR10 gene in the genome). In other embodiments, the polynucleotides of the invention do not contain the coding sequence of more than 1000, 500, 250, 100, 50, 25, 20, 15, 10, 5, 4, 3. 2, or I genomic flanking gene(s).

As indicated, nucleic acid molecules of the present invention which encode a TR10 polypeptide may include, but are not limited to the coding sequence for the mature polypeptide, by itself; the coding sequence for the mature polypeptide and additional sequences, such as those encoding a leader or secretory sequence, such as a pre-, or pro- or prepro- protein sequence; the coding sequence of the mature polypeptide, with or without the aforementioned additional coding sequences, together with additional, non-coding sequences, including for example, but not limited to introns and non-coding 5' and 3' sequences, such as the transcribed, non-translated sequences that play a role in transcription, mRNA processing - including splicing and polyadenylation signals, for example - ribosome binding and stability of mRNA; additional coding sequence which codes for additional amino acids, such as those which provide additional functionalities. Thus, for instance, the polypeptide may be fused to a marker sequence, such as a peptide, which facilitates purification of the fused polypeptide. In certain preferred embodiments of this aspect of the invention, the marker sequence is a hexa-histidine peptide, such as the tag provided in a pQE vector (Qiagen, Inc,), among others, many of which are commercially available. As described in Gentz *et al., Proc. Natl. Acad. Sci. USA 86:* 821-824 (1989), for instance, hexa-histidine provides for convenient purification of the fusion protein. The "HA" tag is another peptide useful for purification which corresponds to an epitope derived from the influenza hemagglutinin protein, which has been described by Wilson *et al., Cell 37*:767-778 (1984). As discussed below, other such fusion proteins include the TR10 receptor fused to Fc at the N- or C-terminus.

The present invention further relates to variants of the nucleic acid molecules of the present invention, which encode portions, analogs, or derivatives of the TR10 receptor. Variants may occur naturally, such as a natural allelic variant. By an "allelic variant" is intended one of several alternate forms of a gene occupying a given locus on a chromosome of an organism. *Genes II,* Lewin, B., ed., John Wiley & Sons, New York (1985). Non-naturally occurring variants may be produced using art-known mutagenesis techniques.

Such variants include those produced by nucleotide substitutions, deletions or additions which may involve one or more nucleotides. The variants may be altered in coding or non-coding regions or both. Alterations in the coding regions may produce conservative or non-conservative amino acid substitutions, deletions, or additions. Especially preferred among these are silent substitutions, additions, and deletions, which do not alter the properties and activities of the TR10 receptor or portions thereof. Also especially preferred in this regard are conservative substitutions.

Further embodiments of the invention include isolated nucleic acid molecules comprising or, alternatively, consisting of a polynucleotide having a nucleotide sequence at least 80%, 85%, or 90% identical, and more preferably at least 95%, 96%, 97%, 98%, or 99% identical to: (a) a nucleotide sequence encoding the polypeptide having the amino acid sequence in SEQ ID NO:2; (b) a nucleotide sequence encoding the polypeptide having the amino acid sequence in SEQ ID NO: 2, but lacking the amino terminal methionine; (c) a nucleotide sequence encoding the polypeptide having the amino acid sequence at positions about I to about 331 in SEQ ID NO:2; (d) a nucleotide sequence encoding the polypeptide having the amino acid sequence encoded by the cDNA clone contained in ATCC Deposit No. 209040; (e) a nucleotide sequence encoding the mature TR10 polypeptide having the amino acid sequence encoded by the cDNA clone contained in ATCC Deposit No. 209040; (f) a nucleotide sequence encoding the TR10 receptor extracellular domain; (g) a nucleotide sequence encoding the TR10 cysteine rich domain; (h) a nucleotide sequence encoding the TR10 receptor transmembrane domain; (i) a nucleotide sequence encoding the TR10 receptor intracellular domain; (j) a nucleotide sequence encoding the TR10 receptor extracellular and intracellular domains with all or part of the transmembrane domain deleted; (k) a nucleotide sequence encoding the TR10 receptor partial death domain; and (l) a nucleotide sequence complementary to any of the nucleotide sequences in (a), (b), (c), (d), (e), (f), (g), (h), (i), (j) or (k) above.

By a polynucleotide having a nucleotide sequence at least, for example, 95% "identical" to a reference nucleotide sequence encoding a TR10 polypeptide is intended that the nucleotide sequence of the polynucleotide is identical to the reference sequence except that the polynucleotide sequence may include up to five mismatches per each 100 nucleotides of the reference nucleotide sequence encoding the TR10 polypeptide. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mismatches of the reference sequence may occur at the 5' or 3' terminal positions of the reference nucleotide sequence or anywhere between those terminal positions, interspersed either individually among nucleotides in the reference sequence or in one or more contiguous groups within the reference sequence. The reference (query) sequence may be the entire TR10 encoding nucleotide sequence shown in Figures 1A-D (SEQ ID NO:1) or any TR10 polynucleotide fragment (e.g., a polynucleotide encoding the amino acid sequence of any of the TR10 N- and/or C- terminal deletions described herein), variant, derivative or analog, as described herein.

As a practical matter, whether any particular nucleic acid molecule is at least 80%, 85%, 90%, 95%, 96%, 97%, 98% or 99% identical to for instance, the nucleotide sequence shown in SEQ ID NO: 1 or to the nucleotide sequence of the deposited cDNA clone can be determined conventionally using known computer programs such as the Bestfit program (Wisconsin Sequence Analysis Package, Version 8 for Unix, Genetics Computer Group, University Research Park, 575 Science Drive, Madison, W1 53711). Bestfit uses the local homology algorithm of Smith and Waterman, *Advances in Applied Mathematics 2*: 482-489 (1981), to find the best segment of homology between two sequences. When using Bestfit or any other sequence alignment program to determine whether a particular sequence is, for instance, 95% identical to a reference sequence according to the present invention, the parameters are set, of course, such that the percentage of identity is calculated over the full length of the reference nucleotide sequence and that gaps in homology of up to 5% of the total number of nucleotides in the reference sequence are allowed.

In a specific embodiment, the identity between a reference (query) sequence (a sequence of the present invention) and a subject sequence, also referred to as a global sequence alignment, is determined using the FASTDB computer program based on the algorithm of Brutlag et al. (Comp. App. Biosci. 6:237-245 (1990)). Preferred parameters used in a FASTDB alignment of DNA sequences to calculate percent identity are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the length of the subject nucleotide sequence, whichever is shorter. According to this embodiment, if the subject sequence is shorter than the query sequence because of 5' or 3' deletions, not because of internal deletions, a manual correction is made to the results to take into consideration the fact that the FASTDB program does not account for 5' and 3' truncations of the subject sequence when calculating percent identity. For subject sequences truncated at the 5' or 3' ends, relative to the query sequence, the percent identity is corrected by calculating the number of bases of the query sequence that are 5' and 3' of the subject sequence, which are not matched/aligned, as a percent of the total bases of the query sequence. A determination of whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of this embodiment. Only bases outside the 5' and 3' bases of the subject sequence, as displayed by the FASTDB alignment, which are not matched/aligned with the query sequence, are calculated for the purposes of manually adjusting the percent identity score. For example, a 90 base subject sequence is aligned to a 100 base query sequence to determine percent identity. The deletions occur at the 5' end of the subject sequence and therefore, the FASTDB alignment does not show a matched/alignment of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequence are manually corrected for. No other manual corrections are made for the purposes of this embodiment.

The present application is directed to nucleic acid molecules at least 80%, 85%, 90%, 95%, 96%, 97%, 98%, or 99% identical to the nucleic acid sequence for example, shown in SEQ ID NO: 1, or to the nucleic acid sequence of the deposited cDNA, irrespective of whether they encode a polypeptide having TR10 receptor activity. This is because even where a particular nucleic acid molecule does not encode a polypeptide having TR10 functional activity, one of skill in the art would still know how to use the nucleic acid molecule, for instance, as a hybridization probe or a polymerase chain reaction (PCR) primer. Uses of the nucleic acid molecules of the present invention that do not encode a polypeptide having TR10 receptor activity include, *inter alia:* (1) isolating the TR10 receptor gene or allelic variants thereof in a cDNA library; (2) *in situ* hybridization (e.g., "FISH") to metaphase chromosomal spreads to provide precise chromosomal location of the TR10 receptor gene, as described in Verma *et al., Human Chromosomes: A Manual of Basic Techniques,* Pergamon Press, New York (1988); and (3) Northern Blot analysis for detecting TR10 receptor mRNA expression in specific tissues.

Preferred, however, are nucleic acid molecules having sequences at least 90%, 80%, 85%, 95%, 96%, 97%, 98% or 99% identical to for example, the nucleic acid sequence shown in SEQ ID NO:1, or to the nucleic acid sequence of the deposited cDNA, which do, in fact, encode a polypeptide having TR10 receptor functional activity. By "a polypeptide having TR10 functional receptor activity" is intended polypeptides exhibiting activity similar, but not necessarily identical, to an activity of the TR10 receptor of the invention (either the full-length protein or, preferably, the mature protein), as measured in a particular biological assay. For example, TR10 functional receptor activity can be measured using the cell death assays performed essentially as previously described (A.M. Chinnaiyan *et al., Cell 81:* 505-512 (1995); M.P. Boldin *et al., J. Biol. Chem. 270*:7795-8(1995); F.C. Kischkel *et al., EMBO 14*:5579-5588 (1995); A.M. Chinnaiyan *et al.. J. Biol. Chem. 271:* 4961-4965 (1996)) and as set forth in Example 5, below. In MCF7 cells, plasmids encoding full-length TR10 or a candidate death domain containing receptor are co-transfected with the pLantern reporter construct encoding green fluorescent protein. Nuclei of cells transfected with TR10 will exhibit apoptotic morphology as assessed by DAPI staining. Similar to TNFR-1 and Fas/APO-1 (M. Muzio *et al., Cell 85*:817-827 (1996); M. P. Boldin *et al., Cell 85*:803-815 (1996); M. Tewari *et al., J. Biol. Chem. 270*:3255-60 (1995)). TR 10-induced apoptosis is blocked by the inhibitors of ICE-like proteases, CrmA and z-VAD-fmk. In addition, apoptosis induced by TR10 is also blocked by dominant negative versions of FADD (FADD-DN) or FLICE (FLICE-DN/MACHalC360S).

Of course, due to the degeneracy of the genetic code, one of ordinary skill in the art will immediately recognize that a large number of the nucleic acid molecules having a sequence at least 80%, 85%, 90%, 95%, 96%, 97%, 98%, or 99% identical to, for example, the nucleic acid sequence of the deposited cDNA or the nucleic acid sequence shown in SEQ ID NO: will encode a polypeptide "having TR10 receptor functional activity." In fact, since degenerate variants of these nucleotide sequences all encode the same polypeptide, this will be clear to the skilled artisan even without performing the above described comparison assay. It will be further recognized in the art that, for such nucleic acid molecules that are not degenerate variants, a reasonable number will also encode a polypeptide having TR10 receptor activity. This is because the skilled artisan is fully aware of amino acid substitutions that are either less likely or not likely to significantly effect protein function (e.g., replacing one aliphatic amino acid with a second aliphatic amino acid).

For example, guidance concerning how to make phenotypically silent amino acid substitutions is provided in J.U. Bowie *et al.,* "Deciphering the Message in Protein Sequences: Tolerance to Amino Acid Substitutions," *Science 247*:1306-1310 (1990), wherein the authors indicate that proteins are surprisingly tolerant of amino acid substitutions.

***Polynucleotide assays***

This invention is also related to the use of TR10 polynucleotides to detect complementary polynucleotides such as, for example, as a diagnostic reagent. Detection of a mutated form of TR10 associated with a dysfunction will provide a diagnostic tool that can add or define a diagnosis of a disease or susceptibility to a disease which results from under-expression over-expression or altered expression of TR10 or a soluble form thereof, such as, for example, tumors or autoimmune disease.

Individuals carrying mutations in the TR10 gene may be detected at the DNA level by a variety of techniques. Nucleic acids for diagnosis may be obtained from a patient's cells, such as from blood, urine, saliva, tissue biopsy and autopsy material. The genomic DNA may be used directly for detection or may be amplified enzymatically by using PCR prior to analysis. (Saiki *et al., Nature 324*:163-166 (1986)). RNA or cDNA may also be used in the same ways. As an example, PCR primers complementary to the nucleic acid encoding TR10 can be used to identify and analyze TR10 expression and mutations. For example, deletions and insertions can be detected by a change in size of the amplified product in comparison to the normal genotype. Point mutations can be identified by hybridizing amplified DNA to radiolabeled TR10 RNA or alternatively, radiolabeled TR10 antisense DNA sequences. Perfectly matched sequences can be distinguished from mismatched duplexes by RNase A digestion or by differences in melting temperatures.

Sequence differences between a reference gene and genes having mutations also may be revealed by direct DNA sequencing. In addition, cloned DNA segments may be employed as probes to detect specific DNA segments. The sensitivity of such methods can be greatly enhanced by appropriate use of PCR or another amplification method. For example, a sequencing primer is used with double-stranded PCR product or a single-stranded template molecule generated by a modified PCR. The sequence determination is performed by conventional procedures with radiolabeled nucleotide or by automatic sequencing procedures with fluorescent-tags.

Genetic testing based on DNA sequence differences may be achieved by detection of alteration in electrophoretic mobility of DNA fragments in gels, with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperatures (see, e.g., Myers *et al., Science 230*:1242 (1985)).

Sequence changes at specific locations also may be revealed by nuclease protection assays, such as RNase and S1 protection or the chemical cleavage method (e.g., *Cotton et al., Proc. Natl. Acad. Sci. USA 85:* 4397-4401 (1985)).

Thus, the detection of a specific DNA sequence may be achieved by methods such as hybridization, RNase protection, chemical cleavage, direct DNA sequencing or the use of restriction enzymes, (e.g., restriction fragment length polymorphisms ("RFLP") and Southern blotting of genomic DNA.

In addition to more conventional gel-electrophoresis and DNA sequencing, mutations also can be detected by *in situ* analysis.

***Vectors and Host Cells***

The present invention also relates to vectors which include the isolated DNA molecules of the present invention, host cells which are genetically engineered with the recombinant vectors and/or nucleic acids of the invention and the production of TR10 polypeptides or fragments thereof by recombinant techniques.

Host cells can be genetically engineered to incorporate nucleic acid molecules and express polypeptides of the present invention. The polynucleotides may be introduced alone or with other polynucleotides. Such other polynucleotides may be introduced independently, co-introduced or introduced joined to the polynucleotides of the invention.

In accordance with the present invention the vector may be, for example, a plasmid vector, a single or double-stranded phage vector, a single or double-stranded RNA or DNA viral vector. Such vectors may be introduced into cells as polynucleotides, preferably DNA, by well known techniques for introducing DNA and RNA into cells. Viral vectors may be replication competent or replication defective. In the latter case viral propagation generally will occur only in complementing host cells.

Preferred among vectors, in certain respects, are those for expression of polynucleotides and polypeptides of the present invention. Generally, such vectors comprise cis-acting control regions effective for expression in a host operatively linked to the polynucleotide to be expressed. Appropriate trans-acting factors either are supplied by the host, supplied by a complementing vector or supplied by the vector itself upon introduction into the host.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged *in vitro* using an appropriate packaging cell line and then transduced into host cells.

The DNA insert should be operatively linked to an appropriate promoter, such as the phage lambda PL promoter, the *E. coli lac, trp* and *tac* promoters, the SV40 early and late promoters and promoters of retroviral LTRs, to name a few. Other suitable promoters will be known to the skilled artisan. The expression constructs will further contain sites for transcription initiation, termination and, in the transcribed region, a ribosome binding site for translation. The coding portion of the mature transcripts expressed by the constructs will preferably include a translation initiating at the beginning and a termination codon (UAA, UGA or UAG) appropriately positioned at the end of the polypeptide to be translated.

As indicated, the expression vectors will preferably include at least one selectable marker. Such markers include dihydrofolate reductase or neomycin resistance for eukaryotic cell culture and tetracycline or ampicillin resistance genes for culturing in *E. coli* and other bacteria. Representative examples of appropriate hosts include, but are not limited to, bacterial cells, such as *E. coli, Streptomyces* and *Salmonella typhimurium* cells; fungal cells, such as yeast cells; insect cells such as *Drosophila* S2 and *Spodoptera* Sf9 cells; animal cells such as CHO, COS and Bowes melanoma cells; and plant cells. Appropriate culture mediums and conditions for the above-described host cells are known in the art.

Among vectors preferred for use in bacteria include pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

The present invention also relates to host cells containing the above-described vector constructs described herein, and additionally encompasses host cells containing nucleotide sequences of the invention that are operably associated with one or more heterologous control regions (e.g., promoter and/or enhancer) using techniques known of in the art. The host cell can be a higher eukaryotic cell, such as a mammalian cell (e.g., a human derived cell), or a lower eukaryotic cell, such as a yeast cell, or the host cell can be a prokaryotic cell, such as a bacterial cell. The host strain may be chosen which modulates the expression of the inserted gene sequences, or modifies and processes the gene product in the specific fashion desired. Expression from certain promoters can be elevated in the presence of certain inducers; thus expression of the genetically engineered polypeptide may be controlled. Furthermore, different host cells have characteristics and specific mechanisms for the translational and post-translational processing and modification (e.g., phosphorylation, cleavage) of proteins. Appropriate cell lines can be chosen to ensure the desired modifications and processing of the foreign protein expressed.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals, such as Davis *et al., Basic Methods In Molecular*

*Biology* (1986).

In addition to encompassing host cells containing the vector constructs discussed herein, the invention also encompasses primary, secondary, and immortalized host cells of vertebrate origin, particularly mammalian origin, that have been engineered to delete or replace endogenous genetic material (e.g., TR10 coding sequence), and/or to include genetic material (e.g., heterologous polynucleotide sequences) that is operably associated with TR10 polynucleotides of the invention, and which activates, alters, and/or amplifies endogenous TR10 polynucleotides. For example, techniques known in the art may be used to operably associate heterologous control regions (e.g., promoter and/or enhancer) and endogenous TR10 polynucleotide sequences via homologous recombination (see, e.g., US Patent Number 5,641,670, issued June 24, 1997; International Publication Number WO 96/29411, published September 26, 1996; International Publication Number WO 94/12650, published August 4, 1994; Koller *et al., Proc. Natl. Acad. Sci. USA 86*:8932-8935 (1989); and Zijlstra *et al., Nature 342*:435-438 (1989), the disclosures of each of which are incorporated by reference in their entireties).

The polypeptide may be expressed in a modified form, such as a fusion protein (comprising the polypeptide joined via a peptide bond to a heterologous protein sequence (of a different protein)), and may include not only secretion signals but also additional heterologous functional regions. Alternatively, such a fusion protein can be made by protein synthetic techniques, e.g., by use of a peptide synthesizer. Thus, a region of additional amino acids, particularly charged amino acids, may be added to the N-terminus of the polypeptide to improve stability and persistence in the host cell, during purification or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. For example, in one embodiment, polynucleotides encoding TR10 polypeptides of the invention may be fused to the pelB pectate lyase signal sequence to increase the efficiency to expression and purification of such polypeptides in Gram-negative bacteria. See, US Patent Nos. 5,576,195 and 5,846,818, the contents of which are herein incorporated by reference in their entireties.

A preferred fusion protein comprises a heterologous region from immunoglobulin that is useful to solubilize proteins. For example, EP-A-O 464 533 (Canadian counterpart 2045869) discloses fusion proteins comprising various portions of constant region of immunoglobin molecules together with another human protein or part thereof. In many cases, the Fc part in a fusion protein is thoroughly advantageous for use in therapy and diagnosis and thus results, for example, in improved pharmacokinetic properties (EP-A 0232 262). On the other hand, for some uses, it would be desirable to be able to delete the Fc part after the fusion protein has been expressed, detected and purified in the advantageous manner described. This is the case when the Fc portion proves to be a hindrance to use in therapy and diagnosis, for example, when the fusion protein is to be used as an antigen for immunizations. In drug discovery, for example, human proteins, such as the hIL5-receptor, have been fused with Fc portions for the purpose of high-throughput screening assays to identify antagonists of hIL-5. See, D. *Bennett et al., Journal of Molecular Recognition* 8:52-58 (1995) and K. Johanson et *al., The Journal of Biological Chemistry* 270:16:9459-9471 (1995).

The TR10 polypeptides of the invention can be recovered and purified from chemical synthesis and recombinant cell cultures by standard methods which include, but are not limited to, ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably high performance liquid chromatography ("HPLC") is employed for purification. Well known techniques for refolding protein may be employed to regenerate active conformation when the polypeptide is denatured during isolation and/or purification.

TR 10 receptor polynucleotides and polypeptides may be used in accordance with the present invention for a variety of applications, particularly those that make use of the chemical and biological properties of TR10. Among these are applications in treatment of tumors, resistance to parasites, bacteria and viruses, to induce proliferation of T-cells, endothelial cells and certain hematopoietic cells, to treat restenosis, graft vs. host disease, to regulate anti-viral responses and to prevent certain autoimmune diseases after stimulation of TR10 by an agonist. Additional applications relate to diagnosis and to treatment of disorders of cells, tissues and organisms. These aspects of the invention are discussed further below.

## ***Transgenics and "knock-outs"***

The TR10 proteins of the invention can also be expressed in transgenic animals. Animals of any species, including, but not limited to, mice, rats, rabbits, hamsters, guinea pigs, pigs, micro-pigs, goats, sheep, cows and non-human primates, e.g., baboons, monkeys, and chimpanzees may be used to generate transgenic animals. In a specific embodiment, techniques described herein or otherwise known in the art, are used to express polypeptides of the invention in humans, as part of a gene therapy protocol.

Any technique known in the art may be used to introduce the transgene (i.e., nucleic acids of the invention) into animals to produce the founder lines of transgenic animals. Such techniques include, but are not limited to, pronuclear microinjection (Paterson *et al., Appl. Microbiol. Biotechnol. 40*:691-698 (1994); Carver *et al., Biotechnology (NY) 11*: 1263-1270 (1993); Wright *et al., Biotechnology (NY) 9*:830-834 (1991); and Hoppe et al., US Patent Number 4,873,191 (1989)); retrovirus mediated gene transfer into germ lines (Van der Putten *et al., Proc. Natl. Acad. Sci., USA 82*:6148-6152 (1985)), blastocysts or embryos; gene targeting in embryonic stem cells (Thompson *et al., Cell 56*:313-321 (1989)), electroporation of cells or embryos (Lo. *Mol Cell. Biol. 3*:1803-1814 (1983)); introduction of the polynucleotides of the invention using a gene gun (see, e.g., Ulmer *et al., Science 259*:1745 (1993); introducing nucleic acid constructs into embryonic pleuripotent stem cells and transferring the stem cells back into the blastocyst: and sperm-mediated gene transfer (Lavitrano *et al., Cell 57*:717-723 (1989); etc. For a review of such techniques, see Gordon, "Transgenic Animals," *Intl. Rev. Cytol. 115:*171-229 (1989), which is incorporated by reference herein in its entirety. Further, the contents of each of the documents recited in this paragraph is herein incorporated by reference in its entirety. Gordon. "Transgenic Animals," Intl. Rev. Cytol. 115:171-229 (1989), which is incorporated by reference herein in its entirety. See also. U.S. Patent No. 5,464,764 (Capecchi, et al., Positive-Negative Selection Methods and Vectors); U.S. Patent No. 5,631,153 (Capecchi. et al., Cells and Non-Human Organisms Containing Predetermined Genomic Modifications and Positive-Negative Selection Methods and Vectors for Making Same); U.S. Patent No. 4,736,866 (Leder, et al., Transgenic Non-Human Animals); and U.S. Patent No. 4,873,191 (Wagner, et al., Genetic Transformation of Zygotes); each of which is hereby incorporated by reference in its entirety.

Any technique known in the art may be used to produce transgenic clones containing polynucleotides of the invention, for example, nuclear transfer into enucleated oocytes of nuclei from cultured embryonic, fetal, or adult cells induced to quiescence (Campell *et al., Nature 380*:64-66 (1996); Wilmut *et al., Nature 385*:810-813 (1997)), each of which is herein incorporated by reference in its entirety).

The present invention provides for transgenic animals that carry the transgene in all their cells, as well as animals which carry the transgene in some, but not all their cells, i.e., mosaic animals or chimeric animals. The transgene may be integrated as a single transgene or as multiple copies such as in concatamers, e.g., head-to-head tandems or head-to-tail tandems. The transgene may also be selectively introduced into and activated in a particular cell type by following, for example, the teaching of Lasko *et al. (Proc. Natl. Acad. Sci. USA 89*:6232-6236 (1992)). The regulatory sequences required for such a cell-type specific activation will depend upon the particular cell type of interest, and will be apparent to those of skill in the art. When it is desired that the polynucleotide transgene be integrated into the chromosomal site of the endogenous gene, gene targeting is preferred. Briefly, when such a technique is to be utilized, vectors containing some nucleotide sequences homologous to the endogenous gene are designed for the purpose of integrating, via homologous recombination with chromosomal sequences, into and disrupting the function of the nucleotide sequence of the endogenous gene. The transgene may also be selectively introduced into a particular cell type, thus inactivating the endogenous gene in only that cell type, by following, for example, the teaching of Gu *et al. (Science 265:*103-106 (1994)). The regulatory sequences required for such a cell-type specific inactivation will depend upon the particular cell type of interest, and will be apparent to those of skill in the art. The contents of each of the documents recited in this paragraph is herein incorporated by reference in its entirety.

Once transgenic animals have been generated, the expression of the recombinant gene may be assayed utilizing standard techniques. Initial screening may be accomplished by Southern blot analysis or PCR techniques to analyze animal tissues to verify that integration of the transgene has taken place. The level of mRNA expression of the transgene in the tissues of the transgenic animals may also be assessed using techniques which include, but are not limited to, Northern blot analysis of tissue samples obtained from the animal, *in situ* hybridization analysis, and reverse transcriptase-PCR (rt-PCR). Samples of transgenic gene-expressing tissue may also be evaluated immunocytochemically or immunohistochemically using antibodies specific for the transgene product.

Once the founder animals are produced, they may be bred, inbred, outbred, or crossbred to produce colonies of the particular animal. Examples of such breeding strategies include, but are not limited to: outbreeding of founder animals with more than one integration site in order to establish separate lines; inbreeding of separate lines in order to produce compound transgenics that express the transgene at higher levels because of the effects of additive expression of each transgene; crossing of heterozygous transgenic animals to produce animals homozygous for a given integration site in order to both augment expression and eliminate the need for screening of animals by DNA analysis; crossing of separate homozygous lines to produce compound heterozygous or homozygous lines; and breeding to place the transgene on a distinct background that is appropriate for an experimental model of interest.

Transgenic and "knock-out" animals of the invention have uses which include, but are not limited to, animal model systems useful in elaborating the biological function of TR10 polypeptides, studying conditions and/or disorders associated with aberrant TR10 expression, and in screening for compounds effective in ameliorating such conditions and/or disorders.

In further embodiments of the invention, cells that are genetically engineered to express the proteins of the invention, or alternatively, that are genetically engineered not to express the proteins of the invention (e.g., knockouts) are administered to a patient *in vivo.* Such cells may be obtained from the patient (i.e., animal, including human) or an MHC compatible donor and can include, but are not limited to fibroblasts, bone marrow cells, blood cells (e.g., lymphocytes), adipocytes, muscle cells, endothelial cells, etc. The cells are genetically engineered *in vitro* using recombinant DNA techniques to introduce the coding sequence of polypeptides of the invention into the cells, or alternatively, to disrupt the coding sequence and/or endogenous regulatory sequence associated with the polypeptides of the invention, e.g., by transduction (using viral vectors, and preferably vectors that integrate the transgene into the cell genome) or transfection procedures, including, but not limited to, the use of plasmids, cosmids, YACs, naked DNA. electroporation, liposomes, etc. The coding sequence of the polypeptides of the invention can be placed under the control of a strong constitutive or inducible promoter or promoter/enhancer to achieve expression, and preferably secretion, of the polypeptides of the invention. The engineered cells which express and preferably secrete the polypeptides of the invention can be introduced into the patient systemically, e.g., in the circulation, or intraperitoneally. Alternatively, the cells can be incorporated into a matrix and implanted in the body, e.g., genetically engineered fibroblasts can be implanted as part of a skin graft; genetically engineered endothelial cells can be implanted as part of a lymphatic or vascular graft. (See, for example, Anderson et al. US Patent Number 5,399,349; and Mulligan & Wilson, US Patent Number 5,460,959. each of which is incorporated by reference herein in its entirety).

When the cells to be administered are non-autologous or non-MHC compatible cells, they can be administered using well known techniques which prevent the development of a host immune response against the introduced cells. For example, the cells may be introduced in an encapsulated form which, while allowing for an exchange of components with the immediate extracellular environment, does not allow the introduced cells to be recognized by the host immune system.

### *TR10 Receptor Polypeptides and Fragments*

The TR10 proteins (polypeptides) of the invention may be in monomers or multimers (i.e., dimers, trimers, tetramers, and higher multimers). Accordingly, the present invention relates to monomers and multimers of the TR10 proteins (polypeptides) of the invention, their preparation, and compositions (preferably, pharmaceutical compositions) containing them. In specific embodiments, the polypeptides of the invention are monomers, dimers, trimers or tetramers. In additional embodiments, the multimers of the invention are at least dimers, at least trimers, or at least tetramers.

Multimers encompassed by the invention may be homomers or heteromers. As used herein, the term homomer, refers to a multimer containing only TR10 proteins of the invention (including TR10 fragments, variants, and fusion proteins, as described herein). These homomers may contain TR10 proteins having identical or different polypeptide sequences. In a specific embodiment, a homomer of the invention is a multimer containing only TR10 proteins having an identical polypeptide sequence. In another specific embodiment, a homomer of the invention is a multimer containing TR10 proteins having different polypeptide sequences. In specific embodiments, the multimer of the invention is a homodimer (e.g., containing TR10 proteins having identical or different polypeptide sequences) or a homotrimer (e.g., containing TR10 proteins having identical or different polypeptide sequences). In additional embodiments, the homomeric multimer of the invention is at least a homodimer, at least a homotrimer, or at least a homotetramer.

As used herein, the term heteromer refers to a multimer containing heterologous proteins (i.e., proteins containing only polypeptide sequences that do not correspond to a polypeptide sequences encoded by the TR10 gene) in addition to the TR10 proteins of the invention. In a specific embodiment, the multimer of the invention is a heterodimer, a heterotrimer, or a heterotetramer. In additional embodiments, the homomeric multimer of the invention is at least a homodimer, at least a homotrimer, or at least a homotetramer.

Multimers of the invention may be the result of hydrophobic, hydrophilic, ionic and/or covalent associations and/or may be indirectly linked, by for example, liposome formation. Thus, in one embodiment, multimers of the invention, such as, for example, homodimers or homotrimers, are formed when proteins of the invention contact one another in solution. In another embodiment, heteromultimers of the invention, such as, for example, heterotrimers or heterotetramers, are formed when proteins of the invention contact antibodies to the polypeptides of the invention (including antibodies to the heterologous polypeptide sequence in a fusion protein of the invention) in solution. In other embodiments, multimers of the invention are formed by covalent associations with and/or between the TR10 proteins of the invention. Such covalent associations may involve one or more amino acid residues contained in the polypeptide sequence of the protein (e.g., the polypeptide sequence recited in SEQ ID NO:2 or the polypeptide encoded by the deposited cDNA clone). In one instance, the covalent associations are cross-linking between cysteine residues located within the polypeptide sequences of the proteins which interact in the native (i.e., naturally occurring) polypeptide. In another instance, the covalent associations are the consequence of chemical or recombinant manipulation. Alternatively, such covalent associations may involve one or more amino acid residues contained in the heterologous polypeptide sequence in a TR10 fusion protein. In one example, covalent associations are between the heterologous sequence contained in a fusion protein of the invention (see, e.g., US Patent Number 5,478,925). In a specific example, the covalent associations are between the heterologous sequence contained in a TR10-Fc fusion protein of the invention (as described herein). In another specific example, covalent associations of fusion proteins of the invention are between heterologous polypeptide sequences from another TNF family ligand/receptor member that is capable of forming covalently associated multimers, such as for example, oseteoprotegerin (see, e.g., International Publication No. WO 98/49305, the contents of which are herein incorporated by reference in its entirety). In another embodiment, two or more TR10 polypeptides of the invention are joined through synthetic linkers (e.g., peptide, carbohydrate or soluble polymer linkers). Examples include those peptide linkers described in U.S. Pat. No. 5,073,627 (hereby incorporated by reference). Proteins comprising multiple TR10 polypeptides separated by peptide linkers may be produced using conventional recombinant DNA technology.

Another method for preparing multimer TR10 polypeptides of the invention involves use of TR10 polypeptides fused to a leucine zipper or isoleucine polypeptide sequence. Leucine zipper domains and isoleucine zipper domains are polypeptides that promote multimerization of the proteins in which they are found. Leucine zippers were originally identified in several DNA-binding proteins (Landschulz et al., *Science* 240:1759, (1988)), and have since been found in a variety of different proteins. Among the known leucine zippers are naturally occurring peptides and derivatives thereof that dimerize or trimerize. Examples of leucine zipper domains suitable for producing soluble multimeric TR10 proteins are those described in PCT application WO 94/10308, hereby incorporated by reference. Recombinant fusion proteins comprising a soluble TR10 polypeptide fused to a peptide that dimerizes or trimerizes in solution are expressed in suitable host cells, and the resulting soluble multimeric TR10 is recovered from the culture supernatant using techniques known in the art.

Certain members of the TNF family of proteins are believed to exist in trimeric form (Beutler and Huffel, *Science* 264:667, 1994; Banner et al., *Cell* 73:431, 1993). Thus, trimeric TR10 may offer the advantage of enhanced biological activity. Preferred leucine zipper moieties are those that preferentially form trimers. One example is a leucine zipper derived from lung surfactant protein D (SPD), as described in Hoppe et al. (*FEBS Letters* 344:191, (1994)) and in U.S. patent application Ser. No. 08/446,922, hereby incorporated by reference. Other peptides derived from naturally occurring trimeric proteins may be employed in preparing trimeric TR10.

In further preferred embodiments, TR10 polynucleotides of the invention are fused to a polynucleotide encoding a "FLAG" polypeptide. Thus, a TR10-FLAG fusion protein is encompassed by the present invention. The FLAG antigenic polypeptide may be fused to a TR10 polypeptide of the invention at either or both the amino or the carboxy terminus. In preferred embodiments, a TR10-FLAG fusion protein is expressed from a pFLAG-CMV-5a or a pFLAG-CMV-1 expression vector (available from Sigma, St. Louis, MO, USA). *See,* Andersson, S., *et al., J. Biol. Chem.* 264:8222-29 (1989); Thomsen, D. R., *et al., Proc. Natl. Acad. Sci. USA,* 81:659-63 (1984); and Kozak, M., *Nature* 308:241 (1984) (each of which is hereby incorporated by reference). In further preferred embodiments, a TR10-FLAG fusion protein is detectable by anti-FLAG monoclonal antibodies (also available from Sigma).

In another example, proteins of the invention are associated by interactions between FLAG polypeptide sequence contained in FLAG-TR10 fusion proteins of the invention. In a further embodiment, associated proteins of the invention are associated by interactions between heterologous polypeptide sequence contained in FLAG-TR10 fusion proteins of the invention and anti-FLAG antibody.

The multimers of the invention may be generated using chemical techniques known in the art. For example, proteins desired to be contained in the multimers of the invention may be chemically cross-linked using linker molecules and linker molecule length optimization techniques known in the art (see, e.g., US Patent Number 5,478,925, which is herein incorporated by reference in its entirety). Additionally, multimers of the invention may be generated using techniques known in the art to form one or more inter-molecule cross-links between the cysteine residues located within the polypeptide sequence of the proteins desired to be contained in the multimer (see, e.g., US Patent Number 5,478,925, which is herein incorporated by reference in its entirety). Further, proteins of the invention may be routinely modified by the addition of cysteine or biotin to the C terminus or N-terminus of the polypeptide sequence of the protein and techniques known in the art may be applied to generate multimers containing one or more of these modified proteins (see, e.g., US Patent Number 5,478,925, which is herein incorporated by reference in its entirety). Additionally, techniques known in the art may be applied to generate liposomes containing the protein components desired to be contained in the multimer of the invention (see, e.g., US Patent Number 5,478,925, which is herein incorporated by reference in its entirety).

Alternatively, multimers of the invention may be generated using genetic engineering techniques known in the art. In one embodiment, proteins contained in multimers of the invention are produced recombinantly using fusion protein technology described herein or otherwise known in the art (see, e.g., US Patent Number 5,478,925, which is herein incorporated by reference in its entirety). In a specific embodiment, polynucleotides coding for a homodimer of the invention are generated by ligating a polynucleotide sequence encoding a polypeptide of the invention to a sequence encoding a linker polypeptide and then further to a synthetic polynucleotide encoding the translated product of the polypeptide in the reverse orientation from the original C-terminus to the N-terminus (lacking the leader sequence) (see, e.g., US Patent Number 5,478,925, which is herein incorporated by reference in its entirety). In another embodiment, recombinant techniques described herein or otherwise known in the art are applied to generate recombinant polypeptides of the invention which contain a transmembrane domain and which can be incorporated by membrane reconstitution techniques into liposomes (see, e.g., US Patent Number 5,478,925, which is herein incorporated by reference in its entirety).

The polypeptides of the present invention are preferably provided in an isolated form. By "isolated polypeptide" is intended a polypeptide removed from its native environment. Thus, a polypeptide produced and/or contained within a recombinant host cell is considered isolated for purposes of the present invention. Also intended as an "isolated polypeptide" are polypeptides that have been purified, partially or substantially, from a recombinant host cell. For example, a recombinantly produced version of the TR 10 polypeptide can be substantially purified by the one-step method described in Smith and Johnson, *Gene 67*:31-40 (1988).

Accordingly, in one embodiment, the invention provides an isolated TR10 polypeptide having the amino acid sequence encoded by the deposited cDNA, or the amino acid sequence in SEQ ID NO:2, or a peptide or polypeptide comprising a portion of the above polypeptides.

Polypeptide fragments of the present invention include polypeptides comprising or alternatively, consisting of, an amino acid sequence contained in SEQ ID NO:2, encoded by the cDNA contained in the deposited clone, or encoded by nucleic acids which hybridize (e.g., under stringent hybridization conditions) to the nucleotide sequence contained in the deposited clone, or shown in Figures 1A-D (SEQ ID NO:1) or the complementary strand thereto. Protein fragments may be "free-standing," or comprised within a larger polypeptide of which the fragment forms a part or region, most preferably as a single continuous region. Representative examples of polypeptide fragments of the invention, include, for example, fragments that comprise or alternatively, consist of from about amino acid residues: -55 to -1, 1 to 50, 51 to 100, 101 to 157, 158 to 175, 176 to 226, 227 to 277, and/or 278 to 331 of SEQ ID NO:2. Moreover, polypeptide fragments can be at least 10, 20, 30, 40, 50, 60, 70, 80, 90, 100, 110, 120, 130, 140, 150, 175 or 200 amino acids in length. Polynucleotides encoding these polypeptides are also encompassed by the invention. In this context "about" includes the particularly recited ranges, larger or smaller by several (5, 4, 3, 2, or 1) amino acids, at either extreme or at both extremes.

In specific embodiments, polypeptide fragments of the invention comprise, or alternatively consist of, amino acid residues: -55 to -1,1 to 157, 158 to 175, 176 to 331, and/or 298 to 308 as depicted in SEQ ID NO:2. Polynucleotides encoding these polypeptides are also encompassed by the invention.

In additional embodiments, the polypeptide fragments of the invention comprise, or alternatively consist of, one or more TR10 domains. Preferred polypeptide fragments of the present invention include a member selected from the group: (a) a polypeptide comprising or alternatively, consisting of, the TR10 extracellular domain (predicted to constitute amino acid residues from about 56 to about 212 Figures 1A-D, or from about 1 to about 157 in SEQ ID NO:2); (b) a polypeptide comprising or alternatively, consisting of, the TR10 cysteine rich domain (predicted to constitute amino acid residues from about 81 to about 182 Figures 1A-D, or from about 26 to about 127 in SEQ ID NO:2); (c) a polypeptide comprising or alternatively, consisting of, the TR10 transmembrane domain (predicted to constitute amino acid residues from about 213 to about 230 Figures 1A-D, or from about 158 to about 175 SEQ ID NO:2); (d) a polypeptide comprising or alternatively, consisting of, the TR10 intracellular domain (predicted to constitute amino acid residues from about 231 to about 386 Figures 1 A-D, or from about 176 to about 331 in SEQ ID NO:2); (e) a polypeptide comprising or alternatively, consisting of, the TR10 partial death domain (predicted to constitute amino acid residues from about 353 to about 363 in Figures 1A-D, or from about 298 to about 308 in SEQ ID NO:2); (f) a polypeptide comprising, or alternatively, consisting of, one, two, three, four or more, epitope bearing portions of the TR10 receptor protein (g) any combination of polypeptides (a)-(f). Polynucleotides encoding these polypeptides are also encompassed by the invention.

As discussed above, it is believed that one or both of the extracellular cysteine rich motifs of TR10 is important for interactions between TR10 and its ligands (e.g., TRAIL). Accordingly, in preferred embodiments, polypeptide fragments of the invention comprise, or alternatively consist of amino acid residues 26 to 80, and/or 81 to 127 of SEQ ID NO:2 (which corresponds to amino acid residues 81 to 135 and/or 136 to 182 of Figures 1A-D). In a specific embodiment the polypeptides of the invention comprise, or alternatively consist of both of the extracellular cysteine rich motifs disclosed in Figures 1A-D.

Among the especially preferred fragments of the invention are fragments characterized by structural or functional attributes of TR10. Such fragments include amino acid residues that comprise alpha-helix and alpha-helix forming regions ("alpha-regions"), beta-sheet and beta-sheet-forming regions ("beta-regions"), turn and turn-forming regions ("turn-regions"), coil and coil-forming regions ("coil-regions"), hydrophilic regions, hydrophobic regions, alpha amphipathic regions, beta amphipathic regions, surface forming regions, and high antigenic index regions (i.e., containing four or more contiguous amino acids having an antigenic index of greater than or equal to 1.5, as identified using the default parameters of the Jameson-Wolf program) of complete (i.e., full-length) TR10 (SEQ ID NO:2). Certain preferred regions are those set out in Figure 3 and include, but are not limited to, regions of the aforementioned types identified by analysis of the amino acid sequence depicted in Figures 1A-D (SEQ ID NO:2), such preferred regions include; Garnier-Robson predicted alpha-regions, beta-regions, turn-regions, and coil-regions; Chou-Fasman predicted alpha-regions, beta-regions, and turn-regions; Kyte-Doolittle predicted hydrophilic and Hopp-Woods predicted hydrophobic regions; Eisenberg alpha and beta amphipathic regions; Emini surface-forming regions; and Jameson-Wolf high antigenic index regions, as predicted using the default parameters of these computer programs. Polynucleotides encoding these polypeptides are also encompassed by the invention.

As mentioned above, even if deletion of one or more amino acids from the N-terminus of a protein results in modification of loss of one or more biological functions of the protein, other functional activities (e.g., biological activities, ability to multimerize, ability to bind TR10 ligand) may still be retained. For example, the ability of shortened TR10 muteins to induce and/or bind to antibodies which recognize the complete or mature forms of the polypeptides generally will be retained when less than the majority of the residues of the complete or mature polypeptide are removed from the N-terminus. Whether a particular polypeptide lacking N-terminal residues of a complete polypeptide retains such immunologic activities can readily be determined by routine methods described herein and otherwise known in the art. It is not unlikely that an TR10 mutein with a large number of deleted N-terminal amino acid residues may retain some biological or immunogenic activities. In fact, peptides composed of as few as six TR10 amino acid residues may often evoke an immune response.

Accordingly, the present invention further provides polypeptides having one or more residues deleted from the amino terminus of the TR10 amino acid sequence shown in Figures 1A-D, up to the alanine residue at position number 382 and polynucleotides encoding such polypeptides. In particular, the present invention provides polypeptides comprising the amino acid sequence of residues n'-386 of Figures 1A-D, where n' is an integer from 2 to 381 corresponding to the position of the amino acid residue in Figures 1A-D (which is identical to the sequence shown as SEQ ID NO:2, with the exception that the amino acid residues in Figures 1A-D are numbered consecutively from 1 through 386 from the N-terminus to the C-terminus, while the amino acid residues in SEQ ID NO:2 are numbered consecutively from -55 through 331 to reflect the position of the predicted signal peptide).

More in particular, the invention provides polynucleotides encoding polypeptides comprising, or alternatively consisting of, the amino acid sequence of residues of G-2 to L-386; L-3 to L-386; W-4 to L-386; G-5 to L-386; Q-6 to L-386; S-7 to L-386; V-8 to L-386; P-9 to L-386; T-10 to L-386; A-11 to L-386; S-12 to L-386; S-13 to L-386; A-14 to L-386; R-15 to L-386; A-16 to L-386; G-17 to L-386; R-18 to L-386; Y-19 to L-386; P-20 to L-386; G-21 to L-386; A-22 to L-386; R-23 to L-386; T-24 to L-386; A-25 to L-386; S-26 to L-386; G-27 to L-386; T-28 to L-386; R-29 to L-386; P-30 to L-386; W-31 to L-386; L-32 to L-386; L-33 to L-386; D-34 to L-386; P-35 to L-386; K-36 to L-386; 1-37 to L-386; L-38 to L-386; K-39 to L-386; F-40 to L-386; V-41 to L-386; V-42 to L-386; F-43 to L-386; I-44 to L-386; V-45 to L-386; A-46 to L-386; V-47 to L-386; L-48 to L-386; L-49 to L-386; P-50 to L-386; V-51 to L-386; R-52 to L-386; V-53 to L-386; D-54 to L-386; S-55 to L-386; A-56 to L-386; T-57 to L-386; I-58 to L-386; P-59 to L-386; R-60 to L-386; Q-61 to L-386; D-62 to L-386; E-63 to L-386; V-64 to L-386; P-65 to L-386; Q-66 to L-386; Q-67 to L-386; T-68 to L-386; V-69 to L-386; A-70 to L-386; P-71 to L-386; Q-72 to L-386; Q-73 to L-386; Q-74 to L-386; R-75 to L-386; R-76 to L-386; S-77 to L-386; L-78 to L-386; K-79 to L-386; E-80 to L-386; E-81 to L-386; E-82 to L-386; C-83 to L-386; P-84 to L-386; A-85 to L-386; G-86 to L-386; S-87 to L-386; H-88 to L-386; R-89 to L-386; S-90 to L-386; E-91 to L-386; Y-92 to L-386; T-93 to L-386; G-94 to L-386: A-95 to L-386: C-96 to L-386: N-97 to L-386; P-98 to L-386; C-99 to L-386; T-100 to L-386: E-101 to L-386; G-102 to L-386; V -103 to L-386; D-104 to L-386; Y-105 to L-386; T-106 to L-386; I-107 to L-386; A-108 to L-386; S-109 to L-386; N-110 to L-386; N-111 to L-386; L-112 to L-386; P-113 to L-386; S-114 to L-386; C- 115 to L-386; L-116 to L-386; L-117 to L-386; C-118 to L-386; T-119 to L-386; V-120 to L-386; C-121 to L-386; K-122 to L-386; S-123 to L-386; G-124 to L-386; Q-125 to L-386; T-126 to L-386; N-127 to L-386; K-128 to L-386; S-129 to L-386; S-130 to L-386; C-131 to L-386; T-132 to L-386; T-133 to L-386; T-134 to L-386; R-135 to L-386; D-136 to L-386; T-137 to L-386; V-138 to L-386; C-139 to L-386; Q-140 to L-386; C- 141 to L-386; E-142 to L-386; K-143 to L-386; G-144 to L-386; S-145 to L-386; F-146 to L-386; Q-147 to L-386; D-148 to L-386; K-149 to L-386; N-150 to L-386; S-151 to L-386; P-152 to L-386; E-153 to L-386; M-154 to L-386; C-155 to L-386; R-156 to L-386; T-157 to L-386; C-158 to L-386; R-159 to L-386; T-160 to L-386; G-161 to L-386; C-162 to L-386; P-163 to L-386; R-164 to L-386; G-165 to L-386; M-166 to L-386; V-167 to L-386; K-168 to L-386; V-169 to L-386; S-170 to L-386; N-171 to L-386; C-172 to L-386; T-173 to L-386; P-174 to L-386; R-175 to L-386; S-176 to L-386; D-177 to L-386; I-178 to L-386; K-179 to L-386; C-180 to L-386; K-181 to L-386; N-182 to L-386; E-183 to L-386; S-184 to L-386; A-185 to L-386; A-186 to L-386; S-187 to L-386; S- 188 to L-386; T-189 to L-386; G-190 to L-386; K-191 to L-386; T-192 to L-386; P-193 to L-386; A-194 to L-386; A-195 to L-386; E-196 to L-386; E-197 to L-386; T-198 to L-386; V-199 to L-386; T-200 to L-386; T-201 to L-386; 1-202 to L-386; L-203 to L-386; G-204 to L-386; M-205 to L-386; L-206 to L-386; A-207 to L-386; S-208 to L-386; P-209 to L-386; Y-210 to L-386; H-211 to L-386; Y-212 to L-386; L-213 to L-386; 1-214 to L-386;I-215 to L-386; I-216 to L-386; V-217 to L-386; V-218 to L-386; L-219 to L-386; V-220 to L-386; I-221 to L-386;I-222 to L-386; L-223 to L-386; A-224 to L-386; V-225 to L-386; V-226 to L-386; V-227 to L-386; V-228 to L-386; G-229 to L-386; F-230 to L-386; S-231 to L-386; C-232 to L-386; R-233 to L-386; K-234 to L-386; K-235 to L-386; F-236 to L-386; 1-237 to L-386; S-238 to L-386; Y-239 to L-386; L-240 to L-386; K-241 to L-386; G-242 to L-386; 1-243 to L-386; C-244 to L-386; S-245 to L-386; G-246 to L-386; G-247 to L-386; G-248 to L-386; G-249 to L-386; G-250 to L-386; P-251 to L-386; E-252 to L-386; R-253 to L-386; V-254 to L-386; H-255 to L-386; R-256 to L-386; V-257 to L-386; L-258 to L-386; F-259 to L-386; R-260 to L-386; R-261 to L-386; R-262 to L-386; S-263 to L-386; C-264 to L-386; P-265 to L-386; S-266 to L-386; R-267 to L-386; V-268 to L-386; P-269 to L-386; G-270 to L-386; A-271 to L-386; E-272 to L-386; D-273 to L-386; N-274 to L-386; A-275 to L-386; R-276 to L-386; N-277 to L-386; E-278 to L-386; T-279 to L-386; L-280 to L-386; S-281 to L-386; N-282 to L-386; R-283 to L-386; Y-284 to L-386; L-285 to L-386; Q-286 to L-386; P-287 to L-386; T-288 to L-386; Q-289 to L-386; V-290 to L-386; S-291 to L-386; E-292 to L-386; Q-293 to L-386; E-294 to L-386; 1-295 to L-386; Q-296 to L-386;

G-297 to L-386; Q-298 to L-386; E-299 to L-386: L-300 to L-386; A-301 to L-386: E-302 to L-386; L-303 to L-386; T-304 to L-386; G-305 to L-386; V-306 to L-386; T-307 to L-386; V-308 to L-386; E-309 to L-386; S-310 to L-386; P-311 to L-386; E-312 to L-386: E-313 to L-386: P-314 to L-386; Q-315 to L-386: R-316 to L-386; L-317 to L-386; L-318 to L-386; E-319 to L-386; Q-320 to L-386; A-321 to L-386; E-322 to L-386; A-323 to L-386; E-324 to L-386: G-325 to L-386; C-326 to L-386; Q-327 to L-386; R-328 to L-386; R-329 to L-386; R-330 to L-386; L-331 to L-386; L-332 to L-386; V-333 to L-386; P-334 to L-386; V-335 to L-386; N-336 to L-386; D-337 to L-386; A-338 to L-386: D-339 to L-386; S-340 to L-386; A-341 to L-386; D-342 to L-386; 1-343 to L-386; S-344 to L-386; T-345 to L-386; L-346 to L-386; L-347 to L-386; D-348 to L-386; A-349 to L-386; S-350 to L-386; A-351 to L-386; T-352 to L-386: L-353 to L-386; E-354 to L-386; E-355 to L-386; G-356 to L-386; H-357 to L-386; A-358 to L-386; K-359 to L-386; E-360 to L-386; T-361 to L-386; 1-362 to L-386; Q-363 to L-386; D-364 to L-386; Q-365 to L-386; L-366 to L-386; V-367 to L-386; G-368 to L-386; S-369 to L-386; E-370 to L-386; K-371 to L-386; L-372 to L-386; F-373 to L-386; Y-374 to L-386; E-375 to L-386; E-376 to L-386; D-377 to L-386; E-378 to L-386; A-379 to L-386; G-380 to L-386; and S-381 to L-386 of the TR10 sequence shown in Figures 1A-D (which is identical to the sequence shown as SEQ ID NO:2, with the exception that the amino acid residues in Figures 1A-D are numbered consecutively from 1 through 386 from the N-terminus to the C-terminus, while the amino acid residues in SEQ ID NO:2 are numbered consecutively from -55 through 331 to reflect the position of the predicted signal peptide). Polypeptides encoded by these polynucleotides are also encompassed by the invention.

In another embodiment, N-terminal deletions of the TR10 polypeptide can be described by the general formula $n^2$-212, where $n^2$ is a number from 2 to 207, corresponding to the position of amino acid identified in Figures 1A-D (which is identical to the sequence shown as SEQ ID NO:2, with the exception that the amino acid residues in Figures 1A-D are numbered consecutively from 1 through 386 from the N-terminus to the C-terminus, while the amino acid residues in SEQ ID NO:2 are numbered consecutively from -55 through 331 to reflect the position of the predicted signal peptide). Preferably, N-terminal deletions of the TR10 polypeptide of the invention shown as SEQ ID NO:2 include polypeptides comprising, or alternatively consisting of, the amino acid sequence of residues: G-2 to Y-212; L-3 to Y-212; W-4 to Y-212; G-5 to Y-212; Q-6 to Y-212; S-7 to Y-212; V-8 to Y-212; P-9 to Y-212; T-10 to Y-212; A-11 to Y-212: S-12 to Y-212; S-13 to Y-212; A-14 to Y-212; R-15 to Y-212; A-16 to Y-212; G-17 to Y-212: R-18 to Y-212; Y-19 to Y-212; P-20 to Y-212; G-21 to Y-212; A-22 to Y-212; R-23 to Y-212; T-24 to Y-212; A-25 to Y-212; S-26 to Y-212; G-27 to Y-212; T-28 to Y-212; R-29 to Y-212; P-30 to Y-212; W-31 to Y-212; L-32 to Y-212; L-33 to Y-212; D-34 to Y-212; P-35 to Y-212; K-36 to Y-212; 1-37 to Y-212; L-38 to Y-212; K-39 to Y-212; F-40 to Y-212; V-41 to Y-212; V-42 to Y-212; F-43 to Y-212; I-44 to Y-212; V-45 to Y-212; A-46 to Y-212; V-47 to Y-212; L-48 to Y-212: L-49 to Y-212; P-50 to Y-212: V-51 to Y-212; R-52 to Y-212; V-53 to Y-212; D-54 to Y-212; S-55 to Y-212; A-56 to Y-212; T-57 to Y-212; 1-58 to Y-212; P-59 to Y-212; R-60 to Y-212; Q-61 to Y-212; D-62 to Y-212; E-63 to Y-212; V-64 to Y-212; P-65 to Y-212; Q-66 to Y-212; Q-67 to Y-212; T-68 to Y-212; V-69 to Y-212; A-70 to Y-212; P-71 to Y-212; Q-72 to Y-212; Q-73 to Y-212; Q-74 to Y-212; R-75 to Y-212; R-76 to Y-212; S-77 to Y-212; L-78 to Y-212; K-79 to Y-212; E-80 to Y-212; E-81 to Y-212; E-82 to Y-212; C-83 to Y-212; P-84 to Y-212; A-85 to Y-212; G-86 to Y-212; S-87 to Y-212; H-88 to Y-212; R-89 to Y-212; S-90 to Y-212; E-91 to Y-212; Y-92 to Y-212; T-93 to Y-212; G-94 to Y-212; A-95 to Y-212; C-96 to Y-212; N-97 to Y-212; P-98 to Y-212; C-99 to Y-212; T-100 to Y-212; E-101 to Y-212; G-102 to Y-212; V-103 to Y-212; D-104 to Y-212; Y-105 to Y-212; T-106 to Y-212; 1-107 to Y-212; A-108 to Y-212; S-109 to Y-212; N-110 to Y-212; N-111 to Y-212; L-112 to Y-212: P-113 to Y-212; S-114 to Y-212; C-115 to Y-212; L-116 to Y-212; L-117 to Y-212; C-118 to Y-212; T-119 to Y-212; V-120 to Y-212; C-121 to Y-212; K-122 to Y-212; S-123 to Y-212; G-124 to Y-212; Q-125 to Y-212; T-126 to Y-212; N-127 to Y-212; K-128 to Y-212; S-129 to Y-212; S-130 to Y-212; C-131 to Y-212; T-132 to Y-212; T-133 to Y-212; T-134 to Y-212; R-135 to Y-212; D-136 to Y-212; T-137 to Y-212; V-138 to Y-212; C-139 to Y-212; Q-140 to Y-212; C-141 to Y-212; E-142 to Y-212; K-143 to Y-212; G-144 to Y-212; S-145 to Y-212; F-146 to Y-212; Q-147 to Y-212; D-148 to Y-212; K-149 to Y-212; N-150 to Y-212; S-151 to Y-212; P-152 to Y-212; E-153 to Y-212; M-154 to Y-212; C-155 to Y-212; R-156 to Y-212; T-157 to Y-212; C-158 to Y-212; R-159 to Y-212; T-160 to Y-212; G-161 to Y-212; C-162 to Y-212; P-163 to Y-212; R-164 to Y-212; G-165 to Y-212; M-166 to Y-212; V-167 to Y-212; K-168 to Y-212; V-169 to Y-212; S-170 to Y-212; N-171 to Y-212; C-172 to Y-212; T-173 to Y-212; P-174 to Y-212; R-175 to Y-212; S-176 to Y-212; D-177 to Y-212;I-178 to Y-212; K-179 to Y-212; C-180 to Y-212; K-181 to Y-212; N-182 to Y-212; E-183 to Y-212; S-184 to Y-212; A-185 to Y-212; A-186 to Y-212; S-187 to Y-212; S-188 to Y-212; T-189 to Y-212; G-190 to Y-212; K-191 to Y-212; T-192 to Y-212; P-193 to Y-212; A-194 to Y-212; A-195 to Y-212; E-196 to Y-212; E-197 to Y-212; T-198 to Y-212; V-199 to Y-212; T-200 to Y-212; T-201 to Y-212; I-202 to Y-212; L-203 to Y-212; G-204 to Y-212; M-205 to Y-212; L-206 to Y-2I2; and A-207 to Y-212 of the TR 10 extracellular domain sequence shown in Figures 1A-D. The invention is also directed to nucleic acid molecules comprising or, alternatively, consisting of a nucleotide sequence at least 80%, 85%, 90%, 92%, 95%, 96%, 97%, 98%, or 99% identical to the polynucleotide sequences encoding the TR10 polypeptides described above. The invention also encompasses these nucleotide sequences fused to a heterologous nucleotide sequence. Polypeptides encoded by these polynucleotides are also encompassed by the invention.

Also as mentioned above, even if deletion of one or more amino acids from the C-terminus of a protein results in modification of loss of one or more biological functions of the protein, other functional activities (e.g., biological activities (e.g., ability to inhibit TRAIL induced cell death *in vivo* or *in vitro,* and/or regulate (e.g., inhibit) B cell proliferation, and/or regulate hematopoiesis), ability to multimerize, ability to bind TR10 ligand (e.g., TRAIL, and/or ligands on the surface of NK cells and/or endothelial cells) may still be retained. For example the ability of the shortened TR10 mutein to induce and/or bind to antibodies which recognize the complete or mature forms of the polypeptide generally will be retained when less than the majority of the residues of the complete or mature polypeptide are removed from the C-terminus. Whether a particular polypeptide tacking C-terminal residues of a complete polypeptide retains such immunologic activities can readily be determined by routine methods described herein and otherwise known in the art. It is not unlikely that an TR10 mutein with a large number of deleted C-terminal amino acid residues may retain some biological or immunogenic activities. In fact, peptides composed of as few as six TR10 amino acid residues may often evoke an immune response.

Accordingly, the present invention further provides polypeptides having one or more residues deleted from the carboxy terminus of the amino acid sequence of the TR10 polypeptide shown in Figures 1A-D, up to the glutamine residue at position number 61, and polynucleotides encoding such polypeptides. In particular, the present invention provides polypeptides comprising the amino acid sequence of residues 56-m' of Figures 1A-D, where m' is an integer from 61 to 385 corresponding to the position of the amino acid residue in Figures 1A-D (which is identical to the sequence shown as SEQ ID NO:2, with the exception that the amino acid residues in Figures 1A-D are numbered consecutively from 1 through 386 from the N-terminus to the C-terminus, while the amino acid residues in SEQ ID NO:2 are numbered consecutively from -55 through 331 to reflect the position of the predicted signal peptide).

More in particular, the invention provides polynucleotides encoding polypeptides comprising, or alternatively consisting of, the amino acid sequence of residues A-56 to C-385; A-56 to S-384; A-56 to T-383; A-56 to A-382; A-56 to S-381; A-56 to G-380; A-56 to A-379; A-56 to E-378; A-56 to D-377; A-56 to E-376; A-56 to E-375; A-56 to Y-374; A-56 to F-373; A-56 to L-372; A-56 to K-371; A-56 to E-370; A-56 to S-369; A-56 to G-368; A-56 to V-367; A-56 to L-366; A-56 to Q-365; A-56 to D-364; A-56 to Q-363; A-56 to 1-362; A-56 to T-361; A-56 to E-360; A-56 to K-359; A-56 to A-358; A-56 to H-357; A-56 to G-356; A-56 to E-355; A-56 to E-354; A-56 to L-353; A-56 to T-352; A-56 to A-35 1: A-56 to S-350; A-56 to A-349; A-56 to D-348; A-56 to L-347; A-56 to L-346; A-56 to T-345; A-56 to S-344; A-56 to 1-343; A-56 to D-342; A-56 to A-341; A-56 to S-340; A-56 to D-339; A-56 to A-338; A-56 to D-337; A-56 to N-336; A-56 to V-335; A-56 to P-334; A-56 to V-333; A-56 to L-332; A-56 to L-331; A-56 to R-330; A-56 to R-329; A-56 to R-328; A-56 to Q-327; A-56 to C-326; A-56 to G-325; A-56 to E-324; A-56 to A-323; A-56 to E-322; A-56 to A-321; A-56 to Q-320; A-56 to E-319; A-56 to L-318; A-56 to L-317; A-56 to R-316; A-56 to Q-315; A-56 to P-314; A-56 to E-313; A-56 to E-312; A-56 to P-311; A-56 to S-310; A-56 to E-309; A-56 to V-308; A-56 to T-307; A-56 to V-306; A-56 to G-305; A-56 to T-304; A-56 to L-303; A-56 to E-302; A-56 to A-301; A-56 to L-300; A-56 to E-299; A-56 to Q-298; A-56 to G-297; A-56 to Q-296; A-56 to I-295; A-56 to E-294; A-56 to Q-293; A-56 to E-292; A-56 to S-291; A-56 to V-290; A-56 to Q-289; A-56 to T-288; A-56 to P-287; A-56 to Q-286; A-56 to L-285; A-56 to Y-284; A-56 to R-283; A-56 to N-282: A-56 to S-281; A-56 to L-280; A-56 to T-279; A-56 to E-278; A-56 to N-277; A-56 to R-276; A-56 to A-275; A-56 to N-274; A-56 to D-273; A-56 to E-272; A-56 to A-271; A-56 to G-270; A-56 to P-269; A-56 to V-268; A-56 to R-267; A-56 to S-266; A-56 to P-265; A-56 to C-264; A-56 to S-263; A-56 to R-262; A-56 to R-261; A-56 to R-260; A-56 to F-259; A-56 to L-258; A-56 to V-257; A-56 to R-256; A-56 to H-255; A-56 to V-254; A-56 to R-253; A-56 to E-252; A-56 to P-251; A-56 to G-250; A-56 to G-249; A-56 to G-248; A-56 to G-247; A-56 to G-246; A-56 to S-245; A-56 to C-244; A-56 to I-243; A-56 to G-242; A-56 to K-241; A-56 to L-240; A-56 to Y-239; A-56 to S-238; A-56 to I-237; A-56 to F-236; A-56 to K-235; A-56 to K-234; A-56 to R-233; A-56 to C-232; A-56 to S-231; A-56 to F-230; A-56 to G-229; A-56 to V-228; A-56 to V-227; A-56 to V-226; A-56 to V-225; A-56 to A-224; A-56 to L-223; A-56 to 1-222; A-56 to I-221; A-56 to V-220; A-56 to L-219; A-56 to V-218; A-56 to V-217; A-56 to I-216; A-56 to I-215; A-56 to I-214; A-56 to L-213; A-56 to Y-212; A-56 to H-211; A-56 to Y-210; A-56 to P-209; A-56 to S-208; A-56 to A-207; A-56 to L-206; A-56 to M-205; A-56 to G-204; A-56 to L-203; A-56 to 1-202; A-56 to T-201; A-56 to T-200; A-56 to V-199; A-56 to T-198; A-56 to E-197; A-56 to E-196; A-56 to A-195; A-56 to A-194; A-56 to P-193; A-56 to T-192; A-56 to K-191; A-56 to G-190; A-56 to T-189; A-56 to S-188; A-56 to S-187; A-56 to A-186; A-56 to A-185; A-56 to S-184; A-56 to E-183; A-56 to N-182; A-56 to K-181; A-56 to C-180; A-56 to K-179; A-56 to 1-178; A-56 to D-177; A-56 to S-176; A-56 to R-175; A-56 to P-174; A-56 to T-173; A-56 to C-172; A-56 to N-171; A-56 to S-170; A-56 to V-169; A-56 to K-168; A-56 to V-167: A-56 to M-166; A-56 to G-165; A-56 to R-164; A-56 to P-163; A-56 to C-162; A-56 to G-161; A-56 to T-160; A-56 to R-159; A-56 to C-158; A-56 to T-157; A-56 to R-156; A-56 to C-155; A-56 to M-154; A-56 to E-153; A-56 to P-152; A-56 to S-151; A-56 to N-150; A-56 to K-149; A-56 to D-148; A-56 to Q-147; A-56 to F-146; A-56 to S-145; A-56 to G-144; A-56 to K-143 ; A-56 to E-142; A-56 to C-141; A-56 to Q-140; A-56 to C-139; A-56 to V-138; A-56 to T-137; A-56 to D-136; A-56 to R-135; A-56 to T-134; A-56 to T-133; A-56 to T-132; A-56 to C-131: A-56 to S-130; A-56 to S-129: A-56 to K-128; A-56 to N-127; A-56 to T-126; A-56 to Q-125; A-56 to G-124; A-56 to S-123: A-56 to K-122; A-56 to C-121-, A-56 to V-120; A-56 to T-119; A-56 to C-118; A-56 to L-117; A-56 to L-116; A-56 to C-115; A-56 to S-114; A-56 to P-113 ; A-56 to L-112; A-56 to N-111; A-56 to N- 110; A-56 to S-109; A-56 to A-108; A-56 to 1-107; A-56 to T-106; A-56 to Y-105; A-56 to D-104; A-56 to V-103; A-56 to G-102; A-56 to E-101; A-56 to T-100; A-56 to C-99; A-56 to P-98: A-56 to N-97; A-56 to C-96; A-56 to A-95; A-56 to G-94: A-56 to T-93; A-56 to Y-92; A-56 to E-91; A-56 to S-90; A-56 to R-89; A-56 to H-88; A-56 to S-87; A-56 to G-86: A-56 to A-85: A-56 to P-84; A-56 to C-83; A-56 to E-82; A-56 to E-81; A-56 to E-80; A-56 to K-79; A-56 to L-78; A-56 to S-77; A-56 to R-76; A-56 to R-75; A-56 to Q-74; A-56 to Q-73; A-56 to Q-72: A-56 to P-71; A-56 to A-70; A-56 to V-69; A-56 to T-68: A-56 to Q-67; A-56 to Q-66; A-56 to P-65; A-56 to V-64; A-56 to E-63; A-56 to D-62; and A-56 to Q-61 of the TR10 sequence shown in Figures 1A-D (which is identical to the sequence shown as SEQ ID NO:2, with the exception that the amino acid residues in Figures 1A-D are numbered consecutively from 1 through 386 from the N-terminus to the C-terminus, while the amino acid residues in SEQ ID NO:2 are numbered consecutively from -55 through 331 to reflect the position of the predicted signal peptide). Polypeptides encoded by these polynucleotides are also encompassed by the invention.

The invention also provides polypeptides having one or more amino acids deleted from both the amino and the carboxyl termini, which may be described generally as having residues $n^1$- $m^1$ and/or $n^2$- $m^1$ of Figures 1A-D (i.e.. SEQ ID NO:2), where $n^1$, $n^2$, and $m^1$ are integers as described above. Thus, any of the above listed N- or C-terminal deletions can be combined to produce a N- and C-terminal deleted TR10 polypeptide.

It will be recognized in the art that some amino acid sequences of TR10 can be varied without significant effect on the structure or function of the protein. If such differences in sequence are contemplated, it should be remembered that there will be critical areas on the protein which determine activity. Thus, the invention further includes variations of the TR10 receptor, which show substantial TR10 receptor activity or which include regions of TR10 proteins, such as the protein portions discussed herein. Such mutants include deletions, insertions, inversions, repeats, and type substitutions. As indicated above, guidance concerning which amino acid changes are likely to be phenotypically silent can be found in J.U. Bowie *et al., Science 247*:1306-1310 (1990).

Thus, the fragment, derivative, or analog of the polypeptide of SEQ ID NO:2, or that encoded by the deposited cDNA, may be (i) one in which at least one or more of the amino acid residues are substituted with a conserved or non-conserved amino acid residue (preferably a conserved amino acid residue(s), and more preferably at least one but less than ten conserved amino acid residues) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethylene glycol), or (iv) one in which the additional amino acids are fused to the mature polypeptide, such as an IgG Fc fusion region peptide or leader or secretory sequence or a sequence which is employed for purification of the mature polypeptide or a proprotein sequence. Such fragments, derivatives and analogs are deemed to be within the scope of those skilled in the art from the teachings herein.

Of particular interest are substitutions of charged amino acids with another charged amino acid and with neutral or negatively charged amino acids. The latter results in proteins with reduced positive charge to improve the characteristics of the TR10 receptor protein. The prevention of aggregation is highly desirable. Aggregation of proteins not only results in a loss of activity but can also be problematic when preparing pharmaceutical formulations, because they can be immunogenic. (Pinckard *et al., Clin Exp. Immunol. 2*:331-340 (1967); Robbins *et al., Diabetes 36*:838-845 (1987); Cleland *et al. Crit. Rev. Therapeutic Drug Carrier Systems 10*:307-377 (1993)).

The replacement of amino acids can also change the selectivity of binding to cell surface receptors. *Ostade et al., Nature 361*:266-268 (1993), describes certain mutations resulting in selective binding of TNF-α to only one of the two known types of TNF receptors. Thus, the TR10 receptor of the present invention may include one or more amino acid substitutions, deletions, or additions, either from natural mutations or human manipulation.

As indicated, changes are preferably of a minor nature, such as conservative amino acid substitutions that do not significantly affect the folding or activity of the protein (see Table II).

**TABLE II. Conservative Amino Acid Substitutions**

| | |
|---|---|
| Aromatic | Phenylalanine<br>Tryptophan<br>Tyrosine |
| Hydrophobic | Leucine<br>Isoleucine<br>Valine |
| Polar | Glutamine<br>Asparagine |

Table continued

| | |
|---|---|
| Basic | Arginine<br>Lysine<br>Histidine |
| Acidic | Aspartic Acid<br>Glutamic Acid |
| Small | Alanine<br>Serine<br>Threonine<br>Methionine<br>Glycine |

In specific embodiments, the number of substitutions, additions or deletions in the amino acid sequence of Figures 1A-D and/or any of the polypeptide fragments described herein (e.g., the extracellular domain or intracellular domain) is 75, 70, 60, 50, 40, 35, 30, 25, 20, 15, 10, 9, 8, 7, 6, 5, 4, 3, 2, 1 or 30-20, 20-15, 20-10, 15-10, 10-1, 5-10, 1-5, 1-3 or 1-2.

Amino acids in the TR 10 protein of the present invention that are essential for function can be identified by methods known in the art, such as site-directed mutagenesis or alanine-scanning mutagenesis (Cunningham and Wells, *Science 244*:1081-1085 (1989). The latter procedure introduces single alanine mutations at every residue in the molecule. The resulting mutant molecules are then tested for biological activity such as receptor binding or *in vitro* proliferative activity. Sites that are critical for ligand-receptor binding can also be determined by structural analysis such as crystallization, nuclear magnetic resonance or photoaffinity labeling *(Smith et al., J. Mol. Biol. 224*:899-904 (1992) and de Vos *et al. Science 255:*306-312 (1992)).

To improve or alter the characteristics of TR10 polypeptides, protein engineering may be employed. Recombinant DNA technology known to those skilled in the art can be used to create novel mutant proteins or "muteins including single or multiple amino acid substitutions, deletions, additions or fusion proteins. Such modified polypeptides can show, e.g., enhanced activity or increased stability. In addition, they may be purified in higher yields and show better solubility than the corresponding natural polypeptide, at least under certain purification and storage conditions.

Non-naturally occurring variants may be produced using art-known mutagenesis techniques, which include, but are not limited to oligonucleotide mediated mutagenesis, alanine scanning, PCR mutagenesis, site directed mutagenesis (see e.g., Carter *et al., Nucl. Acids Res. 13*:4331 (1986); and Zoller *et al., Nucl. Acids Res. 10*:6487 (1982)), cassette mutagenesis (see e.g., Wells *et al., Gene 34*:315 (1985)), restriction selection mutagenesis (see e.g., Wells *et al., Philos. Trans. R. Soc. London SerA 317*:415 (1986)).

Thus, the invention also encompasses TR10 derivatives and analogs that have one or more amino acid residues deleted, added, or substituted to generate TR10 polypeptides that are better suited for expression, scale up, etc., in the host cells chosen. For example, cysteine residues can be deleted or substituted with another amino acid residue in order to eliminate disulfide bridges; N-linked glycosylation sites can be altered or eliminated to achieve, for example, expression of a homogeneous product that is more easily recovered and purified from yeast hosts which are known to hyperglycosylate N-linked sites. To this end, a variety of amino acid substitutions at one or both of the first or third amino acid positions on any one or more of the glycosylation recognitions sequences in the TR10 polypeptides of the invention, and/or an amino acid deletion at the second position of any one or more such recognition sequences will prevent glycosylation of the TR10 at the modified tripeptide sequence (see, e.g., Miyajimo *et al., EMBO J 5*(6):1193-1197). Additionally, one or more of the amino acid residues of the polypeptides of the invention (e.g., arginine and lysine residues) may be deleted or substituted with another residue to eliminate undesired processing by proteases such as, for example, furins or kexins.

The polypeptides of the present invention include a polypeptide comprising, or alternatively, consisting of the polypeptide encoded by the deposited cDNA including the leader; a polypeptide comprising, or alternatively, consisting of the mature polypeptide encoded by the deposited cDNA minus the leader (i.e., the mature protein); a polypeptide comprising, or alternatively, consisting of amino acids from about - 55 to about 331 in SEQ ID NO:2; a polypeptide comprising, or alternatively, consisting of amino acids from about - 54 to about 331 in SEQ ID NO:2; a polypeptide comprising, or alternatively, consisting of amino acids from about 1 to about 33 in SEQ ID NO:2; a polypeptide comprising, or alternatively, consisting of the extracellular domain; a polypeptide comprising, or alternatively, consisting of the cysteine rich domain; a polypeptide comprising, or alternatively, consisting of the transmembrane domain; a polypeptide comprising, or alternatively, consisting of the intracellular domain; a polypeptide comprising, or alternatively, consisting of the extracellular and intracellular domains with all or part of the transmembrane domain deleted: and a polypeptide comprising, or alternatively, consisting of the partial death domain: as well as polypeptides which are at least 80% identical, more preferably at least 80%, 85%, 90%, or 95% identical, still more preferably at least 96%, 97%, 98%, or 99% identical to the polypeptides described above (e.g., the polypeptide encoded by the deposited cDNA clone, the polypeptide of Figures 1A-D (SEQ ID NO:2)), and also include portions of such polypeptides with at least 30 amino acids and more preferably at least 50 amino acids. Polynucleotides encoding these polypeptides are also encompassed by the invention.

By a polypeptide having an amino acid sequence at least, for example. 95% "identical" to a reference amino acid sequence of a TR10 polypeptide is intended that the amino acid sequence of the polypeptide is identical to the reference sequence except that the polypeptide sequence may include up to five amino acid alterations per each 100 amino acids of the reference amino acid of the TR10 receptor. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 80%, 85%, 90%, 95%, 96%, 97%, 98%, or 99% identical to, for instance, the amino acid sequence shown in SEQ ID NO:2, or to the amino acid sequence encoded by the deposited cDNA clone, can be determined conventionally using known computer programs such the Bestfit program (Wisconsin Sequence Analysis Package, Version 8 for Unix, Genetics Computer Group, University Research Park, 575 Science Drive, Madison, WI 53711). When using Bestfit or any other sequence alignment program to determine whether a particular sequence is, for instance, 95% identical to a reference sequence according to the present invention, the parameters are set, of course, such that the percentage of identity is calculated over the full length of the reference amino acid sequence and that gaps in homology of up to 5% of the total number of amino acid residues in the reference sequence are allowed.

In a specific embodiment, the identity between a reference (query) sequence (a sequence of the present invention) and a subject sequence, also referred to as a global sequence alignment, is determined using the FASTDB computer program based on the algorithm of Brutlag et al. *(Comp. App. Biosci.* 6:237-245 (1990)). Preferred parameters used in a FASTDB amino acid alignment are: Matrix=PAM 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter. According to this embodiment. if the subject sequence is shorter than the query sequence due to N- or C-terminal deletions, not because of internal deletions, a manual correction is made to the results to take into consideration the fact that the FASTDB program does not account for N- and C-terminal truncations of the subject sequence when calculating global percent identity. For subject sequences truncated at the N-and C-termini, relative to the query sequence, the percent identity is corrected by calculating the number of residues of the query sequence that are N- and C-terminal of the subject sequence, which are not matched/aligned with a corresponding subject residue, as a percent of the total bases of the query sequence. A determination of whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of this embodiment. Only residues to the N- and C-termini of the subject sequence, which are not matched/ aligned with the query sequence, are considered for the purposes of manually adjusting the percent identity score. That is, only query residue positions outside the farthest N- and C-terminal residues of the subject sequence. For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C- termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequence are manually corrected for. No other manual corrections are made for the purposes of this embodiment.

In additional embodiments, polynucleotides of the invention comprise, or alternatively consist of, a polynucleotide sequence at least 80%, 85%,90%, 95%, 96%, 97%, 98% or 99% identical to the polynucleotide sequence encoding one or both of the extracellular cysteine rich motifs of TR10 disclosed in Figures 1A-D (amino acid residues 26 to 80, and/or 81 to 127 of SEQ ID NO:2; corresponding to amino acid residues 81 to 135, and/or 136 to 182 of Figures 1A-D)). In another embodiment, the invention provides an isolated nucleic acid molecule comprising a polynucleotide which hybridizes under stringent hybridization conditions to one or both of the TR10 extracellular cysteine rich motifs. The present invention also encompasses the above polynucleotide/nucleic acid sequences fused to a heterologous polynucleotide sequence. Polypeptides encoded by these nucleic acids and/or polynucleotide sequences are also encompassed by the invention.

The present application is also directed to proteins cotaining polypeptides at least 80%, 85%, 90%, 95%, 96%, 97%, 98% or 99% identical to the TR10 polypeptide sequence set forth as $n^1$-$m^1$, and/or $n^2$- $m^1$ herein. In preferred embodiments, the application is directed to proteins containing polypeptides at least 80%, 85%, 90%, 95%, 96%, 97%, 98% or 99% identical to polypeptides having the amino acid sequence of the specific TR10 N- and C-terminal deletions recited herein. Polynucleotides encoding these polypeptides are also encompassed by the invention.

In certain preferred embodiments, TR10 proteins of the invention comprise fusion proteins as described above wherein the TR10 polypeptides are those described as n'-m', and/or $n^2$- $m^1$ herein. In preferred embodiments, the application is directed to nucleic acid molecules at least 80%, 85%, 90%, 95%, 96%, 97%, 98% or 99% identical to the nucleic acid sequences encoding polypeptides having the amino acid sequence of the specific N- and C-terminal deletions recited herein. Polynucleotides encoding these polypeptides are also encompassed by the invention.

Polypeptides of the present invention include naturally purified products, products of chemical synthetic procedures, and products produced by recombinant techniques from a prokaryotic or eukaryotic host, including, for example, bacterial, yeast, higher plant, insect and mammalian cells using, for example, the recombinant compositions and methods described above. Depending upon the host employed in a recombinant production procedure, the polypeptides of the present invention may be glycosylated or non-glycosylated. In addition, polypeptides of the invention may also include an initial modified methionine residue, in some cases as a result of host-mediated processes.

In addition, proteins of the invention can be chemically synthesized using techniques known in the art (e.g., see Creighton, Proteins: Structures and Molecular Principles, W.H. Freeman & Co., N.Y. (1983), and Hunkapiller, *et al., Nature 310:*105-111 (1984)). For example, a peptide corresponding to a fragment of the TR10 polypeptides of the invention can be synthesized by use of a peptide synthesizer. Furthermore, if desired, nonclassical amino acids or chemical amino acid analogs can be introduced as a substitution or addition into the TR10 polypeptide sequence. Non-classical amino acids include, but are not limited to, to the D-isomers of the common amino acids, 2,4-diaminobutyric acid, a-amino isobutyric acid, 4-aminobutyric acid, Abu, 2-amino butyric acid, g-Abu, e-Ahx, 6-amino hexanoic acid, Aib, 2-amino isobutyric acid, 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, b-alanine, fluoro-amino acids, designer amino acids such as b-methyl amino acids, Ca-methyl amino acids, Na-methyl amino acids, and amino acid analogs in general. Furthermore, the amino acid can be D (dextrorotary) or L (levorotary).

The invention additionally, encompasses TR10 polypeptides which are differentially modified during or after translation, e.g., by glycosylation, acetylation, phosphorylation, amidation, derivatization by known protecting/blocking groups, proteolytic cleavage, linkage to an antibody molecule or other cellular ligand, etc. Any of numerous chemical modifications may be carried out by known techniques, including but not limited to, specific chemical cleavage by cyanogen bromide, trypsin, chymotrypsin, papain, V8 protease, $NaBH_{4.}$ acetylation, formylation, oxidation, reduction, metabolic synthesis in the presence of tunicamycin; etc.

Additional post-translational modifications encompassed by the invention include, for example, e.g., N-linked or 0-linked carbohydrate chains, processing of N-terminal or C-terminal ends), attachment of chemical moieties to the amino acid backbone, chemical modifications of N-linked or O-linked carbohydrate chains, and addition or deletion of an N-terminal methionine residue as a result of procaryotic host cell expression. The polypeptides may also be modified with a detectable label, such as an enzymatic, fluorescent, isotopic or affinity label to allow for detection and isolation of the protein.

Also provided by the invention are chemically modified derivatives of TR10 which may provide additional advantages such as increased solubility, stability and circulating time of the polypeptide, or decreased immunogenicity (see U. S. Patent No. 4,179,337). The chemical moieties for derivitization may be selected from water soluble polymers such as polyethylene glycol, ethylene glycol/propylene glycol copolymers, carboxymethylcellulose, dextran, polyvinyl alcohol and the like. The polypeptides may be modified at random positions within the molecule, or at predetermined positions within the molecule and may include one, two, three or more attached chemical moieties.

The polymer may be of any molecular weight, and may be branched or unbranched. For polyethylene glycol, the preferred molecular weight is between about 1 kDa and about 100 kDa (the term "about" indicating that in preparations of polyethylene glycol, some molecules will weigh more, some less, than the stated molecular weight) for ease in handling and manufacturing. Other sizes may be used. depending on the desired therapeutic profile (e.g., the duration of sustained release desired, the effects, if any on biological activity, the ease in handling, the degree or lack of antigenicity and other known effects of the polyethylene glycol to a therapeutic protein or analog). For example, the polyethylene glycol may have an average molecular weight of about 200, 500, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 15,500. 16,000, 16,500, 17,000, 17,500, 18,000, 18,500, 19,000, 19,500, 20,000, 25,000, 30,000, 35,000, 40,000, 50,000, 55,000, 60,000, 65,000, 70,000, 75,000, 80,000, 85.000, 90,000, 95,000, or 100,000 kDa.

As noted above, the polyethylene glycol may have a branched structure. Branched polyethylene glycols are described, for example, in U.S. Patent No. 5,643,575; Morpurgo *et al., Appl. Biochem. Biotechnol. 56*:59-72 (1996); Vorobjev *et al., Nucleosides Nucleotides 18*:2745-2750 (1999); and Caliceti *et al., Bioconjug. Chem. 10*:638-646 (1999), the disclosures of each of which are incorporated herein by reference.

The polyethylene glycol molecules (or other chemical moieties) should be attached to the protein with consideration of effects on functional or antigenic domains of the protein. There are a number of attachment methods available to those skilled in the art, *e.g.*, EP 0 401 384, herein incorporated by reference (coupling PEG to G-CSF), see also Malik *et al., Exp. Hematol. 20*:1028-1035 (1992) (reporting pegylation of GM-CSF using tresyl chloride). For example, polyethylene glycol may be covalently bound through amino acid residues via a reactive group, such as, a free amino or carboxyl group. Reactive groups are those to which an activated polyethylene glycol molecule may be bound. The amino acid residues having a free amino group may include lysine residues and the N-terminal amino acid residues; those having a free carboxyl group may include aspartic acid residues, glutamic acid residues and the C-terminal amino acid residue. Sulfhydryl groups may also be used as a reactive group for attaching the polyethylene glycol molecules. Preferred for therapeutic purposes is attachment at an amino group, such as attachment at the N-terminus or lysine group.

As suggested above, polyethylene glycol may be attached to proteins via linkage to any of a number of amino acid residues. For example, polyethylene glycol can be linked to a proteins via covalent bonds to lysine, histidine, aspartic acid, glutamic acid, or cysteine residues. One or more reaction chemistries may be employed to attach polyethylene glycol to specific amino acid residues (e.g., lysine, histidine, aspartic acid, glutamic acid, or cysteine) of the protein or to more than one type of amino acid residue (e.g., lysine, histidine, aspartic acid, glutamic acid, cysteine and combinations thereof) of the protein.

One may specifically desire proteins chemically modified at the N-terminus. Using polyethylene glycol as an illustration of the present composition, one may select from a variety of polyethylene glycol molecules (by molecular weight, branching, etc.), the proportion of polyethylene glycol molecules to protein (or peptide) molecules in the reaction mix, the type of pegylation reaction to be performed, and the method of obtaining the selected N-terminally pegylated protein. The method of obtaining the N-terminally pegylated preparation *(i.e.,* separating this moiety from other monopegylated moieties if necessary) may be by purification of the N-terminally pegylated material from a population of pegylated protein molecules. Selective proteins chemically modified at the N-terminus modification may be accomplished by reductive alkylation which exploits differential reactivity of different types of primary amino groups (lysine versus the N-terminal) available for derivatization in a particular protein. Under the appropriate reaction conditions, substantially selective derivatization of the protein at the N-terminus with a carbonyl group containing polymer is achieved.

As indicated above, pegylation of the proteins of the invention may be accomplished by any number of means. For example, polyethylene glycol may be attached to the protein either directly or by an intervening linker. Linkerless systems for attaching polyethylene glycol to proteins are described in Delgado *et al., Crit. Rev. Thera. Drug Carrier Sys. 9*:249-304 (1992); Francis *et al., Intern. J. of Hematol. 68*:1-18 (1998); U.S. Patent No. 4,002,531; U.S. Patent No. 5,349,052; WO 95/06058: and WO 98/32466, the disclosures of each of which are incorporated herein by reference.

One system for attaching polyethylene glycol directly to amino acid residues of proteins without an intervening linker employs tresylated MPEG, which is produced by the modification of monmethoxy polyethylene glycol (MPEG) using tresylchloride ($ClSO_2CH_2CF_3$). Upon reaction of protein with tresylated MPEG, polyethylene glycol is directly attached to amine groups of the protein. Thus, the invention includes protein-polyethylene glycol conjugates produced by reacting proteins of the invention with a polyethylene glycol molecule having a 2,2,2-trifluoreothane sulphonyl group.

Polyethylene glycol can also be attached to proteins using a number of different intervening linkers. For example, U.S. Patent No. 5,612,460, the entire disclosure of which is incorporated herein by reference, discloses urethane linkers for connecting polyethylene glycol to proteins. Protein-polyethylene glycol conjugates wherein the polyethylene glycol is attached to the protein by a linker can also be produced by reaction of proteins with compounds such as MPEG-succinimidylsuccinate, MPEG activated with 1,1'-carbonyldiimidazole, MPEG-2,4,5-trichloropenylcarbonate, MPEG-p-nitrophenolcarbonate, and various MPEG-succinate derivatives. A number additional polyethylene glycol derivatives and reaction chemistries for attaching polyethylene glycol to proteins are described in WO 98/32466, the entire disclosure of which is incorporated herein by reference. Pegylated protein products produced using the reaction chemistries set out herein are included within the scope of the invention.

The number of polyethylene glycol moieties attached to each protein of the invention (*i.e.*, the degree of substitution) may also vary. For example, the pegylated proteins of the invention may be linked, on average, to 1, 2, 3, 4, 5, 6, 7, 8, 9, 10, 12, 15, 17, 20, or more polyethylene glycol molecules. Similarly, the average degree of substitution within ranges such as 1-3, 2-4, 3-5, 4-6, 5-7, 6-8, 7-9, 8-10, 9-11, 10-12, 11-13, 12-14, 13-15, 14-16, 15-17, 16-18, 17-19, or 18-20 polyethylene glycol moieties per protein molecule. Methods for determining the degree of substitution are discussed, for example, in Delgado *et al., Crit. Rev. Thera. Drug Carrier Sys. 9*:249-304 (1992).

As mentioned the TR10 proteins of the invention may be modified by either natural processes, such as post-translational processing, or by chemical modification techniques which are well known in the art. It will be appreciated that the same type of modification may be present in the same or varying degrees at several sites in a given TR10 polypeptide. TR10 polypeptides may be branched, for example, as a result of ubiquitination, and they may be cyclic, with or without branching. Cyclic, branched, and branched cyclic TR10 polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter *et al., Meth Enzymol 182*:626-646 (1990); Rattan *et al., Ann NY Acad Sci 663*:48-62 (1992)).

***Epitopes***

The present invention encompasses polypeptides comprising, or alternatively consisting of, an epitope of the polypeptide having an amino acid sequence of SEQ ID NO:2, or an epitope of the polypeptide sequence encoded by a polynucleotide sequence contained in the deposited clone having ATCC accession number 209040 or encoded by a polynucleotide that hybridizes to the complement of the sequence of SEQ ID NO: or contained in the deposited clone having ATCC accession number 209040 under stringent hybridization conditions or lower stringency hybridization conditions as defined *supra.* The present invention further encompasses polynucleotide sequences encoding an epitope of a polypeptide sequence of the invention (such as, for example, the sequence disclosed in SEQ ID NO:1), polynucleotide sequences of the complementary strand of a polynucleotide sequence encoding an epitope of the invention, and polynucleotide sequences which hybridize to the complementary strand under stringent hybridization conditions or lower stringency hybridization conditions defined *supra.*

The term "epitopes," as used herein, refers to portions of a polypeptide having antigenic or immunogenic activity in an animal, preferably a mammal, and most preferably in a human. In a preferred embodiment, the present invention encompasses a polypeptide comprising an epitope, as well as the polynucleotide encoding this polypeptide. An "immunogenic epitope," as used herein, is defined as a portion of a protein that elicits an antibody response in an animal, as determined by any method known in the art, for example, by the methods for generating antibodies described infra. *(See,* for example, Geysen et al., Proc. Natl. Acad. Sci. USA 81:3998-4002 (1983)). The term "antigenic epitope," as used herein, is defined as a portion of a protein to which an antibody can immunospecifically bind its antigen as determined by any method well known in the art, for example, by the immunoassays described herein. Immunospecific binding excludes non-specific binding but does not necessarily exclude cross-reactivity with other antigens. Antigenic epitopes need not necessarily be immunogenic.

Fragments that function as epitopes may be produced by any conventional means. *(See, e.g.,* Houghten, Proc. Natl. Acad. Sci. USA 82:5131-5135 (1985), further described in U.S. Patent No. 4,631,211).

In the present invention, antigenic epitopes preferably contain a sequence of at least 4, at least 5, at least 6, at least 7, more preferably at least 8, at least 9, at least 10, at least 15, at least 20, at least 25, and, most preferably, between about 15 to about 30 amino acids. Preferred polypeptides comprising immunogenic or antigenic epitopes are at least 10, 15, 20, 25, 30, 35, 40, 45, 50, 55, 60, 65, 70, 75, 80, 85, 90, 95, or 100 amino acid residues in length. Antigenic epitopes are useful, for example, to raise antibodies, including monoclonal antibodies, that specifically bind the epitope. Antigenic epitopes can be used as the target molecules in immunoassays. (See, for instance, Wilson et al.. Cell 37:767-778 (1984): Sutcliffe et al.. *Science* 219:660-666 (1983)).

Non-limiting examples of antigenic polypeptides or peptides that can be used to generate TR10 receptor-specific antibodies include: a polypeptide comprising amino acid residues from about 57 to about 113 in Figures 1A-D (2 to 58 in SEQ ID NO:2); a polypeptide comprising amino acid residues from about 130 to about 197 in Figures 1A-D (75 to 142 in SEQ ID NO:2), and a polypeptide comprising amino acid residues from about 250 to about 283 in Figures 1A-D (195 to 228 in SEQ ID NO:2). As indicated above, the inventors have determined that the above polypeptide fragments are antigenic regions of the TR10 receptor protein. Thus, the invention encompasses polypeptides comprising or, alternatively, consisting of one, two, or three of these amino acid sequences, as well as polynucleotides encoding these amino acid sequences.

Similarly, immunogenic epitopes can be used, for example, to induce antibodies according to methods well known in the art. *(See,* for instance, Sutcliffe et al., *supra;* Wilson et al., *supra;* Chow et al., Proc. Natl. Acad. Sci. USA 82:910-914; and Bittle et al., J. Gen. Virol. 66:2347-2354 (1985). The polypeptides comprising one or more immunogenic epitopes may be presented for eliciting an antibody response together with a carrier protein, such as an albumin, to an animal system (such as, for example, rabbit or mouse), or, if the polypeptide is of sufficient length (at least about 25 amino acids), the polypeptide may be presented without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting).

Epitope-bearing polypeptides of the present invention may be used to induce antibodies according to methods well known in the art including, but not limited to, *in* vivo immunization, *in vitro* immunization, and phage display methods. *See,* e.g., Sutcliffe et al., *supra;* Wilson et al., *supra,* and Bittle et al., J. Gen. V irol., 66:2347-2354 (1985). If *in vivo* immunization is used, animals may be immunized with free peptide; however, anti-peptide antibody titer may be boosted by coupling the peptide to a macromolecular carrier, such as keyhole limpet hemacyanin (KLH) or tetanus toxoid. For instance, peptides containing cysteine residues may be coupled to a carrier using a linker such as maleimidobenzoyl-N-hydroxysuccinimide ester (MBS), while other peptides may be coupled to carriers using a more general linking agent such as glutaraldehyde. Animals such as, for example, rabbits, rats, and mice are immunized with either free or carrier-coupled peptides, for instance, by intraperitoneal and/or intradermal injection of emulsions containing about 100 micrograms of peptide or carrier protein and Freund's adjuvant or any other adjuvant known for stimulating an immune response. Several booster injections may be needed, for instance, at intervals of about two weeks, to provide a useful titer of anti-peptide antibody that can be detected, for example, by ELISA assay using free peptide adsorbed to a solid surface. The titer of anti-peptide antibodies in serum from an immunized animal may be increased by selection of anti-peptide antibodies, for instance, by adsorption to the peptide on a solid support and elution of the selected antibodies according to methods well known in the art.

As one of skill in the art will appreciate, and as discussed above, the polypeptides of the present invention comprising an immunogenic or antigenic epitope described herein (e.g., corresponding to a portion of the extracellular domain, such as, for example, amino acid residues 1 to 149 of SEQ ID NO:2) can be fused to other polypeptide sequences. For example, the polypeptides of the present invention may be fused with the constant domain of immunoglobulins (IgA, IgE, IgG, IgM), or portions thereof (CH1, CH2, CH3, or any combination thereof and portions thereof) resulting in chimeric polypeptides. Such fusion proteins may facilitate purification and may increase half-life *in vivo.* This has been shown for chimeric proteins consisting of the first two domains of the human CD4-polypeptide and various domains of the constant regions of the heavy or light chains of mammalian immunoglobulins. See, e.g., EP 394,827; Traunecker et al., Nature, 331:84-86 (1988). IgG Fusion proteins that have a disulfide-linked dimeric structure due to the IgG portion desulfide bonds have also been found to be more efficient in binding and neutralizing other molecules than monomeric polypeptides or fragments thereof alone. See, e.g., Fountoulakis et al., J. Biochem., 270:3958-3964 (1995). Nucleic acids encoding the above epitopes can also be recombined with a gene of interest as an epitope tag (e.g., the hemagglutinin ("HA") tag or flag tag) to aid in detection and purification of the expressed polypeptide. For example, a system described by Janknecht et al. allows for the ready purification of non-denatured fusion proteins expressed in human cell lines (Janknecht et al., 1991, Proc. Natl. Acad. Sci. USA 88:8972- 897). In this system, the gene of interest is subcloned into a vaccinia recombination plasmid such that the open reading frame of the gene is translationally fused to an amino-terminal tag consisting of six histidine residues. The tag serves as a matrix-binding domain for the fusion protein. Extracts from cells infected with the recombinant vaccinia virus are loaded onto $Ni^{2+}$ nitriloacetic acid-agarose column and histidine-tagged proteins can be selectively eluted with imidazole-containing buffers.

Preferred Fc fusions of the present invention include, but are not limited to constructs comprising, or alternatively consisting of, amino acid residues -55 to 157, -55 to 149, -45 to 157. -35 to 157, -15 to 157, -5 to 157, 1 to 157, 1 to 155, 1 to 149, 1 to 140, 10 to 157, 10 to 149, and/or 10 to 140 of SEQ ID NO:2.

Additional fusion proteins of the invention may be generated through the techniques of gene-shuffling, motif-shuffling, exon-shuffling, and/or codon-shuffling (collectively referred to as "DNA shuffling"). DNA shuffling may be employed to modulate the activities of polypeptides of the invention, such methods can be used to generate polypeptides with altered activity, as well as agonists and antagonists of the polypeptides. See, generally, U.S. Patent Nos. 5,605,793; 5,811,238; 5,830,721; 5,834,252; and 5,837,458, and Patten et al., Curr. Opinion Biotechnol. 8:724-33 (1997); Harayama, Trends Biotechnol. 16(2):76-82 (1998); Hansson, et al., J. Mol. Biol. 287:265-76 (1999); and Lorenzo and Blasco, Biotechniques 24(2):308- 13 (1998) (each of these patents and publications are hereby incorporated by reference in its entirety). In one embodiment, alteration of polynucleotides corresponding to SEQ ID NO: 1 and the polypeptides encoded by these polynucleotides may be achieved by DNA shuffling. DNA shuffling involves the assembly of two or more DNA segments by homologous or site-specific recombination to generate variation in the polynucleotide sequence. In another embodiment, polynucleotides of the invention, or the encoded polypeptides, may be altered by being subjected to random mutagenesis by error-prone PCR, random nucleotide insertion or other methods prior to recombination. In another embodiment, one or more components, motifs, sections, parts, domains, fragments, etc., of a polynucleotide coding a polypeptide of the invention may be recombined with one or more components, motifs, sections, parts, domains, fragments, etc. of one or more heterologous molecules.

### *Polypeptide Assays*

The present invention also relates to diagnostic assays such as quantitative and diagnostic assays for detecting levels of TR10 receptor protein, or the soluble form thereof, in cells and tissues, including determination of normal and abnormal levels. Thus, for instance, a diagnostic assay in accordance with the invention for detecting over-expression of TR10, or soluble form thereof, compared to normal control tissue samples may be used to detect the presence of tumors, for example. Assay techniques that can be used to determine levels of a protein, such as a TR10 protein of the present invention, or a soluble form thereof, in a sample derived from a host are well-known to those of skill in the art. Such assay methods include radioimmunoassays, competitive-binding assays, Western Blot analysis and ELISA assays.

Assaying TR10 protein levels in a biological sample can occur using any art-known method. By "biological sample" is intended any biological sample obtained from an individual, cell line, tissue culture, or other source containing TR 10 receptor protein or mRNA. Preferred for assaying TR10 protein levels in a biological sample are antibody-based techniques. For example, TR10 protein expression in tissues can be studied with classical immunohistological methods. (M. Jalkanen *et al., J. Cell. Biol. 101*:976-985 (1985); M. Jalkanen *et al., J. Cell. Biol. 105*:3087-3096 (1987)). Other antibody-based methods useful for detecting TR10 receptor gene expression include immunoassays, such as the enzyme linked immunosorbent assay (ELISA) and the radioimmunoassay (RIA).

Suitable antibody assay labels are known in the art and include enzyme labels, such as, glucose oxidase; radioisotopes, such as iodine ($^{131}I$, $^{125}I$, $^{123}I$, $^{121}I$), carbon ($^{14}C$), sulfur ($^{35}S$), tritium ($^{3}H$), indium ($^{115m}In$, $^{113}In$, $^{112}In$, $^{111}In$), and technetium ($^{99}Tc$, $^{99m}Tc$), thallium ($^{201}Ti$), gallium ($^{68}Ga$, $^{67}Ga$), palladium ($^{103}Pd$), molybdenum ($^{99}Mo$), xenon ($^{133}Xe$), fluorine ($^{18}F$), $^{153}Sm$, $^{177}Lu$, $^{159}Gd$, $^{149}Pm$, $^{140}La$, $^{175}Yb$, $^{166}Ho$, $^{90}Y$, $^{47}Sc$, $^{186}Re$, $^{188}Re$, $^{142}Pr$, $^{105}Rh$, $^{97}Ru$; luminescent labels, such as luminol; and fluorescent labels, such as fluorescein and rhodamine, and biotin.

### *Antibodies*

The present invention further relates to antibodies and T-cell antigen receptors (TCR) which immunospecifically bind a polypeptide, preferably an epitope, of the present invention (as determined by immunoassays well known in the art for assaying specific antibody-antigen binding). Antibodies of the invention include, but are not limited to, polyclonal, monoclonal, multispecific, human, humanized or chimeric antibodies, single chain antibodies, Fab fragments, F(ab') fragments, fragments produced by a Fab expression library, anti-idiotypic (anti-Id) antibodies (including, e.g., anti-Id antibodies to antibodies of the invention), and epitope-binding fragments of any of the above. The term "antibody," as used herein, refers to immunoglobulin molecules and immunologically active portions of immunoglobulin molecules, i.e., molecules that contain an antigen binding site that immunospecifically binds an antigen. The immunoglobulin molecules of the invention can be of any type (e.g., IgG, IgE, IgM, IgD, IgA and IgY), class (e.g., IgG1, IgG2, IgG3, IgG4, IgA1 and IgA2) or subclass of immunoglobulin molecule.

Most preferably the antibodies are human antigen-binding antibody fragments of the present invention and include, but are not limited to, Fab, Fab' and F(ab')2, Fd, single-chain Fvs (scFv), single-chain antibodies, disulfide-linked Fvs (sdFv) and fragments comprising either a VL or VH domain. Antigen-binding antibody fragments, including single-chain antibodies, may comprise the variable region(s) alone or in combination with the entirety or a portion of the following: hinge region, CH1, CH2, and CH3 domains. Also included in the invention are antigen-binding fragments also comprising any combination of variable region(s) with a hinge region, CH1, CH2, and CH3 domains. The antibodies of the invention may be from any animal origin including birds and mammals. Preferably, the antibodies are human, murine, donkey, ship rabbit, goat, guinea pig, camel, horse, or chicken. As used herein, "human" antibodies include antibodies having the amino acid sequence of a human immunoglobulin and include antibodies isolated from human immunoglobulin libraries or from animals transgenic for one or more human immunoglobulin and that do not express endogenous immunoglobulins, as described infra and, for example in, U.S. Patent No. 5,939,598 by Kucherlapati et al.

The antibodies of the present invention may be monospecific, bispecific, trispecific or of greater multispecificity. Multispecific antibodies may be specific for different epitopes of a polypeptide of the present invention or may be specific for both a polypeptide of the present invention as well as for a heterologous epitope, such as a heterologous polypeptide or solid support material. See, e.g., PCT publications WO 93/17715; WO 92/08802; WO 91/00360; WO 92/05793; Tutt, et al., J. Immunol. 147:60-69 (1991); U.S. Patent Nos. 4,474,893; 4,714,681; 4,925,648; 5,573,920; 5,601,819; Kostelny et al., J. Immunol. 148:1547-1553 (1992).

Antibodies of the present invention may be described or specified in terms of the epitope(s) or portion(s) of a polypeptide of the present invention that they recognize or specifically bind. The epitope(s) or polypeptide portion(s) may be specified as described herein, e.g., by N-terminal and C-terminal positions, by size in contiguous amino acid residues, or listed in the Tables and Figures. Antibodies that specifically bind any epitope or polypeptide of the present invention may also be excluded. Therefore, the present invention includes antibodies that specifically bind polypeptides of the present invention, and allows for the exclusion of the same.

Antibodies of the present invention may also be described or specified in terms of their cross-reactivity. Antibodies that do not bind any other analog, ortholog, or homolog of a polypeptide of the present invention are included. Antibodies that bind polypeptides with at least 95%, at least 90%, at least 85%, at least 80%, at least 75%, at least 70%, at least 65%, at least 60%, at least 55%, and at least 50% identity (as calculated using methods known in the art and described herein) to a polypeptide of the present invention are also included in the present invention. Antibodies that do not bind polypeptides with less than 95%, less than 90%, less than 85%, less than 80%, less than 75%, less than 70%, less than 65%, less than 60%, less than 55%, and less than 50% identity (as calculated using methods known in the art and described herein) to a polypeptide of the present invention are also included in the present invention. Further included in the present invention are antibodies that bind polypeptides encoded by polynucleotides which hybridize to a polynucleotide of the present invention under stringent hybridization conditions (as described herein). Antibodies of the present invention may also be described or specified in terms of their binding affinity to a polypeptide of the invention. Preferred binding affinities include those with a dissociation constant or Kd less than $5X10^{-2}M$, $10^{-2}M$, $5X10^{-3}M$, $10^{-3}M$, $5X10^{-4}M$, $10^{-4}$ M, $5X10^{-5}M$, $10^{-5}M$, $5X10^{-6}M$, $10^{-6}M$, $5X10^{-7}M$, $10^{-7}M$, $5X10^{-8}M$, $10^{-8}M$, $5X10^{-9}M$, $10^{-9}M$, $5X10^{-10}M$, $10^{-10}M$. $5X10^{-11}M$, $10^{-11}M$, $5X10^{-12}M$, $10^{-12}M$. $5X10^{-13}M$, $10^{-13}M$, 5X $10^{-14}M$, $10^{-14}M$, 5X $10^{-15}M$, and $10^{-15}M$.

The invention also provides antibodies that competitively inhibit binding of an antibody to an epitope of the invention as determined by any method known in the art for determining competitive binding, for example, the immunoassays described herein. In preferred embodiments, the antibody competitively inhibits binding to the epitope by at least 90%, at least 80%, at least 70%, at least 60%, or at least 50%.

Antibodies of the present invention may act as agonists or antagonists of the polypeptides of the present invention. For example, the present invention includes antibodies which disrupt the receptor/ligand interactions with the polypeptides of the invention either partially or fully. The invention features both receptor-specific antibodies and ligand-specific antibodies. The invention also features receptor-specific antibodies which do not prevent ligand binding but prevent receptor activation. Receptor activation (i.e., signaling) may be determined by techniques described herein or otherwise known in the art. For example, receptor activation can be determined by detecting the phosphorylation (e.g., tyrosine or serine/threonine) of the receptor or its substrate by immunoprecipitation followed by western blot analysis (for example, as described supra). In specific embodiments, antibodies are provided that inhibit ligand or receptor activity by at least 90%, at least 80%, at least 70%, at least 60%, or at least 50% of the activity in absence of the antibody.

The invention also features receptor-specific antibodies which both prevent ligand binding and receptor activation as well as antibodies that recognize the receptorligand complex, and, preferably, do not specifically recognize the unbound receptor or the unbound ligand. Likewise, included in the invention are neutralizing antibodies which bind the ligand and prevent binding of the ligand to the receptor, as well as antibodies which bind the ligand, thereby preventing receptor activation, but do not prevent the ligand from binding the receptor. Further included in the invention are antibodies which activate the receptor. These antibodies may act as receptor agonists, i.e., potentiate or activate either all or a subset of the biological activities of the ligand-mediated receptor activation. The antibodies may be specified as agonists, antagonists or inverse agonists for biological activities comprising the specific biological activities of the peptides of the invention disclosed herein. The above antibody agonists can be made using methods known in the art. See, e.g., PCT publication WO 96/40281; U.S. Patent No. 5,811,097; Deng et al., Blood 92(6):1981-1988 (1998); Chen, et al., Cancer Res. 58(16):3668-3678 (1998); Harrop et al., J. Immunol. 161(4):1786-1794 (1998); Zhu et al., Cancer Res. 58(15): 3209-3214 (1998); Yoon, et al., J. Immunol. 160(7):3170-3179 (1998); Prat et al., J. Cell. Sci. 111 (Pt2):237-247 (1998); Pitard et al., J. Immunol. Methods 205(2):177-190 (1997); Liautard et al., Cytokine 9(4):233-241 (1997); Carlson et al., J. Biol. Chem. 272(17):11295-11301 (1997); Taryman et al., Neuron 14(4):755-762 (1995); Muller et al., Structure 6(9): 1153-1167 (1998); Bartunek et al., Cytokine 8(1):14-20 (1996) (which are all incorporated by reference herein in their entireties).

Antibodies of the present invention may be used, for example, but not limited to, to purify, detect, and target the polypeptides of the present invention, including both in vitro and in vivo diagnostic and therapeutic methods. For example, the antibodies have use in immunoassays for qualitatively and quantitatively measuring levels of the polypeptides of the present invention in biological samples. See, e.g., Harlow et al., Antibodies: A Laboratory Manual, (Cold Spring Harbor Laboratory Press, 2nd ed. 1988) (incorporated by reference herein in its entirety).

As discussed in more detail below, the antibodies of the present invention may be used either alone or in combination with other compositions. The antibodies may further be recombinantly fused to a heterologous polypeptide at the N- or C-terminus or chemically conjugated (including covalently and non-covalently conjugations) to polypeptides or other compositions. For example, antibodies of the present invention may be recombinantly fused or conjugated to molecules useful as labels in detection assays and effector molecules such as heterologous polypeptides, drugs, or toxins. See, e.g., PCT publications WO 92/08495; WO 91/14438; WO 89/12624; U.S. Patent No. 5,314,995; and EP 396,387.

The antibodies of the invention include derivatives that are modified, i.e, by the covalent attachment of any type of molecule to the antibody such that covalent attachment does not prevent the antibody from generating an anti-idiotypic response. For example, but not by way of limitation, the antibody derivatives include antibodies that have been modified, e.g., by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the derivative may contain one or more non-classical amino acids.

The antibodies of the present invention may be generated by any suitable method known in the art. Polyclonal antibodies to an antigen-of- interest can be produced by various procedures well known in the art. For example, a polypeptide of the invention can be administered to various host animals including, but not limited to, rabbits, mice, rats, etc. to induce the production of sera containing polyclonal antibodies specific for the antigen. Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum. Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981) (said references incorporated by reference in their entireties). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well-known in the art and are discussed in detail in Example 6, below. Briefly, mice can be immunized with a polypeptide of the invention or a cell expressing such peptide. Once an immune response is detected, e.g., antibodies specific for the antigen are detected in the mouse serum, the mouse spleen is harvested and splenocytes isolated. The splenocytes are then fused by well-known techniques to any suitable myeloma cells, for example cells from cell line SP20 available from the ATCC. Hybridomas are selected and cloned by limited dilution. The hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

Accordingly, the present invention provides methods of generating monoclonal antibodies as well as antibodies produced by the method comprising culturing a hybridoma cell secreting an antibody of the invention wherein, preferably, the hybridoma is generated by fusing splenocytes isolated from a mouse immunized with an antigen of the invention with myeloma cells and then screening the hybridomas resulting from the fusion for hybridoma clones that secrete an antibody able to bind a polypeptide of the invention.

Antibody fragments that recognize specific epitopes may be generated by known techniques. For example, Fab and F(ab')2 fragments of the invention may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH 1 domain of the heavy chain.

For example, the antibodies of the present invention can also be generated using various phage display methods known in the art. In phage display methods, functional antibody domains are displayed on the surface of phage particles which carry the polynucleotide sequences encoding them. In a particular, such phage can be utilized to display antigen-binding domains expressed from a repertoire or combinatorial antibody library (e.g., human or murine). Phage expressing an antigen binding domain that binds the antigen of interest can be selected or identified with antigen, e.g., using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Examples of phage display methods that can be used to make the antibodies of the present invention include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT application No. PCT/GB91/01134; PCT publications WO 90/02809; WO 91/10737; WO 92/01047; WO 92/18619; WO 93/11236; WO 95/15982; WO 95/20401; and U.S. Patent Nos. 5,698,426; 5,223,409; 5.403,484; 5,580,717; 5.427,908; 5,750,753; 5,821,047; 5,571,698; 5,427,908; 5,516,637; 5,780,225; 5,658,727; 5,733,743 and 5,969,108; each of which is incorporated herein by reference in its entirety.

As described in the above references, after phage selection, the antibody coding regions from the phase can be isolated and used to generate whole antibodies, including human antibodies, or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')2 fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240: 1041-1043 (1988) (said references incorporated by reference in their entireties).

Examples of techniques which can be used to produce single-chain Fvs and antibodies include those described in U.S. Patents 4,946,778 and 5,258,498; Huston et al., Methods in Enzymology 203:46-88 (1991); Shu et al., PNAS 90:7995-7999 (1993); and Skerra et al., Science 240:1038-1040 (1988). For some uses, including *in vivo* use of antibodies in humans and in vitro detection assays, it may be preferable to use chimeric, humanized, or human antibodies. A chimeric antibody is a molecule in which different portions of the antibody are derived from different animal species, such as antibodies having a variable region derived from a murine monoclonal antibody and a human immunoglobulin constant region. Methods for producing chimeric antibodies are known in the art. See e.g., Morrison, Science 229:1202 (1985); Oi et al., BioTechniques 4:214 (1986); Gillies et al., (1989) J. Immunol. Methods 125:191-202; U.S. Patent Nos. 5,807,715; 4,816,567; and 4,816397, which are incorporated herein by reference in their entireties. Humanized antibodies are antibody molecules from non-human species antibody that binds the desired antigen having one or more complementarity determining regions (CDRs) from the non-human species and framework regions from a human immunoglobulin molecule. Often, framework residues in the human framework regions will be substituted with the corresponding residue from the CDR donor antibody to alter, preferably improve, antigen binding. These framework substitutions are identified by methods well known in the art, e.g., by modeling of the interactions of the CDR and framework residues to identify framework residues important for antigen binding and sequence comparison to identify unusual framework residues at particular positions. (See, e.g., Queen et al., U.S. Patent No. 5,585,089: Riechmann et al., Nature 332:323 (1988), which are incorporated herein by reference in their entireties.) Antibodies can be humanized using a variety of techniques known in the art including, for example, CDR-grafting (EP 239,400; PCT publication WO 91/09967; U.S. Patent Nos. 5,225,539: 5,530,101; and 5,585,089), veneering or resurfacing (EP 592,106; EP 519,596; Padlan, Molecular Immunology 28(4/5):489-498 (1991); Studnicka et al., Protein Engineering 7(6):805-814 (1994); Roguska. et al., PNAS 91: 969-973 (1994)), and chain shuffling (U.S. Patent No. 5,565,332).

Completely human antibodies are particularly desirable for therapeutic treatment of human patients. Human antibodies can be made by a variety of methods known in the art including phage display methods described above using antibody libraries derived from human immunoglobulin sequences. See also, U.S. Patent Nos. 4,444,887 and 4,716,111; and PCT publications WO 98/46645, WO 98/50433, WO 98/24893, WO 98/16654, WO 96/34096, WO 96/33735, and WO 91/10741; each of which is incorporated herein by reference in its entirety.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring that express human antibodies. The transgenic mice are immunized in the normal fashion with a selected antigen, e.g., all or a portion of a polypeptide of the invention. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13: 65-93). For a detailed discussion of this technology for producing human antibodies and human monoclonal antibodies and protocols for producing such antibodies, see, e.g., PCT publications WO 98/24893; WO 96/34096; WO 96/33735; U.S. Patent Nos. 5,413,923; 5.625,126; 5,633,425; 5,569,825; 5.66 1 ,016: 5,545,806; 5,814,318; and 5,939,598, which are incorporated by reference herein in their entirety. In addition, companies such as Abgenix, Inc. (Freemont, CA) and Genpharm (San Jose, CA) can be engaged to provide human antibodies directed against a selected antigen using technology similar to that described above.

Completely human antibodies which recognize a selected epitope can be generated using a technique referred to as "guided selection." In this approach a selected non-human monoclonal antibody, e.g., a mouse antibody, is used to guide the selection of a completely human antibody recognizing the same epitope. (Jespers et al., Bio/technology 12: 899-903 (1988)).

Further, antibodies to the polypeptides of the invention can, in turn, be utilized to generate anti-idiotype antibodies that "mimic" polypeptides of the invention using techniques well known to those skilled in the art. (See, e.g., Greenspan & Bona, FASEB J. 7(5):437-444; (1989) and Nissinoff, J. Immunol. 147(8):2429-2438 (1991)). For example, antibodies which bind to and competitively inhibit polypeptide multimerization and/or binding of a polypeptide of the invention to a ligand can be used to generate anti-idiotypes that "mimic" the polypeptide multimerization and/or binding domain and, as a consequence, bind to and neutralize polypeptide and/or its ligand. Such neutralizing anti-idiotypes or Fab fragments of such anti-idiotypes can be used in therapeutic regimens to neutralize polypeptide ligand. For example, such anti-idiotypic antibodies can be used to bind a polypeptide of the invention and/or to bind its ligands/receptors, and thereby block its biological activity.

### *Polynucleotides Encoding Antibodies*

The invention further provides polynucleotides comprising a nucleotide sequence encoding an antibody of the invention and fragments thereof. The invention also encompasses polynucleotides that hybridize under stringent or lower stringency hybridization conditions, e.g., as defined supra, to polynucleotides that encode an antibody, preferably, that specifically binds to a polypeptide of the invention, preferably, an antibody that binds to a polypeptide having the amino acid sequence of SEQ ID NO:2.

The polynucleotides may be obtained, and the nucleotide sequence of the polynucleotides determined, by any method known in the art. For example, if the nucleotide sequence of the antibody is known, a polynucleotide encoding the antibody may be assembled from chemically synthesized oligonucleotides (e.g., as described in Kutmeier et al., BioTechniques 17:242 (1994)), which, briefly, involves the synthesis of overlapping oligonucleotides containing portions of the sequence encoding the antibody, annealing and ligation of those oligonucleotides, and then amplification of the ligated oligonucleotides by PCR.

Alternatively, a polynucleotide encoding an antibody may be generated from nucleic acid from a suitable source-If a clone containing a nucleic acid encoding a particular antibody is not available, but the sequence of the antibody molecule is known, a nucleic acid encoding the immunoglobulin may be obtained from a suitable source (e.g., an antibody cDNA library, or a cDNA library generated from, or nucleic acid, preferably poly A+ RNA, isolated from, any tissue or cells expressing the antibody, such as hybridoma cells selected to express an antibody of the invention) by PCR amplification using synthetic primers hybridizable to the 3' and 5' ends of the sequence or by cloning using an oligonucleotide probe specific for the particular gene sequence to identify, e.g., a cDNA clone from a cDNA library that encodes the antibody. Amplified nucleic acids generated by PCR may then be cloned into replicable cloning vectors using any method well known in the art.

Once the nucleotide sequence and corresponding amino acid sequence of the antibody is determined, the nucleotide sequence of the antibody may be manipulated using methods well known in the art for the manipulation of nucleotide sequences, e.g., recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, NY and Ausubel et al., eds., 1998, Current Protocols in Molecular Biology, John Wiley & Sons, NY, which are both incorporated by reference herein in their entireties ), to generate antibodies having a different amino acid sequence, for example to create amino acid substitutions, deletions, and/or insertions.

In a specific embodiment, the amino acid sequence of the heavy and/or light chain variable domains may be inspected to identify the sequences of the complementarity determining regions (CDRs) by methods that are well know in the art, e.g., by comparison to known amino acid sequences of other heavy and light chain variable regions to determine the regions of sequence hypervariability. Using routine recombinant DNA techniques, one or more of the CDRs may be inserted within framework regions, e.g., into human framework regions to humanize a non-human antibody, as described supra. The framework regions may be naturally occurring or consensus framework regions, and preferably human framework regions (see, e.g., Chothia et al., J. Mol. Biol. 278: 457-479 (1998) for a listing of human framework regions). Preferably, the polynucleotide generated by the combination of the framework regions and CDRs encodes an antibody that specifically binds a polypeptide of the invention. Preferably, as discussed supra, one or more amino acid substitutions may be made within the framework regions, and, preferably, the amino acid substitutions improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present invention and within the skill of the art.

In addition, techniques developed for the production of "chimeric antibodies" (Morrison et al., 1984, Proc. Natl. Acad. Sci. 81:851-855; Neuberger et al., 1984, Nature 312:604-608; Takeda et al., 1985, Nature 314:452-454) by splicing genes from a mouse antibody molecule of appropriate antigen specificity together with genes from a human antibody molecule of appropriate biological activity can be used. As described supra, a chimeric antibody is a molecule in which different portions are derived from different animal species, such as those having a variable region derived from a murine mAb and a human immunoglobulin constant region, e.g., humanized antibodies.

Alternatively, techniques described for the production of single chain antibodies (U.S. Patent No. 4,694,778; Bird, 1988, Science 242:423- 42; Huston et al., 1988, Proc. Natl. Acad. Sci. USA 85:5879-5883; and Ward et al., 1989, Nature 334:544-54) can be adapted to produce single chain antibodies. Single chain antibodies are formed by linking the heavy and light chain fragments of the Fv region via an amino acid bridge, resulting in a single chain polypeptide. Techniques for the assembly of functional Fv fragments in E. coli may also be used (Skerra et al., 1988, Science 242: 1038- 1041).

### *Methods of Producing Antibodies*

The antibodies of the invention can be produced by any method known in the art for the synthesis of antibodies, in particular, by chemical synthesis or preferably, by recombinant expression techniques.

Recombinant expression of an antibody of the invention, or fragment, derivative or analog thereof, e.g., a heavy or light chain of an antibody of the invention, requires construction of an expression vector containing a polynucleotide that encodes the antibody. Once a polynucleotide encoding an antibody molecule or a heavy or light chain of an antibody, or portion thereof (preferably containing the heavy or light chain variable domain), of the invention has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, in vitro recombinant DNA techniques, synthetic techniques, and in vivo genetic recombination. The invention, thus, provides replicable vectors comprising a nucleotide sequence encoding an antibody molecule of the invention, or a heavy or light chain thereof, or a heavy or light chain variable domain, operably linked to a promoter. Such vectors may include the nucleotide sequence encoding the constant region of the antibody molecule (see, e.g., PCT Publication WO 86/05807; PCT Publication WO 89/01036; and U.S. Patent No. 5,122,464) and the variable domain of the antibody may be cloned into such a vector for expression of the entire heavy or light chain.

The expression vector is transferred to a host cell by conventional techniques and the transfected cells are then cultured by conventional techniques to produce an antibody of the invention. Thus, the invention includes host cells containing a polynucleotide encoding an antibody of the invention, or a heavy or light chain thereof, operably linked to a heterologous promoter. In preferred embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibody molecules of the invention. Such host-expression systems represent vehicles by which the coding sequences of interest may be produced and subsequently purified, but also represent cells which may, when transformed or transfected with the appropriate nucleotide coding sequences, express an antibody molecule of the invention in situ. These include but are not limited to microorganisms such as bacteria (e.g., E. coli, B. subtilis) transformed with recombinant bacteriophage DNA, plasmid DNA or cosmid DNA expression vectors containing antibody coding sequences; yeast (e.g., Saccharomyces, Pichia) transformed with recombinant yeast expression vectors containing antibody coding sequences; insect cell systems infected with recombinant virus expression vectors (e.g., baculovirus) containing antibody coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing antibody coding sequences; or mammalian cell systems (e.g., COS, CHO, BHK, 293, 3T3 cells) harboring recombinant expression constructs containing promoters derived from the genome of mammalian cells (e.g., metallothionein promoter) or from mammalian viruses (e.g., the adenovirus late promoter; the vaccinia virus 7.5K promoter). Preferably, bacterial cells such as Escherichia coli, and more preferably, eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; Cockett et al., 1990, Bio/Technology 8:2).

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited, to the E. coli expression vector pUR278 (Ruther et al., 1983, EMBO J. 2:1791 ), in which the antibody coding sequence may be ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, Autographa californica nuclear polyhedrosis virus (AcNPV) is used as a vector to express foreign genes. The virus grows in Spodoptera frugiperda cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome by in vitro or in vivo recombination. Insertion in a non- essential region of the viral genome (e.g., region El or E3) will result in a recombinant virus that is viable and capable of expressing the antibody molecule in infected hosts. (e.g., see Logan & Shenk, 1984, Proc. Natl. Acad. Sci. USA 81:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bittner et al., 1987, Methods in Enzymol. 153:51-54.4).

In addition, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 3T3, W138, and in particular, breast cancer cell lines such as, for example, BT483, Hs578T, HTB2. BT20 and T47D, and normal mammary gland cell line such as, for example, CRL7030 and Hs578Bst.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compounds that interact directly or indirectly with the antibody molecule.

A number of selection systems may be used, including but not limited to the herpes simplex virus thymidine kinase (Wigler et al., 1977, Cell 11:223), hypoxanthine-guanine phosphoribosyltransferase (Szybalska & Szybalski, 192, Proc. Natl. Acad. Sci. USA 48:202), and adenine phosphoribosyltransferase (Lowy et al., 1980, Cell 22:817) genes can be employed in tk-, hgprt- or aprt- cells, respectively. Also, antimetabolite resistance can be used as the basis of selection for the following genes: dhfr, which confers resistance to methotrexate (Wigler et al., 1980, Natl. Acad. Sci. USA 77: 357; O'Hare et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 Clinical Pharmacy 12:488-505; Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62: 191-217; May, 1993, TIB TECH 11(5):155-215); and hygro, which confers resistance to hygromycin (Santerre et al., 1984, Gene 30:147). Methods commonly known in the art of recombinant DNA technology which can be used are described in Ausubel et al. (eds.), 1993, Current Protocols in Molecular Biology, John Wiley & Sons, NY; Kriegler, 1990, Gene Transfer and Expression, A Laboratory Manual, Stockton Press, NY; and in Chapters 12 and 13, Dracopoli et al. (eds), 1994, Current Protocols in Human Genetics, John Wiley & Sons, NY.; Colberre-Garapin et al., 1981, J. Mol. Biol 150:1, which are incorporated by reference herein in their entireties.

The expression levels of an antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, Nature 322:52: Kohler, 1980, Proc. Natl. Acad. Sci. USA 77:2197). The coding sequences for the heavy and light chains may comprise cDNA or genomic DNA.

Once an antibody molecule of the invention has been recombinantly expressed, it may be purified by any method known in the art for purification of an immunoglobulin molecule, for example, by chromatography (e.g., ion exchange, affinity, particularly by affinity for the specific antigen after Protein A, and sizing column chromatography), centrifugation, differential solubility, or by any other standard technique for the purification of proteins.

***Antibody Cojugates***

The present invention encompasses antibodies recombinantly fused or chemically conjugated (including both covalently and non-covalently conjugations) to a polypeptide (orportion thereof, preferably at least 10, 20 or 50 amino acids of the polypeptide) of the present invention to generate fusion proteins. The fusion does not necessarily need to be direct, but may occur through linker sequences. The antibodies may be specific for antigens other than polypeptides (or portion thereof, preferably at least 10, 20 or 50 amino acids of the polypeptide) of the present invention. For example, antibodies may be used to target the polypeptides of the present invention to particular cell types, either in vitro or in vivo, by fusing or conjugating the polypeptides of the present invention to antibodies specific for particular cell surface receptors. Antibodies fused or conjugated to the polypeptides of the present invention may also be used in in vitro immunoassays and purification methods using methods known in the art. See e.g., Harbor et al., supra, and PCT publication WO 93/21232; EP 439,095; Naramura et al., Immunol. Lett. 39:91-99 (1994); U.S. Patent 5,474,981; Gillies et al., PNAS 89:1428-1432 (1992); Fell et al., J. Immunol. 146:2446-2452(1991), which are incorporated by reference in their entireties.

The present invention further includes compositions comprising the polypeptides of the present invention fused or conjugated to antibody domains other than the variable regions. For example, the polypeptides of the present invention may be fused or conjugated to an antibody Fc region, or portion thereof. The antibody portion fused to a polypeptide of the present invention may comprise the constant region, hinge region, CH1 domain, CH2 domain, and CH3 domain or any combination of whole domains or portions thereof. The polypeptides may also be fused or conjugated to the above antibody portions to form multimers. For example, Fc portions fused to the polypeptides of the present invention can form dimers through disulfide bonding between the Fc portions. Higher multimeric forms can be made by fusing the polypeptides to portions of IgA and IgM. Methods for fusing or conjugating the polypeptides of the present invention to antibody portions are known in the art. See, e.g., U.S. Patent Nos. 5,336,603; 5,622,929; 5,359,046; 5,349,053; 5,447,851; 5,112,946; EP 307,434; EP 367,166; PCT publications WO 96/04388; WO 91/06570; Ashkenazi et al., Proc. Natl. Acad. Sci. USA 88:10535-10539 (1991); Zheng et al., J. Immunol. 154:5590-5600 (1995); and Vil et al., Proc. Natl. Acad. Sci. USA 89:11337- 11341(1992) (said references incorporated by reference in their entireties).

As discussed, supra, the polypeptides of the present invention may be fused or conjugated to the above antibody portions to increase the in vivo half life of the polypeptides or for use in immunoassays using methods known in the art. Further, the polypeptides of the present invention may be fused or conjugated to the above antibody portions to facilitate purification. One reported example describes chimeric proteins consisting of the first two domains of the human CD4-polypeptide and various domains of the constant regions of the heavy or light chains of mammalian immunoglobulins. (EP 394,827; Traunecker et al., Nature 331:84-86 (1988). The polypeptides of the present invention fused or conjugated to an antibody having disulfide- linked dimeric structures (due to the IgG) may also be more efficient in binding and neutralizing other molecules, than the monomeric secreted protein or protein fragment alone. (Fountoulakis et al., J. Biochem. 270:3958-3964 (1995)). In many cases, the Fc part in a fusion protein is beneficial in therapy and diagnosis, and thus can result in. for example, improved pharmacokinetic properties. (EP A 232,262). Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270:9459-9471 (1995)0.

Moreover, the antibodies or fragments thereof of the present invention can be fused to marker sequences, such as a peptide to facilitates their purification. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86: 821-824 (1989), for instance, hexa-histidine provides for convenient purification of the fusion protein. Other peptide tags useful for purification include, but are not limited to, the "HA" tag, which corresponds to an epitope derived from the influenza hemagglutinin protein (Wilson et al., Cell 37:767 (1984)) and the "flag" tag.

The present invention further encompasses antibodies or fragments thereof conjugated to a diagnostic or therapeutic agent. The antibodies can be used diagnostically to, for example, monitor the development or progression of a tumor as part of a clinical testing procedure to, e.g., determine the efficacy of a given treatment regimen. Detection can be facilitated by coupline the antibody to a detectable substance. Examples of detectable substances include various enzymes, prosthetic groups, fluorescent materials, luminescent materials, bioluminescent materials, radioactive materials, positron emitting metals using various positron emission tomographies, and nonradioactive paramagnetic metal ions. See, for example, U.S. Patent No. 4,741,900 for metal ions which can be conjugated to antibodies for use as diagnostics according to the present invention. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin: and examples of suitable radioactive material include iodine ($^{131}I$, $^{125}I$, $^{123}I$, $^{121}I$), carbon ($^{14}C$), sulfur ($^{35}S$), tritium ($^{3}H$), indium ($^{115m}In$, $^{113m}In$, $^{112}In$, $^{111}In$), and technetium ($^{99}Tc$, $^{99m}Tc$), thallium ($^{201}Ti$), gallium ($^{68}Ga$, $^{67}Ga$), palladium ($^{103}Pd$), molybdenum ($^{99}Mo$), xenon ($^{133}Xe$), fluorine ($^{18}F$), $^{153}Sm$, $^{177}Lu$, $^{159}Gd$, $^{149}Pm$, $^{140}La$, $^{175}Yb$, $^{166}Ho$, $^{90}Y$, $^{47}Sc$, $^{186}Re$, $^{188}Re$, $^{142}Pr$, $^{105}Rh$, $^{97}Ru$.

Further, an antibody or fragment thereof may be conjugated to a therapeutic moiety such as a cytotoxin, e.g., a cytostatic or cytocidal agent, a therapeutic agent or a radioactive metal ion. A cytotoxin or cytotoxic agent includes any agent that is detrimental to cells. Examples include paclitaxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyctothosphamide, busulfan, dibromomannitol, streptozotocin, mitomycin C, and cis- dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

The conjugates of the invention can be used for modifying a given biological response, the therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, a-interferon, β-interferon, nerve growth factor, platelet derived growth factor, tissue plasminogen activator, a thrombotic agent or an anti- angiogenic agent, e.g., angiostatin or endostatin; or, biological response modifiers such as, for example, lymphokines, interleukin-1 ("IL-1 "), interleukin-2 ("IL-2"), interleukin-6 ("IL-6"), granulocyte macrophase colony stimulating factor ("GM-CSF"), granulocyte colony stimulating factor ("G-CSF"), or other growth factors.

Antibodies may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

Techniques for conjugating such therapeutic moiety to antibodies are well known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev. 62:119-58 ( 1982).

Alternatively, an antibody can be conjugated to a second antibody to form an antibody heteroconjugate as described by Segal in U.S. Patent No. 4,676,980, which is incorporated herein by reference in its entirety.

An antibody, with or without a therapeutic moiety conjugated to it, administered alone or in combination with cytotoxic factor(s) and/or cytokine(s) can be used as a therapeutic.

***Assays For Antibody Binding***

The antibodies of the invention may be assayed for immunospecific binding by any method known in the art.

The immunoassays which can be used include but are not limited to competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (see, e.g., Ausubel et al, eds, 1994, Current Protocols in Molecular Biology, Vol. 1, John Wiley & Sons, Inc., New York, which is incorporated by reference herein in its entirety). Exemplary immunoassays are described briefly below (but are not intended by way of limitation).

Immunoprecipitation protocols generally comprise lysing a population of cells in a lysis buffer such as RIPA buffer (1% NP-40 or Triton X- 100, 1 % sodium deoxycholate, 0.1 % SDS, 0.15 M NaCl, 0.01 M sodium phosphate at pH 7.2, 1% Trasylol) supplemented with protein phosphatase and/or protease inhibitors (e.g., EDTA, PMSF, aprotinin, sodium vanadate), adding the antibody of interest to the cell lysate, incubating for a period of time (e.g., 1-4 hours) at 4° C, adding protein A and/or protein G sepharose beads to the cell lysate, incubating for about an hour or more at 4° C, washing the beads in lysis buffer and resuspending the beads in SDS/sample buffer. The ability of the antibody of interest to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with sepharose beads). For further discussion regarding immunoprecipitation protocols see, e.g., Ausubel et al, eds, 1994, Current Protocols in Molecular Biology, Vol. 1, John Wiley & Sons, Inc., New York at 10.16.1.

Western blot analysis generally comprises preparing protein samples, electrophoresis of the protein samples in a polyacrylamide gel (e.g., 8%- 20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 1251) diluted in blocking buffer, washing the membrane in wash buffer, and detecting the presence of the antigen. One of skill in the art would be knowledgeable as to the parameters that can be modified to increase the signal detected and to reduce the background noise. For further discussion regarding western blot protocols see, e.g., Ausubel et al, eds, 1994, Current Protocols in Molecular Biology, Vol. 1, John Wiley & Sons, Inc., New York at 10.8.1.

ELISAs comprise preparing antigen, coating the well of a 96 well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound: instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art. For further discussion regarding ELISAs see, e.g., Ausubel et al, eds, 1994, Current Protocols in Molecular Biology, Vol. 1, John Wiley & Sons, Inc., New York at 11.2.1.

The binding affinity of an antibody to an antigen and the off-rate of an antibody-antigen interaction can be determined by competitive binding assays. One example of a competitive binding assay is a radioimmunoassay comprising the incubation of labeled antigen (e.g., 3H or 1251) with the antibody of interest in the presence of increasing amounts of unlabeled antigen, and the detection of the antibody bound to the labeled antigen. The affinity of the antibody of interest for a particular antigen and the binding off-rates can be determined from the data by scatchard plot analysis. Competition with a second antibody can also be determined using radioimmunoassays. In this case, the antigen is incubated with antibody of interest is conjugated to a labeled compound (e.g., 3H or 125I) in the presence of increasing amounts of an unlabeled second antibody.

### *Antibody-Based Therapeutic Uses*

The present invention is further directed to antibody-based therapies which involve administering antibodies of the invention to an animal, preferably a mammal, and most preferably a human, patient for treating one or more of the described disorders. Therapeutic compounds of the invention include, but are not limited to, antibodies of the invention (including fragments, analogs and derivatives thereof as described herein) and nucleic acids encoding antibodies of the invention (including fragments, analogs and derivatives thereof as described herein). The antibodies of the invention can be used to treat, inhibit or prevent diseases and disorders associated with aberrant expression and/or activity of a polypeptide of the invention, including, but not limited to, [ insert diseases and disorders]. The treatment and/or prevention of diseases and disorders associated with aberrant expression and/or activity of a polypeptide of the invention includes, but is not limited to, alleviating symptoms associated with those diseases and disorders. Antibodies of the invention may be provided in pharmaceutically acceptable compositions as known in the art or as described herein.

A summary of the ways in which the antibodies of the present invention may be used therapeutically includes binding polynucleotides or polypeptides of the present invention locally or systemically in the body or by direct cytotoxicity of the antibody, e.g. as mediated by complement (CDC) or by effector cells (ADCC). Some of these approaches are described in more detail below. Armed with the teachings provided herein, one of ordinary skill in the art will know how to use the antibodies of the present invention for diagnostic, monitoring or therapeutic purposes without undue experimentation.

The antibodies of this invention may be advantageously utilized in combination with other monoclonal or chimeric antibodies, or with lymphokines or hematopoietic growth factors (such as, e.g., IL-2, IL-3 and IL-7), for example, which serve to increase the number or activity of effector cells which interact with the antibodies.

The antibodies of the invention may be administered alone or in combination with other types of treatments (e.g., radiation therapy, chemotherapy, hormonal therapy, immunotherapy and anti-tumor agents). Generally, administration of products of a species origin or species reactivity (in the case of antibodies) that is the same species as that of the patient is preferred. Thus, in a preferred embodiment, human antibodies, fragments derivatives, analogs, or nucleic acids, are administered to a human patient for therapy or prophylaxis.

It is preferred to use high affinity and/or potent in vivo inhibiting and/or neutralizing antibodies against polypeptides or polynucleotides of the present invention, fragments or regions thereof, for both immunoassays directed to and therapy of disorders related to polynucleotides or polypeptides, including fragments thereof, of the present invention. Such antibodies, fragments, or regions, will preferably have an affinity for polynucleotides or polypeptides, including fragments thereof. Preferred binding affinities include those with a dissociation constant or Kd less than 5 X 10-6 M, 10-6 M, 5 X 10-7 M, 10-7 M, 5 X 10-8 M, 10-8 M, 5 X 10-9 M, 10-9 M, 5 X 10-10 M, 10-10 M, 5 X 10-11 M, 10-11 M, 5 X 10-12 M, 10-12 M, 5 X 10-13 M, 10- 13 M, 5 X 10-14 M, 10-14 M, 5 X 10-15 M, and 10-15 M.

### *Antibody-Based Gene Therapy*

In a specific embodiment, nucleic acids comprising sequences encoding antibodies or functional derivatives thereof, are administered to treat, inhibit or prevent a disease or disorder associated with aberrant expression and/or activity of a polypeptide of the invention, by way of gene therapy. Gene therapy refers to therapy performed by the administration to a subject of an expressed or expressible nucleic acid. In this embodiment of the invention, the nucleic acids produce their encoded protein that mediates a therapeutic effect.

Any of the methods for gene therapy available in the art can be used according to the present invention. Exemplary methods are described below.

For general reviews of the methods of gene therapy, see Goldspiel et al., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62:191-217; May, 1993, TIBTECH 11(5): 155-215). Methods commonly known in the art of recombinant DNA technology which can be used are described in Ausubel et al. (eds.), 1993, Current Protocols in Molecular Biology, John Wiley & Sons, NY; and Kriegler, 1990, Gene Transfer and Expression, A Laboratory Manual, Stockton Press, NY.

In a preferred aspect, the compound comprises nucleic acid sequences encoding an antibody, said nucleic acid sequences being part of expression vectors that express the antibody or fragments or chimeric proteins or heavy or light chains thereof in a suitable host In particular, such nucleic acid sequences have promoters operably linked to the antibody coding region, said promoter being inducible or constitutive, and, optionally, tissue- specific. In another particular embodiment, nucleic acid molecules are used in which the antibody coding sequences and any other desired sequences are flanked by regions that promote homologous recombination at a desired site in the genome, thus providing for intrachromosomal expression of the antibody nucleic acids (Koller and Smithies, 1989, Proc. Natl. Acad. Sci. USA 86:8932-8935; Zijlstra et al., 1989, Nature 342:435-438). In specific embodiments, the expressed antibody molecule is a single chain antibody; alternatively, the nucleic acid sequences include sequences encoding both the heavy and light chains, or fragments thereof, of the antibody.

Delivery of the nucleic acids into a patient may be either direct, in which case the patient is directly exposed to the nucleic acid or nucleic acid- carrying vectors, or indirect, in which case, cells are first transformed with the nucleic acids in vitro, then transplanted into the patient. These two approaches are known, respectively, as in vivo or ex vivo gene therapy.

In a specific embodiment, the nucleic acid sequences are directly administered in vivo, where it is expressed to produce the encoded product. This can be accomplished by any of numerous methods known in the art, e.g., by constructing them as part of an appropriate nucleic acid expression vector and administering it so that they become intracellular, e.g., by infection using defective or attenuated retrovirals or other viral vectors (see U.S. Patent No. 4,980,286), or by direct injection of naked DNA, or by use of microparticle bombardment (e.g., a gene gun; Biolistic, Dupont), or coating with lipids or cell-surface receptors or transfecting agents, encapsulation in liposomes, microparticles, or microcapsules, or by administering them in linkage to a peptide which is known to enter the nucleus, by administering it in linkage to a ligand subject to receptor-mediated endocytosis (see, e.g., Wu and Wu, 1987, J. Biol. Chem. 262: 4429-4432) (which can be used to target cell types specifically expressing the receptors), etc. In another embodiment, nucleic acid-ligand complexes can be formed in which the ligand comprises a fusogenic viral peptide to disrupt endosomes, allowing the nucleic acid to avoid lysosomal degradation. In yet another embodiment, the nucleic acid can be targeted in vivo for cell specific uptake and expression, by targeting a specific receptor (see, e.g., PCT Publications WO 92/06180 dated April 16, 1992 (Wu et al.); WO 92/22635 dated December 23, 1992 (Wilson et al.); WO92/20316 dated November 26, 1992 (Findeis et al.); WO93/14188 dated July 22, 1993 (Clarke et al.), WO 93/20221 dated October 14,1993 (Young)). Alternatively, the nucleic acid can be introduced intracellularly and incorporated within host cell DNA for expression, by homologous recombination (Koller and Smithies, 1989, Proc. Natl. Acad. Sci. USA 86:8932-8935; Zijlstra et al., 1989, Nature 342:435-438).

In a specific embodiment, viral vectors that contains nucleic acid sequences encoding an antibody of the invention are used. For example, a retroviral vector can be used (see Miller et al., 1993, Meth. Enzymol. 217:581-599). These retroviral vectors have been to delete retroviral sequences that are not necessary for packaging of the viral genome and integration into host cell DNA. The nucleic acid sequences encoding the antibody to be used in gene therapy are cloned into one or more vectors, which facilitates delivery of the gene into a patient. More detail about retroviral vectors can be found in Boesen et al., 1994, Biotherapy 6:291-302, which describes the use of a retroviral vector to deliver the mdr1 gene to hematopoietic stem cells in order to make the stem cells more resistant to chemotherapy. Other references illustrating the use of retroviral vectors in gene therapy are: Clowes et al., 1994, J. Clin. Invest. 93:644-651; Kiem et al., 1994, Blood 83:1467-1473: Salmons and Gunzberg, 1993, Human Gene Therapy 4:129-141: and Grossman and Wilson. 1993, Curr. Opin. in Genetics and Devel. 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, 1993, Current Opinion in Genetics and Development 3:499-503 present a review of adenovirus-based gene therapy. Bout et al., 1994, Human Gene Therapy 5:3-10 demonstrated the use of adenovirus vectors to transfer genes to the respiratory epithelia of rhesus monkeys. Other instances of the use of adenoviruses in gene therapy can be found in Rosenfeld et al., 1991, Science 252:431-434; Rosenfeld et al., 1992, Cell 68:143- 155; Mastrangeli et al., 1993, J. Clin. Invest. 91:225-234; PCT Publication WO94/12649; and Wang, et al., 1995, Gene Therapy 2:775-783. In a preferred embodiment, adenovirus vectors are used.

Adeno-associated virus (AAV) has also been proposed for use in gene therapy (Walsh et al., 1993, Proc. Soc. Exp. Biol. Med. 204:289-300; U.S. Patent No. 5,436,146).

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a patient.

In this embodiment, the nucleic acid is introduced into a cell prior to administration in vivo of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, e.g., Loeffler and Behr, 1993, Meth. Enzymol. 217:599-618; Cohen et al., 1993, Meth. Enzymol. 217:618-644; Cline, 1985, Pharmac. Ther. 29: 69-92) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a patient by various methods known in the art. Recombinant blood cells (e.g., hematopoietic stem or progenitor cells) are preferably administered intravenously. The amount of cells envisioned for use depends on the desired effect, patient state, etc., and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as Tlymphocytes, Blymphocytes, monocytes, macrophages, neutrophils, eosinophils, megakaryocytes, granulocytes; various stem or progenitor cells, in particular hematopoietic stem or progenitor cells, e.g., as obtained from bone marrow, umbilical cord blood, peripheral blood, fetal liver, etc.

In a preferred embodiment, the cell used for gene therapy is autologous to the patient.

In an embodiment in which recombinant cells are used in gene therapy, nucleic acid sequences encoding an antibody are introduced into the cells such that they are expressible by the cells or their progeny, and the recombinant cells are then administered in vivo for therapeutic effect. In a specific embodiment, stem or progenitor cells are used. Any stem and/or progenitor cells which can be isolated and maintained in vitro can potentially be used in accordance with this embodiment of the present invention (see e.g. PCT Publication WO 94/08598, dated April 28, 1994; Stemple and Anderson, 1992, Cell 71:973-985; Rheinwald, 1980, Meth. Cell Bio. 21A:229; and Pittelkow and Scott, 1986, Mayo Clinic Proc. 61:771).

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

***Demonstration of Antibody-Based Therapeutic or Prophylactic Activity***

The compounds or pharmaceutical compositions of the invention are preferably tested in vitro, and then in vivo for the desired therapeutic or prophylactic activity, prior to use in humans. For example, in vitro assays to demonstrate the therapeutic or prophylactic utility of a compound or pharmaceutical composition include, the effect of a compound on a cell line or a patient tissue sample. The effect of the compound or composition on the cell line and/or tissue sample can be determined utilizing techniques known to those of skill in the art including, but not limited to, rosette formation assays and cell lysis assays. In accordance with the invention, in vitro assays which can be used to determine whether administration of a specific compound is indicated, include in vitro cell culture assays in which a patient tissue sample is grown in culture, and exposed to or otherwise administered a compound, and the effect of such compound upon the tissue sample is observed.

***Antibody-Based Therapeutic/Prophylactic Administration and Composition***

The invention provides methods of treatment, inhibition and prophylaxis by administration to a subject of an effective amount of a compound or pharmaceutical composition of the invention, preferably an antibody of the invention. In a preferred aspect, the compound is substantially purified (e.g., substantially free from substances that limit its effect or produce undesired side-effects). The subject is preferably an animal, including but not limited to animals such as cows, pigs, horses, chickens, cats, dogs, etc., and is preferably a mammal, and most preferably human.

Formulations and methods of administration that can be employed when the compound comprises a nucleic acid or an immunoglobulin are described above; additional appropriate formulations and routes of administration can be selected from among those described herein below.

Various delivery systems are known and can be used to administer a compound of the invention, e.g., encapsulation in liposomes, microparticles, microcapsules, recombinant cells capable of expressing the compound, receptor-mediated endocytosis (see, e.g., Wu and Wu, 1987, J. Biol. Chem. 262:4429-4432), construction of a nucleic acid as part of a retroviral or other vector, etc. Methods of introduction include but are not limited to intradermal, intramuscular, intraperitoneal, intravenous, subcutaneous, intranasal, epidural, and oral routes. The compounds or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the pharmaceutical compounds or compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compounds or compositions of the invention locally to the area in need of treatment; this may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. Preferably, when administering a protein, including an antibody, of the invention, care must be taken to use materials to which the protein does not absorb.

In another embodiment, the compound or composition can be delivered in a vesicle, in particular a liposome (see Langer, 1990, Science 249:1527-1533; Treat et al., in Liposomes in the Therapy of Infectious Disease and Cancer, Lopez-Berestein and Fidler (eds.), Liss, New York, pp. 353- 365 (1989); Lopez-Berestein, ibid., pp. 317-327; see generally ibid.)

In yet another embodiment, the compound or composition can be delivered in a controlled release system. In one embodiment, a pump may be used (see Langer, supra; Sefton, 1987, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J., 1983, Macromol. Sci. Rev. Macromol. Chem. 23:61; see also Levy et al., 1985, Science 228:190; During et al., 1989, Ann. Neurol. 25:351; Howard et al., 1989, J.Neurosurg. 71:105). In yet another embodiment, a controlled release system can be placed in proximity of the therapeutic target, i.e., the brain, thus requiring only a fraction of the systemic dose (see, e.g., Goodson, in Medical Applications of Controlled Release. supra. vol. 2, pp. 115-138 (1984)).

Other controlled release systems are discussed in the review by Langer (1990, Science 249:1527-1533).

In a specific embodiment where the compound of the invention is a nucleic acid encoding a protein, the nucleic acid can be administered in vivo to promote expression of its encoded protein, by constructing it as part of an appropriate nucleic acid expression vector and administering it so that it becomes intracellular, e.g., by use of a retroviral vector (see U.S. Patent No. 4,980,286), or by direct injection, or by use of microparticle bombardment (e.g., a gene gun; Biolistic, Dupont). or coating with lipids or cell-surface receptors or transfecting agents, or by administering it in linkage to a homeobox- like peptide which is known to enter the nucleus (see e.g., Joliot et al., 1991, Proc. Natl. Acad. Sci. USA 88: 1864-1868), etc. Alternatively, a nucleic acid can be introduced intracellularly and incorporated within host cell DNA for expression, by homologous recombination.

The present invention also provides pharmaceutical compositions. Such compositions comprise a therapeutically effective amount of a compound, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol. lactose, starch, magnesium stearate, sodium saccharine. cellulose, magnesium carbonate, etc. Examples of suitable pharmaceutical carriers are described in "Remington's Pharmaceutical Sciences" by E.W. Martin. Such compositions will contain a therapeutically effective amount of the compound, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The amount of the compound of the invention which will be effective in the treatment, inhibition and prevention of a disease or disorder associated with aberrant expression and/or activity of a polypeptide of the invention can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems.

For antibodies. the dosage administered to a patient is typically 0. 1 mg/kg to 100 mg/kg of the patient's body weight. Preferably, the dosage administered to a patient is between 0. 1 mg/kg and 20 mg/kg of the patient's body weight, more preferably I mg/kg to 10 mg/kg of the patient's body weight. Generally, human antibodies have a longer half-life within the human body than antibodies from other species due to the immune response to the foreign polypeptides. Thus, lower dosages of human antibodies and less frequent administration is often possible. Further, the dosage and frequency of administration of antibodies of the invention may be reduced by enhancing uptake and tissue penetration (e.g., into the brain) of the antibodies by modifications such as, for example, lipidation.

The invention also provides a pharmaceutical pack or kit comprising one or more containers filled with one or more of the ingredients of the pharmaceutical compositions of the invention. Optionally associated with such container (s) can be a notice in the form prescribed by a governmental agency regulating the manufacture, use or sale of pharmaceuticals or biological products, which notice reflects approval by the agency of manufacture, use or sale for human administration.

***Antibody-Based Diagnosis and Imaging***

Labeled antibodies, and derivatives and analogs thereof, which specifically bind to a polypeptide of interest can be used for diagnostic purposes to detect, diagnose, or monitor diseases and/or disorders associated with the aberrant expression and/or activity of a polypeptide of the invention. The invention provides for the detection of aberrant expression of a polypeptide of interest, comprising (a) assaying the expression of the polypeptide of interest in cells or body fluid of an individual using one or more antibodies specific to the polypeptide interest and (b) comparing the level of gene expression with a standard gene expression level, whereby an increase or decrease in the assayed polypeptide gene expression level compared to the standard expression level is indicative of aberrant expression.

The invention provides a diagnostic assay for diagnosising a disorder, comprising (a) assaying the expression of the polypeptide of interest in cells or body fluid of an individual using one or more antibodies specific to the polypeptide interest and (b) comparing the level of gene expression with a standard gene expression level, whereby an increase or decrease in the assayed polypeptide gene expression level compared to the standard expression level is indicative of a particular disorder. With respect to cancer, the presence of a relatively high amount of transcript in biopsied tissue from an individual may indicate a predisposition for the development of the disease, or may provide a means for detecting the disease prior to the appearance of actual clinical symptoms. A more definitive diagnosis of this type may allow health professionals to employ preventative measures or aggressive treatment earlier thereby preventing the development or further progression of the cancer.

Antibodies of the invention can be used to assay protein levels in a biological sample using classical immunohistological methods known to those of skill in the art (e.g., see Jalkanen, M., et al., J. Cell. Biol. 101:976-985 (1985); Jalkanen, M., et al., J. Cell . Biol. 105:3087-3096 (1987)). Other antibody-based methods useful for detecting protein gene expression include immunoassays, such as the enzyme linked immunosorbent assay (ELISA) and the radioimmunoassay (R1A). Suitable antibody assay labels are known in the art and include enzyme labels, such as, glucose oxidase; radioisotopes, such as iodine ($^{131}I$, $^{125}I$, $^{123}I$, $^{121}I$), carbon ($^{14}C$), sulfur ($^{35}S$), tritium ($^{3}H$), indium ($^{115m}In$, $^{113m}In$, $^{112}In$, $^{111}In$), and technetium ($^{99}Tc$, $^{99m}Tc$), thallium ($^{201}Ti$), gallium ($^{68}Ga$, $^{67}Ga$), palladium ($^{103}Pd$), molybdenum ($^{99}Mo$), xenon ($^{133}Xe$), fluorine ($^{18}F$), $^{153}Sm$, $^{177}Lu$, $^{159}Gd$, $^{149}Pm$, $^{140}La$, $^{175}Yb$, $^{166}Ho$, $^{90}Y$, $^{47}SC$, $^{186}Re$, $^{188}Re$, $^{142}Pr$, $^{105}Rh$, $^{97}Ru$; luminescent labels, such as luminol; and fluorescent labels, such as fluorescein and rhodamine, and biotin.

One aspect of the invention is the detection and diagnosis of a disease or disorder associated with aberrant expression of a polypeptide of the interest in an animal, preferably a mammal and most preferably a human. In one embodiment, diagnosis comprises: a) administering (for example, parenterally, subcutaneously, or intraperitoneally) to a subject an effective amount of a labeled molecule which specifically binds to the polypeptide of interest; b) waiting for a time interval following the administering for permitting the labeled molecule to preferentially concentrate at sites in the subject where the polypeptide is expressed (and for unbound labeled molecule to be cleared to background level); c) determining background level; and d) detecting the labeled molecule in the subject, such that detection of labeled molecule above the background level indicates that the subject has a particular disease or disorder associated with aberrant expression of the polypeptide of interest. Background level can be determined by various methods including, comparing the amount of labeled molecule detected to a standard value previously determined for a particular system.

It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds.. Masson Publishing Inc. (1982).

Depending on several variables, including the type of label used and the mode of administration, the time interval following the administration for permitting the labeled molecule to preferentially concentrate at sites in the subject and for unbound labeled molecule to be cleared to background level is 6 to 48 hours or 6 to 24 hours or 6 to 12 hours. In another embodiment the time interval following administration is 5 to 20 days or 5 to 10 days.

In an embodiment, monitoring of the disease or disorder is carried out by repeating the method for diagnosing the disease or disease, for example, one month after initial diagnosis, six months after initial diagnosis, one year after initial diagnosis, etc.

Presence of the labeled molecule can be detected in the patient using methods known in the art for in vivo scanning. These methods depend upon the type of label used. Skilled artisans will be able to determine the appropriate method for detecting a particular label. Methods and devices that may be used in the diagnostic methods of the invention include, but are not limited to, computed tomography (CT), whole body scan such as position emission tomography (PET), magnetic resonance imaging (MRI), and sonography.

In a specific embodiment, the molecule is labeled with a radioisotope and is detected in the patient using a radiation responsive surgical instrument (Thurston et al., U.S. Patent No. 5,441,050). In another embodiment, the molecule is labeled with a fluorescent compound and is detected in the patient using a fluorescence responsive scanning instrument. In another embodiment, the molecule is labeled with a positron emitting metal and is detected in the patent using positron emission-tomography. In yet another embodiment, the molecule is labeled with a paramagnetic label and is detected in a patient using magnetic resonance imaging (MRI).

### *Antibody-Based Kits*

The present invention provides kits that can be used in the above methods. In one embodiment, a kit comprises an antibody of the invention, preferably a purified antibody, in one or more containers. In a specific embodiment, the kits of the present invention contain a substantially isolated polypeptide comprising an epitope which is specifically immunoreactive with an antibody included in the kit. Preferably, the kits of the present invention further comprise a control antibody which does not react with the polypeptide of interest. In another specific embodiment, the kits of the present invention contain a means for detecting the binding of an antibody to a polypeptide of interest (e.g., the antibody may be conjugated to a detectable substrate such as a fluorescent compound, an enzymatic substrate, a radioactive compound or a luminescent compound, or a second antibody which recognizes the first antibody may be conjugated to a detectable substrate).

In another specific embodiment of the present invention, the kit is a diagnostic kit for use in screening serum containing antibodies specific against proliferative and/or cancerous polynucleotides and polypeptides. Such a kit may include a control antibody that does not react with the polypeptide of interest. Such a kit may include a substantially isolated polypeptide antigen comprising an epitope which is specifically immunoreactive with at least one anti-polypeptide antigen antibody. Further, such a kit includes means for detecting the binding of said antibody to the antigen (e.g., the antibody may be conjugated to a fluorescent compound such as fluorescein or rhodamine which can be detected by flow cytometry). In specific embodiments, the kit may include a recombinantly produced or chemically synthesized polypeptide antigen. The polypeptide antigen of the kit may also be attached to a solid support.

In a more specific embodiment the detecting means of the above-described kit includes a solid support to which said polypeptide antigen is attached. Such a kit may also include a non-attached reporter-labeled anti-human antibody. In this embodiment, binding of the antibody to the polypeptide antigen can be detected by binding of the said reporter-labeled antibody.

In an additional embodiment, the invention includes a diagnostic kit for use in screening serum containing antigens of the polypeptide of the invention. The diagnostic kit includes a substantially isolated antibody specifically immunoreactive with polypeptide or polynucleotide antigens, and means for detecting the binding of the polynucleotide or polypeptide antigen to the antibody. In one embodiment, the antibody is attached to a solid support. In a specific embodiment, the antibody may be a monoclonal antibody. The detecting means of the kit may include a second, labeled monoclonal antibody. Alternatively, or in addition, the detecting means may include a labeled, competing antigen.

In one diagnostic configuration, test serum is reacted with a solid phase reagent having a surface-bound antigen obtained by the methods of the present invention. After binding with specific antigen antibody to the reagent and removing unbound serum components by washing, the reagent is reacted with reporter-labeled anti-human antibody to bind reporter to the reagent in proportion to the amount of bound anti-antigen antibody on the solid support. The reagent is again washed to remove unbound labeled antibody, and the amount of reporter associated with the reagent is determined. Typically, the reporter is an enzyme which is detected by incubating the solid phase in the presence of a suitable fluorometric, luminescent or colorimetric substrate (Sigma, St. Louis, MO).

The solid surface reagent in the above assay is prepared by known techniques for attaching protein material to solid support material, such as polymeric beads, dip sticks, 96-well plate or filter material. These attachment methods generally include non-specific adsorption of the protein to the support or covalent attachment of the protein, typically through a free amine group, to a chemically reactive group on the solid support, such as an activated carboxyl, hydroxyl, or aldehyde group. Alternatively, streptavidin coated plates can be used in conjunction with biotinylated antigen(s).

Thus, the invention provides an assay system or kit for carrying out this diagnostic method. The kit generally includes a support with surface- bound recombinant antigens, and a reporter-labeled anti-human antibody for detecting surface-bound anti-antigen antibody.

***Therapeutic Compositions and Methods***

The Tumor Necrosis Factor (TNF) family ligands are known to be among the most pleiotropic cytokines, inducing a large number of cellular responses, including cytotoxicity, anti-viral activity, immunoregulatory activities, and the transcriptional regulation of several genes (D.V. Goeddel *et al.,* "Tumor Necrosis Factors: Gene Structure and Biological Activities," *Symp. Quant. Biol. 51*:597- 609 (1986), Cold Spring Harbor; B. Beutler and A. Cerami, *Annu. Rev. Biochem. 57*:505-518 (1988); L.J. Old, *Sci. Am. 258*:59-75 (1988); W. Fiers, *FEBS Lett. 285*:199-224 (1991)). The TNF-family ligands induce such various cellular responses by binding to TNF-family receptors, including the TR10 of the present invention.

TR10 polynucleotides, polypeptides, agonists and/or antagonists of the invention may be administered to a patient (e.g., mammal, preferably human) afflicted with any disease or disorder mediated (directly or indirectly) by defective, or deficient levels of, TRIO. Alternatively, a gene therapy approach may be applied to treat such diseases or disorders. In one embodiment of the invention. TR10 polynucleotide sequences are used to detect mutein TR10 genes, including defective genes. Mutein genes may be identified in *in vitro* diagnostic assays, and by comparison of the TR 10 nucleotide sequence disclosed herein with that of a TR10 gene obtained from a patient suspected of harboring a defect in this gene. Defective genes may be replaced with normal TR10-encoding genes using techniques known to one skilled in the art.

In another embodiment, the TR10 polypeptides, polynucleotides, agonists and/or antagonists of the present invention are used as research tools for studying the phenotypic effects that result from inhibiting TRAIL/TR10 interactions on various cell types. TR10 polypeptides and antagonists (e.g. monoclonal antibodies to TR10) also may be used in in vitro assays for detecting TRAIL or TR10 or the interactions thereof.

The therapeutic compositions and methods described in this section include those antibody-based composition and methods described in detail above. For example, the agonists and antagonists, and methods of using such agonists and antagoists, include the antibodies and their uses described above.

It has been reported that certain ligands of the TNF family (of which TRAIL is a member) bind to more than one distinct cell surface receptor protein. For example, a receptor protein designated DR4 reportedly binds TRAIL, but is distinct from the TR10 of the present invention (Pan *et al., Science 276*:111-113, (1997); hereby incorporated by reference). In another embodiment, a purified TR10 polypeptide, agonist and/or antagonist is used to inhibit binding of TRAIL to endogenous cell surface TR10. By competing for TRAIL binding, soluble TR10 polypeptides of the present invention may be employed to inhibit the interaction of TRAIL not only with cell surface TR10, but also with TRAIL receptor proteins distinct from TR10. Thus, in a further embodiment, TR10 polynucleotides, polypeptides, agonists and/or antagonists of the invention are used to inhibit a functional activity of TRAIL, in *in vitro* or in vivo procedures. By inhibiting binding of TRAIL to cell surface receptors, TR10 also inhibits biological effects that result from the binding of TRAIL to endogenous receptors. Various forms of TR10 may be employed, including, for example, the above-described TR10 fragments, derivatives, and variants that are capable of binding TRAIL. In a preferred embodiment, a soluble TR10, is employed to inhibit a functional activity of TRAIL, e.g., to inhibit TRAIL-mediated apoptosis of cells susceptible to such apoptosis. Thus, in an additional embodiment, TR10 is administered to a mammal (e.g., a human) to treat a TRAIL-mediated disorder. Such TRAIL-mediated disorders include conditions caused (directly or indirectly) or exacerbated by TRAIL.

Cells which express the TR10 polypeptide and are believed to have a potent cellular response to TR10 ligands include fetal liver, PBL, lung, kidney, small intestine, colon, keratinocytes, endothelial cells, and monocyte activated tissue. By "a cellular response to a TNF-family ligand" is intended any genotypic, phenotypic, and/or morphologic change to a cell, cell line, tissue, tissue culture or patient that is induced by a TNF-family ligand. As indicated, such cellular responses include not only normal physiological responses to TNF-family ligands, but also diseases associated with increased apoptosis or the inhibition of apoptosis. Apoptosis-programmed cell death-is a physiological mechanism involved in the deletion of peripheral T lymphocytes of the immune system and its dysregulation can lead to a number of different pathogenic processes (J.C. Ameisen, *AIDS 8*:1197-1213 (1994); P.H. *Krammer et al., Curr. Opin. Immunol. 6*:279-289 (1994)).

Diseases associated with increased cell survival, or the inhibition of apoptosis, and that may be treated or prevented by the polynucleotides, polypeptides and/or agonists or antagonists of the invention include, but are not limited to, cancers (such as follicular lymphomas, carcinomas with p53 mutations, and hormone-dependent tumors, including, but not limited to colon cancer, cardiac tumors, pancreatic cancer, melanoma, retinoblastoma, glioblastoma, lung cancer, intestinal cancer, testicular cancer, stomach cancer, neuroblastoma, myxoma, myoma, lymphoma, endothelioma, osteoblastoma, osteoclastoma, osteosarcoma, chondrosarcoma, adenoma, breast cancer, prostrate cancer, Kaposi's sarcoma and ovarian cancer); autoimmune disorders (such as, multiple sclerosis, Sjogren's syndrome, Hashimoto's thyroiditis, biliary cirrhosis, Behcet's disease, Crohn's disease, polymyositis, systemic lupus erythematosus and immune-related glomerulonephritis rheumatoid arthritis); viral infections (such as herpes viruses, pox viruses and adenoviruses); inflammation; graft vs. host disease; acute graft rejection and chronic graft rejection. In preferred embodiments, TR10 polynucleotides, polypeptides, and/or antagonists of the invention are used to inhibit growth, progression, and/or metasis of cancers, in particular those listed above, or in the paragraph that follows.

Additional diseases or conditions associated with increased cell survival and that may be treated or prevented by the polynucleotides, polypeptides and/or agonists or antagonists of the invention include, but are not limited to, progression, and/or metastases of malignancies and related disorders such as leukemia (including acute leukemias (e.g., acute lymphocytic leukemia, acute myelocytic leukemia (including myeloblastic, promyelocytic, myelomonocytic, monocytic, and erythroleukemia)) and chronic leukemias (e.g., chronic myelocytic (granulocytic) leukemia and chronic lymphocytic leukemia)), polycythemia vera, lymphomas (e.g., Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma, and retinoblastoma.

Thus, in preferred embodiments TR10 polynucleotides or polypeptides of the invention and agonists or antagonists thereof, are used to treat or prevent autoimmune diseases and/or inhibit the growth, progression, and/or metastasis of cancers, including, but not limited to, those cancers disclosed herein, such as, for example, lymphocytic leukemias (including, for example, MLL and chronic lymphocytic leukemia (CLL)) and follicular lymphomas. In another embodiment TR10 polynucleotides or polypeptides of the invention and/or agonists or antagonists thereof, are used to activate, differentiate or proliferate cancerous cells or tissue (e.g., B cell lineage related cancers (e.g., CLL and MLL), lymphocytic leukemia, or lymphoma) and thereby render the cells more vulnerable to cancer therapy (e.g., chemotherapy or radiation therapy).

Diseases associated with increased apoptosis and that may be treated or prevented by the polynucleotides, polypeptides and/or agonists or antagonists of the invention include, but are not limited to, AIDS; neurodegenerative disorders (such as Alzheimer's disease, Parkinson's disease, Amyotrophic lateral sclerosis, Retinitis pigmentosa, Cerebellar degeneration and brain tumor or prior associated disease); autoimmune disorders (such as, multiple sclerosis, Sjogren's syndrome, Hashimoto's thyroiditis, biliary cirrhosis, Behcet's disease, Crohn's disease, polymyositis, systemic lupus erythematosus and immune-related glomerulonephritis and rheumatoid arthritis); myelodysplastic syndromes (such as aplastic anemia), graft v. host disease, ischemic injury (such as that caused by myocardial infarction, stroke and reperfusion injury), liver injury (such as hepatitis related liver injury, ischemia/reperfusion injury, cholestosis (bile duct injury) and liver cancer); toxin-induced liver disease (such as that caused by alcohol), septic shock, cachexia and anorexia. In preferred embodiments, TR10 polynucleotides, polypeptides and/or agonists are used to treat the diseases and disorders listed above.

Many of the pathologies associated with HIV are mediated by apoptosis, including HIV-induced nephropathy and HIV encephalitis. Thus, in additional preferred embodiments, TR10 polynucleotides, polypeptides, and/or TR10 agonists or antagonists of the invention are used to treat AIDS and pathologies associated with AIDS.

Another embodiment of the present invention is directed to the use of TR10 to reduce TRAIL-mediated death of T cells in HIV-infected patients. The state of immunodeficiency that defines AIDS is secondary to a decrease in the number and function of $CD4^-$ T-lymphocytes. Recent reports estimate the daily loss of $CD4^+$ T cells to be between 3.5 x $10^7$ and 2 x $10^9$ cells (Wei *et al., Nature 373*:117-122 (1995)). One cause of $CD4^+$ T cell depletion in the setting of HIV infection is believed to be HIV-induced apoptosis (see, for example, Meyaard *et al., Science 257*:217-219, 1992; Groux *et al., J Exp. Med., 175*:331, 1992; and Oyaizu et al., in *Cell Activation and Apoptosis in HIV Infection,* Andrieu and Lu, Eds., Plenum Press, New York, 1995, pp. 101-1 14). Indeed, HIV-induced apoptotic cell death has been demonstrated not only *in vitro* but also, more importantly, in infected individuals (J.C. Ameisen, *AIDS 8*:1197-1213 (1994); T.H. Finkel and N.K. Banda, *Curr. Opin. Immunol. 6*:605-615(1995); C.A. Muro-Cacho *et al., J. Immunol.* 154:5555-5566 (1995)). Furthermore, apoptosis and $CD4^+$ T-lymphocyte depletion is tightly correlated in different animal models of AIDS (T. *Brunner et al., Nature 373*:441-444 (1995); M.L. Gougeon *et al., AIDS Res. Hum. Retroviruses 9*:553-563 (1993)) and, apoptosis is not observed in those animal models in which viral replication does not result in AIDS. *Id.*

Further data indicates that uninfected but primed or activated T lymphocytes from HIV-infected individuals undergo apoptosis after encountering the TNF-family ligand FasL. Using monocytic cell lines that result in death following HIV infection, it has been demonstrated that infection of U937 cells with HIV results in the *de novo* expression of FasL and that FasL mediates HIV-induced apoptosis (A.D. Badley *et al., J. Virol. 70:* 199-206 (1996)). Further, the TNF-family ligand was detectable in uninfected macrophages and its expression was upregulated following HIV infection resulting in selective killing of uninfected CD4T-lymphocytes. *Id.* Thus, by the invention, a method for treating $HIV^+$ individuals is provided which involves administering TR10 and/or TR10 agonists of the present invention to reduce selective killing of $CD4^+$ T-lymphocytes. Modes of administration and dosages are discussed in detail below.

Activated human T cells are induced to undergo programmed cell death (apoptosis) upon triggering through the CD3/T cell receptor complex, a process termed activated-induced cell death (AICD). AICD of $CD4^+$ T cells isolated from HIV-Infected asymptomatic individuals has been reported (Groux et al., *supra*). Thus, AICD may play a role in the depletion of $CD4^+$ T cells and the progression to AIDS in HIV-infected individuals. Thus, the present invention provides a method of inhibiting TRAIL-mediated T cell death in HIV patients, comprising administering a TR10 polypeptide of the invention (preferably, a soluble TR10 polypeptide) to the patients. In one embodiment, the patient is asymptomatic when treatment with TR10 commences. If desired, prior to treatment, peripheral blood T cells may be extracted from an HIV patient, and tested for susceptibility to TRAIL-mediated cell death by procedures known in the art. In one embodiment, a patient's blood or plasma is contacted with TR10 *ex* vivo. The TR10 may be bound to a suitable chromatography matrix by procedures known in the art. The patient's blood or plasma flows through a chromatography column containing TR10 bound to the matrix, before being returned to the patient. The immobilized TR10 binds TRAIL, thus removing TRAIL protein from the patient's blood.

In additional embodiments a TR10 polypeptide of the invention is administered in combination with other inhibitors of T cell apoptosis. For example, Fas-mediated apoptosis also has been implicated in loss of T cells in HIV individuals (Katsikis *et al., J. Exp. Med. 181*:2029-2036 (1995)). Thus, a patient susceptible to both Fas ligand mediated and TRAIL mediated T cell death may be treated with both an agent that blocks TRAIL/TRAIL receptor interactions and an agent that blocks Fas-ligand/Fas interactions. Suitable agents for blocking binding of Fas-ligand to Fas include, but are not limited to, soluble Fas polypeptides; mulitmeric forms of soluble Fas polypeptides (e.g., dimers of sFas/Fc); anti-Fas antibodies that bind Fas without transducing the biological signal that results in apoptosis; anti-Fas-ligand antibodies that block binding of Fas-ligand to Fas; and muteins of Fas-ligand that bind Fas but do not transduce the biological signal that results in apoptosis. Preferably, the antibodies employed according to this method are monoclonal antibodies. Examples of suitable agents for blocking Fas-ligand/Fas interactions, including blocking anti-Fas monoclonal antibodies, are described in International application publication number WO95/10540, hereby incorporated by reference.

Suitable agents, which also block binding of TRAIL to a TRAIL receptor that may be administered with the polynucleotides and/or polypeptides of the present invention include, but are not limited to, soluble TRAIL receptor polypeptides (e.g., a soluble form of OPG, DR4 (International application publication number WO 98/32856); TR5 (International application publication number WO 98/30693); and DR5 (International application publication number WO 98/41629)); multimeric forms of soluble TRAIL receptor polypeptides; and TRAIL receptor antibodies that bind the TRAIL receptor without transducing the biological signal that results in apoptosis, anti-TRAIL antibodies that block binding of TRAIL to one or more TRAIL receptors, and muteins of TRAIL that bind TRAIL receptors but do not transduce the biological signal that results in apoptosis. Preferably, the antibodies employed according to this method are monoclonal antibodies.

TR10 polypeptides or polynucleotides encoding TR10 of the invention may be used to treat cardiovascular disorders, including peripheral artery disease, such as limb ischemia.

Cardiovascular disorders include cardiovascular abnormalities, such as arterio-arterial fistula, arteriovenous fistula, cerebral arteriovenous malformations, congenital heart defects, pulmonary atresia, and Scimitar Syndrome. Congenital heart defects include aortic coarctation, cor triatriatum, coronary vessel anomalies, crisscross heart. dextrocardia, patent ductus arteriosus, Ebstein's anomaly, Eisenmenger complex, hypoplastic left heart syndrome, levocardia, tetralogy of fallot, transposition of great vessels. double outlet right ventricle, tricuspid atresia, persistent truncus arteriosus, and heart septal defects, such as aortopulmonary septal defect, endocardial cushion defects. Lutembacher's Syndrome, trilogy of Fallot, ventricular heart septal defects, and conditions characterized by clotting of small blood vessels.

Cardiovascular disorders also include heart disease, such as arrhythmias, carcinoid heart disease, high cardiac output, low cardiac output, cardiac tamponade, endocarditis (including bacterial), heart aneurysm, cardiac arrest, congestive heart failure, congestive cardiomyopathy, paroxysmal dyspnea, cardiac edema, heart hypertrophy, congestive cardiomyopathy, left ventricular hypertrophy, right ventricular hypertrophy, post-infarction heart rupture, ventricular septal rupture, heart valve diseases, myocardial diseases, myocardial ischemia, pericardial effusion, pericarditis (including constrictive and tuberculous), pneumopericardium, postpericardiotomy syndrome, pulmonary heart disease, rheumatic heart disease, ventricular dysfunction, hyperemia, cardiovascular pregnancy complications, Scimitar Syndrome, cardiovascular syphilis, and cardiovascular tuberculosis.

Arrhythmias include sinus arrhythmia, atrial fibrillation, atrial flutter, bradycardia, extrasystole, Adams-Stokes Syndrome, bundle-branch block, sinoatrial block, long QT syndrome, parasystole. Lown-Ganong-Levine Syndrome, Mahaim-type pre-excitation syndrome, Wolff-Parkinson-White syndrome, sick sinus syndrome, tachycardias, and ventricular fibrillation. Tachycardias include paroxysmal tachycardia, supraventricular tachycardia, accelerated idioventricular rhythm, atrioventricular nodal reentry tachycardia, ectopic atrial tachycardia, ectopic junctional tachycardia, sinoatrial nodal reentry tachycardia, sinus tachycardia, Torsades de Pointes, and ventricular tachycardia.

Heart valve disease include aortic valve insufficiency, aortic valve stenosis, hear murmurs, aortic valve prolapse, mitral valve prolapse, tricuspid valve prolapse, mitral valve insufficiency, mitral valve stenosis, pulmonary atresia, pulmonary valve insufficiency, pulmonary valve stenosis, tricuspid atresia, tricuspid valve insufficiency, and tricuspid valve stenosis.

Myocardial diseases include alcoholic cardiomyopathy, congestive cardiomyopathy, hypertrophic cardiomyopathy, aortic subvalvular stenosis, pulmonary subvalvular stenosis, restrictive cardiomyopathy, Chagas cardiomyopathy, endocardial fibroelastosis, endomyocardial fibrosis, Kearns Syndrome, myocardial reperfusion injury, and myocarditis.

Myocardial ischemias include coronary disease, such as angina pectoris, coronary aneurysm, coronary arteriosclerosis, coronary thrombosis, coronary vasospasm, myocardial infarction and myocardial stunning.

Cardiovascular diseases also include vascular diseases such as aneurysms, angiodysplasia, angiomatosis, bacillary angiomatosis. Hippel-Lindau Disease, Klippel-Trenaunay-Weber Syndrome, Sturge-Weber Syndrome, angioneurotic edema, aortic diseases, Takayasu's Arteritis, aortitis, Leriche's Syndrome, arterial occlusive diseases, arteritis, enarteritis, polyarteritis nodosa, cerebrovascular disorders, diabetic angiopathies, diabetic retinopathy, embolisms, thrombosis, erythromelalgia, hemorrhoids, hepatic veno-occlusive disease, hypertension, hypotension, ischemia, peripheral vascular diseases, phlebitis, pulmonary veno-occlusive disease. Raynaud's disease, CREST syndrome, retinal vein occlusion, Scimitar syndrome, superior vena cava syndrome, telangiectasia, atacia telangiectasia, hereditary hemorrhagic telanciectasia, varicocele, varicose veins, varicose ulcer, vasculitis, thrombotic microangiopathies (e.g., thrombotic thrombocytopenic purpura (TTP) and hemolytic-uremic syndrome (HUS)), and venous insufficiency.

Aneurysms include dissecting aneurysms, false aneurysms, infected aneurysms, ruptured aneurysms, aortic aneurysms, cerebral aneurysms, coronary aneurysms, heart aneurysms, and iliac aneurysms.

Arterial occlusive diseases include arteriosclerosis, intermittent claudication, carotid stenosis, fibromuscular dysplasias, mesenteric vascular occlusion, Moyamoya disease, renal artery obstruction, retinal artery occlusion, and thromboangiitis obliterans.

Cerebrovascular disorders include carotid artery diseases, cerebral amyloid angiopathy, cerebral aneurysm, cerebral anoxia, cerebral arteriosclerosis, cerebral arteriovenous malformation, cerebral artery diseases, cerebral embolism and thrombosis, carotid artery thrombosis, sinus thrombosis, Wallenberg's syndrome, cerebral hemorrhage, epidural hematoma, subdural hematoma, subaraxhnoid hemorrhage, cerebral infarction, cerebral ischemia (including transient), subclavian steal syndrome, periventricular leukomalacia, vascular headache, cluster headache, migraine, and vertebrobasilar insufficiency.

Embolisms include air embolisms, amniotic fluid embolisms, cholesterol embolisms, blue toe syndrome, fat embolisms, pulmonary embolisms, and thromoboembolisms. Thrombosis include coronary thrombosis, hepatic vein thrombosis, retinal vein occlusion, carotid artery thrombosis, sinus thrombosis, Wallenberg's syndrome, and thrombophlebitis.

Ischemia includes cerebral ischemia, ischemic colitis, compartment syndromes, anterior compartment syndrome, myocardial ischemia, reperfusion injuries, and peripheral limb ischemia. Vasculitis includes aortitis, arteritis, Behcet's Syndrome, Churg-Strauss Syndrome, mucocutaneous lymph node syndrome, thromboangiitis obliterans, hypersensitivity vasculitis, Schoenlein-Henoch purpura, allergic cutaneous vasculitis, and Wegener's granulomatosis.

The naturally occurring balance between endogenous stimulators and inhibitors of angiogenesis is one in which inhibitory influences predominate. Rastinejad *et al., Cell* 56:345-355 (1989). In those rare instances in which neovascularization occurs under normal physiological conditions, such as wound healing, organ regeneration, embryonic development, and female reproductive processes, angiogenesis is stringently regulated and spatially and temporally delimited. Under conditions of pathological angiogenesis such as that characterizing solid tumor growth, these regulatory controls fail. Unregulated angiogenesis becomes pathologic and sustains progression of many neoplastic and non-neoplastic diseases. A number of serious diseases are dominated by abnormal neovascularization including solid tumor growth and metastases, arthritis, some types of eye disorders, and psoriasis. See, e.g., reviews by Moses *et al., Biotech. 9*:630-634 (1991). Folkman *et al., N. Engl. J. Med.. 333*:1757-1763 ( 1995); *Auerbach et al., J. Microvasc. Res. 29*: 401-411 (1985); Folkman. Advances in Cancer Research, eds. Klein and Weinhouse, Academic Press, New York, pp. 175-203 (1985); Patz, *Am. J. Opthalmol. 94*:715-743 (1982): and Folkman *et al., Science 221*:719-725 (1983). In a number of pathological conditions, the process of angiogenesis contributes to the disease state. For example, significant data have accumulated which suggest that the growth of solid tumors is dependent on angiogenesis. Folkman and Klagsbrun, *Science 235*:442-447 (1987).

The present invention provides for treatment of diseases or disorders associated with neovascularization by administration of the TR10 polynucleotides and/or polypeptides of the invention (including TR10 agonists and/or antagonists). Malignant and metastatic conditions which can be treated with the polynucleotides and polypeptides of the invention include, but are not limited to those malignancies, solid tumors, and cancers described herein and otherwise known in the art (for a review of such disorders, see Fishman et al., Medicine, 2d Ed., J. B. Lippincott Co., Philadelphia (1985)).

Additionally, ocular disorders associated with neovascularization which can be treated with the TR10 polynucleotides and polypeptides of the present invention (including TR10 agonists and TR10 antagonists) include, but are not limited to: neovascular glaucoma, diabetic retinopathy, retinoblastoma, retrolental fibroplasia, uveitis, retinopathy of prematurity macular degeneration, corneal graft neovascularization, as well as other eye inflammatory diseases, ocular tumors and diseases associated with choroidal or iris neovascularization. See, e.g., reviews by Waltman *et al., Am. J. Ophthal. 85*:704-710 (1978) and Gartner *et al., Surv. Ophthal. 22*:291-312 (1978).

Additionally, disorders which can be treated with the TR10 polynucleotides and polypeptides of the present invention (including TR10 agonists and TR10 antagonists) include, but are not limited to, hemangioma, arthritis, psoriasis, angiofibroma, atherosclerotic plaques, delayed wound healing, granulations, hemophilic joints, hypertrophic scars, nonunion fractures, Osler-Weber syndrome, pyogenic granuloma, scleroderma, trachoma, and vascular adhesions.

The polynucleotides and/or polypeptides of the invention and/or agonists and/or antagonists thereof, can also be employed to inhibit the proliferation and differentiation of hematopoietic cells and therefore may be employed to protect bone marrow stem cells from chemotherapeutic agents during chemotherapy. This antiproliferative effect may allow administration of higher doses of chemotherapeutic agents and, therefore, more effective chemotherapeutic treatment.

The polynucleotides and/or polypeptides of the invention and/or agonists and/or antagonists thereof, may also be employed for the expansion of immature hematopoeitic progenitor cells, for example, granulocytes, macrophages or monocytes (e.g., C-kit+, Sca-1+), by temporarily preventing their differentiation. These bone marrow cells may be cultured *in vitro.* Thus, TR10 may be useful as a modulator of hematopoietic stem cells *in vitro* for the purpose of bone marrow transplantation and/or gene therapy. Since stem cells are rare and are most useful for introducing genes into for gene therapy, TR10 can be used to isolate enriched populations of stem cells. Stem cells can be enriched by culturing cells in the presence of cytotoxins, such as 5-Fu, which kills rapidly dividing cells, where as the stem cells will be protected by TR10. These stem cells can be returned to a bone marrow transplant patient or can then be used for transfection of the desired gene for gene therapy. In addition, TR10 can be injected into animals which results in the release of stem cells from the bone marrow of the animal into the peripheral blood. These stem cells can be isolated for the purpose of autologous bone marrow transplantation or manipulation for gene therapy. After the patient has finished chemotherapy or radiation treatment, the isolated stem cells can be returned to the patient.

In a specific embodiment, polynucleotides and/or polypeptides of the invention and/or angonists and/or antagonists thereof may be used to increase the concentration of blood cells in individuals in need of such increase (i.e., in hematopoietin therapy). Conditions that may be ameliorated by administering the compositions of the invention include, but are not limited to, neutropenia, anemia, and thrombocytopenia.

In a specific embodiment, the polynucleotides and/or polypeptides of the invention (and/or agonists or antagonists thereof) are used in erythropoietin therapy, which is directed toward supplementing the oxygen carrying capacity of blood. Polynucleotides and/or polypeptides of the invention (and/or agonists or antagonists thereof) may be used to treat or prevent diseases or conditions in patients generally requiring blood transfusions, such as, for example, trauma victims, surgical patients, dialysis patients, and patients with a variety of blood composition-affecting disorders, such as, for example, hemophilia, cystic fibrosis, pregnancy, menstrual disorders, early anemia of prematurity, spinal cord injury, aging, various neoplastic disease states, and the like. Examples of patient conditions that require supplementation of the oxygen carrying capacity of blood and which are within the scope of this invention, include, but are not limited to: treatment of blood disorders characterized by low or defective red blood cell production, anemia associated with chronic renal failure, stimulation of reticulocyte response, development of ferrokinetic effects (such as plasma iron turnover effects and marrow transit time effects), erythrocyte mass changes, stimulation of hemoglobin C synthesis, and increasing levels of hematocrit in vertebrates. The invention also provides for treatment to enhance the oxyoen-carrying capacity of an individual, such as for example, an individual encountering hypoxic environmental conditions.

Polynucleotides and/or polypeptides of the invention and/or angonists and/or antagonists thereof may be used in treatment of myeloid leukemias.

TR10 polynucleotides or polypeptides, or agonists of TR10, can be used in the treatment of infectious agents. For example, by increasing the immune response, particularly increasing the proliferation and differentiation of B cells, infectious diseases may be treated. The immune response may be increased by either enhancing an existing immune response, or by initiating a new immune response. Alternatively, TR10 polynucleotides or polypeptides, or agonists or antagonists of TR10, may also directly inhibit the infectious agent, without necessarily eliciting an immune response.

Viruses are one example of an infectious agent that can cause disease or symptoms that can be treated by TR10 polynucleotides or polypeptides, or agonists of TR10. Examples of viruses, include, but are not limited to the following DNA and RNA viruses and viral families: Arbovirus, Adenoviridae, Arenaviridae, Arterivirus, Birnaviridae, Bunyaviridae, Caliciviridae, Circoviridae, Coronaviridae, Dengue, EBV, HIV, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza A, Influenza B, and parainfluenza), Papiloma virus, Papovaviridae, Parvoviridae, Picornaviridae, Poxviridae (such as Smallpox or Vaccinia), Reoviridae (e.g., Rotavirus), Retroviridae (HTLVI, HTLV-II, Lentivirus), and Togaviridae (e.g., Rubivirus). Viruses falling within these families can cause a variety of diseases or symptoms, including, but not limited to: arthritis, bronchiollitis, respiratory syncytial virus, encephalitis, eye infections (e.g., conjunctivitis, keratitis), chronic fatigue syndrome, hepatitis (A, B, C, E, Chronic Active, Delta), Japanese B encephalitis, Junin, Chikungunya, Rift Valley fever, yellow fever, meningitis, opportunistic infections (e.g., AIDS), pneumonia, Burkitt's Lymphoma, chickenpox, hemorrhagic fever, Measles, Mumps, Parainfluenza, Rabies, the common cold, Polio, leukemia, Rubella, sexually transmitted diseases, skin diseases (e.g., Kaposi's, warts), and viremia. TR10 polynucleotides or polypeptides, or agonists or antagonists of TR10, can be used to treat or detect any of these symptoms or diseases. In specific embodiments, TR10 polynucleotides, polypeptides, or agonists are used to treat: meningitis, Dengue, EBV, and/or hepatitis (e.g., hepatitis B). In an additional specific embodiment TR10 polynucleotides, polypeptides, or agonists are used to treat patients nonresponsive to one or more other commercially available hepatitis vaccines. In a further specific embodiment, TR10 polynucleotides, polypeptides, or agonists are used to treat AIDS.

Similarly, bacterial or fungal agents that can cause disease or symptoms and that can be treated by TR10 polynucleotides or polypeptides, or agonists or antagonists of TR10, include, but not limited to, the following Gram-Negative and Gram-positive bacteria and bacterial families and fungi: Actinomycetales (e.g., Corynebacterium, Mycobacterium, Norcardia), Cryptococcus neoformans, Aspergillosis, Bacillaceae (e.g., Anthrax, Clostridium), Bacteroidaceae, Blastomycosis, Bordetella, Borrelia (e.g., Borrelia burgdorferi, Brucellosis, Candidiasis. Campylobacter, Coccidioidomycosis, Cryptococcosis, Dermatocycoses, E. coli (e.g., Enterotoxigenic E. coli and Enterohemorrhagic E. coli), Enterobacteriaceae (Klebsiella, Salmonella (e.g., Salmonella typhi, and Salmonella paratyphi), Serratia, Yersinia), Erysipelothrix, Helicobacter, Legionellosis, Leptospirosis, Listeria, Mycoplasmatales, Mycobacterium leprae, Vibrio cholerae, Neisseriaceae (e.g., Acinetobacter, Gonorrhea, Menigococcal), Meisseria meningitidis, Pasteurellacea Infections (e.g., Actinobacillus, Heamophilus (e.g., Heamophilus influenza type B), Pasteurella), Pseudomonas, Rickettsiaceae, Chlamydiaceae, Syphilis, Shigella spp., Staphylococcal, Meningiococcal, Pneumococcal and Streptococcal (e.g., Streptococcus pneumoniae and Group B Streptococcus). These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis (e.g., mengitis types A and B), Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections. TR10 polynucleotides or polypeptides, or agonists or antagonists of TR 10, can be used to treat or detect any of these symptoms or diseases. In specific embodiments, TR10 polynucleotides, polypeptides, or agonists thereof are used to treat: tetanus, Diptheria, botulism, and/or meningitis type B.

Moreover, parasitic agents causing disease or symptoms that can be treated by TR10 polynucleotides or polypeptides, or agonists of TR10, include, but not limited to, the following families or class: Amebiasis, Babesiosis, Coccidiosis, Cryptosporidiosis, Dientamoebiasis. Dourine. Ectoparasitic, Giardiasis, Helminthiasis. Leishmaniasis, Theileriasis, Toxoplasmosis, Trypanosomiasis, and Trichomonas and Sporozoans (e.g., Plasmodium virax, Plasmodium falciparium, Plasmodium malariae and Plasmodium ovale). These parasites can cause a variety of diseases or symptoms, including, but not limited to: Scabies, Trombiculiasis, eye infections, intestinal disease (e.g., dysentery, giardiasis), liver disease, lung disease, opportunistic infections (e.g., AIDS related), malaria, pregnancy complications, and toxoplasmosis. TR10 polynucleotides or polypeptides, or agonists or antagonists of TR 10, can be used to treat or detect any of these symptoms or diseases. In specific embodiments, TR10 polynucleotides, polypeptides, or agonists thereof are used to treat malaria.

An additional condition, disease or symptom that can be treated by TR10 polynucleotides or polypeptides, or agonists of TR10, is osteomyelitis.

Preferably, treatment using TR10 polynucleotides or polypeptides, or agonists of TR10, could either be by administering an effective amount of TR10 polypeptide to the patient, or by removing cells from the patient, supplying the cells with TR10 polynucleotide, and returning the engineered cells to the patient (ex vivo therapy). Moreover, as further discussed herein, the TR10 polypeptide or polynucleotide can be used as an adjuvant in a vaccine to raise an immune response against infectious disease.

Additional preferred embodiments of the invention include, but are not limited to, the use of TR10 polypeptides and functional agonists in the following applications:

Administration to an animal (e.g., mouse, rat, rabbit, hamster, guinea pig, pigs, micro-pig, chicken, camel, goat, horse, cow, sheep, dog, cat, non-human primate, and human, most preferably human) to boost the immune system to produce increased quantities of one or more antibodies (e.g., IgG, IgA, IgM, and IgE), to induce higher affinity antibody production (e.g., IgG, IgA, IgM, and IgE), and/or to increase an immune response.

Administration to an animal (including, but not limited to, those listed above, and also including transgenic animals) incapable of producing functional endogenous antibody molecules or having an otherwise compromised endogenous immune system, but which is capable of producing human immunoglobulin molecules by means of a reconstituted or partially reconsituted immune system from another animal (see, e.g., published PCT Application Nos. WO98/24893, WO/9634096, WO/9633735, and WO/9110741.

A vaccine adjuvant that enhances immune responsiveness to specific antigen.

An adjuvant to enhance tumor-specific immune responses.

An adjuvant to enhance anti-viral immune responses. Anti-viral immune responses that may be enhanced using the compositions of the invention as an adjuvant, include virus and virus associated diseases or symptoms described herein or otherwise known in the art. In specific embodiments, the compositions of the invention are used as an adjuvant to enhance an immune response to a virus, disease, or symptom selected from the group consisting of: AIDS, meningitis, Dengue, EBV, and hepatitis (e.g., hepatitis B). In another specific embodiment, the compositions of the invention are used as an adjuvant to enhance an immune response to a virus, disease, or symptom selected from the group consisting of: HIV/AIDS. Respiratory syncytial virus. Dengue, Rotavirus. Japanese B encephalitis, Influenza A and B, Parainfluenza, Measles, Cytomegalovirus, Rabies. Junin, Chikungunya, Rift Valley fever. Herpes simplex, and yellow fever.

An adjuvant to enhance anti-bacterial or anti-fungal immune responses. Anti-bacterial or anti-fungal immune responses that may be enhanced using the compositions of the invention as an adjuvant, include bacteria or fungus and bacteria or fungus associated diseases or symptoms described herein or otherwise known in the art. In specific embodiments, the compositions of the invention are used as an adjuvant to enhance an immune response to a bacteria or fungus, disease, or symptom selected from the group consisting of: tetanus, Diphtheria, botulism, and meningitis type B. In another specific embodiment, the compositions of the invention are used as an adjuvant to enhance an immune response to a bacteria or fungus, disease, or symptom selected from the group consisting of: Vibrio cholerae, Mycobacterium leprae, Salmonella typhi, Salmonella paratyphi, Meisseria meningitidis, Streptococcus pneumoniae, Group B streptococcus, Shigella spp., Enterotoxigenic Escherichia coli, Enterohemorrhagic E. coli, Borrelia burgdorferi, and Plasmodium (malaria).

An adjuvant to enhance anti-parasitic immune responses. Anti-parasitic immune responses that may be enhanced using the compositions of the invention as an adjuvant, include parasite and parasite associated diseases or symptoms described herein or otherwise known in the art. In specific embodiments, the compositions of the invention are used as an adjuvant to enhance an immune response to a parasite. In another specific embodiment, the compositions of the invention are used as an adjuvant to enhance an immune response to Plasmodium (malaria).

As a stimulator of B cell responsiveness to pathogens.

As an agent that elevates the immune status of an individual prior to their receipt of immunosuppressive therapies.

As an agent to induce higher affinity antibodies.

As an agent to increase serum immunoglobulin concentrations.

As an agent to accelerate recovery of immunocompromised individuals.

As an agent to boost immunoresponsiveness among aged populations.

As an immune system enhancer prior to, during, or after bone marrow transplant and/or other transplants (e.g., allogeneic or xenogeneic organ transplantation). With respect to transplantation, compositions of the invention may be administered prior to, concomitant with, and/or after transplantation. In a specific embodiment, compositions of the invention are administered after transplantation, prior to the beginning of recovery of T-cell populations. In another specific embodiment, compositions of the invention are first administered after transplantation after the beginning of recovery of T cell populations, but prior to full recovery of B cell populations.

As an agent to boost immunoresponsiveness among B cell immunodeficient individuals. B cell immunodeficiencies that may be ameliorated or treated by administering the TR10 polypeptides or polynucleotides of the invention, or agonists thereof, include, but are not limited to, SCID, congenital agammaglobulinemia, common variable immunodeficiency, Wiskott-Aldrich Syndrome, X-linked immunodeficiency with hyper 1gM, and severe combined immunodeficiency.

As an agent to boost immunoresponsiveness among individuals having an acquired loss of B cell function. Conditions resulting in an acquired loss of B cell function that may be ameliorated or treated by administering the TR10 polypeptides or polynucleotides of the invention, or agonists thereof, include, but are not limited to, HIV Infection, AIDS, bone marrow transplant, and B cell chronic lymphocytic leukemia (CLL).

As an agent to boost immunoresponsiveness among individuals having a temporary immune deficiency. Conditions resulting in a temporary immune deficiency that may be ameliorated or treated by administering the TR10 polypeptides or polynucleotides of the invention, or agonists thereof, include, but are not limited to, recovery from viral infections (e.g., influenza), conditions associated with malnutrition, recovery from infectious mononucleosis, or conditions associated with stress, recovery from measles, recovery from blood transfusion, recovery from surgery.

As a regulator of antigen presentation by monocytes, dendritic cells, and/or B-cells. In one embodiment, TRIO (in soluble, membrane-bound or transmembrane forms) enhances antigen presentation or antagonizes antigen presentation in vitro or in vivo. Moreover, in related embodiments, said enhancement or antagonization of antigen presentation may be useful as an anti-tumor treatment or to modulate the immune system.

As an agent to direct an individuals immune system towards development of a humoral response (i.e. TH2) as opposed to a TH1 cellular response.

As a means to induce tumor proliferation and thus make it more susceptible to anti-neoplastic agents. For example, multiple myeloma is a slowly dividing disease and is thus refractory to virtually all anti-neoplastic regimens. If these cells were forced to proliferate more rapidly their susceptibility profile would likely change.

As a stimulator of B cell production in pathologies such as AIDS, chronic lymphocyte disorder and/or Common Variable Immunodificiency;

As a therapy for generation and/or regeneration of lymphoid tissues following surgery, trauma or genetic defect.

As a gene-based therapy for genetically inherited disorders resulting in immuno-incompetence such as observed among SCID patients.

As an antigen for the generation of antibodies to inhibit or enhance TR10 mediated responses.

As a means of activating T cells.

As pretreatment of bone marrow samples prior to transplant. Such treatment would increase B cell representation and thus accelerate recover.

As a means of regulating secreted cytokines that are elicited by TR10.

TR10 polypeptides or polynucleotides of the invention, or agonists may be used to modulate IgE concentrations in vitro or in vivo.

Additinoally, TR 10 polypeptides or polynucleotides of the invention, or agonists thereof, may be used to treat or prevent IgE-mediated allergic reactions. Such allergic reactions include, but are not limited to, asthma, rhinitis, and eczema.

All of the above described applications as they may apply to veterinary medicine.

Antagonists of TR10 include binding and/or inhibitory antibodies, antisense nucleic acids, ribozymes or soluble forms of the TR10 receptor(s) (e.g,. the TR10-Fc molecule described in Example 38). These would be expected to reverse many of the activities of the ligand described above as well as find clinical or practical application as:

A means of blocking various aspects of immune responses to foreign agents or self. Examples include autoimmune disorders such as lupus, and arthritis, as well as immunoresponsiveness to skin allergies, inflammation, bowel disease, injury and pathogens. Although our current data speaks directly to the potential role of TR10 in B cell and T cell related pathologies, it remains possible that other cell types may gain expression or responsiveness to TR2. Thus, TR10 may, like CD40 and its ligand, be regulated by the status of the immune system and the microenvironment in which the cell is located.

A therapy for preventing the B cell proliferation and Ig secretion associated with autoimmune diseases such as idiopathic thrombocytopenic purpura, systemic lupus erythramatosus and MS.

An inhibitor of graft versus host disease or transplant rejection.

A therapy for B cell malignancies such as ALL, Hodgkins disease, non-Hodgkins lymphoma, Chronic lymphocyte leukemia, plasmacytomas, multiple myeloma, Burkitt's lymphoma, and EBV-transformed diseases.

A therapy for chronic hypersammagtobuiinemeia evident in such diseases as monoclonalgammopathy of undetermined significance (MGUS), Waldenstrom's disease, related idiopathic monoclonalgammopathies, and plasmacytomas.

A therapy for decreasing cellular proliferation of Large B-cell Lymphomas.

A means of decreasing the involvement of B cells and Ig associated with Chronic Myelogenous Leukemia.

An immunosuppressive agent(s).

TR10 polypeptides or polynucleotides of the invention, or antagonists may be used to modulate IgE concentrations in vitro or in vivo.

In another embodiment, administration of TR10 polypeptides or polynucleotides of the invention, or antagonists thereof, may be used to treat or prevent IgE-mediated allergic reactions including, but not limited to, asthma, rhinitis, and eczema.

The above-recited applications have uses in a wide variety of hosts. Such hosts include, but are not limited to, human, murine, rabbit, goat, guinea pig, camel, horse, mouse, rat, hamster, pig, micro-pig, chicken, goat, cow, sheep, dog, cat, non-human primate, and human. In specific embodiments, the host is a mouse, rabbit, goat, guinea pig, chicken, rat, hamster, pig, sheep, dog or cat. In preferred embodiments, the host is a mammal. In most preferred embodiments, the host is a human.

The agonists and antagonists may be employed in a composition with a pharmaceutically acceptable carrier, e.g., as described herein.

Polynucleotides and/or polypeptides of the invention and/or agonists and/or antagonists thereof are useful in the diagnosis and treatment or prevention of a wide range of diseases and/or conditions. Such diseases and conditions include, but are not limited to, cancer (e.g., immune cell related cancers, breast cancer, prostate cancer, ovarian cancer, follicular lymphoma, cancer associated with mutation or alteration of p53, brain tumor, bladder cancer, uterocervical cancer, colon cancer, colorectal cancer, non-small cell carcinoma of the lung, small cell carcinoma of the lung, stomach cancer, etc.), lymphoproliferative disorders (e.g., lymphadenopathy), microbial (e.g., viral, bacterial, etc.) infection (e.g., HIV-1 infection, HIV-2 infection, herpesvirus infection (including, but not limited to, HSV-1, HSV-2, CMV, VZV, HHV-6, HHV-7, EBV), adenovirus infection, poxvirus infection, human papilloma virus infection, hepatitis infection (e.g., HAV, HBV, HCV, etc.), *Helicobacter pylori* infection, invasive Staphylococcia, etc.), parasitic infection, nephritis, bone disease (e.g., osteoporosis), atherosclerosis, pain, cardiovascular disorders (e.g., neovascularization, hypovascularization or reduced circulation (e.g., ischemic disease (e.g., myocardial infarction, stroke, etc.), AIDS, allergy, inflammation, neurodegenerative disease (e.g., Alzheimer's disease, Parkinson's disease, amyotrophic lateral sclerosis, pigmentary retinitis, cerebellar degeneration etc.), graft rejection (acute and chronic), graft vs. host disease, diseases due to osteomyelodysplasia (e.g.. aplastic anemia, etc.), joint tissue destruction in rheumatism, liver disease (e.g., acute and chronic hepatitis, liver injury, and cirrhosis), autoimmune disease (e.g., multiple sclerosis, rheumatoid arthritis. systemic lupus erythematosus, immune complex glomerulonephritis, autoimmune diabetes, autoimmune thrombocytopenic purpura, Grave's disease, Hashimoto's thyroiditis, etc.), cardiomyopathy (e.g., dilated cardiomyopathy), diabetes, diabetic complications (e.g. , diabetic nephropathy, diabetic neuropathy, diabetic retinopathy), influenza, asthma, psoriasis, glomerulonephritis, septic shock, and ulcerative colitis.

Polynucleotides and/or polypeptides of the invention and/or agonists and/or antagonists thereof are useful in promoting angiogenesis, regulating hematopoiesis and wound healing (e.g., wounds, burns, and bone fractures).

Polynucleotides and/or polypeptides of the invention and/or agonists and/or antagonists thereof are also useful as an adjuvant to enhance immune responsiveness to specific antigen, anti-viral immune responses.

More generally, polynucleotides and/or polypeptides of the invention and/or agonists and/or antagonists thereof are useful in regulating (i.e., elevating or reducing) immune response. For example, polynucleotides and/or polypeptides of the invention may be useful in preparation or recovery from surgery, trauma, radiation therapy, chemotherapy, and transplantation, or may be used to boost immune response and/or recovery in the elderly and immunocompromised individuals. Alternatively, polynucleotides and/or polypeptides of the invention and/or agonists and/or antagonists thereof are useful as immunosuppressive agents, for example in the treatment or prevention of autoimmune disorders. In specific embodiments, polynucleotides and/or polypeptides of the invention are used to treat or prevent chronic inflammatory, allergic or autoimmune conditions, such as those described herein or are otherwise known in the art.

In one aspect, the present invention is directed to a method for enhancing apoptosis induced by a TNF-family ligand, which involves administering to a cell which expresses the TR10polypeptide an effective amount of TR10 ligand, analog or an agonist capable of increasing TR10 mediated signaling. Preferably, TR10 mediated signaling is increased to treat a disease wherein decreased apoptosis or decreased cytokine and adhesion molecule expression is exhibited. An agonist can include soluble forms of TR10 and monoclonal antibodies directed against the TR10 polypeptide.

In a further aspect, the present invention is directed to a method for inhibiting apoptosis induced by a TNF-family ligand, which involves administering to a cell which expresses the TR10 polypeptide an effective amount of an antagonist capable of decreasing TR10 mediated signaling. Preferably, TR10 mediated signaling is decreased to treat a disease wherein increased apoptosis or NFkB expression is exhibited. An antagonist can include soluble forms of TR10 and monoclonal antibodies directed against the TR10 polypeptide.

By "agonist" is intended naturally occurring and synthetic compounds capable of enhancing or potentiating apoptosis. By "antagonist" is intended naturally occurring and synthetic compounds capable of inhibiting apoptosis. Whether any candidate "agonist" or "antagonist" of the present invention can enhance or inhibit apoptosis can be determined using art-known TNF-family ligand/receptor cellular response assays, including those described in more detail below.

One such screening procedure involves the use of melanophores which are transfected to express the receptor of the present invention. Such a screening technique is described in PCT WO 92/01810, published February 6, 1992. Such an assay may be employed, for example, for screening for a compound which inhibits (or enhances) activation of the receptor polypeptide of the present invention by contacting the melanophore cells which encode the receptor with both a TNF-family ligand and the candidate antagonist (or agonist). Inhibition or enhancement of the signal generated by the ligand indicates that the compound is an antagonist or agonist of the ligand/receptor signaling pathway.

Other screening techniques include the use of cells which express the receptor (for example, transfected CHO cells) in a system which measures extracellular pH changes caused by receptor activation. For example, compounds may be contacted with a cell which expresses the receptor polypeptide of the present invention and a second messenger response, e.g., signal transduction or pH changes, may be measured to determine whether the potential compound activates or inhibits the receptor.

Another such screening technique involves introducing RNA encoding the receptor into *Xenopus* oocytes to transiently express the receptor. The receptor oocytes may then be contacted with the receptor ligand and a compound to be screened, followed by detection of inhibition or activation of a calcium signal in the case of screening for compounds which are thought to inhibit activation of the receptor.

Another screening technique well known in the art involves expressing in cells a construct wherein the receptor is linked to a phospholipase C or D. Exemplary cells include endothelial cells, smooth muscle cells, embryonic kidney cells, etc. The screening may be accomplished as hereinabove described by detecting activation of the receptor or inhibition of activation of the receptor from the phospholipase signal.

Another method involves screening for compounds which inhibit activation of the receptor polypeptide of the present invention antagonists by determining inhibition of binding of labeled ligand to cells which have the receptor on the surface thereof. Such a method involves transfecting a eukaryotic cell with DNA encoding the receptor such that the cell expresses the receptor on its surface and contacting the cell with a compound in the presence of a labeled form of a known ligand. The ligand can be labeled, e.g., by radioactivity. The amount of labeled ligand bound to the receptors is measured, e.g., by measuring radioactivity of the receptors. If the compound binds to the receptor as determined by a reduction of labeled ligand which binds to the receptors. the binding of labeled ligand to the receptor is inhibited.

Further screening assays for agonists and antagonists of the present invention are described in L.A.Tartaglia and D.V. Goeddel. J. *Biol. Chem. 267*:4304-4307(1992).

Thus, in a further aspect, a screening method is provided for determining whether a candidate agonist or antagonist is capable of enhancing or inhibiting a cellular response to a TNF-family ligand. The method involves contacting cells which express the TR10 polypeptide with a candidate compound and a TNF-family ligand, assaying a cellular response, and comparing the cellular response to a standard cellular response, the standard being assayed when contact is made with the ligand in absence of the candidate compound, whereby an increased cellular response over the standard indicates that the candidate compound is an agonist of the ligand/receptor signaling pathway and a decreased cellular response compared to the standard indicates that the candidate compound is an antagonist of the ligand/receptor signaling pathway. By "assaying a cellular response" is intended qualitatively or quantitatively measuring a cellular response to a candidate compound and/or a TNF-family ligand (e.g., determining or estimating an increase or decrease in T cell proliferation or tritiated thymidine labeling). By the invention, a cell expressing the TR10 polypeptide can be contacted with either an endogenous or exogenously administered TNF-family ligand.

Antagonist according to the present invention include naturally occurring and synthetic compounds such as, for example, TNF family ligand peptide fragments, transforming growth factor, neurotransmitters (such as glutamate, dopamine, *N*- methyl-D-aspartate), tumor suppressors (p53), cytolytic T cells and antimetabolites. Preferred agonists include chemotherapeutic drugs such as, for example, cisplatin, doxorubicin, bleomycin, cytosine arabinoside, nitrogen mustard, methotrexate and vincristine. Others include ethanol and - amyloid peptide. *(Science 267*:1457-1458 (1995)). Further preferred agonists include polyclonal and monoclonal antibodies raised against the TR10 polypeptide, or a fragment thereof. Such agonist antibodies raised against a TNF-family receptor are disclosed in L.A. Tartaglia *et al., Proc. Natl. Acad. Sci. USA 88*:9292-9296 (1991); and L.A.Tartaglia and D.V.Goeddel, *J. Biol. Chem. 267*:4304- 4307 (1992). See, also, PCT Application WO 94/09137.

Agonists according to the present invention include naturally occurring and synthetic compounds such as, for example, the CD40 ligand, neutral amino acids, zinc, estrogen, androgens, viral genes (such as Adenovirus *EIB.* Baculovirus *p35* and *IAP.* Cowpox virus *crmA*, Epstein-Barr virus *BHRF1, LMP-1,* African swine fever virus *LMW5-HL,* and Herpesvirus yl 34.5). calpain inhibitors, cysteine protease inhibitors and tumor promoters (such as PMA, Phenobarbital, and □-Hexachlorocyclohexane).

Other potential antagonists include antisense molecules. Antisense technology can be used to control gene expression through antisense DNA or RNA or through triple-helix formation. Antisense techniques are discussed, for example, in Okano, *J. Neurochem. 56:560* (1991); *Oligodeoxynucleotides as Antisense Inhibitors of Gene Expression,* CRC Press, Boca Raton, FL (1988). Triple helix formation is discussed in, for instance *Lee et al., Nucleic Acids Research 6*:3073 (1979); Cooney *et al., Science 241*:456 (1988); and Dervan *et al., Science 251*:1360 (1991). The methods are based on binding of a polynucleotide to a complementary DNA or RNA.

In specific embodiments, antagonists according to the present invention are nucleic acids corresponding to the sequences contained in TR10, or the complementary strand thereof, and/or to nucleotide sequences contained in the deposited clone ATCC Deposit No. 209040. In one embodiment, antisense sequence is generated internally by the organism, in another embodiment, the antisense sequence is separately administered (see, for example, Okano H*. et al., J. Neurochem.* 56:560 (1991), and Oligodeoxynucleotides as Antisense Inhibitors of Gene Expression, CRC Press, Boca Raton, FL (1988). Antisense technology can be used to control gene expression through antisense DNA or RNA, or through triple-helix formation. Antisense techniques are discussed for example, in Okano, *Neurochem.* 56:560 (1991); Oligodeoxynucleotides as Antisense Inhibitors of Gene Expression, CRC Press, Boca Raton, FL (1988). Triple helix formation is discussed in, for instance, *Lee et al., Nucleic Acids Research 6*:3073 (1979); Cooney *et al., Science 241*: 456 (1988); and Dervan *et al., Science 251*:1300 (1991). The methods are based on binding of a polynucleotide to a complementary DNA or RNA.

For example, the 5' coding portion of a polynucleotide that encodes the mature polypeptide of the present invention may be used to design an antisense RNA oligonucleotide of from about 10 to 40 base pairs in length. A DNA oligonucleotide is designed to be complementary to a region of the gene involved in transcription thereby preventing transcription and the production of the receptor. The antisense RNA oligonucleotide hybridizes to the mRNA *in vivo* and blocks translation of the mRNA molecule into receptor polypeptide. The oligonucleotides described above can also be delivered to cells such that the antisense RNA or DNA may be expressed *in vivo* to inhibit production of the receptor.

In one embodiment, the TR10 antisense nucleic acid of the invention is produced intracellularly by transcription from an exogenous sequence. For example, a vector or a portion thereof, is transcribed, producing an antisense nucleic acid (RNA) of the invention. Such a vector would contain a sequence encoding the TR10 antisense nucleic acid. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others know in the art, used for replication and expression in vertebrate cells. Expression of the sequence encoding TR10,or fragments thereof, can be by any promoter known in the art to act in vertebrate, preferably human cells. Such promoters can be inducible or constitutive. Such promoters include, but are not limited to, the SV40 early promoter region (Bemoist and Chambon, *Nature 29*:304-310 (1981), the promoter contained in the 3' long terminal repeat of Rous sarcoma virus (Yamamoto *et al., Cell 22*:787-797 (1980), the herpes thymidine promoter *(Wagner et al., Proc. Natl. Acad. Sci. U.S.A. 78*:1441-1445 (1981), the regulatory sequences of the metallothionein gene (Brinster, *et al., Nature 296*:39-42 (1982)), etc.

The antisense nucleic acids of the invention comprise a sequence complementary to at least a portion of an RNA transcript of a TR10 gene. However, absolute complementarity, although preferred, is not required. A sequence "complementary to at least a portion of an RNA," referred to herein, means a sequence having sufficient complementarity to be able to hybridize with the RNA, forming a stable duplex; in the case of double stranded TR10 antisense nucleic acids, a single strand of the duplex DNA may thus be tested, or triplex formation may be assayed. The ability to hybridize will depend on both the degree of complementarity and the length of the antisense nucleic acid Generally, the larger the hybridizing nucleic acid, the more base mismatches with a TR10 RNA it may contain and still form a stable duplex (or triplex as the case may be). One skilled in the art can ascertain a tolerable degree of mismatch by use of standard procedures to determine the melting point of the hybridized complex.

Oligonucleotides that are complementary to the 5' end of the message, e.g., the 5' untranslated sequence up to and including the AUG initiation codon, should work most efficiently at inhibiting translation. However, sequences complementary to the 3' untranslated sequences of mRNAs have been shown to be effective at inhibiting translation of mRNAs as well. See generally, Wagner, R., *Nature 372*:333-335 (1994). Thus, oligonucleotides complementary to either the 5'- or 3'- non- translated, non-coding regions of the TR10 shown in Figures 1A-D could be used in an antisense approach to inhibit translation of endogenous TR10 mRNA. Oligonucleotides complementary to the 5' untranslated region of the mRNA should include the complement of the AUG start codon. Antisense oligonucleotides complementary to mRNA coding regions are less efficient inhibitors of translation but could be used in accordance with the invention. Whether designed to hybridize to the 5'-, 3'- or coding region of TR10 mRNA, antisense nucleic acids should be at least six nucleotides in length, and are preferably oligonucleotides ranging from 6 to about 50 nucleotides in length. In specific aspects the oligonucleotide is at least 10 nucleotides, at least 17 nucleotides, at least 25 nucleotides or at least 50 nucleotides.

The polynucleotides of the invention can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (e.g., for targeting host cell receptors in vivo), or agents facilitating transport across the cell membrane (see, e.g., Letsinger *et al., Proc. Natl. Acad. Sci. U.S.A. 86*:6553-6556 (1989); Lemaitre *et al., Proc. Natl. Acad. Sci. 84*:648-652 (1987); PCT Publication No. WO88/09810, published December 15, 1988) or the blood-brain barrier (see, e.g., PCT Publication No. WO89/10134, published April 25, 1988), hybridization-triggered cleavage agents. (See, e.g., Krol *et al., BioTechniques 6*:958-976 (1988)) or intercalating agents. (See, e.g., Zon, *Pharm. Res. 5*:539-549 (1988)). To this end, the oligonucleotide may be conjugated to another molecule, e.g., a peptide, hybridization triggered cross-linking agent, transport agent, hybridization-triggered cleavage agent, etc.

The antisense oligonucteotide may comprise at least one modified base moiety which is selected from the group including, but not limited to, 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyl adenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil. 5-methoxyaminomethyl-2-thiouracil, beta-D-mannosylqueosine, 5-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypfopyl) uracil, (acp3) w, and 2,6-diaminopurine.

The antisense oligonucleotide may also comprise at least one modified sugar moiety selected from the group including, but not limited to, arabinose, 2-fluoroarabinose, xylulose, and hexose.

In yet another embodiment, the antisense oligonucleotide comprises at least one modified phosphate backbone selected from the group including, but not limited to, a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier *et al., Nucl. Acids Res. 15*:6625-6641 ( 1987)). The oligonucleotide is a 2'-0-methylribonucleotide (Inoue *et al., Nucl. Acids Res. 15*:6131-6148 (1987)), or a chimeric RNA-DNA analogue *(*Inoue *et al., FEBS Lett. 215*:327-330 (1987)).

Polynucleotides of the invention may be synthesized by standard methods known in the art, e.g. by use of an automated DNA synthesizer (such as are commercially available from Biosearch, Applied Biosystems, etc.). As examples, phosphorothioate oligonucleotides may be synthesized by the method of Stein et al. *(Nucl. Acids Res. 16*:3209 (1988)), methylphosphonate oligonucleotides can be prepared by use of controlled pore glass polymer supports (Sarin *et al., Proc. Natl. Acad. Sci. U.S.A. 85*:7448-7451 ( 1988)), etc.

While antisense nucleotides complementary to the TR10 coding region sequence could be used, those complementary to the transcribed untranslated region are most preferred.

Potential antagonists according to the invention also include catalytic RNA, or a ribozyme (See, e.g., PCT International Publication WO 90/11364, published October 4, 1990; Sarver *et al, Science 247*:1222-1225 (1990). While ribozymes that cleave mRNA at site specific recognition sequences can be used to destroy TR 10 mRNAs, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach, *Nature 334*:585-591 (1988). There are numerous potential hammerhead ribozyme cleavage sites within the nucleotide sequence of TR10 (Figures 1A-D (SEQ ID NO:1)). Preferably, the ribozyme is engineered so that the cleavage recognition site is located near the 5' end of the TR10 mRNA; i.e., to increase efficiency and minimize the intracellular accumulation of non-functional mRNA transcripts.

As in the antisense approach, the ribozymes of the invention can be composed of modified oligonucleotides (e.g., for improved stability, targeting, etc.) and should be delivered to cells which express TR10 *in vivo.* DNA constructs encoding the ribozyme may be introduced into the cell in the same manner as described above for the introduction of antisense encoding DNA. A preferred method of delivery involves using a DNA construct "encoding" the ribozyme under the control of a strong constitutive promoter, such as. for example, pol III or pol II promoter, so that transfected cells will produce sufficient quantities of the ribozyme to destroy endogenous TR 10 messages and inhibit translation. Since ribozymes unlike antisense molecules, are catalytic, a lower intracellular concentration is required for efficiency.

Endogenous gene expression can also be reduced by inactivating or "knocking out" the TR10 gene and/or its promoter using targeted homologous recombination. (E.g., see Smithies *et al., Nature 317*:230-234 (1985); Thomas & Capecchi, *Cell 51*:503-512 (1987); Thompson *et al., Cell 5*:313-321 (1989); each of which is incorporated by reference herein in its entirety). For example, a mutant, non-functional polynucleotide of the invention (or a completely unrelated DNA sequence) flanked by DNA homologous to the endogenous polynucleotide sequence (either the coding regions or regulatory regions of the gene) can be used, with or without a selectable marker and/or a negative selectable marker, to transfect cells that express polypeptides of the invention in vivo. In another embodiment, techniques known in the art are used to generate knockouts in cells that contain, but do not express the gene of interest. Insertion of the DNA construct, via targeted homologous recombination, results in inactivation of the targeted gene. Such approaches are particularly suited in research and agricultural fields where modifications to embryonic stem cells can be used to generate animal offspring with an inactive targeted gene (e.g., see Thomas & Capecchi 1987 and Thompson 1989, *supra*). However this approach can be routinely adapted for use in humans provided the recombinant DNA constructs are directly administered or targeted to the required site *in vivo* using appropriate viral vectors that will be apparent to those of skill in the art. The contents of each of the documents recited in this paragraph is herein incorporated by reference in its entirety.

The techniques of gene-shuffling, motif-shuffling, exon-shuffling. and/or codon-shuffling (collectively referred to as "DNA shuffling") may be employed to modulate the activities of TR10 thereby effectively generating agonists and antagonists of TR10. *See generally,* U.S. Patent Nos. 5,605,793, 5,811,238, 5,830,721, 5,834,252, and 5,837,458, and Patten *et al., Curr. Opinion Biotechnol. 8*:724-33 (1997); Harayama. *Trends Biotechnol. 16*(2):76-82 (1998); Hansson *et al., J. Mol. Biol. 287*:265-76 (1999); and Lorenzo and Blasco, *Biotechniques 24*(2):308-13 (1998) (each of these patents and publications are hereby incorporated by reference). In one embodiment, alteration of TR10 polynucleotides and corresponding polypeptides may be achieved by DNA shuffling. DNA shuffling involves the assembly of two or more DNA segments into a desired TR10 molecule by homologous, or site-specific, recombination. In another embodiment, TRIO polynucleotides and corresponding polypeptides may be alterred by being subjected to random mutagenesis by error-prone PCR, random nucleotide insertion or other methods prior to recombination. In another embodiment, one or more components, motifs, sections, parts, domains, fragments, etc., of TR10 may be recombined with one or more components, motifs, sections, parts, domains, fragments, etc. of one or more heterologous molecules. In preferred embodiments, the heterologous molecules are include, but are not limited to, TNF-alpha, lymphotoxin-alpha (LT-alpha, also known as TNF-beta), LT-beta (found in complex heterotrimer LT-alpha2-beta), OPGL, FasL, CD27L, CD30L, CD40L, 4-1BBL, DcR3, OX40L, TNF-gamma (International Publication No. WO 96/14328), TRAIL, AIM-II (International Publication No. WO 97/34911), APRIL (*J. Exp. Med. 188*(6):1185-1190), endokine-alpha (International Publication No. WO 98/07880), neutrakine alpha (International Publication No.WO98/18921), TR6 (International Publication No. WO 98/30694), OPG, OX40, and nerve growth factor (NGF), and soluble forms of Fas, CD30, CD27, CD40 and 4-IBB, TR2 (International Publication No. WO 96/34095), DR3 (International Publication No. WO 97/33904), DR4 (International Publication No. WO 98/32856), TR5 (International Publication No. WO 98/30693), TR6 (International Publication No. WO 98/30694), TR7 (International Publication No. WO 98/41629), TRANK, TR9 (International Publication No. WO 98/56892), 312C2 (International Publication No. WO 98/06842), and TR12, and soluble forms CD154, CD70, and CD153. In further preferred embodiments, the heterologous molecules are any member of the TNF family.

In other embodiments, antagonists according to the present invention include soluble forms of TR10 (e.g., fragments of the TR10 shown in Figures 1A-D (SEQ ID NO:2) that include the ligand binding domain from the extracellular region of the full length receptor). Such soluble forms of the TR10, which may be naturally occurring or synthetic, antagonize TR10 mediated signaling by competing with the cell surface bound forms of the receptor for binding to TNF-family ligands. Antagonists of the present invention also include antibodies specific for TNF-family ligands and TR10-Fc fusion proteins.

By a"TNF-family ligand" is intended naturally occurring, recombinant, and synthetic ligands that are capable of binding to a member of the TNF receptor family and inducing and/or blocking the ligand/receptor signaling pathway. Members of the TNF ligand family include, but are not limited to, TNF-alpha, lymphotoxin-alpha (LT-alpha, also known as TNF-beta), LT-beta (found in complex heterotrimer LT-alpha2-beta), OPGL, FasL, CD27L, CD30L, CD40L, 4-1BBL, DcR3, OX40L, TNF-gamma (International Publication No. WO 96/14328), TRAIL, AIM-II (International Publication No. WO 97/34911 ), APRIL (*J. Exp. Med. 188*(6):1185-1190), endokine-alpha (International Publication No. WO 98/07880), TR6 (International Publication No. WO 98/30694), OPG, OX40, and nerve growth factor (NGF), and soluble forms of Fas, CD30, CD27, CD40 and 4-IBB, TR2 (International Publication No. WO 96/34095), DR3 (International Publication No. WO 97/33904), DR4 (International Publication No. WO 98/32856), TR5 (International Publication No. WO 98/30693), TR6 (International Publication No. WO 98/30694), TR7 (International Publication No. WO 98/41629), TRANK, TR9 (International Publication No. WO 98/56892), 312C2 (International Publication No. WO 98/06842). and TR12, and soluble forms CD154, CD70, and CD153.

TNF-$\alpha$ has been shown to protect mice from infection with herpes simplex virus type 1 (HSV-1). Rossol-Voth et al., J .Gen. Virol. 72:143-147 (1991). The mechanism of the protective effect of TNF-$\alpha$ is unknown but appears to involve neither interferons nor NK cell killing. One member of the family has been shown to mediate HSV-1 entry into cells. Montgomery *et al., Eur. Cytokine Newt. 7*:159 (1996). Further, antibodies specific for the extracellular domain of this block HSV-1 entry into cells. Thus, TR10 antagonists of the present invention include both TR10 amino acid sequences and antibodies capable of preventing mediated viral entry into cells. Such sequences and antibodies can function by either competing with cell surface localized for binding to virus or by directly blocking binding of virus to cell surface receptors.

Antibodies according to the present invention may be prepared by any of a variety of methods using TR10 immunogens of the present invention. As indicated, such TR10 immunogens include the full length TR10 polypeptide (which may or may not include the leader sequence) and TR10 polypeptide fragments such as the extracellular domain, the cysteine rich domain, the ligand binding domain, the transmembrane domain, the intracellular domain and the incomplete death domain, or any combination thereof.

Polyclonal and monoclonal antibody agonists or antagonists according to the present invention can be raised according to the methods disclosed herein and and/or known in the art, such as, for example, those methods described in Tartaglia and Goeddel, *J. Biol. Chem. 267*(7):4304-4307(1992); Tartaglia *et al., Cell 73*:213-216 (1993), and PCT Application WO 94/09137 (the contents of each of these three publications are herein incorporated by reference in their entireties), and are preferably specific to TR10 polypeptides of the invention having the amino acid sequence of SEQ ID NO:2.

An agonists according to the present invention include soluble forms of TR10, i.e., TR10 fragments that include the ligand binding domain from the extracellular region of the full length receptor. Such soluble forms of the receptor, which may be naturally occurring or synthetic, antagonize TR10 mediated signaling by competing with the cell surface TR10 for binding to TNF-family ligands. However, soluble TR10 may bind to apoptosis inducing ligands such as TRAIL and more effectively compete for TRAIL binding reducing the available TRAIL for binding to receptors with functional death domains. Thus, soluble forms of the receptor that include the ligand binding domain are novel cytokines capable of inhibiting apoptosis induced by TNF-family ligands. These are preferably expressed as dimers or trimers, since these have been shown to be superior to monomeric forms of soluble receptor as antagonists, e.g., IgGFc-TNF receptor family fusions. Other such cytokines are known in the art and include Fas B (a soluble form of the mouse Fas receptor) that acts physiologically to limit apoptosis induced by Fas ligand (D.P. Hughes and 1.N. Crispe, J. *Exp. Med. 182*:1395-1401 (1995)).

Proteins and other compounds which bind the TR 10 domains are also candidate agonists and antagonists according to the present invention. Such binding compounds can be "captured" using the yeast two-hybrid system (Fields and Song, *Nature 340*:245-246 (1989)). A modified version of the yeast two- hybrid system has been described by Roger Brent and his colleagues (J. Gyuris, *Cell 75*:791-803 (1993); A.S. Zervos *et al., Cell 72*:223-232 (1993)). Preferably, the yeast two-hybrid system is used according to the present invention to capture compounds which bind to either the TR10 ligand binding domain or to the TR10 intracellular domain. Such compounds are good candidate agonists and antagonists of the present invention.

In rejection of an allograft, the immune system of the recipient animal has not previously been primed to respond because the immune system for the most part is only primed by environmental antigens. Tissues from other members of the same species have not been presented in the same way that, for example, viruses and bacteria have been presented. In the case of allograft rejection, immunosuppressive regimens are designed to prevent the immune system from reaching the effector stage. However, the immune profile of xenograft rejection may resemble disease recurrence more that allograft rejection. In the case of disease recurrence, the immune system has already been activated, as evidenced by destruction of the native islet cells. Therefore, in disease recurrence, the immune system is already at the effector stage. Antagonists of the present invention are able to suppress the immune response to both allografts and xenografts because lymphocytes activated and differentiated into effector cells will express the TR10 polypeptide, and thereby are susceptible to compounds which enhance apoptosis. Thus, the present invention further provides a method for creating immune privileged tissues.

TR10 antagonists of the invention can further be used in the treatment of inflammatory diseases, such as inflammatory bowel disease, rheumatoid arthritis, osteoarthritis, psoriasis, and septicemia. In addition, due to lymphoblast expression of TR10, soluble TR10 agonist or antagonist mABs may be used to treat this form of cancer.

***Modes of Administration***

The agonist or antagonists described herein can be administered *in vitro, ex vivo,* or *in vivo* to cells which express the receptor of the present invention. By administration of an "effective amount" of an agonist or antagonist is intended an amount of the compound that is sufficient to enhance or inhibit a cellular response to a TNF-family ligand and include polypeptides. In particular, by administration of an "effective amount" of an agonist or antagonists is intended an amount effective to enhance or inhibit TR10 mediated apoptosis. Of course, where it is desired for apoptosis to be enhanced, an agonist according to the present invention can be co-administered with a TNF-family ligand. One of ordinary skill will appreciate that effective amounts of an agonist or antagonist can be determined empirically and may be employed in pure form or in pharmaceutically acceptable salt, ester or prodrug form. The agonist or antagonist may be administered in compositions in combination with one or more pharmaceutically acceptable excipients.

It will be understood that, when administered to a human patient, the total daily usage of the compounds and compositions of the present invention will be decided by the attending physician within the scope of sound medical judgment. The specific therapeutically effective dose level for any particular patient will depend upon factors well known in the medical arts.

As a general proposition, the total pharmaceutically effective amount of TR10 polypeptide administered parenterally per dose will be in the range of about 1 ug/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the TR10 polypeptide is typically administered at a dose rate of about 1 ug/kg/hour to about 50 ug/kg/hour, either by 1-4 injections per day or by continuous subcutaneous infusions, for example, using a mini-pump. An intravenous bag solution may also be employed.

Dosaging may also be arranged in a patient specific manner to provide a predetermined concentration of an agonist or antagonist in the blood, as determined by the RIA technique. Thus patient dosaging may be adjusted to achieve regular on-going trough blood levels, as measured by RIA, on the order of from 50 to 1000 ng/ml, preferably 150 to 500 ng/ml.

Pharmaceutical compositions containing the TR10 polypeptide of the invention may be administered orally, rectally, parenterally, intracistemally, intravaginally, intraperitoneally, topically (as by powders, ointments, drops or transdermal patch), bucally, or as an oral or nasal spray. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

Pharmaceutical compositions of the present invention for parenteral injection can comprise pharmaceutically acceptable sterile aqueous or nonaqueous solutions, dispersions, suspensions or emulsions as well as sterile powders for reconstitution into sterile injectable solutions or dispersions just prior to use. The composition, if desired, can also contain minor amounts of wetting or emulsifying agents, or pH buffering agents. These compositions can take the form of solutions, suspensions, emulsion, tablets, pills, capsules, powders, sustained-release formulations and the like.

In addition to soluble TR10 polypeptides, TR10 polypeptides containing the transmembrane region can also be used when appropriately solubilized by including detergents, such as CHAPS or NP-40, with buffer.

TR10 compositions of the invention are also suitably administered by sustained-release systems. Suitable examples of sustained-release compositions include suitable polymeric materials (such as, for example, semi-permeable polymer matrices in the form of shaped articles, e.g., films, or mirocapsules), suitable hydrophobic materials (for example as an emulsion in an acceptable oil) or ion exchange resins, and sparingly soluble derivatives (such as, for example, a sparingly soluble salt).

Sustained-release matrices include polylactides (U.S. Pat. No. 3,773,919, EP 58,481), copolymers of L-glutamic acid and gamma-ethyl-L-glutamate (Sidman, U. et al., *Biopolymers 22*:547-556 (1983)), poly (2- hydroxyethyl methacrylate) (R. Langer et al., *J. Biomed. Mater. Res. 15*:167-277 (1981), and R. Langer, *Chem. Tech. 12*:98-105 ( 1982)), ethylene vinyl acetate (R. Langer et al., Id.) or poly-D- (-)-3-hydroxybutyric acid (EP 133,988).

Sustained-release compositions also include liposomally entrapped compositions of the invention *(see* generally, Langer, *Science 249*:1527-1533 (1990); Treat et al., in *Liposomes in the Therapy of Infectious Disease and Cancer,* Lopez-Berestein and Fidler (eds.), Liss, New York, pp. 317 -327 and 353-365 (1989)). Liposomes containing TR10 polypeptide my be prepared by methods known per se: DE 3,218,121; Epstein et al., Proc. Natl. Acad. Sci. (USA) 82: 3688-3692 (1985); Hwang et al., Proc. Natl. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal TR10 polypeptide therapy.

In yet an additional embodiment, the compositions of the invention are delivered by way of a pump *(see* Langer, *supra;* Sefton, CRC Crit. Ref. Biomed. Eng. 14:201 (1987); Buchwald et al., Surgery 88:507 (1980); Saudek et al., N. Engl. J. Med. 321:574 (1989)).

Other controlled release systems are discussed in the review by Langer *(Science* 249:1527-1533 (1990)).

The compositions of the invention may be administered alone or in combination with other adjuvants. Adjuvants that may be administered with the compositions of the invention include, but are not limited to, alum, alum plus deoxycholate (ImmunoAg), MTP-PE (Biocine Corp.), QS21 (Genentech, Inc.), BCG, and MPL. In a specific embodiment, compositions of the invention are admninistered in combination with alum. In another specific embodiment, compositions of the invention are administered in combination with QS-21. Further adjuvants that may be administered with the compositions of the invention include, but are not limited to, Monophosphoryl lipid immunomodulator, AdjuVax 100a, QS-18, CRL1005, Aluminum salts, MF-59, and Virosomal adjuvant technology. Vaccines that may be administered with the compositions of the invention include, but are not limited to, vaccines directed toward protection against MMR (measles, mumps, rubella), polio, varicella, tetanus/diptheria, hepatitis A, hepatitis B, haemophilus influenzae B, whooping cough, pneumonia, influenza, Lyme's Disease, rotavirus, cholera, yellow fever, Japanese encephalitis, poliomyelitis, rabies, typhoid fever, and pertussis. Combinations may be administered either concomitantly, e.g., as an admixture, separately but simultaneously or concurrently; or sequentially. This includes presentations in which the combined agents are administered together as a therapeutic mixture, and also procedures in which the combined agents are administered separately but simultaneously, e.g., as through separate intravenous lines into the same individual. Administration "in combination" further includes the separate administration of one of the compounds or agents given first, followed by the second.

The compositions of the invention may be administered alone or in combination with other therapeutic agents. Therapeutic agents that may be administered in combination with the compositions of the invention, include but are not limited to, other members of the TNF family, chemotherapeutic agents, antibiotics, antivirals, steroidal and non-steroidal anti-inflammatories, conventional immunotherapeutic agents, cytokines, chemokines and/or growth factors. Combinations may be administered either concomitantly, e.g., as an admixture, separately but simultaneously or concurrently; or sequentially. This includes presentations in which the combined agents are administered together as a therapeutic mixture, and also procedures in which the combined agents are administered separately but simultaneously, e.g., as through separate intravenous lines into the same individual. Administration "in combination" further includes the separate administration of one of the compounds or agents given first, followed by the second.

In one embodiment, the compositions of the invention are administered in combination with other members of the TNF family. TNF, TNF-related or TNF-like molecules that may be administered with the compositions of the invention include, but are not limited to, soluble forms of TNF-alpha, lymphotoxin-alpha (LT-alpha, also known as TNF-beta), LT-beta (found in complex heterotrimer LT-alpha2-beta), OPGL, FasL, CD27L, CD30L, CD40L, 4-1BBL, DcR3, OX40L, TNF-gamma (International Publication No. WO 96/14328), TRAIL, AIM-II (International Publication No. WO 97/34911),

APRIL (*J. Exp. Med. 188*(6):1 185-1190), endokine-alpha (International Publication No. WO 98/07880), TR6 (International Publication No. WO 98/30694), OPG, OX40, and nerve growth factor (NGF), and soluble forms of Fas, CD30, CD27, CD40 and 4-IBB, TR2 (International Publication No. WO 9b/34095), DR3 (International Publication No. WO 97/33904), DR4 (International Publication No. WO 98/32856), TR5 (International Publication No. WO 98/30693), TR6 (International Publication No. WO 98/30694), TR7 (International Publication No. WO 98/41629), TRANK, TR9 (International Publication No. WO 98/56892), 312C2 (International Publication No. WO 98/06842), and TR12, and soluble forms CD154, CD70, and CD153.

In certain embodiments, compositions of the invention are administered in combination with antiretroviral agents, nucleoside reverse transcriptase inhibitors, non-nucleoside reverse transcriptase inhibitors, and/or protease inhibitors. Nucleoside reverse transcriptase inhibitors that may be administered in combination with the compositions of the invention, include, but are not limited to, RETROVIR™ (zidovudine/AZT), VIDEX™ (didanosine/ddl), HIVID™ (zalcitabine/ddC), ZERIT™ (stavudine/d4T), EPIYIR™ (lamivudine/3TC), and COMBIVIR™ (zidovudine/lamivudine). Non-nucleoside reverse transcriptase inhibitors that may be administered in combination with the compositions of the invention, include, but are not limited to, VIRAMUNE™ (nevirapine), RESCRIPTOR™ (delavirdine), and SUSTIVA'" (efavirenz). Protease inhibitors that may be administered in combination with the compositions of the invention, include, but are not limited to, CRIXIVAN™ (indinavir), NORVIR™ (ritonavir), INVIRASE™ (saquinavir), and VIRACEPT™ (nelfinavir). In a specific embodiment, antiretroviral agents, nucleoside reverse transcriptase inhibitors, non-nucleoside reverse transcriptase inhibitors, and/or protease inhibitors may be used in any combination with compositions of the invention to treat AIDS and/or to prevent or treat HIV infection.

In other embodiments, compositions of the invention may be administered in combination with anti-opportunistic infection agents. Anti-opportunistic agents that may be administered in combination with the compositions of the invention, include. but are not limited to, TRIMETHOPRIM-SULFAMETHOXAZOLE™, DAPSONE™, PENTAMIDINE™, ATOVAQUONE™, ISONIAZID™, RIFAMPIN™, PYRAZINAMIDE™, ETHAMBUTOL™, RIFABUTIN™, CLARITHROMYCIN™, AZITHROMYCIN™, GANCICLOVIR™, FOSCARNET™, CIDOFOVIR™, FLUCONAZOLE™, ITRACONAZOLE™, KETOCONAZOLE™, ACYCLOVIR™, FAMCICOLVIR™, PYRIMETHAMINE™, LEUCOVORIN™, NEUPOGEN™ (filgrastim/G-CSF), and LEUKINE™ (sargramostim/GM-CSF). In a specific embodiment, compositions of the invention are used in any combination with TRIMETHOPRIM-SULFAMETHOXAZOLE™, DAPSONE™, PENTAMIDINE™, and/or ATOVAQUONE™ to prophylactically treat or prevent an opportunistic *Pneumocystis carinii* pneumonia infection. In another specific embodiment, compositions of the invention are used in any combination with ISONIAZID™, RIFAMPIN™, PYRAZINAMIDE™, and/or ETHAMBUTOL™ to prophylactically treat or prevent an opportunistic *Mycobacterium avium* complex infection. In another specific embodiment, compositions of the invention are used in any combination with RIFABUTIN'", CLARITHROMYCIN™, and/or AZITHROMYCIN™ to prophylactically treat or prevent an opportunistic *Mycobacterium tuberculosis* infection. In another specific embodiment, compositions of the invention are used in any combination with GANCICLOVIR™, FOSCARNET™, and/or CIDOFOVIR™ to prophylactically treat or prevent an opportunistic cytomegalovirus infection. In another specific embodiment, compositions of the invention are used in any combination with FLUCONAZOLE™, ITRACONAZOLE™, and/or KETOCONAZOLE™ to prophylactically treat or prevent an opportunistic fungal infection. In another specific embodiment, compositions of the invention are used in any combination with ACYCLOVIR™ and/or FAMCICOLVIR™ to prophylactically treat or prevent an opportunistic herpes simplex virus type I and/or type II infection. In another specific embodiment, compositions of the invention are used in any combination with PYRIMETHAMINE™ and/or LEUCOVORIN™ to prophylactically treat or prevent an opportunistic *Toxoplasma gondii* infection. In another specific embodiment, compositions of the invention are used in any combination with LEUCOVORIN™ and/or NEUPOGEN™ to prophylactically treat or prevent an opportunistic bacterial infection.

In a further embodiment, the compositions of the invention are administered in combination with an antiviral agent. Antiviral agents that may be administered with the compositions of the invention include, but are not limited to, acyclovir, ribavirin, amantadine, and remantidine. In a further embodiment, the compositions of the invention are administered in combination with an antibiotic agent. Antibiotic agents that may be administered with the compositions of the invention include, but are not limited to, amoxicillin. aminoglycosides, beta-lactam (glycopeptide), beta-lactamases, Clindamycin, chloramphenicol, cephalosporins, ciprofloxacin, ciprofloxacin, erythromycin, fluoroquinolones, macrolides, metronidazole, penicillins, quinolones, rifampin, streptomycin, sulfonamide, tetracyclines, trimethoprim, trimethoprim-sulfamthoxazole, and vancomycin.

Conventional nonspecific immunosuppressive agents, that may be administered in combination with the compositions of the invention include, but are not limited to, steroids, cyclosporine, cyclosporine analogs, cyclophosphamide methylprednisone, prednisone, azathioprine, FK-506, 15-deoxyspergualin, and other immunosuppressive agents that act by suppressing the function of responding T cells.

Additional immunosuppressants preparations that may be administered with the compositions of the invention include, but are not limited to, ORTHOCLONE™ (OKT3), SANDIMMUNE™/NEORAL™/SANGDYA™ (cyclosporin), PROGRAF™ (tacrolimus), CELLCEPT™ (mycophenolate), Azathioprine, glucorticosteroids, and RAPAMUNE™ (sirolimus). In a specific embodiment, immunosuppressants may be used to prevent rejection of organ or bone marrow transplantation.

In an additional embodiment, compositions of the invention are administered alone or in combination with one or more intravenous immune globulin preparations. Intravenous immune globulin preparations that may be administered with the compositions of the invention include, but not limited to, GAMMAR™, IVEEGAM™, SANDOGLOBULIN™, GAMMAGARD S/D™, and GAMIMUNE™. In a specific embodiment, compositions of the invention are administered in combination with intravenous immune globulin preparations in transplantation therapy (e.g., bone marrow transplant).

In an additional embodiment, the compositions of the invention are administered alone or in combination with an anti-inflammatory agent. Anti-inflammatory agents that may be administered with the compositions of the invention include. but are not limited to, glucocorticoids and the nonsteroidal anti-inflammatories, aminoarylcarboxylic acid derivatives, arylacetic acid derivatives, arylbutyric acid derivatives, arylcarboxylic acids, arylpropionic acid derivatives, pyrazoles, pyrazolones, salicylic acid derivatives, thiazinecarboxamides, e-acetamidocaproic acid. S-adenosylmethionine, 3-amino-4-hydroxybutyric acid, amixetrine, bendazac, benzydamine, bucolome. difenpiramide, ditazol, emorfazone, guaiazulene, nabumetone, nimesulide, orgotein, oxaceprol, paranyline, perisoxal, pifoxime, proquazone, proxazole, and tenidap.

In another embodiment, compostions of the invention are administered in combination with a chemotherapeutic agent. Chemotherapeutic agents that may be administered with the compositions of the invention include, but are not limited to, antibiotic derivatives (e.g., doxorubicin, bleomycin, daunorubicin, and dactinomycin); antiestrogens (e.g., tamoxifen); antimetabolites (e.g., fluorouracil, 5-FU, methotrexate, floxuridine, interferon alpha-2b, glutamic acid, plicamycin, mercaptopurine, and 6-thioguanine); cytotoxic agents (e.g., carmustine, BCNU, lomustine, CCNU, cytosine arabinoside, cyclophosphamide, estramustine, hydroxyurea, procarbazine, mitomycin, busulfan, cis-platin, and vincristine sulfate); hormones (e.g., medroxyprogesterone, estramustine phosphate sodium, ethinyl estradiol, estradiol, megestrol acetate, methyltestosterone, diethylstilbestrol diphosphate, chlorotrianisene, and testolactone); nitrogen mustard derivatives (e.g., mephalen, chorambucil, mechlorethamine (nitrogen mustard) and thiotepa); steroids and combinations (e.g., bethamethasone sodium phosphate); and others (e.g., dicarbazine, asparaginase, mitotane, vincristine sulfate, vinblastine sulfate, and etoposide).

In a specific embodiment, compositions of the invention are administered in combination with CHOP (cyclophosphamide, doxorubicin, vincristine, and prednisone) or any combination of the components of CHOP. In another embodiment, compositions of the invention are administered in combination with Rituximab. In a further embodiment, compositions of the invention are administered with Rituxmab and CHOP, or Rituxmab and any combination of the components of CHOP.

In an additional embodiment, the compositions of the invention are administered in combination with cytokines. Cytokines that may be administered with the compositions of the invention include, but are not limited to, GM-CSF, G-CSF, IL-1 alpha, IL-1beta, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, anti-CD40, CD40L, IFN-gamma and TNF-alpha. In one embodiment, the compositions of the invention are administered in combination with one or more chemokines. In specific embodiments, the compositions of the invention are administered in combination with an $\alpha$(CxC) chemokine selected from the group consisting of gamma-interferon inducible protein-10 ($\gamma$IP-10), interleukin-8 (IL-8), platelet factor-4 (PF4), neutrophil activating protein (NAP-2), GRO-$\alpha$,.GRO-$\beta$, GRO-$\gamma$, neutrophil-activating peptide (ENA-78), granulocyte chemoattractant protein-2 (GCP-2), and stromal cell-derived factor- I (SDF-1, or pre-B cell stimulatory factor (PBSF)); and/or a $\beta$(CC) chemokine selected from the group consisting of: RANTES (regulated on activation, normal T expressed and secreted), macrophage inflammatory protein-1 alpha (MIP-1$\alpha$), macrophage inflammatory protein-1 beta (MIP-1$\beta$), monocyte chemotactic protein-1 (MCP-1), monocyte chemotactic protein-2 (MCP-2), monocyte chemotactic protein-3 (MCP-3), monocyte chemotactic protein-4 (MCP-4) macrophage inflammatory protein-1 gamma (MIP-1$\gamma$), macrophage inflammatory protein-3 alpha (MIP-3$\alpha$), macrophage inflammatory protein-3 beta (MIP-3$\beta$), macrophage inflammatory protein-4 (MIP-4/DC-CK-1/PARC), eotaxin, Exodus, and I-309; and/or the $\gamma$(C) chemokine, lymphotactin.

In a preferred embodiment, the compositions of the invention are administered in combination with Stem Cell Factor or IL-3. In a most preferred embodiment the compositions of the invention are administered in combination with Stem Cell Factor and IL-3.

In an additional embodiment, the compositions of the invention are administered in combination with Fibroblast Growth Factors. Fibroblast Growth Factors that may be administered with the compositions of the invention include, but are not limited to, FGF-1, FGF-2, FGF-3, FGF-4, FGF-5, FGF-6, FGF-7, FGF-8, FGF-9, FGF-10, FGF-11, FGF-12, FGF-13, FGF-14, and FGF-15.

The invention also encompasses combining the polynucleotides and/or polypeptides of the invention (and/or agonists or antagonists thereof) with other proposed or conventional hematopoietic therapies. Thus, for example, the polynucleotides and/or polypeptides of the invention (and/or agonists or antagonists thereof) can be combined with compounds that singly exhibit erythropoietic stimulatory effects, such as erythropoietin, testosterone, progenitor cell stimulators, insulin-like growth factor, prostaglandins, serotonin, cyclic AMP, prolactin, and triiodothyzonine. Also encompassed are combinations of the compositions of the invention with compounds generally used to treat aplastic anemia, such as, for example, methenolene, stanozolol, and nandrolone; to treat iron-deficiency anemia, such as, for example, iron preparations; to treat malignant anemia, such as, for example, vitamin $B_{12}$ and/or folic acid; and to treat hemolytic anemia, such as, for example, adrenocortical steroids, e.g., corticoids. See e.g., Resegotti et al., Panminerva Medica, 23:243-248 (1981): Kurtz, FEBS Letters, 14a:105-108 (1982): McGonigle et al., Kidney Int., 25:437-444 (1984); and Pavlovic-Kantera, Expt. Hematol., 8(supp. 8) 283-291 (1980), the contents of each of which are hereby incorporated by reference in their entireties.

Compounds that enhance the effects of or synergize with erythropoietin are also useful as adjuvants herein, and include but are not limited to, adrenergic agonists, thyroid hormones, androgens, hepatic erythropoietic factors, erythrotropins. and erythrogenins, See for e.g., Dunn, "Current Concepts in Erythropoiesis", John Wiley and Sons (Chichester, England, 1983); Kalmani, Kidney Int., 22:383-391 (1982); Shahidi, New Eng. J. Med., 289:72-80 (1973); Urabe et al., J. Exp. Med., 149:1314-1325 (1979); Billat et al., Expt. Hematol., 10:133-140 (1982); Naughton et al., Acta Haemat, 69:171-179 (1983); Cognote et al. in abstract 364, Proceedings 7th Intl. Cong. of Endocrinology (Quebec City, Quebec, July 1-7, 1984); and Rothman et al., 1982, J. Surg. Oncol., 20:105-108 (1982). Methods for stimulating hematopoiesis comprise administering a hematopoietically effective amount (i.e., an amount which effects the formation of blood cells) of a pharmaceutical composition containing polynucleotides and/or poylpeptides of the invention (and/or agonists or antagonists thereof) to a patient. The polynucleotides and/or polypeptides of the invention and/or agonists or antagonists thereof is administered to the patient by any suitable technique, including but not limited to, parenteral, sublingual, topical, intrapulmonary and intranasal, and those techniques further discussed herein. The pharmaceutical composition optionally contains one or more members of the group consisting of erythropoietin, testosterone, progenitor cell stimulators, insulin-like growth factor, prostaglandins, serotonin, cyclic AMP, prolactin, triiodothyzonine, methenolene, stanozolol, and nandrolone, iron preparations, vitamin $B_{12}$, folic acid and/or adrenocortical steroids.

In additional prefered embodiments, the compositions of the invention are administered in combination with hematopoietic growth factors. Hematopoietic growth factors that may be administered with the compositions of the invention included, but are not limited to, LEUKINE™ (SARGRAMOSTIM™) and NEUPOGEN™ (FILGRASTIM™).

In additional embodiments, the compositions of the invention are administered in combination with other therapeutic or prophylactic regimens, such as, for example, radiation therapy.

***Chromosome assays***

The nucleic acid molecules of the present invention are also valuable for chromosome identification. The sequence is specifically targeted to and can hybridize with a particular location on an individual human chromosome. The mapping of DNAs to chromosomes according to the present invention is an important first step in correlating those sequences with genes associated with disease.

In certain preferred embodiments in this regard, the cDNA herein disclosed is used to clone genomic DNA of a TR10 receptor gene. This can be accomplished using a variety of well known techniques and libraries, which generally are available commercially. The genomic DNA is then used for *in situ* chromosome mapping using well known techniques for this purpose.

In addition, in some cases, sequences can be mapped to chromosomes by preparing PCR primers (preferably 15-25 bp) from the cDNA. Computer analysis of the 3' untranslated region of the gene is used to rapidly select primers that do not span more than one exon in the genomic DNA, thus complicating the amplification process. These primers are then used for PCR screening of somatic cell hybrids containing individual human chromosomes.

Fluorescence *in situ* hybridization ("FISH") of a cDNA clone to a metaphase chromosomal spread can be used to provide a precise chromosomal location in one step. This technique can be used with cDNA as short as 50 or 60 bp. For a review of this technique, see Verma *et al., Human Chromosomes: a Manual of Basic Techniques,* Pergamon Press, New York (1988).

Once a sequence has been mapped to a precise chromosomal location, the physical position of the sequence on the chromosome can be correlated with genetic map data. Such data are found, for example, in V. McKusick, *Mendelian Inheritance in Man,* available on line through Johns Hopkins University, Welch Medical Library. The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

Having generally described the invention, the same will be more readily understood by reference to the following examples, which are provided by way of illustration and are not intended as limiting.

*Example 1*

*Expression and Purification of the TR10 Receptor in E. coli*

The bacterial expression vector pQE60 is used for bacterial expression in this example. (QIAGEN, Inc., 9259 Eton Avenue, Chatsworth, CA, 91311). pQE60 encodes ampicillin antibiotic resistance ("Amp'") and contains a bacterial origin of replication ("ori"), an IPTG inducible promoter, a ribosome binding site ("RBS"), six codons encoding histidine residues that allow affinity purification using nickel-nitrilo-tri-acetic acid ("Ni-NTA") affinity resin sold by QIAGEN, Inc., *supra.* and suitable single restriction enzyme cleavage sites. These elements are arranged such that a DNA fragment encoding a polypeptide may be inserted in such as way as to produce that polypeptide with the six His residues (i.e., a "6 X His tag") covalently linked to the carboxyl terminus of that polypeptide. However, in this example, the polypeptide coding sequence is inserted such that translation of the six His codons is prevented and, therefore, the polypeptide is produced with no 6 X His tag.

The DNA sequence encoding the desired portion of the TR10 protein lacking the hydrophobic leader sequence is amplified from the deposited cDNA clone using PCR oligonucleotide primers which anneal to the amino terminal sequences of the desired portion of the TR10 protein and to sequences in the deposited construct 3' to the cDNA coding sequence. Additional nucleotides containing restriction sites to facilitate cloning in the pQE60 vector are added to the 5' and 3' sequences, respectively.

For cloning the mature protein, the 5' primer has the sequence: 5'-CGCCCATGGCCACCATCCCCCGGCAG-3' (SEQ ID NO: 10) containing the underlined NcoI restriction site followed by nucleotides complementary to the amino terminal coding sequence of the mature TR10 sequence in Figures 1A-D. One of ordinary skill in the art would appreciate, of course. that the point in the protein coding sequence where the 5' primer begins may be varied to amplify a desired portion of the complete protein shorter or longer than the mature form.

The 3' primer has the sequence: 5'-CGCAAGCTTTTAGTAGTGATAGGGAGAGGC-3' (SEQ ID NO: 11) containing the underlined HindIII site followed by nucleotides complementary to the 3' end of the non-coding sequence in the TR10 DNA sequence in Figures 1A-D.

The amplified TR10 DNA fragments and the vector pQE60 are digested with Nco I and HindIII and the digested DNAs then ligated together. Insertion of the TR10 protein DNA into the restricted pQE60 vector places the TR10 protein coding region (including its associated stop codon) downstream from the

IPTG-inducible promoter and in-frame with an initiating AUG. The associated stop codon prevents translation of the six histidine codons downstream of the insertion point.

The ligation mixture is transformed into competent *E. coli* cells using standard procedures. Such procedures are described in Sambrook *et al., Molecular Cloning: a Laboratory Manual,* 2nd Ed.; Cold Spring Harbor Laboratory Press, Cold Spring Harbor, N.Y. (1989). *E. coli* strain M15/rep4, containing multiple copies of the plasmid pREP4, which expresses lac repressor and confers kanamycin resistance ("Kan'"), is used in carrying out the illustrative example described herein. This strain, which is only one of many that are suitable for expressing TR10 protein, is available commercially from Qiagen, Inc., *supra.*

Transformants are identified by their ability to grow on LB plates in the presence of ampicillin and kanamycin. Plasmid DNA is isolated from resistant colonies and the identity of the cloned DNA confirmed by restriction analysis, PCR, and DNA sequencing.

Clones containing the desired constructs are grown overnight ("O/N") in liquid culture in LB media supplemented with both ampicillin (100 μg/ml) and kanamycin (25 μg/ml). The O/N culture is used to inoculate a large culture, at a dilution of approximately 1:100 to 1:250. The cells are grown to an optical density at 600nm ("OD600") of between 0.4 and 0.6. Isopropyl-B-D-thiogalactopyranoside ("IPTG") is then added to a final concentration of 1 mM to induce transcription from the *lac* repressor sensitive promoter, by inactivating the *lacl* repressor. Cells subsequently are incubated further for 3 to 4 hours. Cells then are harvested by centrifugation.

The cells are then stirred for 3-4 hours at 4°C in 6M guanidine-HCl, pH8. The cell debris is removed by centrifugation, and the supernatant containing the TR10 is loaded onto a nickel-nitrilo-tri-acetic acid ("NiNTA") affinity resin column (available from QIAGEN, Inc., *supra).* Proteins with a 6 x His tag bind to the NI-NTA resin with high affinity and can be purified in a simple one-step procedure (for details see: The QIAexpressionist, 1995, QIAGEN, Inc., *supra).* Briefly the supernatant is loaded onto the column in 6 M guanidine-HCl, pH8, the column is first washed with 10 volumes of 6 M guanidine-HCl, pH8, then washed with 10 volumes of 6 M guanidine-HCl pH6, and finally the TR10 is eluted with 6 M guanidine-HCl, pH5.

The purified protein is then renatured by dialyzing it against phosphatebuffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH7.4, containing protease inhibitors. The renaturation should be performed over a period of 1.5 hours or more. After renaturation the proteins can be eluted by the addition of 250 mM immidazole.

Immidazole is removed by a final dialyzing step against PBS or 50 mM sodium acetate pH6 buffer plus 200 mM NaCl. The purified protein is stored at 4°C or frozen at -80°C.

***Example 2***

***Cloning and Expression of TR10 in a Baculovirus Expression System***

In this illustrative example, the plasmid shuttle vector pA2 is used to insert the cloned DNA encoding the complete protein, including its naturally associated secretary signal (leader) sequence, into a baculovirus to express the mature TR10 protein, using standard methods as described in Summers *et al., A Manual of Methods for Baculovirus Vectors and Insect Cell Culture Procedures,* Texas Agricultural Experimental Station Bulletin No. 1555 (1987). This expression vector contains the strong polyhedrin promoter of the *Autographa californica* nuclear polyhedrosis virus (AcMNPV) followed by convenient restriction sites such as BamHI and Asp718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate viable virus that express the cloned polynucleotide.

Many other baculovirus vectors could be used in place of the vector above, such as pAc373, pVL941 and pAcIMI, as one skilled in the art would readily appreciate, as long as the construct provides appropriately located signals for transcription, translation, secretion and the like, including a signal peptide and an in-frame AUG as required. Such vectors are described, for instance, in Luckow *et al., Virology 170:*31-39 (1989).

The cDNA sequence encoding the mature TR10 receptor protein in the deposited clone, lacking the AUG initiation codon and the naturally associated leader sequence shown in Figures 1A-D (SEQ ID NO:2), is amplified using PCR oligonucleotide primers corresponding to the 5' and 3' sequences of the gene.

The 5' primer has the sequence 5' CGCGGATCC GCCATCATGGGALTTTGGGGACAA 3' (SEQ ID NO:12) containing the underlined BamHI restriction enzyme site, an efficient signal for initiation of translation in eukaryotic cells, as described by M. Kozak, J. *Mol. Biol. 196*:947- 950 (1987), followed by bases of the sequence of the mature TR10 protein shown in Figures I A-D, beginning with the indicated N-terminus of the mature protein.

The 3' primer for TR10 has the sequence 5' CGCGGTACCTTAGTAGTGATAGGGAGAGGC 3' (SEQ ID NO: 13) containing the underlined Asp718 restriction site followed by nucleotides complementary to the 3' noncoding sequence in Figures 1A-D.

The amplified fragment is isolated from a 1% agarose gel using a commercially available kit ("Geneclean, " BIO 10 1 -Inc., La Jolla. Ca.) The fragment then is digested with BamHl and Asp718 and again is purified on a 1% agarose gel. This fragment is designated "F1."

The plasmid is digested with the restriction enzyme Bam HI and optionally can be dephosphorylated using calf intestinal phosphatase, using routine procedures known in the art. The DNA is then isolated from a 1% agarose gel using a commercially available kit ("Geneclean" BIO 101 Inc., La Jolla, Ca.). The vector DNA is designated herein "V 1."

Fragment F1 and the dephosphorylated plasmid V 1 are ligated together with T4 DNA ligase. *E. coli* HB 101 or other suitable *E. coli* hosts such as XL- 1 Blue (Stratagene Cloning Systems, La Jolla, CA) cells are transformed with the ligation mixture and spread on culture plates. Bacteria are identified that contain the plasmid with the human TR10 gene using the PCR method, in which one of the primers that is used to amplify the gene and the second primer is from well within the vector so that only those bacterial colonies containing the TR10 gene fragment will show amplification of the DNA. The sequence of the cloned fragment is confirmed by DNA sequencing. This plasmid is designated herein pBacTR10.

Five ug of the plasmid pBacTR10 is co-transfected with 1.0 ug of a commercially available linearized baculovirus DNA ("BaculoGold™ baculovirus DNA", Pharmingen, San Diego, CA.), using the lipofectin method described by Feloner *et al., Proc. Natl. Acad. Sci. USA 84*:7413-7417 (1987). 1 ug of BaculoGold™ virus DNA and 5 ug of the plasmid pBacTR10 are mixed in a sterile well of a microliter plate containing 50 ul of serum free Grace's medium (Life Technologies, Inc., Rockville, MD). Afterwards, 10 ul Lipofectin plus 90 ☐l Grace's medium are added, mixed, and incubated for 15 minutes at room temperature. Then, the transfection mixture is added drop-wise to Sf9 insect cells (ATCC CRL 1711) seeded in a 35 mm tissue culture plate with 1 ml Grace's medium without serum. The plate is rocked back and forth to mix the newly added solution. The plate is then incubated for 5 hours at 27°C. After 5 hours, the transfection solution is removed from the plate and 1 ml of Grace's insect medium supplemented with 10% fetal calf serum is added. The plate is put back into an incubator and cultivation is continued at 27°C for four days.

After four days, the supernatant is collected and a plaque assay is performed, as described by Summers and Smith, cited above. An agarose gel with "Blue Gal" (Life Technologies, Inc., Rockville, MD) is used to allow easy identification and isolation of gal-expressing clones, which produce blue-stained plaques. (A detailed description of a "plaque assay" of this type can also be found in the user's guide for insect cell culture and baculovirology distributed by Life Technologies, Inc., Rockville, MD, pages 9-10). After appropriate incubation, blue stained plaques are picked with the tip of a micropipettor (e.g., Eppendorf). The agar containing the recombinant viruses is then resuspended in a microcentrifuge tube containing 200 ul of Grace's medium and the suspension containing the recombinant baculovirus is used to infect Sf9 cells seeded in 35 mm dishes. Four days later the supernatants of these culture dishes are harvested and then they are stored at 4°C. The recombinant virus is called V-TR10.

To verify the expression of the TR10gene, Sf9 cells are grown in Grace's medium supplemented with 10% heat inactivated FBS. The cells are infected with the recombinant baculovirus V-TR10 at a multiplicity of infection ("MOI") of about 2. Six hours later the medium is removed and is replaced with SF900 II medium minus methionine and cysteine (available from Life Technologies, Inc.. Rockville, MD). If radiolabeled proteins are desired, 42 hours later, 5 uCi of $^{35}$S-methionine and 5 uCi $^{35}$S-cysteine (available from Amersham) are added. The cells are further incubated for 16 hours and then they are harvested by centrifugation. The proteins in the supernatant as well as the intracellular proteins are analyzed by SDS-PAGE followed by autoradiography (if radiolabeled). Microsequencing of the amino acid sequence of the amino terminus of purified protein may be used to determine the amino terminal sequence of the mature protein and thus the cleavage point and length of the secretory signal peptide.

***Example 3***

***Cloning and Expression of the TR10 Receptor in Mammalian Cells***

A typical mammalian expression vector contains the promoter element, which mediates the initiation of transcription of mRNA, the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRs) from Retroviruses, e.g. RSV, HTLVI, HIVI and the early promoter of the cytomegalovirus (CMV). However, cellular signals can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). Mammalian host cells that could be used include, human Hela 293, H9 and Jurkat cells, mouse NIH3T3 and C127 cells, Cos 1, Cos 7 and CV1, quail QC1-3 cells, mouse L cells, and Chinese hamster ovary (CHO) cells.

Alternatively, the gene can be expressed in stable cell lines that contain the gene integrated into a chromosome. Co-transfection with a selectable marker such as dhfr, gpt, neomycin, or hygromycin allows the identification and isolation of the transfected cells.

The transfected gene can also be amplified to express large amounts of the encoded protein. The dihydrofolate reductase (DHFR) marker is useful to develop cell lines that carry several hundred or even several thousand copies of the gene of interest. Another useful selection marker is the enzyme glutamine synthase (GS) (Murphy *et al., Biochem. J.* 227:277-279 (1991); Bebbington *et al., Bio/Technology 10:*169-175 (1992)). Using these markers, the mammalian cells are grown in selective medium and the cells with the highest resistance selected. These cell lines contain the amplified gene(s) integrated into a chromosome. Chinese hamster ovary (CHO) cells are often used for the production of proteins.

The expression vectors pC1 and pC4 contain the strong promoter (LTR) of the Rous Sarcoma Virus (Cullen *et al., Molecular and Cellular Biology* 5:438- 447 (March 1985)), plus a fragment of the CMV-enhancer (Boshart *et al., Cell 41*:521-530 (1985)). Multiple cloning sites, e.g., with the restriction enzyme cleavage sites BamHI, XbaI and Asp718, facilitate the cloning of the gene of interest. The vectors contain in addition the 3' intron, the polyadenylation and termination signal of the rat preproinsulin gene.

***Example 3A***

***Cloning and Expression of the Extracellular Soluble Domain of TR10 in COS cells***

The expression plasmid, pTR10-HA, is made by cloning a cDNA encoding TR10 into the expression vector pcDNAI/Amp or pcDNAIII (which can be obtained from Invitrogen, Inc.).

The expression vector pcDNAI/amp contains: (1) an *E. coli* origin of replication effective for propagation in *E. coli* and other prokaryotic cell; (2) an ampicillin resistance gene for selection of plasmid-containing prokaryotic cells; (3) an SV40 origin of replication for propagation in eukaryotic cells; (4) a CMV promoter, a polylinker, an SV40 intron, and a polyadenylation signal arranged so that a cDNA conveniently can be placed under expression control of the CMV promoter and operably linked to the SV40 intron and the polyadenylation signal by means of restriction sites in the polylinker.

A DNA fragment encoding the entire TR10 precursor and a HA tag fused in frame to its 3' end is cloned into the polylinker region of the vector so that recombinant protein expression is directed by the CMV promoter. The HA tag corresponds to an epitope derived from the influenza hemagglutinin protein described by Wilson *et al., Cell 37*:767 (1984). The fusion of the HA tag to the target protein allows easy detection of the recombinant protein with an antibody that recognizes the HA epitope.

The plasmid construction strategy is as follows:

The TR10 cDNA of the deposited clone is amplified using primers that contain convenient restriction sites, much as described above regarding the construction of expression vectors for expression of TR10 in *E. coli..*

To facilitate detection, purification and characterization of the expressed TR10, one of the primers contains a hemagglutinin tag ("HA tag") as described above.

Suitable primers for TR10 include the following, which are used in this example:

The 5' primer, 5' CGCGGATCCGCCATCATGGGACTTTGGGGACAA 3' (SEQ ID NO:12) contains the underlined BamHI site, an ATG start codon and 5 codons thereafter. The 3' primer for TR10, which contains the underlined XbaI site, stop codon, hemagglutinin tag, and the last 19 nucleotides of the 3' coding sequence (at the 3' end), has the following sequence:

5'CGCTCTAGATCAAGCGTAGTCTGGGACGTCGTATGGGTAGTAAGT GATAGGGAGAGGC 3' (SEQ ID NO:14).

The PCR amplified DNA fragment and the vector, pcDNAI/Amp, are digested with BamH1 and XbaI and then ligated. The ligation mixture is transformed into *E. coli* strain SURE (available from Stratagene Cloning Systems, 11099 North Torrey Pines Road, La Jolla, CA 92037) the transformed culture is plated on ampicillin media plates which then are incubated to allow growth of ampicillin resistant colonies. Plasmid DNA is isolated from resistant colonies and examined by restriction analysis and gel sizing for the presence of the TRIO-encoding fragment.

For expression of recombinant TR10, COS cells are transfected with an expression vector, as described above, using DEAE-DEXTRAN, as described, for instance, in Sambrook *et al., Molecular Cloning: a Laboratory Manual,* Cold Spring Laboratory Press, Cold Spring Harbor, NY (1989). Cells are incubated under conditions for expression of TR10 by the vector.

Expression of the TR10-HA fusion protein is detected by radiolabelling and immunoprecipitation. using methods described in, for example Harlow *et al., Antibodies: a Laboratory Manual,* 2nd Ed., Cold Spring Harbor Laboratory Press, Cold Spring Harbor, NY (1988). To this end, two days after transfection, the cells are labeled by incubation in media containing $^{35}$S-cysteine for 8 hours. The cells and the media are collected, and the cells are washed and then lysed with detergent-containing RIPA buffer: 150 mM NaCl, 1% NP-40, 0.1% SDS, 1% NP-40, 0.5% DOC, 50 mM TRIS, pH 7.5, as described by Wilson *et al.* cited above. Proteins are precipitated from the cell lysate and from the culture media using an HA-specific monoclonal antibody. The precipitated proteins then are analyzed by SDS-PAGE gels and autoradiography. An expression product of the expected size is seen in the cell lysate, which is not seen in negative controls.

### *Example 3B*

### *Cloning and Expression of TR10 using the CHO Expression System*

The vector pC4 is used for the expression of the TR10 polypeptide. Plasmid pC4 is a derivative of the plasmid pSV2-dhfr (ATCC Accession No. 37146). The plasmid contains the mouse DHFR gene under control of the SV40 early promoter. Chinese hamster ovary- or other cells lacking dihydrofolate activity that are transfected with these plasmids can be selected by growing the cells in a selective medium (alpha minus MEM, Life Technologies, Rockville, MD) supplemented with the chemotherapeutic agent methotrexate (MTX). The amplification of the DHFR genes in cells resistant to MTX has been well documented (see, e.g., F.W. Alt *et al., J. Biol. Chem. 253*:1357-1370 (1978); J.L. Hamlin and C. Ma, *Biochem. et Biophys. Acta 1097:*107-143 (1990); M.J. Page M.A. Sydenham, *Biotechnology 9*:64-68(1991)). Cells grown in increasing concentrations of MTX develop resistance to the drug by overproducing the target enzyme, DHFR, as a result of amplification of the DHFR gene. If a second gene is linked to the DHFR gene, it is usually co-amplified and over-expressed. It is known in the art that this approach may be used to develop cell lines carrying more than 1,000 copies of the amplified gene(s). Subsequently, when the methotrexate is withdrawn, cell lines are obtained that contain the amplified gene integrated into one or more chromosome(s) of the host cell.

Plasmid pC4 contains, for expressing the gene of interest, the strong promoter of the long terminal repeat (LTR) of the Rous Sarcoma Virus (Cullen *et al., Molecular and Cellular Biology 5*:438-447 (March 1985)), plus a fragment isolated from the enhancer of the immediate early gene of human cytomegalovirus (CMV) *(Boshart et al., Cell 41*:521-530 (1985)). Downstream of the promoter are the following single restriction enzyme cleavage sites that allow the integration of the genes: BamHI, XbaI, and Asp718. Behind these cloning sites, the plasmid contains the 3' intron and the polyadenylation site of the rat preproinsulin gene. Other high efficiency promoters can also be used for the expression, e.g., the human B-actin promoter, the SV40 early or late promoters or the long terminal repeats from other retroviruses, e.g., HIV and HTLVI. Clontech's Tet-Off and Tet-On gene expression systems and similar systems can be used to express the TR10 polypeptide in a regulated way in mammalian cells. For the polyadenylation of the mRNA, other signals, e.g., from the human growth hormone or globin genes, can be used as well.

Stable cell lines carrying a gene of interest integrated into the chromosomes can also be selected upon co-transfection with a selectable marker such as gpt, G418, or hygromycin. It is advantageous to use more than one selectable marker in the beginning, e.g., G418 plus methotrexate.

The plasmid pC4 is digested with the restriction enzyme BamHI and then dephosphorylated using calf intestinal phosphates, by procedures known in the art. The vector is then isolated from a 1% agarose gel.

The DNA sequence encoding the complete TR10 polypeptide is amplified using PCR oligonucleotide primers corresponding to the 5' and 3' sequences of the desired portion of the gene.

The 5' oligonucleotide primer for TR10, containing the underlined BamHI restriction site, a Kozak sequence, and an AUG start codon, has the sequence: 5' CGCGGATCCGCCATCATGGGACTTTGGGGACAA 3' (SEQ ID NO: 12). The 3' primer for TR 10, containing the underlined Asp718 restriction site, has the sequence:

5' CGCGGTACCTTAGTAGTGATAGGGAGAGGC 3' (SEQ ID NO:13).

The amplified fragment is digested with BamHI and then purified again on a 1% agarose gel. The isolated fragment and the dephosphorylated vector are then ligated with T4 DNA ligase. E. coli HB101 or XL-1 Blue cells are then transformed and bacteria are identified that contain the fragment inserted into plasmid pC4 using, for instance, restriction enzyme analysis.

Chinese hamster ovary cells lacking an active DHFR enzyme are used for transfection. Five ug of the expression plasmid pC4 are cotransfected with 0.5 ug of the plasmid pSVneo using the lipofectin method (Felgner *et al., supra).* The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of MTX plus 1 mg/ml G418. After about 10-14 days, single clones are trypsinized and then seeded in 6-well petri dishes or 10 ml flasks using different concentrations of methotrexate (50 nM, 100 nM, 200 nM, 400 nM, 800nM). Clones growing at the highest concentrations of methotrexate are then transferred to new 6-well plates containing even higher concentrations of methotrexate (1 uM, 2 uM, 5 uM, 10 uM, 20 uM). The same procedure is repeated until clones are obtained which grow at a concentration of 100-200 uM. Expression of the desired gene product is analyzed, for instance, by Western blot analysis and SDS-PAGE, or by reversed phase HPLC analysis.

### *Example 4*

### *Protein Fusions of TR10*

TR10 polypeptides of the invention are optionally fused to other proteins. These fusion proteins can be used for a variety of applications. For example, fusion of TR10 polypeptides to His-tag, HA-tag. protein A. IgG domains. and maltose binding protein facilitates purification. (See EP A 394,827; *Traunecker, et al., Nature 331*:84-86 (1988)). Similarly, fusion to IgG-1, IgG-3, and albumin increases the halflife time in vivo. Nuclear localization signals fused to TR10 polypeptides can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made using techniques known in the art or by using or routinely modifying the following protocol, which outlines the fusion of a polypeptide to an IgG molecule.

Briefly, the human Fc portion of the IgG molecule can be PCR amplified, using primers that span the 5' and 3' ends of the sequence described below (SEQ ID NO:16). These primers also preferably contain convenient restriction enzyme sites that will facilitate cloning into an expression vector, preferably a mammalian expression vector.

For example, if the pC4 (Accession No. 209646) expression vector is used, the human Fc portion can be ligated into the BamHI cloning site. Note that the 3' BamHI site should be destroyed. Next, the vector containing the human Fc portion is re-restricted with BamHI, linearizing the vector, and TR10 polynucleotide, isolated by the PCR protocol described in Example 1, is ligated into this BamHI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e.g., WO 96/34891.)

Human IgG Fc region:

GGGATCCGGAGCCCAAATCTTCTGACAAAACTCACACATGCCCACCGTGCCCAGCACCTGAATTCG
AGGGTGCACCGTCAGTCTTCCTCTTCCCCCCAAAACCCAAGGACACCCTCATGATCTCCCGGACTC
CTGAGGTCACATGCGTGGTGGTGGACGTAAGCCACGAAGACCCTGAGGTCAAGTTCAACTGGTACG
TGGACGGCGTGGAGGTGCATAATGCCAAGACAAAGCCGCGGGAGGAGCAGTACAACAGCACGTAC
CGTGTGGTCAGCGTCCTCACCGTCCTGCACCAGGACTGGCTGAATGGCAAGGAGTACAAGTGCAAG
GTCTCCAACAAAGCCCTCCCAACCCCCATCGAGAAAACCATCTCCAAAGCCAAAGGGCAGCCCCG
AGAACCACAGGTGTACACCCTGCCCCCATCCCGGGATGAGCTGACCAAGAACCAGGTCAGCCTGA
CCTGCCTGGTCAAAGGCTTCTATCCAAGCGACATCGCCGTGGAGTGGGAGAGCAATGGGCAGCCGG
AGAACAACTACAAGACCACGCCTCCCGTGCTGGACTCCGACGGCTCCTTCTTCCTCTACAGCAAGC
TCACCGTGGACAAGAGCAGGTGGCAGCAGGGGAACGTCTTCTCATGCTCCGTGATGCATGAGGCTC

TGCACAACCACTACACGCAGAAGAGCCTCTCCCTGTCTCCGGGTAAATGAGTGCGACGGCCGCGA
CTCTAGAGGAT (SEQ ID NO:16)

***Example 5***

***TR10 Induced Apoptosis***

Since the entire sequence of TR10, especially its extracellular cysteine-rich domains, is highly homologous to that of other TRAIL receptors, the ability of TRAIL to bind TR10 and induce apoptosis was assessed.

***Experimental Design***

To facilitate detection, TR10 (corresponding to amino acid residues 56-386 of Figures 1A-D) was cloned into pCMV2FLAG (IBI Kodak) as an in-frame fusion to the signal sequence and FLAG-epitope tag encoded by the vector. The cDNA encoding the extracellular domain of TR10 (corresponding to amino acid residues 56-210 of Figures 1A-D) was obtained by PCR, similar to the methods described above, and subcloned into a modified pCMV1FLAG vector that allowed for in-frame fusion with the Fc portion of human IgG. DR4-Fc, TNRF1-Fc, Fc and soluble TRAIL and TNF alpha expression constructs have been described previously, Pan, G. *et al., Science 276*:111-113 (1997), which is incorporated herein by reference in its entirety.

The receptor-Fc fusions and soluble ligands were prepared and in vivo binding was performed as previously described, Pan G. *et al., Science 276*:111-113 (1997), and Pan G. *et al., Science 277*:815-818 (1997), both of which are incorporated herein by reference in their entirety.

Cell death blocking assays using receptor-Fc fusions were carried out as described previously by Pan G. *et al., Science 276:*111-113 (1997), and Pan G. et *al., Science 277*:815-818 (1997), both of which are incorporated herein by reference in their entirety.

***Results***

The extracellular domain of TR10 was expressed as a secreted chimera fused to the Fc portion of human IgG in 293 cells. Conditioned medium from transfected cells was mixed with bacterially expressed soluble His-FLAG-tagged TRAIL. The resulting complex was precipitated with protein G-Sepharose and bound TRAIL detected by Western blotting with anti-FLAG antibody. Like DR4, DR5, and TR5 (TRID), TR10 bound TRAIL. Corroborating this ability to bind TRAIL was the finding that TR10-Fc, like DR4-Fc, could efficiently block TRAIL-induced apoptosis.

In keeping with TR10 possessing a truncated non-functional death domain was the observation that TR10 overexpression did not cause cell death in Hela cells. and as might be expected, could act as a dominant negative receptor antagonizing TRAIL-induced apoptosis. Therefore, ectopic expression of TR10, like that of the decoy receptor TR5, is capable of substantially attenuating TRAIL-induced cell death, suggesting that TR10 antagonizes TRAIL signaling.

***Example 6***

***Production of an Antibody***

a) Hybridoma Technology

The antibodies of the present invention can be prepared by a variety of methods. (See, Current Protocols, Chapter 2.) As one example of such methods, cells expressing TR10 are administered to an animal to induce the production of sera containing polyclonal antibodies. In a preferred method, a preparation of TR10 protein is prepared and purified to render it substantially free of natural contaminants. Such a preparation is then introduced into an animal in order to produce polyclonal antisera of greater specific activity.

Monoclonal antibodies specific for TR10 protein are prepared using hybridoma technology. (Kohler et al., Nature 256:495 (1975); Kohler et al., Eur. J. Immunol. 6:511 (1976); Kohler et al., Eur. J. Immunol. 6:292 (1976); Hammerling et al., in: Monoclonal Antibodies and T-Cell Hybridomas, Elsevier, N.Y., pp. 563-681 (1981)). In general, an animal (preferably a mouse) is immunized with TR10 polypeptide or, more preferably, with a secreted TR10 polypeptide-expressing cell. Such polypeptide-expressing cells are cultured in any suitable tissue culture medium, preferably in Earle's modified Eagle's medium supplemented with 10% fetal bovine serum (inactivated at about 56°C), and supplemented with about 10 g/l of nonessential amino acids, about 1,000 U/ml of penicillin, and about 100 $\mu$g/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP20), available from the ATCC. After fusion, the resulting hybridoma cells are selectively maintained in HAT medium, and then cloned by limiting dilution as described by Wands et al. (Gastroenterology 80:225-232 (1981). The hybridoma cells obtained through such a selection are then assayed to identify clones which secrete antibodies capable of binding the TR10 polypeptide.

Alternatively, additional antibodies capable of binding to TR10 polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the TR10 protein-specific antibody can be blocked by TR10. Such antibodies comprise anti-idiotypic antibodies to the TR10 protein-specific antibody and are used to immunize an animal to induce formation of further TR10 protein-specific antibodies.

For in vivo use of antibodies in humans, an antibody is "humanized". Such antibodies can be produced using genetic constructs derived from hybridoma cells producing the monoclonal antibodies described above. Methods for producing chimeric and humanized antibodies are known in the art and are discussed infra. (See, for review, Morrison. Science 229:1202 (1985); Oi et al., BioTechniques 4:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).)

b) Isolation Of Antibody Fragments Directed

Against TR10 From A Library Of scFvs

Naturally occurring V-genes isolated from human PBLs are constructed into a library of antibody fragments which contain reactivities against TR10 to which the donor may or may not have been exposed (see e.g., U.S. Patent 5,885,793 incorporated herein by reference in its entirety).

Rescue of the Library. A library of scFvs is constructed from the RNA of human PBLs as described in PCT publication WO 92/01047. To rescue phage displaying antibody fragments, approximately 109 E. coli harboring the phagemid are used to inoculate 50 ml of 2xTY containing 1% glucose and 100 $\mu$g/ml of ampicillin (2xTY-AMP-GLU) and grown to an O.D. of 0.8 with shaking. Five ml of this culture is used to innoculate 50 ml of 2xTY-AMP-GLU, 2 x 108 TU of delta gene 3 helper (M13 delta gene III, see PCT publication WO 92/01047) are added and the culture incubated at 37°C for 45 minutes without shaking and then at 37°C for 45 minutes with shaking. The culture is centrifuged at 4000 r.p.m. for 10 min. and the pellet resuspended in 2 liters of 2xTY containing 100 μg/ml ampicillin and 50 ug/ml kanamycin and grown overnight. Phage are prepared as described in PCT publication WO 92/01047.

M13 delta gene III is prepared as follows: M13 delta gene III helper phage does not encode gene III protein, hence the phage(mid) displaying antibody fragments have a greater avidity of binding to antigen. Infectious M13 delta gene III particles are made by growing the helper phage in cells harboring a pUC19 derivative supplying the wild type gene III protein during phage morphogenesis. The culture is incubated for 1 hour at 37° C without shaking and then for a further hour at 37°C with shaking. Cells are spun down (IEC-Centra 8,400 r.p.m. for 10 min), resuspended in 300 ml 2xTY broth containing 100 μg ampicillin/ml and 25 μg kanamycin/ml (2xTY-AMP-KAN) and grown overnight, shaking at 37°C. Phage particles are purified and concentrated from the culture medium by two PEG-precipitations (Sambrook et al., 1990), resuspended in 2 ml PBS and passed through a 0.45 μm filter (Minisart NML; Sartorius) to give a final concentration of approximately 1013 transducing units/ml (ampicillin-resistant clones).

Panning of the Library. Immunotubes (Nunc) are coated overnight in PBS with 4 ml of either 100 μg/ml or 10 μg/ml of a polypeptide of the present invention. Tubes are blocked with 2% Marvel-PBS for 2 hours at 37°C and then washed 3 times in PBS. Approximately 1013 TU of phage is applied to the tube and incubated for 30 minutes at room temperature tumbling on an over and under turntable and then left to stand for another 1.5 hours. Tubes are washed 10 times with PBS 0.1 % Tween-20 and 10 times with PBS. Phage are eluted by adding 1 ml of 100 mM triethylamine and rotating 15 minutes on an under and over turntable after which the solution is immediately neutralized with 0.5 ml of 1.0M Tris-HCl, pH 7.4. Phage are then used to infect 10 ml of mid-log E. coli TG 1 by incubating eluted phage with bacteria for 30 minutes at 37°C. The E. coli are then plated on TYE plates containing 1 % glucose and 100 μg/ml ampicillin. The resulting bacterial library is then rescued with delta gene 3 helper phage as described above to prepare phage for a subsequent round of selection. This process is then repeated for a total of 4 rounds of affinity purification with tube-washing increased to 20 times with PBS, 0.1 % Tween-20 and 20 times with PBS for rounds 3 and 4.

Characterization of Binders. Eluted phage from the 3rd and 4th rounds of selection are used to infect E. coli HB 2151 and soluble scFv is produced (Marks, et al., 1991) from single colonies for assay. ELISAs are performed with microtitre plates coated with either 10 pg/ml of the polypeptide of the present invention in 50 mM bicarbonate pH 9.6. Clones positive in ELISA are further characterized by PCR fingerprinting (see, e.g., PCT publication WO 92/01047) and then by sequencing.

***Example 7***

***Tissue distribution of TR10 mRNA expression***

Northern blot analysis was carried out to examine TR10 gene expression in human tissues, using methods described by, among others, Sambrook *et al.,* cited above. A cDNA probe containing the entire nucleotide sequence of the TR10 protein (SEQ ID NO:1) was labeled with $^{32}$p using the *redi*prime™ DNA labeling system (Amersham Life Science), according to manufacturer's instructions. After labeling, the probe was purified using a CHROMA SPIN-100 column (Clontech Laboratories, Inc.), according to manufacturer's protocol number PT1200-1. The purified labeled probe was then used to examine various human tissues for TR10 mRNA.

Multiple Tissue Northern (MTN) blots containing various human tissues (H) or human immune system tissues (IM) were obtained from Clontech and were examined with labeled probe using ExpressHyb™ hybridization solution (Clontech) according to manufacturer's protocol number PT1190-1. Following hybridization and washing, the blots were mounted and exposed to film at -70°C overnight, and films developed according to standard procedures. Expression of TR10 was detected in tissues enriched in lymphocytes including peripheral blood leukocytes (PBLs), fetal liver, lung, kidney, small intestine, colon, keratinocytes, endothelial cells, and monocyte activated tissue. It can be envisaged that TR10 plays a role in lymphocyte homeostasis.

***Northern Blot analysis of TR10 in various cell lines Methods***

***Cells***

Unless stated otherwise, cell lines were obtained from the American Type Culture Collection (Rockville, MD). The myeloid (Koeffler *et al.* (1980); Koeffler (1983); Harris and Ralph (1985); and Tucker *et al.* (1987) and B-cell lines (Jonak *et al.* (1922)) studied represent cell types at different stages of the differentiation pathway. KG 1a and PLB 985 cells (Tucker *et al.* (1987)) were obtained from H.P. Koeffler (UCLA School of Medicine). BJA-B was from Z. Jonak (SmithKline Beecham). TF274, a stromal cell line exhibiting osteoblastic features, was generated from the bone marrow of a healthy male donor (Z. Jonak and K.B. Tan, unpublished). Primary carotid artery endothelial cells were purchased from Clonetics Corp. (San Diego, CA) and monocytes were prepared by differential centrifugation of peripheral blood mononuclear cells and adhesion to tissue culture dish. CD19+, CD4+ and CD8+ cells (>90% pure) were isolated with cell type specific immunomagnetic beads (Drynal, Lake Success, NY).

***RNA Analysis***

Total RNA of adult tissues were purchased from Clonetech (Palo Alto, CA). Total RNA was extracted from cell lines (in exponential growth phase) and primary cells with TriReagent (Molecular Research Center, Inc., Cincinnati, OH). 5 to 7.5 ug of total RNA was fractionated in a 1% agarose gel containing formaldehyde cast in a Wide Mini-Sub Cell gel tray (Bio-Rad, Hercules, CA) as described (Sambrook, et al.) with slight modifications. The formaldehyde concentration was reduced to 0.5M and the RNA was stained prior to electrophoresis with 100□g/ml of etidium bromide that was added to the loading buffer. After electrophoresis with continuous buffer recirculation (60 volts/90 min), the gel was photographed and the RNA was transferred quantitatively to Zeta-probe nylon membrane (Biorad, Hercules, CA) by vacuum-blotting with 25 mM NaOH for 90 min. After neutralization for 5-10 min, with 1M Tris-HCl, pH 7.5 containing 3M NaCl, the blots were prehybridized with 50% formamide, 8% dextran sulfate, 6xSSPE, 0.1 % SDS and 100 ug/ml of sheared and denatured salmon sperm DNA for at least 30 min at 42°C. cDNA inserts labeled with $^{32}$P-dCTP by random priming (Stratagene, La Jolla, CA), were denatured with 0.25M NaOH (10 min at 37°C) and added to the prehybridization solution. After 24-65 hr at 42°C, the blots were washed under high stringency conditions (Sambrook, *et al.)* and exposed to X-ray films.

***Results***

Expression of TR10 was assessed by Northern blot in the following cell lines: HL60 (promyelocytic leukemia), Hela cell S3, K562 (chronic myelogeneous leukemia), MOLT4 (lymphoblast leukemia), Raji (Burkitt's lymphoma), SW480 (colorectal adenocarcinoma), A549 (lung carcinoma), and G361 (melanoma), and could only be detected in Hela cell S3, SW480 (colorectal adenocarcinoma), and the A549 (lung carcinoma) cell lines.

***Example 8***

***Method of Determining Alterations in the TR1O Gene***

RNA is isolated from entire families or individual patients presenting with a phenotype of interest (such as a disease). cDNA is then generated from these RNA samples using protocols known in the art. (See, Sambrook.) The cDNA is then used as a template for PCR, employing primers surrounding regions of interest in SEQ ID NO: 1. Suggested PCR conditions consist of 35 cycles at 95° C for 30 seconds; 60-120 seconds at 52-58° C: and 60-120 seconds at 70° C, using buffer solutions described in Sidransky. D., *et al., Science 252*:706 (1991).

PCR products are then sequenced using primers labeled at their 5' end with T4 polynucleotide kinase, employing SequiTherm Polymerase. (Epicentre Technologies). The intron-exon borders of selected exons of TR10 are also determined and genomic PCR products analyzed to confirm the results. PCR products harboring suspected mutations in TR10 is then cloned and sequenced to validate the results of the direct sequencing.

PCR products of TR10 are cloned into T-tailed vectors as described in Holton, T.A. and Graham, M.W., *Nucleic Acids Research, 19*:1156 (1991) and sequenced with T7 polymerase (United States Biochemical). Affected individuals are identified by mutations in TR10 not present in unaffected individuals.

Genomic rearrangements are also observed as a method of determining alterations in the TR10 gene. Genomic clones isolated using techniques known in the art are nick-translated with digoxigenindeoxy-uridine 5'-triphosphate (Boehringer Manheim), and FISH performed as described in Johnson, Cg. *et al., Methods Cell Biol. 35*:73-99 (1991). Hybridization with the labeled probe is carried out using a vast excess of human cot-1 DNA for specific hybridization to the TR10 genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters. (Johnson, Cv. *et al., Genet. Anal. Tech. Appl., 8*:75 ( 1991 ).) Image collection, analysis and chromosomal fractional length measurements are performed using the 1See Graphical Program System. (Inovision Corporation, Durham, NC.) Chromosome alterations of the genomic region of TR10 (hybridized by the probe) are identified as insertions, deletions, and translocations. These TR10 alterations are used as a diagnostic marker for an associated disease.

***Example 9***

***Method of Detecting Abnormal Levels of TR10 in a Biological Sample***

TR10 polypeptides can be detected in a biological sample, and if an increased or decreased level of TR10 is detected, this polypeptide is a marker for a particular phenotype. Methods of detection are numerous, and thus, it is understood that one skilled in the art can modify the following assay to fit their particular needs.

For example. antibody-sandwich ELISAs are used to detect TR10 in a sample, preferably a biological sample. Wells of a microtiter plate are coated with specific antibodies to TR10, at a final concentration of 0.2 to 10 ug/ml. The antibodies are either monoclonal or polyclonal and are produced using technique known in the art. The wells are blocked so that non-specific binding of TR10 to the well is reduced.

The coated wells are then incubated for > 2 hours at RT with a sample containing TR10. Preferably, serial dilutions of the sample should be used to validate results. The plates are then washed three times with deionized or distilled water to remove unbounded TR10.

Next, 50 ul of specific antibody-alkaline phosphatase conjugate, at a concentration of 25-400 ng, is added and incubated for 2 hours at room temperature. The plates are again washed three times with deionized or distilled water to remove unbounded conjugate.

75 ul of 4-methylumbelliferyl phosphate (MUP) or p-nitrophenyl phosphate (NPP) substrate solution is then added to each well and incubated 1 hour at room temperature to allow cleavage of the substrate and flourescence. The flourescence is measured by a microtiter plate reader. A standard curve is preparded using the experimental results from serial dilutions of a control sample with the sample concentration plotted on the X-axis (log scale) and fluorescence or absorbance on the Y-axis (linear scale). The TR10 polypeptide concentration in a sample is then interpolated using the standard curve based on the measured flourescence of that sample.

***Example 10***

***Method of Treating Decreased Levels of TR10***

The present invention relates to a method for treating an individual in need of a decreased level of TR10 biological activity in the body comprising, administering to such an individual a composition comprising a therapeutically effective amount of TR10 antagonist. Preferred antagonists for use in the present invention are TR10-specific antibodies.

Moreover, it will be appreciated that conditions caused by a decrease in the standard or normal expression level of TR10 in an individual can be treated by administering TR10, preferably in a soluble and/or secreted form. Thus, the invention also provides a method of treatment of an individual in need of an increased level of TR10 polypeptide comprising administering to such an individual a pharmaceutical composition comprising an amount of TR10 to increase the biological activity level of TR10 in such an individual.

For example, a patient with decreased levels of TR10 polypeptide receives a daily dose 0.1-100 ug/kg of the polypeptide for six consecutive days. Preferably, the polypeptide is in a soluble and/or secreted form.

***Example 11***

***Method of Treating Increased Levels of TR10***

The present invention also relates to a method for treating an individual in need of an increased level of TR10 biological activity in the body comprising administering to such an individual a composition comprising a therapeutically effective amount of TR10 or an agonist thereof.

Antisense technology is used to inhibit production of TR10. This technology is one example of a method of decreasing levels of TR10 polypeptide, preferably a soluble and/or secreted form, due to a variety of etiologies, such as cancer.

For example, a patient diagnosed with abnormally increased levels of TR10 is administered intravenously antisense polynucleotides at 0.5, 1.0, 1.5, 2.0 and 3.0 mg/kg day for 21 days. This treatment is repeated after a 7-day rest period if the is determined to be well tolerated.

***Example 12***

***Method of Treatment Using Gene Therapy - Ex Vivo***

One method of gene therapy transplants fibroblasts, which are capable of expressing soluble and/or mature TR10 polypeptides, onto a patient. Generally, fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin) is added. The flasks are then incubated at 37 °C for approximately one week.

At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. *et al., DNA, 7*:219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The cDNA encoding TR10 can be amplified using PCR primers which correspond to the 5' and 3' end encoding sequences respectively. Preferably, the 5' primer contains an EcoRI site and the 3' primer includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is then used to transform *E. coli* HB 101, which are then plated onto agar containing kanamycin for the purpose of confirming that the vector contains properly inserted TR10.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the TR10 gene is then added to the media and the packaging cells transduced with the vector. The packaging cells now produce infectious viral particles containing the TR10 gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether TR10 protein is produced.

The engineered fibroblasts are then transplanted onto the host, either alone or after having been grown to confluence on cytodex 3 microcarrier beads.

### *Example 13*

### *Method of Treatment Using Gene Therapy - In Vivo*

Another aspect of the present invention is using in vivo gene therapy methods to treat disorders, diseases and conditions. The gene therapy method relates to the introduction of naked nucleic acid (DNA, RNA, and antisense DNA or RNA) TR10 sequences into an animal to increase or decrease the expression of the TR10 polypeptide. The TR10 polynucleotide may be operatively linked to a promoter or any other genetic elements necessary for the expression of the TR10 polypeptide by the target tissue. Such gene therapy and delivery techniques and methods are known in the art, see, for example, WO90/11092, WO98/11779; U.S. Patent NO. 5693622, 5705151, 5580859; Tabata H. *et al., Cardiovasc. Res. 35*:470-479 (1997); Chao J. *et al., Pharmacol. Res. 35*:517-522 (1997); Wolff J.A. *Neuromuscul. Disord. 7*:314-318 (1997); Schwartz B. *et al., Gene Ther. 3*:405-41 (1996); Tsurumi Y. *et al., Circulation 94*:3281-3290 (1996) (incorporated herein by reference).

The TR10 polynucleotide constructs may be delivered by any method that delivers injectable materials to the cells of an animal, such as, injection into the interstitial space of tissues (heart, muscle, skin, lung, liver, intestine and the like). The TR10 polynucleotide constructs can be delivered in a pharmaceutically acceptable liquid or aqueous carrier.

The term "naked" polynucleotide, DNA or RNA, refers to sequences that are free from any delivery vehicle that acts to assist, promote, or facilitate entry into the cell, including viral sequences, viral particles, liposome formulations, lipofectin or precipitating agents and the like. However, the TR10 polynucleotides may also be delivered in liposome formulations (such as those taught in Felgner P.L., *et al. Ann. NYAcad. Sci. 772*:126-139 (1995), and Abdallah B., *et al. Biol. Cell 85*(1):1-7 (1995)) which can be prepared by methods well known to those skilled in the art.

The TR10 polynucleotide vector constructs used in the gene therapy method are preferably constructs that will not integrate into the host genome nor will they contain sequences that allow for replication. Any strong promoter known to those skilled in the art can be used for driving the expression of DNA. Unlike other gene therapies techniques, one major advantage of introducing naked nucleic acid sequences into target cells is the transitory nature of the polynucleotide synthesis in the cells. Studies have shown that non-replicating DNA sequences can be introduced into cells to provide production of the desired polypeptide for periods of up to six months.

The TR10 polynucleotide construct can be delivered to the interstitial space of tissues within the an animal, including of muscle, skin, brain, lung, liver, spleen, bone marrow, thymus, heart, lymph, blood, bone, cartilage, pancreas, kidney, gall bladder, stomach, intestine, testis, ovary, uterus, rectum, nervous system, eye, gland, and connective tissue. Interstitial space of the tissues comprises the intercellular fluid, mucopolysaccharide matrix among the reticular fibers of organ tissues, elastic fibers in the walls of vessels or chambers, collagen fibers of fibrous tissues, or that same matrix within connective tissue ensheathing muscle cells or in the lacunae of bone. It is similarly the space occupied by the plasma of the circulation and the lymph fluid of the lymphatic channels. Delivery to the interstitial space of muscle tissue is preferred for the reasons discussed below. They may be conveniently delivered by injection into the tissues comprising these cells. They are preferably delivered to and expressed in persistent, non-dividing cells which are differentiated. although delivery and expression may be achieved in non-differentiated or less completely differentiated cells, such as, for example, stem cells of blood or skin fibroblasts. *In vivo* muscle cells are particularly competent in their ability to take up and express polynucleotides.

For the naked TR10 polynucleotide injection, an effective dosage amount of DNA or RNA will be in the range of from about 0.05 g/kg body weight to about 50 mg/kg body weight. Preferably the dosage will be from about 0.005 mg/kg to about 20 mg/kg and more preferably from about 0.05 mg/kg to about 5 mg/kg. Of course. as the artisan of ordinary skill will appreciate, this dosage will vary according to the tissue site of injection. The appropriate and effective dosage of nucleic acid sequence can readily be determined by those of ordinary skill in the art and may depend on the condition being treated and the route of administration. The preferred route of administration is by the parenteral route of injection into the interstitial space of tissues. However, other parenteral routes may also be used, such as, inhalation of an aerosol formulation particularly for delivery to lungs or bronchial tissues, throat or mucous membranes of the nose. In addition, naked TR10 polynucleotide constructs can be delivered to arteries during angioplasty by the catheter used in the procedure.

The dose response effects of injected TR10 polynucleotide in muscle *in vivo* is determined as follows. Suitable TR10 template DNA for production of mRNA coding for TR10 polypeptide is prepared in accordance with a standard recombinant DNA methodology. The template DNA, which may be either circular or linear, is either used as naked DNA or complexed with liposomes. The quadriceps muscles of mice are then injected with various amounts of the template DNA.

Five to six week old female and male Balb/C mice are anesthetized by intraperitoneal injection with 0.3 ml of 2.5% Avertin. A 1.5 cm incision is made on the anterior thigh, and the quadriceps muscle is directly visualized. The TR10 template DNA is injected in 0.1 ml of carrier in a 1 cc syringe through a 27 gauge needle over one minute, approximately 0.5 cm from the distal insertion site of the muscle into the knee and about 0.2 cm deep. A suture is placed over the injection site for future localization, and the skin is closed with stainless steel clips.

After an appropriate incubation time (e.g., 7 days) muscle extracts are prepared by excising the entire quadriceps. Every fifth 15 um cross-section of the individual quadriceps muscles is histochemically stained for TR10 protein expression. A time course for TR10 protein expression may be done in a similar fashion except that quadriceps from different mice are harvested at different times. Persistence of TR10 DNA in muscle following injection may be determined by Southern blot analysis after preparing total cellular DNA and HIRT supernatants from injected and control mice. The results of the above experimentation in mice can be use to extrapolate proper dosages and other treatment parameters in humans and other animals using TR10 naked DNA.

### *Example 14*

### *Gene Therapy Using Endogenous TR10 Gene*

Another method of gene therapy according to the present invention involves operably associating the endogenous TR10 sequence with a promoter via homologous recombination as described, for example, in US Patent Number 5,641,670, issued June 24, 1997; International Publication Number WO 96/29411, published September 26, 1996; International Publication Number WO 94/12650, published August 4, 1994; Koller *et al., Proc. Natl. Acad. Sci. USA 86*: 8932-8935 (1989); and Zijlstra *et al., Nature 342*:435-438 (1989). This method involves the activation of a gene which is present in the target cells, but which is not expressed in the cells, or is expressed at a lower level than desired. Polynucleotide constructs are made which contain a promoter and targeting sequences, which are homologous to the 5' non-coding sequence of endogenous TR10, flanking the promoter. The targeting sequence will be sufficiently near the 5' end of TR10 so the promoter will be operably linked to the endogenous sequence upon homologous recombination. The promoter and the targeting sequences can be amplified using PCR. Preferably, the amplified promoter contains distinct restriction enzyme sites on the 5' and 3' ends. Preferably, the 3' end of the first targeting sequence contains the same restriction enzyme site as the 5' end of the amplified promoter and the 5' end of the second targeting sequence contains the same restriction site as the 3' end of the amplified promoter.

The amplified promoter and the amplified targeting sequences are digested with the appropriate restriction enzymes and subsequently treated with calf intestinal phosphatase. The digested promoter and digested targeting sequences are added together in the presence of T4 DNA ligase. The resulting mixture is maintained under conditions appropriate for ligation of the two fragments. The construct is size fractionated on an agarose gel then purified by phenol extraction and ethanol precipitation.

In this Example, the polynucleotide constructs are administered as naked polynucleotides via electroporation. However, the polynucleotide constructs may also be administered with transfection-facilitating agents, such as liposomes, viral sequences, viral particles, precipitating agents, etc. Such methods of delivery are known in the art.

Once the cells are transfected, homologous recombination will take place which results in the promoter being operably linked to the endogenous TR10 sequence. This results in the expression of TR10in the cell. Expression may be detected by immunological staining, or any other method known in the art.

Fibroblasts are obtained from a subject by skin biopsy. The resulting tissue is placed in DMEM + 10% fetal calf serum. Exponentially growing or early stationary phase fibroblasts are trypsinized and rinsed from the plastic surface with nutrient medium. An aliquot of the cell suspension is removed for counting, and the remaining cells are subjected to centrifugation. The supernatant is aspirated and the pellet is resuspended in 5 ml of electroporation buffer (20 mM HEPES pH 7.3.137 mM NaCl. 5 mM KCl, 0.7 mM Na2 HPO4, 6 mM dextrose). The cells are recentrifuged, the supernatant aspirated, and the cells resuspended in electroporation buffer containing 1 mg/ml acetylated bovine serum albumin. The final cell suspension contains approximately 3X $10^6$ cells/ml. Electroporation should be performed immediately following resuspension.

Plasmid DNA is prepared according to standard techniques. For example, to construct a plasmid for targeting to the TR10 locus, plasmid pUC18 (MBI Fermentas, Amherst, NY) is digested with Hindlll. The CMV promoter is amplified by PCR with an Xbal site on the 5' end and a BamHI site on the 3'end. Two TR10 non-coding sequences are amplified via PCR: one TR10 non-coding sequence (TR10 fragment 1) is amplified with a HindIII site at the 5' end and an Xba site at the 3'end; the other TR10 non-coding sequence (TR10 fragment 2) is amplified with a BamHl site at the 5'end and a HindIII site at the 3'end. The CMV promoter and TR10 fragments are digested with the appropriate enzymes (CMV promoter - Xbal and BamHI; TR10 fragment I - XbaI; TR10 fragment 2 - BamHl) and ligated together. The resulting ligation product is digested with HindIII, and ligated with the Hindlll-digested pUC18 plasmid.

Plasmid DNA is added to a sterile cuvette with a 0.4 cm electrode gap (Bio- Rad). The final DNA concentration is generally at least 120 $\mu g$/ml. 0.5 ml of the cell suspension (containing approximately 1.5.X $10^6$ cells) is then added to the cuvette, and the cell suspension and DNA solutions are gently mixed. Electroporation is performed with a Gene-Pulser apparatus (Bio-Rad). Capacitance and voltage are set at 960 $\mu$F and 250-300 V, respectively. As voltage increases, cell survival decreases, but the percentage of surviving cells that stably incorporate the introduced DNA into their genome increases dramatically. Given these parameters, a pulse time of approximately 14-20 mSec should be observed.

Electroporated cells are maintained at room temperature for approximately 5 min, and the contents of the cuvette are then gently removed with a sterile transfer pipette. The cells are added directly to 10 ml of prewarmed nutrient media (DMEM with 15% calf serum) in a 10 cm dish and incubated at 37°C. The following day, the media is aspirated and replaced with 10 ml of fresh media and incubated for a further 16-24 hours.

The engineered fibroblasts are then injected into the host, either alone or after having been grown to confluence on cytodex 3 microcarrier beads. The fibroblasts now produce the protein product. The fibroblasts can then be introduced into a patient as described above.

### *Example 15*

### *Bioassay for the effect of TR10 polypeptides, agonists, or antagonists on hematopoietic progenitor cells and/ or differentiation.*

Mouse bone marrow cells are used as target cells to examine the effect of TR10 polypeptides of the invention on hematopoietic progenitor cells and/or differentiation. Briefly, unfractionated bone marrow cells are first washed 2X with a serum-free IMDM that is supplemented with 10% (V/V) BIT (Bovine serum albumin, Insulin and Transferrin supplement from Stem Cell Technologies, Vancouver. Canada). The washed cells are then resuspended in the same growth medium and plated in the 96-well tissue culture plate (5 x $10^4$ cells/well) in 0.2 ml of the above medium in the presence or absence of cytokines and TR10. Stem cell factor (SCF) and IL-3 are included as positive mediators of cell proliferation. Cells are allowed to grow in a low oxygen environment (5% $CO_2$, 7% $O^2$, and 88% $N_2$) tissue culture incubator for 6 days. On the sixth day, 0.5 $\mu$Ci of Tritiated thymidine is added to each well and incubation is continued for an additional 16-18 hours, at which point the cells are harvested. The level of radioactivity incorporated into cellular DNA is determined by scintillation spectrometry and reflects the amount of cell proliferation.

The studies described in this example test the activity of TR10 polypeptides of the invention. However, one skilled in the art could easily modify the exemplified studies to test the activity of TR10 polynucleotides (e.g., gene therapy), agonists, and/or antagonists of TR10. Potential agonists would be expected to inhibit hematopoietic cell proliferation in the presence of SCF and/or IL3 and/or to increase the inhibition of cell proliferation in the presence of cytokines and TR 10 in this assay. Potential antagonists would be expected to reduce the inhibition of cell proliferation in the presence of cytokines and TR10 in this assay.

***Example 16***

***Effect of TR10-fc fusion (i.e., chimera) polypeptides on hematopoietic progenitor cells and/or differentiation***

Mouse bone marrow cells were used as target cells to examine the effect of TR10 polypeptides of the invention on hematopoietic progenitor cells and/or differentiation. Briefly, unfractionated bone marrow cells were first washed 2X with a serum-free IMDM that was supplemented with 10% (V/V) BIT (Bovine serum albumin, Insulin and Transferrin supplement from Stem Cell Technologies, Vancouver, Canada). The washed cells were then resuspended in the same growth medium, plated in a 96-well tissue culture plate (5 x $10^{-4}$ cells/well) in 0.2 ml of the above medium in the presence or absence of cytokines and TR10. Stem cell factor (SCF) and IL-3 were included as positive mediators of cell proliferation. Cells were allowed to grow in a low oxygen environment (5% $CO_2$ 7% $O^2$, and 88% $N_2$) tissue culture incubator for 6 days. On the sixth day, 0.5 μCi of Tritiated thymidine was added to each well and incubation was continued for an additional 16-18 hours, at which point the cells were harvested. The level of radioactivity incorporated into cellular DNA was determined by scintillation spectrometry and reflects the amount of cell proliferation.

TR10-Fc chimera, a Fc fusion of the extracellular portion of TR10 (corresponding to Met-1 to Gly-204 in SEQ ID NO: 2) was tested as described above. As expected, cell proliferation was barely detectable (incorporation of radioactivity) in the absence of stem cell factor (SCF). Addition of TR10-Fc chimera in the absence of SCF had no effect. Incubation of cells in the presence of SCF resulted in a slight stimulation of incorporation of thymidine (Figure 4). This result is not surprising since SCF is a known cell survival factor and SCF by itself possesses little mitogenic effect. This SCF stimulated proliferation was inhibited by TR10-Fc chimera at 1,000 and 5,000 ng/ml doses, whereas lower doses of TR10 had little effect (Figure 4). When SCF was included along with IL-3, there was about four-fold increase in the proliferative activity in the absence of TR10 (Figure 4). Addition of TR10 to cells in the presence of SCF plus IL-3 resulted in a dose-dependent decrease in the cell proliferation response (Figure 4). This effect of TR10 is specific since other TRAIL receptor-Fc chimeras (e.g., Fc fusions of OPG, DR4 (International application publication number WO 98/32856); TRS (International application publication number WO 98/30693); and DR5 (International application publication number WO 98/41629)) that were tested had no effect on SCF+IL-3 stimulated proliferative response (data not shown).

***Example 17***

***Bioassay for the effect of TR10 polypeptides, agonists or antagonists on IL-3 and SCF stimulated proliferation and differentiation of hematopoietic progenitor cells.***

To determine if TR10 polypeptides of the invention inhibit specific hematopoietic lineages, mouse bone marrow cells are first washed 2X with a serum-free IMDM that is supplemented with 10% (V/V) BIT (Bovine serum albumin, Insulin and Transferrin supplement from Stem Cell Technologies, Vancouver, Canada). The washed cells are then resuspended in the same growth medium and plated in the 96-well tissue culture plate (5 x$10^4$ cells/well) in 0.2 ml of the above medium in the presence of IL-3 (1 ng/ml) plus SCF (5 ng/ml) with or without TR10. Cells are allowed to grow in a low oxygen environment (5% $CO_2$, 7% $O^2$, and 88% N,) tissue culture incubator, and after 7 days, analyzed for expression of differentiation antigens by staining with various monoclonal antibodies and FACScan.

The studies described in this example test the activity of TR 10 polypeptides of the invention. However, one skilled in the art could easily modify the exemplified studies to test the activity of TR 10 polynucleotides (e.g., gene therapy), agonists, and/or antagonists of TR10. Potential agonists tested in this assay would be expected to inhibit cell proliferation in the presence of cytokines and/or to increase the inhibition of cell proliferation in the presence of cytokines and TR10. Potential antagonists tested in this assay would be expected to reduce the inhibition of cell proliferation in the presence of cytokines and TR10.

***Example 18***

***Effect of TR10-Fc chimera on IL-3 and SCF stimulated proliferation and differentiation of hematopoietic progenitor cells.***

To determine if TR10 polypeptides of the invention inhibit specific hematopoietic lineages, mouse bone marrow cells were first washed 2X with a serum-free IMDM that was supplemented with 10% (V/V) BIT (Bovine serum albumin, Insulin and Transferrin supplement from Stem Cell Technologies, Vancouver, Canada). The washed cells were then resuspended in the same growth medium and plated in 96-well tissue culture plate (5 x $10^4$ cells/well) in 0.2 ml of the above medium in the presence of IL-3 (1 ng/ml) plus SCF (5 ng/ml) with or without TR10-Fc chimera (corresponding to Met-1 to Gly-204 in SEQ ID NO: 2). Cells were allowed to grow in a low oxygen environment (5% $CO_2$, 7% $O^2$, and 88% $N_2$) tissue culture incubator, and after 7 days, analyzed for expression of differentiation antigens by staining with various monoclonal antibodies and FACScan.

Data from two such experiments show that culturing of cells in the presence of the TRIO-Fc chimera resulted in two- to three-fold increase in the number of C-kit and Sca-1 double positive cells. In contrast, the TR10-Fc chimera had no effect on the number of Gr-1, Mac.1, and Gr-1 and Mac.1 double positive populations of cells. Since TR10 is a decoy receptor for TRAIL and TRAIL is known to cause apoptotic cell death, cells in one of the experiments were stained with Annexin, which is a marker for cells undergoing apoptosis, and propidium iodide, which stains the DNA of dead cells. TR10 had no effect on the number of cells undergoing apoptosis (data not shown).

Thus, while TR10-Fc chimera inhibits mouse bone marrow cell proliferation in a dose dependent manner (See, Example 16 and Figure 4), this effect was not associated with an increase in apoptosis. Interestingly, the TR10-Fc chimera inhibition of mouse bone marrow cell proliferation is associated with an increase in the C-kit and Sca-1 double positive cells.

### *Example 19*

### *Effect of TR10 on IL-3 and SCF stimulated proliferation and differentiation of lin-population of bone marrow cells*

A population of mouse bone marrow cells enriched in primitive hematopoietic progenitors can be obtained using a negative selection procedure, where the committed cells of most of the lineages are removed using a panel of monoclonal antibodies (anti cd11b, CD4, CD8, CD45R and Gr-1 antigens) and magnetic beads. The resulting population of cells (lineage depleted cells) are plated (5 x $10^4$ cells/ml) in the presence or absence of TR10 polypeptide of the invention (in a range of concentrations) in a growth medium supplemented with IL-3 (5 ng/ml) plus SCF (100 ng/ml). After seven days of incubation at 37°C in a humidified incubator (5% $CO_2$, 7% $O_2$, and 88% $N_2$ environment), cells are harvested and assayed for the HPP-CFC, and immature progenitors. In addition, cells are analyzed for the expression of certain differentiation antigens by FACScan. Colony data is expressed as mean number of colonies +/- SD) and are obtained from assays performed in six dishes for each population of cells.

### *Example 20*

### *Assays to detect stimulation or inhibition of B cell proliferation and differentiation*

Generation of functional humoral immune responses requires both soluble and cognate signaling between B-lineage cells and their microenvironment. Signals may impart a positive stimulus that allows a B-lineage cell to continue its programmed development, or a negative stimulus that instructs the cell to arrest its current developmental pathway. To date, numerous stimulatory and inhibitory signals have been found to influence B cell responsiveness including IL-2, IL-4, IL5, IL6, IL-7, IL10, IL-13, IL14 and IL15. Interestingly, these signals are by themselves weak effectors but can, in combination with various co-stimulatory proteins, induce activation, proliferation, differentiation, homing, tolerance and death among B cell populations. One of the best studied classes of B-cell co-stimulatory proteins is the TNF-superfamily. Within this family CD40, CD27, and CD30 along with their respective ligands CD154, CD70, and CD153 have been found to regulate a variety of immune responses. Assays which allow for the detection and/or observation of the proliferation and differentiation of these B-cell populations and their precursors are valuable tools in determining the effects various proteins may have on these B-cell populations in terms of proliferation and differentiation. Listed below are two assays designed to allow for the detection of the differentiation, proliferation, or inhibition of B-cell populations and their precursors.

***a. In Vitro* assay-** Purified TR10 polylpeptides of the invention (e.g., soluble TR10) or agonists or antagonists thereof. is assessed for its ability to induce activation, proliferation, differentiation or inhibition and/or death in B-cell populations and their precursors. The activity of TR10 polypeptides, or agonists or antagonists thereof on purified human tonsillar B cells, measured qualitatively over the dose range from 0.1 to 10,000 ng/ml, is assessed in a standard B-lymphocyte co-stimulation assay in which purified tonsillar B cells are cultured in the presence of either formalin-fixed *Staphylococcus aureus* Cowan I (SAC) or immobilized anti-human IgM antibody as the priming agent. Second signals such as IL-2 and IL-15 synergize with SAC and IgM crosslinking to elicit B cell proliferation as measured by tritiated-thymidine incorporation. Novel synergizing agents can be readily identified using this assay.

The assay involves isolating human tonsillar B cells by magnetic bead (MACS) depletion of CD3-positive cells. The resulting cell population is greater than 95% B cells as assessed by expression of CD45R(B220). Various dilutions of each sample are placed into individual wells of a 96-well plate to which are added $10^5$ B-cells suspended in culture medium (RPMI 1640 containing 10% FBS, 5 X $10^{-5}$M βME, 100U/ml penicillin, 10ug/ml streptomycin, and $10^{-5}$ dilution of SAC) in a total volume of 150ul. Proliferation or inhibition is quantitated by a 20h pulse (luCi/well) with $^3$H-thymidine (6.7 Ci/mM) beginning 72h post factor addition. The positive and negative controls are IL2 and medium respectively.

**b. *In Vivo* assay-** BALB/c mice are injected (i.p.) twice per day with buffer only, or 2 mg/Kg of TR10 polypeptide (e.g., soluble TR10) or agonists or antagonists thereof. Mice receive this treatment for 4 consecutive days, at which time they are sacrificed and various tissues and serum collected for analyses. Comparison of H&E sections from normal and TR10 polypeptide-treated spleens identify the results of the activity of TR10 polypeptide on spleen cells, such as the diffusion of peri-arterial lymphatic sheaths, and/or significant increases in the nucleated cel lularity of the red pulp regions, which may indicate the activation of the differentiation and proliferation of B-cell populations. Immunohistochemical studies using a B cell marker, anti-CD45R(B220), are used to determine whether any physiological changes to splenic cells, such as splenic disorganization, are due to increased B-cell representation within loosely defined B-cell zones that infiltrate established T-cell regions.

Flow cytometric analyses of the spleens from TR10 polypeptide -treated mice is used to indicate whether TR 10 polypeptide specifically increases the proportion of ThB+, CD45R(B220)dull B cells over that which is observed in control mice.

Likewise, a predicted consequence of increased mature B-cell representation *in vivo* is a relative increase in serum lg titers. Accordingly, serum IgM and IgA levels are compared between buffer and TR10 polypeptide-treated mice.

The studies described in this example test the activity in TR10 polypeptide. However, one skilled in the art could easily modify the exemplified studies to test the activity of TR10 polynucleotides (e.g.. gene therapy), and agonists, and/or antagonists of TR10.

### *Example 21*

### *T Cell Proliferation Assay*

A CD3-induced proliferation assay is performed on PBMCs and is measured by the uptake of $^3$H-thymidine. The assay is performed as follows. Ninety-six well plates are coated with 100 μl/well of mAb to CD3 (HIT3a, Pharmingen) or isotype-matched control mAb (B33.1) overnight at 4°C (1 μg/ml in .05M bicarbonate buffer, pH 9.5), then washed three times with PBS. PBMC are isolated by F/H gradient centrifugation from human peripheral blood and added to quadruplicate wells (5 x $10^4$/well) of mAb coated plates in RPMI containing 10% FCS and P/S in the presence of varying concentrations of TR10 protein (total volume 200 μl). Relevant protein buffer and medium alone are controls. After 48 hr. culture at 37°C, plates are spun for 2 min. at 1000 rpm and 100 μl of supernatant is removed and stored -20°C for measurement of IL-2 (or other cytokines) if effect on proliferation is observed. Wells are supplemented with 100 μl of medium containing 0.5 μCi of $^3$H-thymidine and cultured at 37°C for 18-24 hr. Wells are harvested and incorporation of $^3$H-thymidine used as a measure of proliferation. Anti-CD3 alone is the positive control for proliferation. IL-2 (100 U/ml) is also used as a control which enhances proliferation. Control antibody which does not induce proliferation of Tcells is used as the negative controls for the effects of TR10 proteins.

The studies described in this example test the activity in TR10 protein. However, one skilled in the art could easily modify the exemplified studies to test the activity of TR10 polynucleotides (e.g., gene therapy), agonists, and/or antagonists of TR10.

### *Example 22*

### *Effect of TR10 on the Expression of MHC Class II, Costimulatory and Adhesion Molecules and Cell Differentiation of monocytes and Monocyte-Derived Human Dendritic Cells*

Dendritic cells are generated by the expansion of proliferating precursors found in the peripheral blood: adherent PBMC or elutriated monocytic fractions are cultured for 7-10 days with GM-CSF (50 ng/ml) and IL-4 (20 ng/ml). These dendritic cells have the characteristic phenotype of immature cells (expression of CD1, CD80, CD86. CD40 and MHC class 11 antigens). Treatment with activating factors, such as TNF-α. causes a rapid change in surface phenotype (increased expression of MHC class I and 11, costimulatory and adhesion molecules, downregulation of FCγRII, upregulation of CD83). These changes correlate with increased antigen-presenting capacity and with functional maturation of the dendritic cells.

FACS analysis of surface antigens is performed as follows. Cells are treated 1-3 days with increasing concentrations of TR10 or LPS (positive control), washed with PBS containing 1% BSA and 0.02 mM sodium azide, and then incubated with 1:20 dilution of appropriate FITC- or PE-labeled monoclonal antibodies for 30 minutes at 4°C. After an additional wash, the labeled cells are analyzed by flow cytometry on a FACScan (Becton Dickinson).

**Effect on the production of cytokines.** Cytokines generated by dendritic cells, in particular IL-12, are important in the initiation of T-cell dependent immune responses. IL-12 strongly influences the development of Th1 helper T-cell immune response, and induces cytotoxic T and NK cell function. An ELISA is used to measure the IL-12 release as follows. Dendritic cells ($10^6$/ml) are treated with increasing concentrations of TR10 for 24 hours. LPS (100 ng/ml) is added to the cell culture as positive control. Supernatants from the cell cultures are then collected and analyzed for IL-12 content using commercial ELISA kit (e.g., R & D Systems (Minneapolis, MN)). The standard protocols provided with the kits are used. Effect on the expression of MHC Class II, costimulatory and adhesion molecules. Three major families of cell surface antigens can be identified on monocytes: adhesion molecules, molecules involved in antigen presentation, and Fc receptor. Modulation of the expression of MHC class II antigens and other costimulatory molecules, such as B7 and ICAM-1, may result in changes in the antigen presenting capacity of monocytes and ability to induce T cell activation. Increase expression of Fc receptors may correlate with improved monocyte cytotoxic activity, cytokine release and phagocytosis.

FACS analysis is used to examine the surface antigens as follows. Monocytes are treated 1-5 days with increasing concentrations of TR10 or LPS (positive control), washed with PBS containing 1% BSA and 0.02 mM sodium azide, and then incubated with 1:20 dilution of appropriate FITC- or PE-labeled monoclonal antibodies for 30 minutes at 4°C. After an additional wash, the labeled cells are analyzed by flow cytometry on a FACScan (Becton Dickinson).

**Monocyte activation and/or increased survival.** Assays for molecules that activate (or alternatively, inactivate) monocytes and/or increase monocyte survival (or alternatively, decrease monocyte survival) are known in the art and may routinely be applied to determine whether a molecule of the invention functions as an inhibitor or activator of monocytes. TR10, agonists, or antagonists of TR10 can be screened using the three assays described below. For each of these assays, Peripheral blood mononuclear cells (PBMC) are purified from single donor leukopacks (American Red Cross, Baltimore, MD) by centrifugation through a Histopaque gradient (Sigma). Monocytes are isolated from PBMC by counterflow centrifugal elutriation.

***1. Monocyte Survival Assay.*** Human peripheral blood monocytes progressively lose viability when cultured in absence of serum or other stimuli. Their death results from internally regulated process (apoptosis). Addition to the culture of activating factors, such as TNF-alpha dramatically improves cell survival and prevents DNA fragmentation. Propidium iodide (PI) staining is used to measure apoptosis as follows. Monocytes are cultured for 48 hours in polypropylene tubes in serum-free medium (positive control), in the presence of 100 ng/ml TNF-alpha (negative control), and in the presence of varying concentrations of the compound to be tested. Cells are suspended at a concentration of 2 x $10^6$/ml in PBS containing PI at a final concentration of 5 $\mu$g/ml, and then incubated at room temperature for 5 minutes before FAC Scan analysis. PI uptake has been demonstrated to correlate with DNA fragmentation in this experimental paradigm.

***2. Effect on cytokine release.*** An important function of monocytes/macrophages is their regulatory activity on other cellular populations of the immune system through the release of cytokines after stimulation. An ELISA to measure cytokine release is performed as follows. Human monocytes are incubated at a density of $5\times10^5$ cells/ml with increasing concentrations of TR10 and under the same conditions, but in the absence of TR10. For IL-12 production, the cells are primed overnight with IFN- y (100 U/ml) in presence of TR10. LPS (10 ng/ml) is then added. Conditioned media are collected after 24h and kept frozen until use. Measurement of TNF-$\alpha$, IL-10, MCP-1 and IL-8 is then performed using a commercially available ELISA kit (e.g., R & D Systems (Minneapolis, MN)) applying the standard protocols provided with the kit.

***3. Oxidative burst.*** Purified monocytes are plated in 96-well plate at 2-1x$10^5$ cell/well. Increasing concentrations of TR10 are added to the wells in a total volume of 0.2 ml culture medium (RPM1 1640 + 10% FCS, glutamine and antibiotics). After 3 days incubation, the plates are centrifuged and the medium is removed from the wells. To the macrophage monolayers, 0.2 ml per well of phenol red solution (140 mM NaCl, 10 mM potassium phosphate buffer pH 7.0, 5.5 mM dextrose, 0.56 mM phenol red and 19 U/mi of HRPO) is added, together with the stimulant (200 nM PMA). The plates are incubated at 37°C for 2 hours and the reaction is stopped by adding 20 $\mu$l 1N NaOH per well. The absorbance is read at 610 nm. To calculate the amount of $H_2O_2$ produced by the macrophages, a standard curve of a $H_2O_2$ solution of known molarity is performed for each experiment.

The studies described in this example test the activity in TR10 protein. However, one skilled in the art could easily modify the exemplified studies to test the activity of TR10 polynucleotides (e.g., gene therapy), agonists, and/or antagonists of TR10.

### *Example 23*

### *The Effect of TR10 on the Growth of Vascular Endothelial Cells*

On day 1, human umbilical vein endothelial cells (HUVEC) are seeded at 2-5x$10^4$ cells/35 mm dish density in M199 medium containing 4% fetal bovine serum (FBS), 16 units/ml heparin, and 50 units/ml endothelial cell growth supplements (ECGS, Biotechnique, Inc.). On day 2, the medium is replaced with M199 containing 10% FBS, 8 units/ml heparin. TR10 protein of SEQ ID NO. 2, and positive controls, such as VEGF and basic FGF (bFGF) are added, at varying concentrations. On days 4 and 6, the medium is replaced. On day 8, cell number is determined with a Coulter Counter. An increase in the number of HUVEC cells indicates that TR10 may proliferate vascular endothelial cells.
The studies described in this example test the activity in TR10 protein. However, one skilled in the art could easily modify the exemplified studies to test the activity of TR10 polynucleotides (e.g., gene therapy), agonists, and/or antagonists of TR10.

### *Example 24*

### *Stimulatory Effect of TR10 on the Proliferation of Vascular Endothelial Cells*

For evaluation of mitogenic activity of growth factors, the colorimetric MTS (3-(4,5-dimethylthiazol-2-yl)-5-(3-carboxymethoxyphenyl)-2-(4-sulfophenyl)2H-tetrazolium) assay with the electron coupling reagent PMS (phenazine methosulfate) was performed (CellTiter 96 AQ, Promega). Cells are seeded in a 96-well plate (5,000 cells/well) in 0.1 ml serum-supplemented medium and are allowed to attach overnight. After serum-starvation for 12 hours in 0.5% FBS, conditions (bFGF, $VEGF_{165}$ or TR10 in 0.5% FBS) with or without Heparin (8 U/ml) are added to wells for 48 hours. 20 mg of MTS/PMS mixture (1:0.05) are added per well and allowed to incubate for 1 hour at 37°C before measuring the absorbance at 490 nm in an ELISA plate reader. Background absorbance from control wells (some media, no cells) is subtracted, and seven wells are performed in parallel for each condition. See, Leak *et al. In Vitro Cell. Dev. Biol.* 30A: 512-518 (1994).

The studies described in this example test the activity in TR10 protein. However, one skilled in the art could easily modify the exemplified studies to test the activity of TR10 polynucleotides (e.g., gene therapy), agonists, and/or antagonists of TR10.

### *Example 25*

### *Inhibition of PDGF-induced Vascular Smooth Muscle Cell Proliferation Stimulatory Effect*

HAoSMC proliferation can be measured, for example, by BrdUrd incorporation. Briefly, subconfluent, quiescent cells grown on the 4-chamber slides are transfected with CRP or FITC-labeled AT2-3LP. Then, the cells are pulsed with 10% calf serum and 6 mg/ml BrdUrd. After 24 h, immunocytochemistry is performed by using BrdUrd Staining Kit (Zymed Laboratories). In brief, the cells are incubated with the biotinylated mouse anti-BrdUrd antibody at 4 °C for 2 h after exposing to denaturing solution and then with the streptavidin-peroxidase and diaminobenzidine. After counterstaining with hematoxylin, the cells are mounted for microscopic examination, and the BrdUrd-positive cells are counted. The BrdUrd index is calculated as a percent of the BrdUrd-positive cells to the total cell number. In addition, the simultaneous detection of the BrdUrd staining (nucleus) and the FITC uptake (cytoplasm) is performed for individual cells by the concomitant use of bright field illumination and dark field-UV fluorescent illumination. See, Hayashida *et al., J. Biol. Chem. 6;271(36)*:21985-21992 (1996).
The studies described in this example test the activity in TR10 protein. However, one skilled in the art could easily modify the exemplified studies to test the activity of TR10 polynucleotides (e.g., gene therapy), agonists, and/or antagonists of TR10.

### *Example 26*

### *Stimulation of Endothelial Migration*

This example will be used to explore the possibility that TR10 may stimulate lymphatic endothelial cell migration.

Endothelial cell migration assays are performed using a 48 well microchemotaxis chamber (Neuroprobe Inc., Cabin John, MD; Falk, W., Goodwin, R. H. J., and Leonard, E. J. "A 48 well micro chemotaxis assembly for rapid and accurate measurement of leukocyte migration." *J. Immunological Methods* 1980;33:239-247). Polyvinylpyrrolidone-free polycarbonate filters with a pore size of 8 um (Nucleopore Corp. Cambridge, MA) are coated with 0.1% gelatin for at least 6 hours at room temperature and dried under sterile air. Test substances are diluted to appropriate concentrations in M199 supplemented with 0.25% bovine serum albumin (BSA), and 25 ul of the final dilution is placed in the lower chamber of the modified Boyden apparatus. Subconfluent, early passage (2-6) HUVEC or BMEC cultures are washed and trypsinized for the minimum time required to achieve cell detachment. After placing the filter between lower and upper chamber, 2.5 x $10^5$ cells suspended in 50 ul M199 containing 1% FBS are seeded in the upper compartment. The apparatus is then incubated for 5 hours at 37°C in a humidified chamber with 5% CO2 to allow cell migration. After the incubation period, the filter is removed and the upper side of the filter with the non-migrated cells is scraped with a rubber policeman. The filters are fixed with methanol and stained with a Giemsa solution (Diff-Quick, Baxter, McGraw Park, IL). Migration is quantified by counting cells of three random high-power fields (40x) in each well, and all groups are performed in quadruplicate.

The studies described in this example test the activity in TR10 protein. However, one skilled in the art could easily modify the exemplified studies to test the activity of TR10 polynucleotides (e.g., gene therapy), agonists, and/or antagonists of TR10.

### *Example 27*

### *Stimulation of Nitric Oxide Production by Endothelial Cells*

Nitric oxide released by the vascular endothelium is believed to be a mediator of vascular endothelium relaxation. Thus, TR10 activity can be assayed by determining nitric oxide production by endothelial cells in response to TR10.

Nitric oxide is measured in 96-well plates of confluent microvascular endothelial cells after 24 hours starvation and a subsequent 4 hr exposure to various levels of a positive control (such as VEGF-1 ) and TRIO. Nitric oxide in the medium is determined by use of the Griess reagent to measure total nitrite after reduction of nitric oxide-derived nitrate by nitrate reductase. The effect of TR10 on nitric oxide release is examined on HUVEC.

Briefly, NO release from cultured HUVEC monolayer is measured with a NO-specific polarographic electrode connected to a NO meter (Iso-NO, World Precision Instruments Inc.). Calibration of the NO element is performed according to the following equation:

$$2\ KNO_2 + 2\ KI + 2\ H_2SO_4\ 6\ 2\ NO + I_2 + 2\ H_2O + 2\ K_2SO_4$$

The standard calibration curve is obtained by adding graded concentrations of KNO, (0, 5, 10, 25, 50, 100, 250, and 500 nmol/L) into the calibration solution containing KI and $H_2SO_4$, The specificity of the Iso-NO electrode to NO is previously determined by measurement of NO from authentic NO gas. The culture medium is removed and HUVECs are washed twice with Dulbecco's phosphate buffered saline. The cells are then bathed in 5 ml of filtered Krebs-Henseleit solution in 6-well plates, and the cell plates are kept on a slide warmer (Lab Line Instruments Inc.) to maintain the temperature at 37°C. The NO sensor probe is inserted vertically into the wells, keeping the tip of the electrode 2 mm under the surface of the solution, before addition of the different conditions. S-nitroso acetyl penicillamin (SNAP) is used as a positive control. The amount of released NO is expressed as picomoles per 1 x $10^6$ endothelial cells. All values reported are means of four to six measurements in each group (number of cell culture wells). See, Leak *et al. Biochem. and Biophys. Res. Comm. 217:96*-105 (1995).

The studies described in this example test the activity in TR10protein. However, one skilled in the art could easily modify the exemplified studies to test the activity of TR10 polynucleotides (e.g., gene therapy), agonists, and/or antagonists of TR10.

### *Example 28*

### *Effect of TR10 on Cord Formation in Angiogenesis*

Another step in angiogenesis is cord formation, marked by differentiation of endothelial cells. This bioassay measures the ability of microvascular endothelial cells to form capillary-like structures (hollow structures) when cultured *in vitro.*

CADMEC (microvascular endothelial cells) are purchased from Cell Applications, Inc. as proliferating (passage 2) cells and are cultured in Cell Applications' CADMEC Growth Medium and used at passage 5. For the *in vitro* angiogenesis assay, the wells of a 48-well cell culture plate are coated with Cell Applications' Attachment Factor Medium (200 μl/well) for 30 min. at 37°C. CADMEC are seeded onto the coated wells at 7,500 cells/well and cultured overnight in Growth Medium. The Growth Medium is then replaced with 300 μg Cell Applications' Chord Formation Medium containing control buffer or TRIO (0.1 to 100 ng/ml) and the cells are cultured for an additional 48 hr. The numbers and lengths of the capillary-like chords are quantitated through use of the Boeckeler VIA-170 video image analyzer. All assays are done in triplicate.

Commercial (R&D) VEGF (50 ng/ml) is used as a positive control. b-esteradiol (1 ng/ml) is used as a negative control. The appropriate buffer (without protein) is also utilized as a control.

The studies described in this example test the activity in TR10 protein. However, one skilled in the art could easily modify the exemplified studies to test the activity of TR10 polynucleotides (e.g., gene therapy), agonists, and/or antagonists of TR10.

### *Example 29*

### *Angiogenic Effect on Chick Chorioallantoic Membrane*

Chick chorioallantoic membrane (CAM) is a well-established system to examine angiogenesis. Blood vessel formation on CAM is easily visible and quantifiable. The ability of TR10 to stimulate angiogenesis in CAM can be examined.

Fertilized eggs of the White Leghorn chick *(Gallus gallus)* and the Japanese quail *(Coturnix coturnix)* are incubated at 37.8°C and 80% humidity. Differentiated CAM of 16-day-old chick and 13-day-old quail embryos is studied with the following methods.

On Day 4 of development, a window is made into the egg shell of chick eggs. The embryos are checked for normal development and the eggs sealed with cellotape. They are further incubated until Day 13. Thermanox coverslips (Nunc, Naperville, IL) are cut into disks of about 5 mm in diameter. Sterile and salt-free growth factors, and the protein to be tested, are dissolved in distilled water and about 3.3 mg/ 5 ml are pipetted on the disks. After air-drying, the inverted disks are applied on CAM. After 3 days, the specimens are fixed in 3% glutaraldehyde and 2% formaldehyde and rinsed in 0.12 M sodium cacodylate buffer. They are photographed with a stereo microscope Wild M8J and embedded for semi- and ultrathin sectioning as described above. Controls are performed with carrier disks alone.

The studies described in this example test the activity in TR10 protein. However, one skilled in the art could easily modify the exemplified studies to test the activity of TR10 polynucleotides (e.g., gene therapy), agonists, and/or antagonists of TR10.

### *Example 30*

### *Angiogenesis Assay Using a Matrigel Implant in Mouse*

In order to establish an *in vivo* model for angiogenesis to test TR10 protein activities, mice and rats are implanted subcutaneously with methylcellulose disks containing either 20 mg of BSA (negative control), 1 mg of TR10, or 0.5 mg of VEGF-1 (positive control). The negative control disks should contain little vascularization, while the positive control disks should show signs of vessel formation.

The studies described in this example test the activity in TR10 protein. However, one skilled in the art could easily modify the exemplified studies to test the activity of TR10 polynucleotides (e.g., gene therapy), agonists, and/or antagonists of TR10.

### *Example 31*

### *Rescue of Ischemia in Rabbit Lower Limb Model*

To study the in vivo effects of TR10 on ischemia, a rabbit hindlimb ischemia model is created by surgical removal of one femoral arteries as described previously (Takeshita, *S. et al., Am J. Pathol 147:1649-1660* (1995)). The excision of the femoral artery results in retrograde propagation of thrombus and occlusion of the external iliac artery. Consequently, blood flow to the ischemic limb is dependent upon collateral vessels originating from the internal iliac artery (Takeshita, S. *et al., Am J. Pathol* 147:1649-1660 (1995)). An interval of 10 days is allowed for post-operative recovery of rabbits and development of endogenous collateral vessels. At 10 day post-operatively (day 0), after performing a baseline angiogram, the internal i liac artery of the ischemic limb is transfected with 500 mg naked TR10 expression plasmid by arterial gene transfer technology using a hydrogel-coated balloon catheter as described (Riessen, *R. et al., Hum Gene Ther.* 4:749-758 (1993); Leclerc, G. *et al., J. Clin. Invest. 90:* 936-944 (1992)). When TR10 is used in the treatment, a single bolus of 500 mg TR10 protein or control is delivered into the internal iliac artery of the ischemic limb over a period of 1 min. through an infusion catheter. On day 30, various parameters are measured in these rabbits: (a) BP ratio - The blood pressure ratio of systolic pressure of the ischemic limb to that of normal limb; (b) Blood Flow and Flow Reserve - Resting FL: the blood flow during undilated condition and Max FL: the blood flow during fully dilated condition (also an indirect measure of the blood vessel amount) and Flow Reserve is reflected by the ratio of max FL: resting FL; (c) Angiographic Score - This is measured by the angiogram of collateral vessels. A score is determined by the percentage of circles in an overlaying grid that with crossing opacified arteries divided by the total number m the rabbit thigh; (d) Capillary density - The number of collateral capillaries determined in light microscopic sections taken from hindlimbs.

The studies described in this example test the activity in TR10 protein. However, one skilled in the art could easily modify the exemplified studies to test the activity of TR10 polynucleotides (e.g., gene therapy), agonists, and/or antagonists of TR10.

***Example 32***

***Rat Ischemic Skin Flap Model***

The evaluation parameters include skin blood flow, skin temperature, and factor VIII immunohistochemistry or endothelial alkaline phosphatase reaction. TR10 expression, during the skin ischemia, is studied using in situ hybridization.

The study in this model is divided into three parts as follows:

a) Ischemic skin
b) Ischemic skin wounds
c) Normal wounds

The experimental protocol includes:

a) Raising a 3x4 cm, single pedicle full-thickness random skin flap (myocutaneous flap over the lower back of the animal).
b) An excisional wounding (4-6 mm in diameter) in the ischemic skin (skin-flap).
c) Topical treatment with TR10 of the excisional wounds (day 0, 1, 2, 3, 4 post-wounding) at the following various dosage ranges: 1 mg to 100 mg.
d) Harvesting the wound tissues at day 3, 5, 7, 10, 14 and 21 post-wounding for histological, immunohistochemical, and in situ studies.

The studies described in this example test the activity in TR10 protein. However, one skilled in the art could easily modify the exemplified studies to test the activity of TR10 polynucleotides (e.g., gene therapy), agonists, and/or antagonists of TR 10.

***Example 33***

***Peripheral Arterial Disease Model***

Anciogenic therapy using TR10 is a novel therapeutic strategy to obtain restoration of blood flow around the ischemia in case of peripheral arterial diseases. The experimental protocol includes:

a) One side of the femoral artery is ligated to create ischemic muscle of the hindlimb, the other side of hindlimb serves as a control.
b) TR10 protein, in a dosage range of 20 mg - 500 mg, is delivered intravenously and/or intramuscularly 3 times (perhaps more) per week for 2-3 weeks.
c) The ischemic muscle tissue is collected after ligation of the femoral artery at 1, 2, and 3 weeks for the analysis of TR10 expression and histology. Biopsy is also performed on the other side of normal muscle of the contralateral hindlimb.

The studies described in this example test the activity in TR10 protein. However, one skilled in the art could easily modify the exemplified studies to test the activity of TR10 polynucleotides (e.g., gene therapy), agonists, and/or antagonists of TR10.

***Example 34***

***Ischemic Myocardial Disease Model***

TR10 is evaluated as a potent mitogen capable of stimulating the development of collateral vessels, and restructuring new vessels after coronary artery occlusion. Alteration of TR10 expression is investigated in *situ.* The experimental protocol includes:

a) The heart is exposed through a left-side thoracotomy in the rat. Immediately, the left coronary artery is occluded with a thin suture (6-0) and the thorax is closed.
b) TR 10 protein, in a dosage range of 20 mg - 500 mg, is delivered intravenously and/or intramuscularly 3 times (perhaps more) per week for 2-4 weeks.
c) Thirty days after the surgery, the heart is removed and cross-sectioned for morphometric and in situ analyzes.

The studies described in this example test the activity in TR10 protein. However, one skilled in the art could easily modify the exemplified studies to test the activity of TR10 polynucleotides (e.g., gene therapy), agonists, and/or antagonists of TR10.

***Example 35***

***Rat Corneal Wound Healing Model***

This animal model shows the effect of TR10 on neovascularization. The experimental protocol includes:

a) Making a 1-1.5 mm long incision from the center of cornea into the stromal layer.
b) Inserting a spatula below the lip of the incision facing the outer comer of the eye.
c) Making a pocket (its base is 1-1.5 mm form the edge of the eye).
d) Positioning a pellet, containing 50ng- 5ug of TR10, within the pocket.
e) TR10 treatment can also be applied topically to the corneal wounds in a dosage range of 20mg - 500mg (daily treatment for five days).

The studies described in this example test the activity in TR10 protein. However, one skilled in the art could easily modify the exemplified studies to test the activity of TR10 polynucleotides (e.g., gene therapy), agonists, and/or antagonists of TR10.

***Example 36***

***Diabetic Mouse and Glucocorticoid-Impaired Wound Healing Models***

***A. Diabetic db+ldb+ Mouse Model.***

To demonstrate that TR10 accelerates the healing process, the genetically diabetic mouse model of wound healing is used. The full thickness wound healing model in the db+/db+ mouse is a well characterized, clinically relevant and reproducible model of impaired wound healing. Healing of the diabetic wound is dependent on formation of granulation tissue and re-epithelialization rather than contraction (Gartner, M.H. *et al., J. Surg. Res. 52*:389 (1992); Greenhalgh, D.G. *et al., Am. J. Pathol. 136*:1235 (1990)).
The diabetic animals have many of the characteristic features observed in Type II diabetes mellitus. Homozygous (db+/db+) mice are obese in comparison to their normal heterozygous (db+/+m) littenmates. Mutant diabetic (db+/db+) mice have a single autosomal recessive mutation on chromosome 4 (db+) (Coleman *et al. Proc. Nati. Acad. Sci. USA 77*:283-293 (1982)). Animals show polyphagia, polydipsia and polyuria. Mutant diabetic mice (db+/db+) have elevated blood glucose, increased or normal insulin levels. and suppressed cell-mediated immunity (Mandel *et al., J. Immunol. 120:1375* (1978): Debray-Sachs, M. *et al., Clin. Exp. Immunol. 51(1)*:1-7 (1983); Leiter *et al., Am. J. of Pathol. 114*:46-55 (1985)). Peripheral neuropathy, myocardial complications, and microvascular lesions, basement membrane thickening and glomerular filtration abnormalities have been described in these animals (Norido, F. *et al., Exp. Neurol. 83*(*2*):221-232 (1984); *Robertson et al., Diabetes 29(1*):60-67 (1980); Giacomelli *et al., Lab Invest. 40(4)*:460-473 (1979); Coleman. D.L., *Diabetes 31 (Suppl):*1-6 (1982)). These homozygous diabetic mice develop hyperglycemia that is resistant to insulin analogous to human type II diabetes (Mandel *et al.,* J. *Immunol. 120*:1375-1377 (1978)).
The characteristics observed in these animals suggests that healing in this model may be similar to the healing observed in human diabetes (Greenhalgh, *et al., Am. J. of Pathol. 136*:1235-1246 (1990)).
Genetically diabetic female C57BL/KsJ (db+/db+) mice and their non-diabetic (db+/+m) heterozygous littermates are used in this study (Jackson Laboratories). The animals are purchased at 6 weeks of age and were 8 weeks old at the beginning of the study. Animals are individually housed and received food and water *ad libitum.* All manipulations are performed using aseptic techniques. The experiments are conducted according to the rules and guidelines of Human Genome Sciences, Inc. Institutional Animal Care and Use Committee and the Guidelines for the Care and Use of Laboratory Animals.

Wounding protocol is performed according to previously reported methods (Tsuboi, R. and Rifkin, D.B., J. *Exp. Med. 172*:245-251 (1990)). Briefly, on the day of wounding, animals are anesthetized with an intraperitoneal injection of Avertin (0.01 mg/mL), 2,2,2-tribromoethanol and 2-methyl-2-butanol dissolved in deionized water. The dorsal region of the animal is shaved and the skin washed with 70% ethanol solution and iodine. The surgical area is dried with sterile gauze prior to wounding. An 8 mm full-thickness wound is then created using a Keyes tissue punch. Immediately following wounding, the surrounding skin is gently stretched to eliminate wound expansion. The wounds are left open for the duration of the experiment. Application of the treatment is given topically for 5 consecutive days commencing on the day of wounding. Prior to treatment, wounds are gently cleansed with sterile saline and gauze sponges.

Wounds are visually examined and photographed at a fixed distance at the day of surgery and at two day intervals thereafter. Wound closure is determined by daily measurement on days 1-5 and on day 8. Wounds are measured horizontally and vertically using a calibrated Jameson caliper. Wounds are considered healed if granulation tissue is no longer visible and the wound is covered by a continuous epithelium.

TR10 is administered using at a range different doses of TR10, from 4mg to 500mg per wound per day for 8 days in vehicle. Vehicle control groups received 50mL of vehicle. solution.

Animals are euthanized on day 8 with an intraperitoneal injection of sodium pentobarbital (300mg/kg). The wounds and surrounding skin are then harvested for histology and immunohistochemistry. Tissue specimens are placed in 10% neutral buffered formalin in tissue cassettes between biopsy sponges for further processing.

Three groups of 10 animals each (5 diabetic and 5 non-diabetic controls) are evaluated: 1) Vehicle placebo control, 2) TR10.

Wound closure is analyzed by measuring the area in the vertical and horizontal axis and obtaining the total square area of the wound. Contraction is then estimated by establishing the differences between the initial wound area (day 0) and that of post treatment (day 8). The wound area on day 1 was $64mm^2$, the corresponding size of the dermal punch. Calculations were made using the following formula:

[Open area on day 8] - [Open area on day 1]/(Open area on day 1 ]

Specimens are fixed in 10% buffered formalin and paraffin embedded blocks are sectioned perpendicular to the wound surface (5mm) and cut using a Reichert-Jung microtome. Routine hematoxylin-eosin (H&E) staining is performed on cross-sections of bisected wounds. Histologic examination of the wounds are used to assess whether the healing process and the morphologic appearance of the repaired skin is altered by treatment with TR10. This assessment included verification of the presence of cell accumulation, inflammatory cells, capillaries, fibroblasts, re-epithelialization and epidermal maturity (Greenhalgh, *D.G. et al., Am. J. Pathol. 136*:1235 (1990)). A calibrated lens micrometer is used by a blinded observer.

Tissue sections are also stained immunohistochemically with a polyclonal rabbit anti-human keratin antibody using ABC Elite detection system. Human skin is used as a positive tissue control while non-immune IgG is used as a negative control. Keratinocyte growth is determined by evaluating the extent of reepithelialization of the wound using a calibrated lens micrometer.

Proliferating cell nuclear antigen/cyclin (PCNA) in skin specimens is demonstrated by using anti-PCNA antibody (1:50) with an ABC Elite detection system. Human colon cancer served as a positive tissue control and human brain tissue is used as a negative tissue control. Each specimen included a section with omission of the primary antibody and substitution with non-immune mouse IgG. Ranking of these sections is based on the extent of proliferation on a scale of 0-8, the lower side of the scale reflecting slight proliferation to the higher side reflecting intense proliferation.

Experimental data are analyzed using an unpaired t test. A p value of $< 0.05$ is considered significant.

### *B. Steroid Impaired Rat Model*

The inhibition of wound healing by steroids has been well documented in various in *vitro* and *in vivo* systems (Wahl, S.M. Glucocorticoids and Wound healing. In: Anti-Inflammatory Steroid Action: Basic and Clinical Aspects. 280-302 (1989); Wahl, *S.M.et al., J. Immunol. 115:* 476-481 (1975); Werb, Z. *et al., J. Exp. Med. 147*:1684-1694 (1978)). Glucocorticoids retard wound healing by inhibiting angiogenesis, decreasing vascular permeability ( Ebert, R.H., *et al., An. Intern. Med. 37*:701-705 (1952)), fibroblast proliferation, and collagen synthesis (Beck, L.S. *et al., Growth Factors. 5*: 295-304 (1991); Haynes, B.F. *et al., J. Clin. Invest. 61:* 703-797 (1978)) and producing a transient reduction of circulating monocytes (Haynes, B.F., *et al., J. Clin. Invest. 61:* 703-797 (1978); Wahl, S. M., "Glucocorticoids and wound healing", *In:* Antiinflammatory Steroid Action: Basic and Clinical Aspects, Academic Press, New York, pp. 280-302 (1989)). The systemic administration of steroids to impaired wound healing is a well establish phenomenon in rats (Beck, L.S. *et al., Growth Factors. 5*: 295-304 (1991); Haynes, B.F., *et al., J. Clin. Invest. 61:* 703-797 (1978); Wahl, S. M.,

"Glucocorticoids and wound healing", *In*: Antiinflammatory Steroid Action: Basic and Clinical Aspects, Academic Press, New York, pp. 280-302 ( 1989); Pierce, G.F. *et al., Proc. Natl. Acad. Sci. USA 86*: 2229-2233 (1989)).

To demonstrate that TRIO can accelerate the healing process, the effects of multiple topical applications of TRIO on full thickness excisional skin wounds in rats in which healing has been impaired by the systemic administration of methylprednisolone is assessed.

Young adult male Sprague Dawley rats weighing 250-300 g (Charles River Laboratories) are used in this example. The animals are purchased at 8 weeks of age and were 9 weeks old at the beginning of the study. The healing response of rats is impaired by the systemic administration of methylprednisolone (17mg/kg/rat intramuscularly) at the time of wounding. Animals are individually housed and received food and water *ad libitum.* All manipulations are performed using aseptic techniques. This study is conducted according to the rules and guidelines of Human Genome Sciences, Inc. Institutional Animal Care and Use Committee and the Guidelines for the Care and Use of Laboratory Animals.

The wounding protocol is followed according to section A, above. On the day of wounding, animals are anesthetized with an intramuscular injection of ketamine (50 mg/kg) and xylazine (5 mg/kg). The dorsal region of the animal is shaved and the skin washed with 70% ethanol and iodine solutions. The surgical area is dried with sterile gauze prior to wounding. An 8 mm full-thickness wound is created using a Keyes tissue punch. The wounds are left open for the duration of the experiment. Applications of the testing materials are given topically once a day for 7 consecutive days commencing on the day of wounding and subsequent to methylprednisolone administration. Prior to treatment, wounds are gently cleansed with sterile saline and gauze sponges.

Wounds are visually examined and photographed at a fixed distance at the day of wounding and at the end of treatment. Wound closure is determined by daily measurement on days 1-5 and on day 8. Wounds are measured horizontally and vertically using a calibrated Jameson caliper. Wounds are considered healed if granulation tissue was no longer visible and the wound is covered by a continuous epithelium.

TR10 is administered using at a range different doses of TR10, from 4mg to 500mg per wound per day for 8 days in vehicle. Vehicle control groups received 50mL of vehicle solution.

Animals are euthanized on day 8 with an intraperitoneal injection of sodium pentobarbital (300mg/kg). The wounds and surrounding skin are then harvested for histology. Tissue specimens are placed in 10% neutral buffered formalin in tissue cassettes between biopsy sponges for further processing.

Four groups of 10 animals each (5 with methylprednisolone and 5 without glucocorticoid) were evaluated: 1) Untreated group 2) Vehicle placebo control 3) TR10 treated groups.

Wound closure is analyzed by measuring the area in the vertical and horizontal axis and obtaining the total area of the wound. Closure is then estimated by establishing the differences between the initial wound area (day 0) and that of post treatment (day 8). The wound area on day 1 was 64mm$^2$, the corresponding size of the dermal punch. Calculations were made using the following formula:

$$[\text{Open area on day 8}] - [\text{Open area on day 1}] / [\text{Open area on day 1}]$$

Specimens are fixed in 10% buffered formalin and paraffin embedded blocks are sectioned perpendicular to the wound surface (5mm) and cut using an Olympus microtome. Routine hematoxylin-eosin (H&E) staining was performed on cross-sections of bisected wounds. Histologic examination of the wounds allows assessment of whether the healing process and the morphologic appearance of the repaired skin was improved by treatment with TR10. A calibrated lens micrometer is used by a blinded observer to determine the distance of the wound gap.

Experimental data are analyzed using an unpaired t test. A p value of $< 0.05$ is considered significant.

The studies described in this example test the activity in TR10 protein. However, one skilled in the art could easily modify the exemplified studies to test the activity of TR10 polynucleotides (e.g., gene therapy), agonists, and/or antagonists of TR10.

***Example 37***

***Lymphadema Animal Model***

The purpose of this experimental approach is to create an appropriate and consistent lymphedema model for testing the therapeutic effects of TR10 in lymphangiogenesis and reestablishment of the lymphatic circulatory system in the rat hind limb. Effectiveness is measured by swelling volume of the affected limb, quantification of the amount of lymphatic vasculature, total blood plasma protein, and histopathology. Acute lymphedema is observed for 7-10 days. Perhaps more importantly, the chronic progress of the edema is followed for up to 3-4 weeks.

Prior to beginning surgery, blood sample is drawn for protein concentration analysis. Male rats weighing approximately ~350g are dosed with Pentobarbital. Subsequently, the right legs are shaved from knee to hip. The shaved area is swabbed with gauze soaked in 70% EtOH. Blood is drawn for serum total protein testing. Circumference and volumetric measurements are made prior to injecting dye into paws after marking 2 measurement levels (0.5 cm above heel, at mid-pt of dorsal paw). The intradermal dorsum of both right and left paws are injected with 0.05 ml of 1% Evan's Blue. Circumference and volumetric measurements are then made following injection of dye into paws.

Using the knee joint as a landmark, a mid-leg inguinal incision is made circumferentially allowing the femoral vessels to be located. Forceps and hemostats are used to dissect and separate the skin flaps. After locating the femoral vessels, the lymphatic vessel that runs along side and underneath the vessel(s) is located. The main lymphatic vessels in this area are then electrically coagulated or suture ligated.

Using a microscope, muscles in back of the leg (near the semitendinosis and adductors) are bluntly dissected. The popliteal lymph node is then located. The 2 proximal and 2 distal lymphatic vessels and distal blood supply of the popliteal node are then and ligated by suturing. The popliteal lymph node, and any accompanying adipose tissue, is then removed by cutting connective tissues.

Care is taken to control any mild bleeding resulting from this procedure. After lymphatics are occluded, the skin flaps are sealed by using liquid skin (Vetbond) (AJ Buck). The separated skin edges are sealed to the underlying muscle tissue while leaving a gap of ~0.5 cm around the leg. Skin also may be anchored by suturing to underlying muscle when necessary.

To avoid infection, animals are housed individually with mesh (no bedding). Recovering animals are checked daily through the optimal edematous peak. which typically occurred by day 5-7. The plateau edematous peak are then observed. To evaluate the intensity of the lymphedema. the circumference and volumes of 2 designated places on each paw before operation and daily for 7 days are measured. The effect plasma proteins on lymphedema is determined and whether protein analysis is a useful testing perimeter is also investigated. The weights of both control and edematous limbs are evaluated at 2 places. Analysis is performed in a blind manner.

Circumference Measurements: Under brief gas anesthetic to prevent limb movement, a cloth tape is used to measure limb circumference. Measurements are done at the ankle bone and dorsal paw by 2 different people then those 2 readings are averaged. Readings are taken from both control and edematous limbs.

Volumetric Measurements: On the day of surgery, animals are anesthetized with Pentobarbital and are tested prior to surgery. For daily volumetrics animals are under brief halothane anesthetic (rapid immobilization and quick recovery), both legs are shaved and equally marked using waterproof marker on legs. Legs are first dipped in water, then dipped into instrument to each marked level then measured by Buxco edema software(Chen/Victor). Data is recorded by one person, while the other is dipping the limb to marked area.

Blood-plasma protein measurements: Blood is drawn, spun, and serum separated prior to surgery and then at conclusion for total protein and Ca2+ comparison.

Limb Weight Comparison: After drawing blood, the animal is prepared for tissue collection. The limbs were amputated using a quillitine, then both experimental and control legs were cut at the ligature and weighed. A second weighing is done as the tibio-cacaneal joint was disarticulated and the foot was weighed.

Histological Preparations: The transverse muscle located behind the knee (popliteal) area is dissected and arranged in a metal mold, filled with freezeGel, dipped into cold methylbutane, placed into labeled sample bags at - 80EC until sectioning. Upon sectioning, the muscle was observed under fluorescent microscopy for lymphatics. Other immuno/histological methods are currently being evaluated.

The studies described in this example test the activity in TR10 protein. However, one skilled in the art could easily modify the exemplified studies to test the activity of TR10 polynucleotides (e.g., gene therapy), agonists, and/or antagonists of TR10.

### *Example 38*

#### *TR10-Fc inhbits B cell proliferation in vitro in a* co-stimulatory assay

A TR10-Fc polypeptide was prepared that consists of a soluble form of TR10 (corresponding to amino acids -55 to 149 of SEQ ID NO:2) linked to the Fc portion of a human LgGt1 immunogloulin molecule. The ability of this protein to alter the proliferative response of human B cells was assessed in a standard co-stimulatory assay. Briefly, human tonsillar B cells were purified by magnetic bead (MACS) depletion of CD3-positive cells. The resulting cell population was routinely greater than 95% B cells as assessed by expression of CD19 and CD20 staining. Various dilutions of rHuNeutrokine-alpha (Internatioanl Application Publication No. WO 98/18921) or the control protein rHuIL2 were placed into individual wells of a 96-well plate to which was added $10^5$ B cells suspended in culture medium (RPMI 1640 containing 10% FBS, 5 X $10^{-5}$M 2ME, 100U/ml penicillin, 10ug/ml streptomycin, and $10^{-5}$ dilution of formalin-fixed *Staphylococcus aureus* Cowan I (SAC) also known as Pansorbin (Pan)) in a total volume of 150ul. TR10-Fc was then added at various concentrations. Plates were then placed in the incubator (37°C 5% $CO_2$, 95% humidity) for three days. Proliferation was quantitated by a 20h pulse (1μCi/well) of $^3$H-thymidine (6.7 Ci/mM) beginning 72h post factor addition. The positive and negative controls are IL2 and medium respectively.

The results of this experiment confirmed that TR10-Fc inhibited B cell proliferation in the co-stimulatory assay using *Staphylococcus Aureus* Cowan I (SAC) as priming agent and Neutrokine-alpha as a second signal (data not shown). It is important to note that other Tumor Necrosis Factor Receptors (TNFR) fusion proteins (e.g., DR4-Fc (Internatioanl Application Publication No. WO 98/32856), TR6-Fc (Internatioanl Application Publication No. WO 98/31799), and TR9-Fc (Internatioanl Application Publication No. WO 98/56892)) did not inhibit proliferation.

## Claims

1. Use of a TR10-fc fusion polypeptide, wherein said TR10 polypeptide is encoded by a polynucleotide selected from the group consisting of:

(a) polynucleotides comprising a nucleotide sequence encoding at least the mature form of the polypeptide having the deduced amino acid sequence as shown in SEQ ID NO:2;
(b) polynucleotides comprising a nucleotide sequence having the coding sequence as shown in SEQ ID NO: 1 encoding at least the mature form of the polypeptide;
(c) polynucleotides comprising a nucleotide sequence encoding the polypeptide having the amino acid sequence of at least the mature form of the polypeptide encoded by the cDNA contained in ATCC 209040;
(d) polynucleotides comprising a nucleotide sequence having the coding sequence of the cDNA contained in ATCC 209040 encoding at least the mature form of the polypeptide;
(e) polynucleotides comprising a nucleotide sequence encoding a polypeptide comprising the amino acids from -55 to 331, from -54 to 331, from 1 to 331, from 158 to 175, from 176 to 331 or from 298 to 308 in SEQ ID NO: 2;
(f) the polynucleotide of (a) to (e) except for at least one conservative amino acid substitution and encoding a TR10 receptor polypeptide;
(g) polynucleotides encoding a TR10 polypeptide comprising amino acid residues from 2 to 58, from 75 to 142 or from 194 to 228 in SEQ ID NO:2;
(h) polynucleotides of at least 50 contiguous nucleotides of nucleotides 109 to 1269 of SEQ ID NO: 1 or of the open reading frame encoded by the cDNA plasmid contained in ATCC Deposit No. 209040;
(i) polynucleotides encoding at least 30 contiguous amino acid residues of positions -55 to +331 of SEQ ID NO: 2 or of the open reading frame encoded by the cDNA plasmid contained in ATCC Deposit No. 209040;
(j) the polynucleotide of (i) encoding at least 50 contiguous amino acid residues;

(k) polynucleotides which are at least 90% identical to a polynucleotide as defined in any one of (a) to (j) and which encode a TRIO polypeptide;
(1) polynucleotides encoding a polypeptide having an amino acid sequence at least 90% identical to the amino acid sequence of a TRIO polypeptide encoded by a polynucleotide of any one of (a) to (j);
(m) polynucleotides which hybridize to the complementary strand of a polynucleotide of any one of (a) to (1); and
(n) polynucleotides encoding the extracellular, transmembrane, intracellular, the partial death domain or the extracellular and intracellular domains with all or part of the transmembrane domain deleted of a polypeptide encoded by a polynucleotide of any one of (a) to (m);

or the complementary strand of such a polynucleotide for the preparation of a pharmaceutical composition for the treatment of a hematopoietic disorder.

**2.** Use of an antibody that binds the polypeptide encoded by the polynucleotide of any one of claim 1(a) to 1(e) for the preparation of a pharmaceutical composition for the treatment of a hematopoietic disorder.

**3.** The use of claim 1. wherein the TR10 polypeptide of the TR10-fc fusion polypeptide contains the mature extracellular domain of the polypeptide having the amino acid sequence of Met-1 to Gly-149 of SEQ ID NO:2.

**4.** Use of a TR10 fusion polypeptide, wherein said TR10 polypeptide is encoded by a polynucleotide selected from the group consisting of:

(a) polynucleotides comprising a nucleotide sequence encoding at least the mature form of the polypeptide having the deduced amino acid sequence as shown in SEQ ID NO:2;
(b) polynucleotides comprising a nucleotide sequence having the coding sequence as shown in SEQ ID NO:1 encoding at least the mature form of the polypeptide;
(c) polynucleotides comprising a nucleotide sequence encoding the polypeptide having the amino acid sequence of at least the mature form of the polypeptide encoded by the cDNA contained in ATCC 209040;
(d) polynucleotides comprising a nucleotide sequence having the coding sequence of the cDNA contained in ATCC 209040 encoding at least the mature form of the polypeptide;
(e) polynucleotides comprising a nucleotide sequence encoding a polypeptide comprising the amino acids from -55 to 331, from -54 to 331, from 1 to 331, from 158 to 175, from 176 to 331 or from 298 to 308 in SEQ ID NO: 2;
(f) the polynucleotide of (a) to (e) except for at least one conservative amino acid substitution and encoding a TR10 receptor polypeptide;
(g) polynucleotides encoding a TR10 polypeptide comprising amino acid residues from 2 to 58, from 75 to 142 or from 194 to 228 in SEQ ID NO:2;
(h) polynucleotides of at least 50 contiguous nucleotides of nucleotides 109 to 1269 of SEQ ID NO: 1 or of the open reading frame encoded by the cDNA plasmid contained in ATCC Deposit No. 209040;
(i) polynucleotides encoding at least 30 contiguous amino acid residues of positions -55 to +331 of SEQ ID NO: 2 or of the open reading frame encoded by the cDNA plasmid contained in ATCC Deposit No. 209040;
(j) the polynucleotide of (i) encoding at least 50 contiguous amino acid residues;
(k) polynucleotides which are at least 90% identical to a polynucleotide as defined in any one of (a) to (j) and which encode a TR10 polypeptide;
(1) polynucleotides encoding a polypeptide having an amino acid sequence at least 90% identical to the amino acid sequence of a TR10 polypeptide encoded by a polynucleotide of any one of (a) to (j);
(m) polynucleotides which hybridize to the complementary strand of a polynucleotide of any one of (a) to (I); and
(n) polynucleotides encoding the extracellular, transmembrane, intracellular, the partial death domain or the extracellular and intracellular domains with all or part of the transmembrane domain deleted of a polypeptide encoded by a polynucleotide of any one of (a) to (m);

or the complementary strand of such a polynucleotide for the preparation of a pharmaceutical composition for the inhibition of hematopoiesis.

**5.** Use of an antibody that binds the polypeptide encoded by the polynucleotide of any one of claim 4(a) to 4(n) for the preparation of a pharmaceutical composition for the inhibition of hematopoiesis.

**6.** The use of claim 4, wherein the TR10 polypeptide of the TR10-Fc fusion polypeptide contains the mature extracellular domain of the polypeptide having the amino acid sequence of Met-1 to Gly-149 of SEQ NO:2.

**7.** Use of an antibody that binds a TR10 fusion polypeptide, wherein said TR10 polypeptide is encoded by a polynucleotide selected from the group consisting of:

(a) polynucleotides comprising a nucleotide sequence encoding at least the mature form of the polypeptide having the deduced amino acid sequence as shown in SEQ ID NO:2;
(b) polynucleotides comprising a nucleotide sequence having the coding sequence as shown in SEQ ID NO:1 encoding at least the mature form of the polypeptide;
(c) polynucleotides comprising a nucleotide sequence encoding the polypeptide having the amino acid sequence of at least the mature form of the polypeptide encoded by the cDNA contained in ATCC 209040;
(d) polynucleotides comprising a nucleotide sequence having the coding sequence of the cDNA contained in ATCC 209040 encoding at least the mature form of the polypeptide;
(e) polynucleotides comprising a nucleotide sequence encoding a polypeptide comprising the amino acids from -55 to 331, from -54 to 331, from 1 to 331, from 158 to 175, from 176 to 331 or from 298 to 308 in SEQ ID NO: 2;
(f) the polynucleotide of (a) to (e) except for at least one conservative amino acid substitution and encoding a TR10 receptor polypeptide;
(g) polynucleotides encoding a TR10 polypeptide comprising amino acid residues from 2 to 58, from 75 to 142 or from 194 to 228 in SEQ ID NO:2;
(h) polynucleotides of at least 50 contiguous nucleotides of nucleotides 109 to 1269 of SEQ ID NO: 1 or of the open reading frame encoded by the cDNA plasmid contained in ATCC Deposit No. 209040;
(i) polynucleotides encoding at least 30 contiguous amino acid residues of positions -55 to +331 of SEQ ID NO: 2 or of the open reading frame encoded by the cDNA plasmid contained in ATCC Deposit No. 209040;
(j) the polynucleotide of (i) encoding at least 50 contiguous amino acid residues;
(k) polynucleotides which are at least 90% identical to a polynucleotide as defined in any one of(a) to and which encode a TR10 polypeptide;
(1) polynucleotides encoding a polypeptide having an amino acid sequence at least 90% identical to the amino acid sequence of a TR10 polypeptide encoded by a polynucleotide of any one of (a) to (j);
(m) polynucleotides which hybridize to the complementary strand of a polynucleotide of any one of (a) to (l); and
(n) polynucleotides encoding the extracellular, transmembrane, intracellular, the partial death domain or the extracellular and intracellular domains with all or part of the transmembrane domain deleted of a polypeptide encoded by a polynucleotide of any one of (a) to (m);

for the preparation of a pharmaceutical composition for the stimulation . of hematopoiesis.

## Patentansprüche

**1.** Verwendung eines TR10-fc-Fusionspolypeptids, wobei das TR10-Polypeptid codiert wird durch ein Polynucleotid, ausgewählt aus der Gruppe bestehend aus:

(a) Polynucleotiden, umfassend eine Nucleotidsequenz, die mindestens die reife Form des Polypeptids mit der abgeleiteten Aminosäuresequenz wie in SEQ ID NO: 2 gezeigt codiert;
(b) Polynucleotiden, umfassend eine Nucleotidsequenz mit der codierenden Sequenz wie in SEQ ID NO: 1 gezeigt, die mindestens die reife Form des Polypeptids codiert;
(c) Polynucleotiden, umfassend eine Nucleotidsequenz, die das Polypeptid mit der Aminosäuresequenz mindestens der reifen Form des Polypeptids codiert, das durch die in ATCC 209040 enthaltene cDNA codiert wird;
(d) Polynucleotiden, umfassend eine Nucleotidsequenz mit der codierenden Sequenz der in ATCC 209040 enthaltenen cDNA, die mindestens die reife Form des Polypeptids codiert;
(e) Polynucleotiden umfassend eine Nucleotidsequenz, die ein Polypeptid codiert, welches die Aminosäuren von -55 bis 331, von -54 bis 331, von 1 bis 331, von 158 bis 175, von 176 bis 331 oder von 298 bis 308 in SEQ ID NO: 2 umfasst;
(f) den Polynucleotiden von (a) bis (e) ausgenommen mindestens einer konservativen Aminosäuresubstitution und ein TR10-Rezeptorpolypeptid codierend;
(g) Polynucleotiden, die ein TR10-Polypeptid codieren, das die Aminosäurereste von 2 bis 58, von 75 bis 142 oder von 194 bis 228 in SEQ ID NO: 2 umfasst;
(h) Polynucleotiden mit mindestens 50 aufeinanderfolgenden Nucleotiden der Nucleotide 109 bis 1269 von SEQ ID NO: 1 oder des offenen Leserahmens, der durch das in ATCC Nr. 209040 enthaltene cDNA-Plasmid codiert wird;
(i) Polynucleotiden, die mindestens 30 aufeinanderfolgende Aminosäurereste der Positionen -55 bis +331 von SEQ ID NO: 2 oder des offenen Leserahmens codieren, der durch das in ATCC 209040 enthaltene cDNA-Plasmid codiert wird;
(j) den Polynucleotiden von (i), die mindestens 50 aufeinanderfolgende Aminosäurereste codieren;
(k) Polynucleotiden, die zu mindestens 90% mit einem Polynucleotid wie in irgendeinem der Punkte (a) bis (j) definiert identisch sind und die ein TR10-Polypeptid codieren;
(l) Polynucleotiden, die ein Polypeptid mit einer Aminosäuresequenz codieren, die zu mindestens 90% mit der Aminosäuresequenz eines TR10-Polypeptids identisch ist, das durch ein Polynucleotid von irgendeinem der Punkte (a) bis (j) codiert wird;
(m) Polynucleotiden, die mit dem komplementären Strang eines Polynucleotids von irgendeinem der Punkte (a) bis (l) hybridisieren; und
(n) Polynucleotiden, codierend die extrazelluläre, transmembrane, intrazelluläre, die partielle Todesdomäne oder die extrazellulären und intrazellulären Domänen, bei denen die ganze oder ein Teil der Transmembrandomäne entfernt ist, von einem Polypeptid, das durch ein Polynucleotid von irgendeinem der Punkte (a) bis (m) codiert wird;

oder des komplementären Strangs eines solchen Polynucleotids für die Herstellung eines Arzneimittels zur Behandlung einer hämatopoietischen Störung.

**2.** Verwendung eines Antikörpers, der das Polypeptid bindet, welches durch das Polynucleotid aus irgendeinem der Ansprüche 1 (a) bis 1 (e) codiert wird, für die Herstellung eines Arzneimittels zur Behandlung einer hämatopoietischen Störung.

**3.** Verwendung nach Anspruch 1, wobei das TR10-Polypeptid des TR10-fc-Fusionspolypeptids die reife extrazelluläre Domäne des Polypeptids mit der Aminosäuresequenz von Met-1 bis Gly-149 von SEQ ID NO: 2 enthält.

**4.** Verwendung eines TR-10-Fusionspolypeptids, wobei das TR10-Polypeptid codiert wird durch ein Polynucleotid, ausgewählt aus der Gruppe bestehend aus:

(a) Polynucleotiden, umfassend eine Nucleotidsequenz, die mindestens die reife Form des Polypeptids mit der abgeleiteten Aminosäuresequenz wie in SEQ ID NO: 2 gezeigt codiert;
(b) Polynucleotiden, umfassend eine Nucleotidsequenz mit der codierenden Sequenz wie in SEQ ID NO: 1 gezeigt, die mindestens die reife Form des Polypeptids codiert;
(c) Polynucleotiden, umfassend eine Nucleotidsequenz, die das Polypeptid mit der Aminosäuresequenz mindestens der reifen Form des Polypeptids codiert, das durch die in ATCC 209040 enthaltene cDNA codiert wird;
(d) Polynucleotiden, umfassend eine Nucleotidsequenz mit der codierenden Sequenz der in ATCC 209040 enthaltenen cDNA, die mindestens die reife Form des Polypeptids codiert;
(e) Polynucleotiden umfassend eine Nucleotidsequenz, die ein Polypeptid codiert, welches die Aminosäuren von -55 bis 331, von -54 bis 331, von 1 bis 331, von 158 bis 175, von 176 bis 331 oder von 298 bis 308 in SEQ ID NO: 2 umfasst;
(f) den Polynucleotiden von (a) bis (e) ausgenommen mindestens einer konservativen Aminosäuresubstitution und ein TR10-Rezeptorpolypeptid codierend;
(g) Polynucleotiden, die ein TR10-Polypeptid codieren, das die Aminosäurereste von 2 bis 58, von 75 bis 142 oder von 194 bis 228 in SEQ ID NO: 2 umfasst;
(h) Polynucleotiden mit mindestens 50 aufeinanderfolgenden Nucleotiden der Nucleotide 109 bis 1269 von SEQ ID NO: 1 oder des offenen Leserahmens, der durch das in ATCC Nr. 209040 enthaltene cDNA-Plasmid codiert wird;
(i) Polynucleotiden, die mindestens 30 aufeinanderfolgende Aminosäurereste der Positionen -55 bis +331 von SEQ ID NO: 2 oder des offenen Leserahmens codieren, der durch das in ATCC 209040 enthaltene cDNA-Plasmid codiert wird;
(j) den Polynucleotiden von (i), die mindestens 50 aufeinanderfolgende Aminosäurereste codieren;
(k) Polynucleotiden, die zu mindestens 90% mit einem Polynucleotid wie in irgendeinem der Punkte (a) bis (j) definiert identisch sind und die ein TR10-Polypeptid codieren;
(l) Polynucleotiden, die ein Polypeptid mit einer Aminosäuresequenz codieren, die zu mindestens 90% mit der Aminosäuresequenz eines TR10-Polypeptids identisch ist, das durch ein Polynucleotid von irgendeinem der Punkte (a) bis (j) codiert wird;
(m) Polynucleotiden, die mit dem komplementären Strang eines Polynucleotids von irgendeinem der Punkte (a) bis (l) hybridisieren; und
(n) Polynucleotiden, codierend die extrazelluläre, transmembrane, intrazelluläre, die partielle Todesdomäne oder die extrazellulären und intrazellulären Domänen, bei denen die ganze oder ein Teil der Transmembrandomäne entfernt ist, von einem Polypeptid, das durch ein Polynucleotid von irgendeinem der Punkte (a) bis (m) codiert wird;

oder des komplementären Strangs eines solchen Polynucleotids für die Herstellung eines Arzneimittels zur Hemmung der Hämatopoese.

5. Verwendung eines Antikörpers, der das Polypeptid bindet, welches durch das Polynucleotid nach einem der Ansprüche 4(a) bis 4(n), codiert wird, für die Herstellung eines Arzneimittels zur Hemmung der Hämatopoese.

6. Verwendung nach Anspruch 4, wobei das TR10-Polypeptid des TR-10-Fc-Fusionspolypeptids die reife extracelluläre Domäne des Polypeptids mit der Aminosäuresequenz von Met-1 bis Gly-149 von SEQ ID NO: 2 enthält.

7. Verwendung eines Antikörpers, der ein TR10-Fusionspolyptid bindet, wobei das TR10-Polypeptid codiert wird durch ein Polynucleotid, ausgewählt aus der Gruppe bestehend aus:

(a) Polynucleotiden, umfassend eine Nucleotidsequenz, die mindestens die reife Form des Polypeptids mit der abgeleiteten Aminosäuresequenz wie in SEQ ID NO: 2 gezeigt codiert;
(b) Polynucleotiden, umfassend eine Nucleotidsequenz mit der codierenden Sequenz wie in SEQ ID NO: 1 gezeigt, die mindestens die reife Form des Polypeptids codiert;
(c) Polynucleotiden, umfassend eine Nucleotidsequenz, die das Polypeptid mit der Aminosäuresequenz mindestens der reifen Form des Polypeptids codiert, das durch die in ATCC 209040 enthaltene cDNA codiert wird;
(d) Polynucleotiden, umfassend eine Nucleotidsequenz mit der codierenden Sequenz der in ATCC 209040 enthaltenen cDNA, die mindestens die reife Form des Polypeptids codiert;
(e) Polynucleotiden umfassend eine Nucleotidsequenz, die ein Polypeptid codiert, welches die Aminosäuren von -55 bis 331, von -54 bis 331, von 1 bis 331, von 158 bis 175, von 176 bis 331 oder von 298 bis 308 in SEQ ID NO: 2 umfasst;
(f) den Polynucleotiden von (a) bis (e) ausgenommen mindestens einer konservativen Aminosäuresubstitution und ein TR10-Rezeptorpolypeptid codierend;
(g) Polynucleotiden, die ein TR10-Polypeptid codieren, das die Aminosäurereste von 2 bis 58, von 75 bis 142 oder von 194 bis 228 in SEQ ID NO: 2 umfasst;
(h) Polynucleotiden mit mindestens 50 aufeinanderfolgenden Nucleotiden der Nucleotide 109 bis 1269 von SEQ ID NO: 1 oder des offenen Leserahmens, der durch das in ATCC Nr. 209040 enthaltene cDNA-Plasmid codiert wird;
(i) Polynucleotiden, die mindestens 30 aufeinanderfolgende Aminosäurereste der Positionen -55 bis +331 von SEQ ID NO: 2 oder des offenen Leserahmens codieren, der durch das in ATCC 209040 enthaltene cDNA-Plasmid codiert wird;
(j) den Polynucleotiden von (i), die mindestens 50 aufeinanderfolgende Aminosäurereste codieren;
(k) Polynucleotiden, die zu mindestens 90% mit einem Polynucleotid wie in irgendeinem der Punkte (a) bis (j) definiert identisch sind und die ein TR10-Polypeptid codieren;
(l) Polynucleotiden, die ein Polypeptid mit einer Aminosäuresequenz codieren, die zu mindestens 90% mit der Aminosäuresequenz eines TR10-Polypeptids identisch ist, das durch ein Polynucleotid von irgendeinem der Punkte (a) bis (j) codiert wird;
(m) Polynucleotiden, die mit dem komplementären Strang eines Polynucleotids von irgendeinem der Punkte (a) bis (l) hybridisieren; und
(n) Polynucleotiden, codierend die extrazelluläre, transmembrane, intrazelluläre, die partielle Todesdomäne oder die extrazellulären und intrazellulären Domänen, bei denen die ganze oder ein Teil der Transmembrandomäne entfernt ist, von einem Polypeptid, das durch ein Polynucleotid von irgendeinem der Punkte (a) bis (m) codiert wird;

für die Herstellung eines Arzneimittels zur Stimulierung von Hämatopoese.

## Revendications

1. Utilisation d'un polypeptide de fusion TR10-fc, dans laquelle ledit polypeptide TR10 est codé par un polynucléotide choisi dans le groupe constitué de :

(a) polynucléotides comprenant une séquence nucléotidique codant au moins pour la forme mature du polypeptide ayant la séquence d'acides aminés déduite telle que représentée dans la SEQ ID n° : 2 ;
(b) polynucléotides comprenant une séquence nucléotidique ayant la séquence codante telle que représentée dans la SEQ ID n° : 1 codant au moins pour la forme mature du polypeptide ;
(c) polynucléotides comprenant une séquence nucléotidique codant pour le polypeptide ayant la séquence d'acides aminés d'au moins la forme mature du polypeptide codé par l'ADNc contenu dans ATCC 209 040 ;
(d) polynucléotides comprenant une séquence nucléotidique ayant la séquence codante de l'ADNc contenu dans ATCC 209 040 codant au moins pour la forme mature du polypeptide ;
(e) polynucléotides comprenant une séquence nucléotidique codant pour un polypeptide comprenant les acides aminés de -55 à 331, de -54 à 331, de 1 à 331, de 158 à 175, de 176 à 331 ou de 298 à 308 dans la SEQ ID n° : 2 ;
(f) le polynucléotide de (a) à (e) sauf pour au moins une substitution d'acide aminé conservatrice et codant pour un polypeptide récepteur TR10 ;
(g) polynucléotides codant pour un polypeptide TR10 comprenant les résidus d'acides aminés de 2 à 58, de 75 à 142 ou de 194 à 228 dans la SEQ ID n° : 2 ;
(h) polynucléotides d'au moins 50 nucléotides contigus des nucléotides 109 à 1269 de la SEQ ID n° : 1 ou du cadre ouvert de lecture codé par le plasmide d'ADNc contenu dans ATCC n° de dépôt 209 040 ;
(i) polynucléotides codant au moins pour 30 résidus d'acides aminés contigus des positions -55 à +331 de la SEQ ID n° : 2 ou du cadre ouvert de lecture codé par le plasmide d'ADNc contenu dans ATCC n° de dépôt 209 040 ;
(j) le polynucléotide de (i) codant au moins pour 50 résidus d'acides aminés contigus ;
(k) polynucléotides qui sont identiques au moins à 90 % à un polynucléotide tel que défini par l'un quelconque de (a) à (j) et qui codent pour un polypeptide TR10 ;
(1) polynucléotides codant pour un polypeptide ayant une séquence d'acides aminés identique au moins à 90 % à la séquence d'acides aminés d'un polypeptide TR10 codé par un polynucléotide de l'un quelconque de (a) à (j) ;
(m) polynucléotides qui s'hybrident au brin complémentaire d'un polynucléotide de l'un quelconque de (a) à (1) ; et
(n) polynucléotides codant pour le domaine extracellulaire, transmembranaire, intracellulaire, le domaine de mort partielle ou les domaines extracellulaire et intracellulaire avec tout ou partie du domaine transmembranaire délété d'un polypeptide codé par un polynucléotidede l'un quelconque de (a) à (m);

ou le brin complémentaire d'un tel polynucléotide pour la préparation d'une composition pharmaceutique pour le traitement d'un trouble hématopoïétique.

**2.** Utilisation d'un anticorps qui se fixe au polypeptide codé par le polynucléotide selon l'une quelconque des revendications 1(a) à 1(e), pour la préparation d'une composition pharmaceutique pour le traitement d'un trouble hématopoïétique.

**3.** Utilisation selon la revendication 1, dans laquelle le polypeptide TR10 du polypeptide de fusion TR10-Fc contient le domaine extracellulaire mature du polypeptide ayant la séquence d'acides aminés de la Met-1 à la Gly-149 de la SEQ ID n° : 2.

**4.** Utilisation d'un polypeptide de fusion TR10, dans laquelle ledit polypeptide TR10 est codé par un polynucléotide choisi dans le groupe constitué de :

(a) polynucléotides comprenant une séquence nucléotidique codant au moins pour la forme mature du polypeptide ayant la séquence d'acides aminés déduite telle que représentée dans la SEQ ID n°: 2;
(b) polynucléotides comprenant une séquence nucléotidique ayant la séquence codante telle que représentée dans la SEQ ID n° : 1 codant au moins pour la forme mature du polypeptide ;
(c) polynucléotides comprenant une séquence nucléotidique codant pour lé polypeptide ayant la séquence d'acides aminés d'au moins la forme mature du polypeptide codé par l'ADNc contenu dans ATCC 209 040 ;
(d) polynucléotides comprenant une séquence nucléotidique ayant la séquence codante de l'ADNc contenu dans ATCC 209 040 codant au moins pour la forme mature du polypeptide ;
(e) polynucléotides comprenant une séquence nucléotidique codant pour un polypeptide comprenant les acides aminés de -55 à 331, de -54 à 331, de 1 à 331, de 158 à 175, de 176 à 331 ou de 298 à 308 dans la SEQ ID n° : 2 ;
(f) le polynucléotide de (a) à (e) sauf pour au moins une substitution d'acide aminé conservatrice et codant pour un polypeptide récepteur TR10 ;
(g) polynucléotides codant pour un polypeptide TR10 comprenant les résidus d'acides aminés de 2 à 58, de 75 à 142 ou de 194 à 228 dans la SEQ ID n° : 2 ;
(h) polynucléotides d'au moins 50 nucléotides contigus des nucléotides 109 à 1269 de la SEQ ID n° : 1 ou du cadre ouvert de lecture codé par le plasmide d'ADNc contenu dans ATCC n° de dépôt 209 040 ;
(i) polynucléotides codant au moins pour 30 résidus d'acides aminés contigus des positions -55 à +331 de la SEQ ID n° : 2 ou du cadre ouvert de lecture codé par le plasmide d'ADNc contenu dans ATCC n° de dépôt 209 040 ;
(j) le polynucléotide de (i) codant au moins pour 50 résidus d'acides aminés contigus ;
(k) polynucléotides qui sont identiques au moins à 90 % à un polynucléotide tel que défini par l'un quelconque de (a) à (j) et qui codent pour un polypeptide TR10 ;
(1) polynucléotides codant pour un polypeptide ayant une séquence d'acides aminés identique au moins à 90 % à la séquence d'acides aminés d'un polypeptide TR10 codé par un polynucléotide de l'un quelconque de (a) à (j) ;
(m) polynucléotides qui s'hybrident au brin complémentaire d'un polynucléotide de l'un quelconque de (a) à (1) ; et
(n) polynucléotides codant pour le domaine extracellulaire, transmembranaire, intracellulaire, le domaine de mort partielle ou les domaines extracellulaire et intracellulaire avec tout ou partie du domaine transmembranaire délété d'un polypeptide codé par un polynucléotide de l'un quelconque de (a) à (m);

ou le brin complémentaire d'un tel polynucléotide pour la préparation d'une composition pharmaceutique pour l'inhibition de l'hématopoïèse.

**5.** Utilisation d'un anticorps qui se fixe au polypeptide codé par le polynucléotide selon l'une quelconque des revendications 4(a) à 4 (n), pour la préparation d'une composition pharmaceutique pour l'inhibition de l'hématopoïèse.

**6.** Utilisation selon la revendication 4, dans laquelle le polypeptide TR10 du polypeptide de fusion TR10-Fc contient le domaine extracellulaire mature du polypeptide ayant la séquence d'acides aminés de la Met-1 à la Gly-149 de la SEQ ID n° : 2.

**7.** Utilisation d'un anticorps qui se fixe au polypeptide de fusion TR10, dans laquelle ledit polypeptide TR10 est codé par un polynucléotide choisi dans le groupe constitué de:

(a) polynucléotides comprenant une séquence nucléotidique codant au moins pour la forme mature du polypeptide ayant la séquence d'acides aminés déduite telle que représentée dans la SEQ ID n° : 2 ;
(b) polynucléotides comprenant une séquence nucléotidique ayant la séquence codante telle que représentée dans la SEQ ID n° : 1 codant au moins pour la forme mature du polypeptide ;
(c) polynucléotides comprenant une séquence nucléotidique codant pour le polypeptide ayant la séquence d'acides aminés d'au moins la forme mature du polypeptide codé par l'ADNc contenu dans ATCC 209 040 ;
(d) polynucléotides comprenant une séquence nucléotidique ayant la séquence codante de l'ADNc contenu dans ATCC 209 040 codant au moins pour la forme mature du polypeptide ;
(e) polynucléotides comprenant une séquence nucléotidique codant pour un polypeptide comprenant les acides aminés de -55 à 331, de -54 à 331, de 1 à 331, de 158 à 175, de 176 à 331 ou de 298 à 308 dans la SEQ ID n° : 2 ;
(f) le polynucléotide de (a) à (e) sauf pour au moins une substitution d'acide aminé conservatrice et codant pour un polypeptide récepteur TR10 ;
(g) polynucléotides codant pour un polypeptide TR10 comprenant les résidus d'acides aminés de 2 à 58, de 75 à 142 ou de 194 à 228 dans la SEQ ID n° : 2 ;
(h) polynucléotides d'au moins 50 nucléotides contigus des nucléotides 109 à 1269 de la SEQ ID n° : 1 ou du cadre ouvert de lecture codé par le plasmide d'ADNc contenu dans ATCC n° de dépôt 209 040;
(i) polynucléotides codant au moins pour 30 résidus d'acides aminés contigus des positions -55 à +331 de la SEQ ID n° : 2 ou du cadre ouvert de lecture codé par le plasmide d'ADNc contenu dans ATCC n° de dépôt 209 040 ;
(j) le polynucléotide de (i) codant au moins pour 50 résidus d'acides aminés contigus ;
(k) polynucléotides qui sont identiques au moins à 90 % à un polynucléotide tel que défini par l'un quelconque de (a) à (j) et qui codent pour un polypeptide TR10;
(1) polynucléotides codant pour un polypeptide ayant une séquence d'acides aminés identique au moins à 90 % à la séquence d'acides aminés d'un polypeptide TR10 codé par un polynucléotide de l'un quelconque de (a) à (j);
(m) polynucléotides qui s'hybrident au brin complémentaire d'un polynucléotide de l'un quelconque de (a) à (1) ; et

(n) polynucléotides codant pour le domaine extracellulaire, transmembranaire, intracellulaire, le domaine de mort partielle ou les domaines extracellulaire et intracellulaire avec tout ou partie du domaine transmembranaire délété d'un polypeptide codé par un polynucléotide de l'un quelconque de (a) à (m);

pour la préparation d'une composition pharmaceutique pour la stimulation de l'hématopoïèse.

# Figure 1A

```
        10                            30                            50
CGACCCACGCGTCCGCCCACGCGTCCGGAGAACCTTTGCACGCGCACAAACTACGGGGAC
        70                            90                           110
GATTTCTGATTGATTTTTGGCGCTTTCGATCCACCCTCCTCCCTTCTCATGGGACTTTGG
                                                      M  G  L  W
       130                           150                           170
GGACAAAGCGTCCCGACCGCCTCGAGCGCTCGAGCAGGGCGCTATCCAGGAGCCAGGACA
G  Q  S  V  P  T  A  S  S  A  R  A  G  R  Y  P  G  A  R  T
       190                           210                           230
GCGTCGGGAACCAGACCATGGCTCCTGGACCCCAAGATCCTTAAGTTCGTCGTCTTCATC
A  S  G  T  R  P  W  L  L  D  P  K  I  L  K  F  V  V  F  I
       250                           270                           290
GTCGCGGTTCTGCTGCCGGTCCGGGTTGACTCTGCCACCATCCCCCGGCAGGACGAAGTT
V  A  V  L  L  P  V  R  V  D  S  A  T  I  P  R  Q  D  E  V
       310                           330                           350
CCCCAGCAGACAGTGGCCCCACAGCAACAGAGGCGCAGCCTCAAGGAGGAGGAGTGTCCA
P  Q  Q  T  V  A  P  Q  Q  Q  R  R  S  L  K  E  E  E  C  P
       370                           390                           410
GCAGGATCTCATAGATCAGAATATACTGGAGCCTGTAACCCGTGCACAGAGGGTGTGGAT
A  G  S  H  R  S  E  Y  T  G  A  C  N  P  C  T  E  G  V  D
       430                           450                           470
TACACCATTGCTTCCAACAATTTGCCTTCTTGCCTGCTATGTACAGTTTGTAAATCAGGT
Y  T  I  A  S  N  N  L  P  S  C  L  L  C  T  V  C  K  S  G
       490                           510                           530
CAAACAAATAAAAGTTCCTGTACCACGACCAGAGACACCGTGTGTCAGTGTGAAAAAGGA
Q  T  N  K  S  S  C  T  T  T  R  D  T  V  C  Q  C  E  K  G
       550                           570                           590
AGCTTCCAGGATAAAAACTCCCCTGAGATGTGCCGGACGTGTAGAACAGGGTGTCCCAGA
S  F  Q  D  K  N  S  P  E  M  C  R  T  C  R  T  G  C  P  R
       610                           630                           650
GGGATGGTCAAGGTCAGTAATTGTACGCCCCGGAGTGACATCAAGTGCAAAAATGAATCA
G  M  V  K  V  S  N  C  T  P  R  S  D  I  K  C  K  N  E  S
       670                           690                           710
GCTGCCAGTTCCACTGGGAAAACCCCAGCAGCGGAGGAGACAGTGACCACCATCCTGGGG
A  A  S  S  T  G  K  T  P  A  A  E  E  T  V  T  T  I  L  G
       730.                          750                           770
ATGCTTGCCTCTCCCTATCACTACCTTATCATCATAGTGGTTTTAGTCATCATTTTAGCT
M  L  A  S  P  Y  H  Y  L  I  I  I  V  V  L  V  I  I  L  A
       790                           810                           830
GTGGTTGTGGTTGGCTTTTCATGTCGGAAGAAATTCATTTCTTACCTCAAAGGCATCTGC
V  V  V  V  G  F  S  C  R  K  K  F  I  S  Y  L  K  G  I  C
       850                           870                           890
TCAGGTGGTGGAGGAGGTCCCGAACGTGTGCACAGAGTCCTTTTCCGGCGGCGTTCATGT
S  G  G  G  G  G  P  E  R  V  H  R  V  L  F  R  R  R  S  C
       910                           930                           950
CCTTCACGAGTTCCTGGGGCGGAGGACAATGCCCGCAACGAGACCCTGAGTAACAGATAC
P  S  R  V  P  G  A  E  D  N  A  R  N  E  T  L  S  N  R  Y
```

# Figure 1B

970 990 1010
TTGCAGCCCACCCAGGTCTCTGAGCAGGAAATCCAAGGTCAGGAGCTGGCAGAGCTAACA
L Q P T Q V S E Q E I Q G Q E L A E L T
1030 1050 1070
GGTGTGACTGTAGAGTCGCCAGAGGAGCCACAGCGTCTGCTGGAACAGGCAGAAGCTGAA
G V T V E S P E E P Q R L L E Q A E A E
1090 1110 1130
GGGTGTCAGAGGAGGAGGCTGCTGGTTCCAGTGAATGACGCTGACTCCGCTGACATCAGC
G C Q R R R L L V P V N D A D S A D I S
1150 1170 1190
ACCTTGCTGGATGCCTCGGCAACACTGGAAGAAGGACATGCAAAGGAAACAATTCAGGAC
*T L L D A S A T L E E G H A K E T I Q* D
1210 1230 1250
CAACTGGTGGGCTCCGAAAAGCTCTTTTATGAAGAAGATGAGGCAGGCTCTGCTACGTCC
Q L V G S E K L F Y E E D E A G S A T S
1270 1290 1310
TGCCTGTGAAAGAATCTCTTCAGGAAACCAGAGCTTCCCTCATTTACCTTTTCTCCTACA
C L *
1330 1350 1370
AAGGGAAGCAGCCTGGAAGAAACAGTCCAGTACTTGACCCATGCCCCAACAAACTCTACT
1390 1410 1430
ATCCAATATGGGGCAGCTTACCAATGGTCCTAGAACTTTGTTAACGCACTTGGAGTAATT
1450 1470 1490
TTTATGAAATACTGCGTGTGATAAGCAAACGGAGAAATTTATATCAGATTCTTGGCTGC
1510 1530 1550
ATAGTTATACGATTGTGTATTAAGGGTCGTTTTAGGCCACATGCGGTGGCTCATGCCTGT
1570 1590 1610
AATCCCAGCACTTTGATAGGCTGAGGCAGGTGGATTGCTTTGAGCTCGGGAGTTTGAGAC
1630 1650 1670
CAGCCTCATCAACACAGTGAAACTCCATCTCAATTTAAAAAGAAAAAAAAGTGGTTTTAG
1690 1710 1730
GATGTCATTCTTTGCAGTTCTTCATCATGAGACAAGTCTTTTTTTCTGCTTCTTATATTG
1750 1770 1790
CAAGCTCCATCTCTACTGGTGTGTGCATTTAATGACATCTAACTACAGATGCCGCACAGC
1810 1830 1850
CACAATGCTTTGCCTTATAGTTTTTTAACTTTAGAACGGGATTATCTTGTTATTACCTGT
1870 1890 1910
ATTTTCAGTTTCGGATATTTTTGACTTAATGATGAGATTATCAAGACGTAGCCCTATGCT
1930 1950 1970
AAGTCATGAGCATATGGACTTACGAGGGTTCGACTTAGAGTTTTGAGCTTTAAGATAGGA
1990 2010 2030
TTATTGGGGCTTACCCCCACCTTAATTAGAGAAACATTTATATTGCTTACTACTGTAGGC
2050 2070 2090
TGTACATCTCTTTTCCGATTTTTGTATAATGATGTAAACATGGAAAAACTTTAGGAAATG
2110 2130 2150
CACTTATTAGGCTGTTTACATGGGTTGCCTGGATACAAATCAGCAGTCAAAAATGACTAA
2170 2190 2210
AAATATAACTAGTGACGGAGGGAGAAATCCTCCCTCTGTGGGAGGCACTTACTGCATTCC

Figure 1C

```
       2230                2250                2270
AGTTCTCCCTCCTGCGCCCTGAGACTGGACCAGGGTTTGATGGCTGGCAGCTTCTCAAGG
       2290                2310                2330
GGCAGCTTGTCTTACTTGTTAATTTTAGAGGTATATAGCCATATTTATTTATAAATAAAT
       2350                2370                2390
ATTTATTTATTTATTTATAAGTAGATGTTTACATATGCCCAGGATTTTGAAGAGCCTGGT
       2410                2430                2450
ATCTTTGGGAAGCCATGTGTCTGGTTTGTCGTGCTGGGACAGTCATGGGACTGCATCTTC
       2470                2490                2510
CGACTTGTCCACAGCAGATGAGGACAGTGAGAATTAAGTTAGATCCGAGACTGCGAAGAG
       2530                2550                2570
CTTCTCTTTCAAGCGCCATTACAGTTGAACGTTAGTGAATCTTGAGCCTCATTTGGGCTC
       2590                2610                2630
AGGGCAGAGCAGGTGTTTATCTGCCCCGGCATCTGCCATGGCATCAAGAGGGAAGAGTGG
       2650                2670                2690
ACGGTGCTTGGGAATGGTGTGAAATGGTTGCCGACTCAGGCATGGATGGGCCCCTCTCGC
       2710                2730                2750
TTCTGGTGGTCTGTGAACTGAGTCCCTGGGATGCCTTTTAGGGCAGAGATTCCTGAGCTG
       2770                2790                2810
CGTTTTAGGGTACAGATTCCCTGTTTGAGGAGCTTGGCCCCTCTGTAAGCATCTGACTCA
       2830                2850                2870
TCTCAGAGATATCAATTCTTAAACACTGTGACAACGGGATCTAAAATGGCTGACACATTT
       2890                2910                2930
GTCCTTGTGTCACGTTCCATTATTTTATTTAAAAACCTCAGTAATCGTTTTAGCTTCTTT
       2950                2970                2990
CCAGCAAACTCTTCTCCACAGTAGCCCAGTCGTGGTAGGATAAATTACGGATATAGTCAT
       3010                3030                3050
TCTAGGGGTTTCAGTCTTTTCCATCTCAAGGCATTGTGTGTTTTGTTCCGGGACTGGTTT
       3070                3090                3110
GGCTGGGACAAAGTTAGAACTGCCTGAAGTTCGCACATTCAGATTGTTGTGTCCATGGAG
       3130                3150                3170
TTTTAGGAGGGGATGGCCTTTCCGGTCTTCGCACTTCCATCCTCTCCCCACTTCCCATCT
       3190                3210                3230
GGCGTCCCACACCTTGTCCCCCTGCACTTCTGGATGACCAGGGTGCTGCTGCCTCCTAGT
       3250                3270                3290
CTTTGCCTTTGCTGGGCCTTCTGTGCAGGAGACTTGGTCTCAAAGCTCAGAGAGAGCCAG
       3310                3330                3350
TCCGGTCCCAGCTCCTTTGTCCCTTCCTCAGAGGCCTTCCTTGAAGATGCATCTAGACTA
       3370                3390                3410
CCAGCCTTATCAGTGTTTAAGCTTATTCCTTTAACATAAGCTTCCTGACAACATGAAATT
       3430                3450                3470
GTTGGGGTTTTTTGGCGTTTGTTGATTTGTTTAGGTTTTGCTTTATACCCGGGCCAAATA
       3490                3510                3530
GCACATAACACCTGGTTATATATGAAATACTCATATGTTTATGACCAAAATAAATATGAA
       3550
ACCTCAAAAAAAAAAAAAAAAAAAA
```

Figure 2

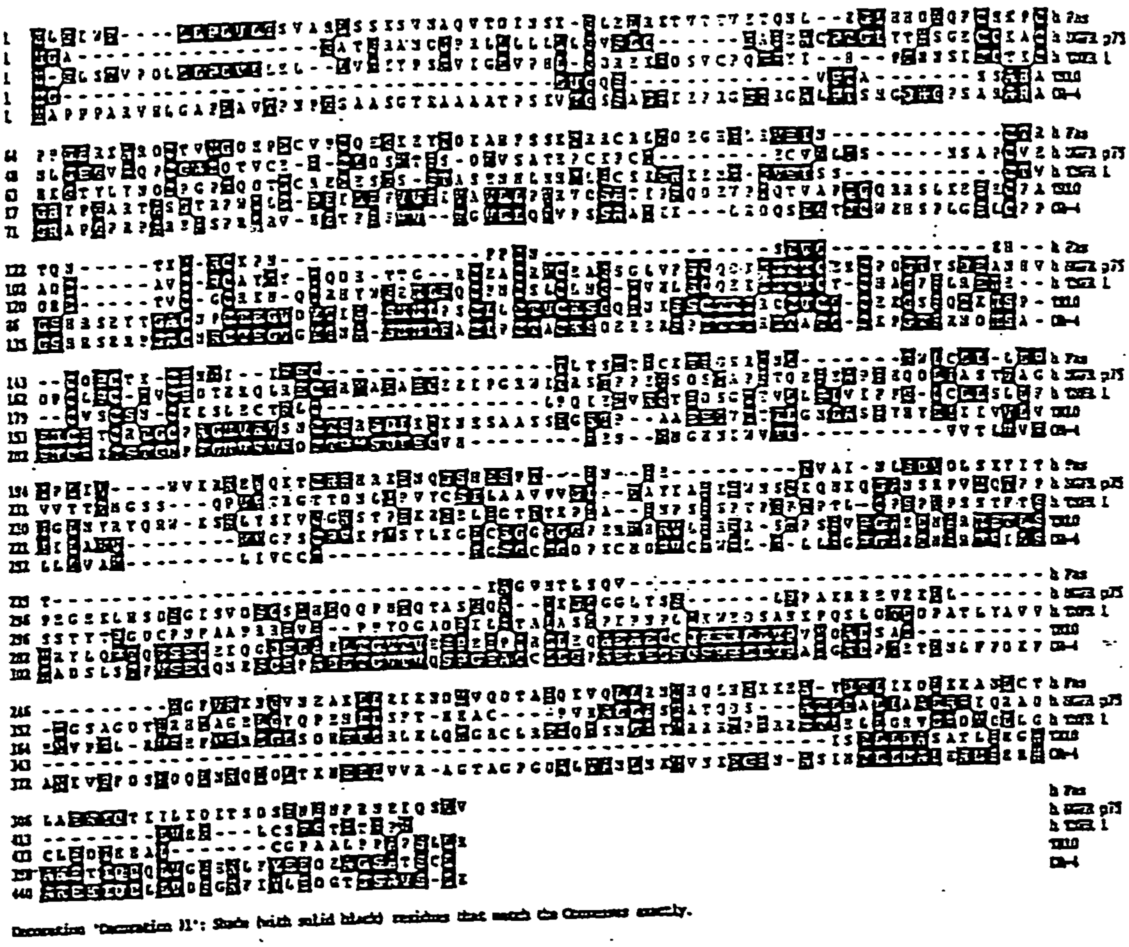

Decoration 'Decoration #1': Shade (with solid black) residues that match the Consensus exactly.

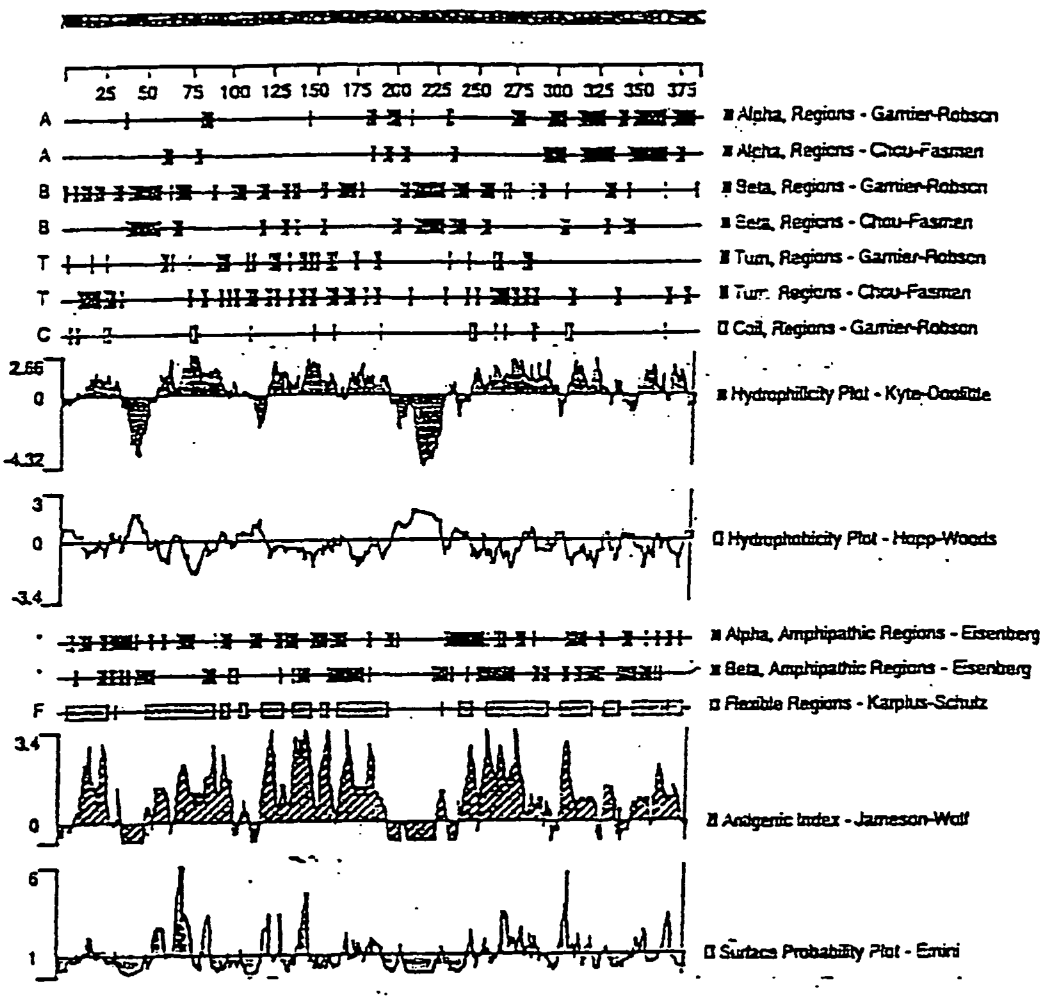
25 50 75 100 125 150 175 200 225 250 275 300 325 350 375
A
Alpha, Regions - Garnier-Robson
A
Alpha, Regions - Chou-Fasman
B
Beta, Regions - Garnier-Robson
B
Beta, Regions - Chou-Fasman
T
Turn, Regions - Garnier-Robson
T
Turn, Regions - Chou-Fasman
C
Coil, Regions - Garnier-Robson
2.66
0
-4.32
Hydrophilicity Plot - Kyte-Doolittle
3
0
-3.4
Hydrophobicity Plot - Hopp-Woods
Alpha, Amphipathic Regions - Eisenberg
Beta, Amphipathic Regions - Eisenberg
F
Flexible Regions - Karplus-Schulz
3.4
0
Antigenic Index - Jameson-Wolf
6
1
Surface Probability Plot - Emini

Figure 4

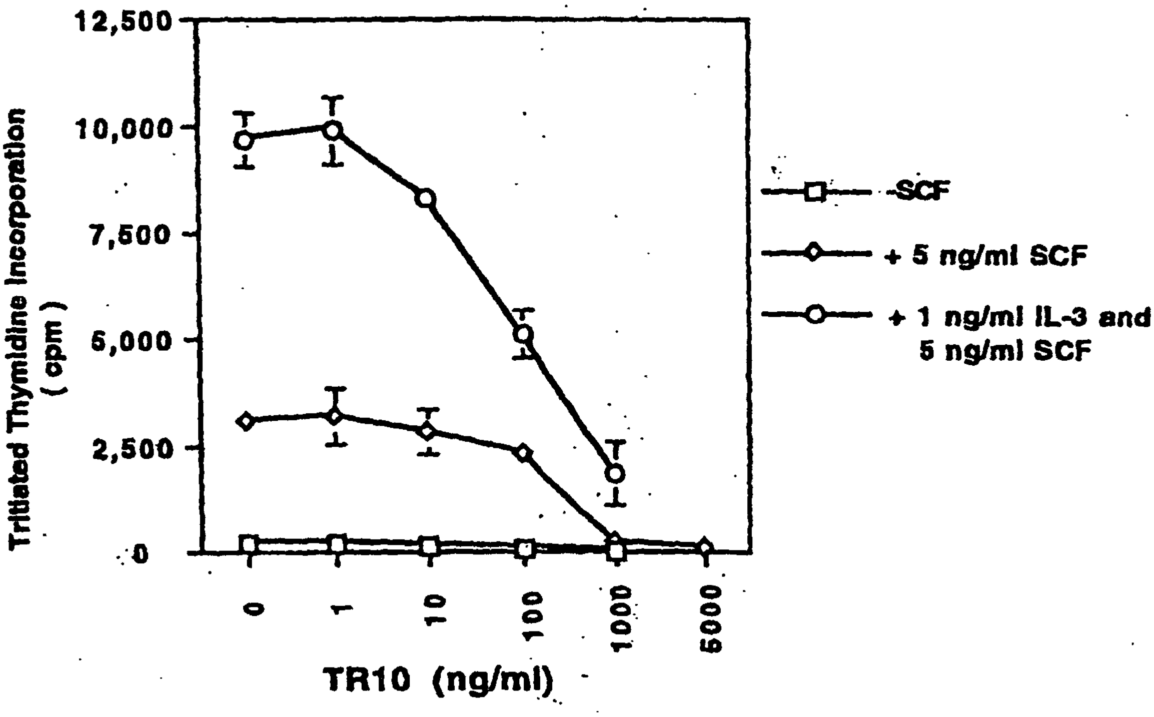
12,500
10,000
7,500
5,000
2,500
0
Tritiated Thymidine Incorporation ( cpm )
0
1
10
100
1000
5000
TR10 (ng/ml)
-SCF
+ 5 ng/ml SCF
+ 1 ng/ml IL-3 and 5 ng/ml SCF

SEQUENCE LISTING

<110> Human Genome Sciences, Inc.

<120> Human Tumor Necrosis Factor Receptor TR10

<130> F 2982 EP

<140> PCT/US00/14554
<141> 2000-05-25

<150> 60/136,786
<151> 1999-05-28

<150> 60/142,563
<151> 1999-07-07

<150> 60/144,023
<151> 1999-07-15

<160> 16

<170> PatentIn Ver. 2.0

<210> 1
<211> 3566
<212> DNA
<213> Homo sapiens

<220>
<221> CDS
<222> (109)..(1266)

<220>
<221> sig_peptide
<222> (109)..(271)

<220>
<221> mat_peptide
<222> (274) (1266)

<400> 1

```
cgacccacgc gtccgcccac gcgtccggag aacctttgca cgcgcacaaa ctacggggac 60

gatttctgat tgatttttgg cgctttcgat ccaccctcct cccttctc atg gga ctt  117
                                                     Met Gly Leu
                                                     -55

tgg gga caa agc gtc ccg acc gcc tcg agc gct cga gca ggg cgc tat   165
Trp Gly Gln Ser Val Pro Thr Ala Ser Ser Ala Arg Ala Gly Arg Tyr
        -50                 -45                 -40

cca gga gcc agg aca gcg tcg gga acc aga cca tgg ctc ctg gac ccc   213
Pro Gly Ala Arg Thr Ala Ser Gly Thr Arg Pro Trp Leu Leu Asp Pro
    -35                 -30                 -25

aag atc ctt aag ttc gtc gtc ttc atc gtc gcg gtt ctg ctg ccg gtc   261
Lys Ile Leu Lys Phe Val Val Phe Ile Val Ala Val Leu Leu Pro Val
-20                 -15                 -10                     -5

cgg gtt gac tct gcc acc atc ccc cgg cag gac gaa gtt ccc cag cag   309
Arg Val Asp Ser Ala Thr Ile Pro Arg Gln Asp Glu Val Pro Gln Gln
            -1   1              5                   10
```

```
aca gtg gcc cca cag caa cag agg cgc agc ctc aag gag gag gag tgt    357
Thr Val Ala Pro Gln Gln Gln Arg Arg Ser Leu Lys Glu Glu Glu Cys
        15                  20                  25

cca gca gga tct cat aga tca gaa tat act gga gcc tgt aac ccg tgc    405
Pro Ala Gly Ser His Arg Ser Glu Tyr Thr Gly Ala Cys Asn Pro Cys
    30                  35                  40

aca gag ggt gtg gat tac acc att gct tcc aac aat ttg cct tct tgc    453
Thr Glu Gly Val Asp Tyr Thr Ile Ala Ser Asn Asn Leu Pro Ser Cys
45                  50                  55                  60

ctg cta tgt aca gtt tgt aaa tca ggt caa aca aat aaa agt tcc tgt    501
Leu Leu Cys Thr Val Cys Lys Ser Gly Gln Thr Asn Lys Ser Ser Cys
                65                  70                  75

acc acg acc aga gac acc gtg tgt cag tgt gaa aaa gga agc ttc cag    549
Thr Thr Thr Arg Asp Thr Val Cys Gln Cys Glu Lys Gly Ser Phe Gln
            80                  85                  90

gat aaa aac tcc cct gag atg tgc cgg acg tgt aga aca ggg tgt ccc    597
Asp Lys Asn Ser Pro Glu Met Cys Arg Thr Cys Arg Thr Gly Cys Pro
        95                  100                 105

aga ggg atg gtc aag gtc agt aat tgt acg ccc cgg agt gac atc aag    645
Arg Gly Met Val Lys Val Ser Asn Cys Thr Pro Arg Ser Asp Ile Lys
    110                 115                 120

tgc aaa aat gaa tca gct gcc agt tcc act ggg aaa acc cca gca gcg    693
Cys Lys Asn Glu Ser Ala Ala Ser Ser Thr Gly Lys Thr Pro Ala Ala
125                 130                 135                 140

gag gag aca gtg acc acc atc ctg ggg atg ctt gcc tct ccc tat cac    741
Glu Glu Thr Val Thr Thr Ile Leu Gly Met Leu Ala Ser Pro Tyr His
                145                 150                 155

tac ctt atc atc ata gtg gtt tta gtc atc att tta gct gtg gtt gtg    789
Tyr Leu Ile Ile Ile Val Val Leu Val Ile Ile Leu Ala Val Val Val
            160                 165                 170

gtt ggc ttt tca tgt cgg aag aaa ttc att tct tac ctc aaa ggc atc    837
Val Gly Phe Ser Cys Arg Lys Lys Phe Ile Ser Tyr Leu Lys Gly Ile
        175                 180                 185

tgc tca ggt ggt gga gga ggt ccc gaa cgt gtg cac aga gtc ctt ttc    885
Cys Ser Gly Gly Gly Gly Gly Pro Glu Arg Val His Arg Val Leu Phe
    190                 195                 200

cgg cgg cgt tca tgt cct tca cga gtt cct ggg gcg gag gac aat gcc    933
Arg Arg Arg Ser Cys Pro Ser Arg Val Pro Gly Ala Glu Asp Asn Ala
205                 210                 215                 220

cgc aac gag acc ctg agt aac aga tac ttg cag ccc acc cag gtc tct    981
Arg Asn Glu Thr Leu Ser Asn Arg Tyr Leu Gln Pro Thr Gln Val Ser
                225                 230                 235

gag cag gaa atc caa ggt cag gag ctg gca gag cta aca ggt gtg act    1029
Glu Gln Glu Ile Gln Gly Gln Glu Leu Ala Glu Leu Thr Gly Val Thr
            240                 245                 250

gta gag tcg cca gag gag cca cag cgt ctg ctg gaa cag gca gaa gct    1077
Val Glu Ser Pro Glu Glu Pro Gln Arg Leu Leu Glu Gln Ala Glu Ala
```

```
        255                 260                 265

gaa ggg tgt cag agg agg agg ctg ctg gtt cca gtg aat gac gct gac    1125
Glu Gly Cys Gln Arg Arg Arg Leu Leu Val Pro Val Asn Asp Ala Asp
    270                 275                 280

tcc gct gac atc agc acc ttg ctg gat gcc tcg gca aca ctg gaa gaa    1173
Ser Ala Asp Ile Ser Thr Leu Leu Asp Ala Ser Ala Thr Leu Glu Glu
285                 290                 295                 300

gga cat gca aag gaa aca att cag gac caa ctg gtg ggc tcc gaa aag    1221
Gly His Ala Lys Glu Thr Ile Gln Asp Gln Leu Val Gly Ser Glu Lys
                305                 310                 315

ctc ttt tat gaa gaa gat gag gca ggc tct gct acg tcc tgc ctg        1266
Leu Phe Tyr Glu Glu Asp Glu Ala Gly Ser Ala Thr Ser Cys Leu
            320                 325                 330

tgaaagaatc tcttcaggaa accagagctt ccctcattta ccttttctcc tacaaaggga 1326
agcagcctgg aagaaacagt ccagtacttg acccatgccc caacaaactc tactatccaa 1386
tatggggcag cttaccaatg gtcctagaac tttgttaacg cacttggagt aatttttatg 1446
aaatactgcg tgtgataagc aaacgggaga aatttatatc agattcttgg ctgcatagtt 1506
atacgattgt gtattaaggg tcgttttagg ccacatgcgg tggctcatgc ctgtaatccc 1566
agcactttga taggctgagg caggtggatt gctttgagct cgggagtttg agaccagcct 1626
catcaacaca gtgaaactcc atctcaattt aaaaagaaaa aaaagtggtt ttaggatgtc 1686
attctttgca gttcttcatc atgagacaag tctttttttc tgcttcttat attgcaagct 1746
ccatctctac tggtgtgtgc atttaatgac atctaactac agatgccgca cagccacaat 1806
gctttgcctt atagtttttt aactttagaa cgggattatc ttgttattac ctgtattttc 1866
agtttcggat atttttgact taatgatgag attatcaaga cgtagcccta tgctaagtca 1926
tgagcatatg gacttacgag ggttcgactt agagttttga gctttaagat aggattattg 1986
gggcttaccc ccaccttaat tagagaaaca tttatattgc ttactactgt aggctgtaca 2046
tctcttttcc gatttttgta taatgatgta aacatggaaa aactttagga aatgcactta 2106
ttaggctgtt tacatgggtt gcctggatac aaatcagcag tcaaaaatga ctaaaaatat 2166
aactagtgac ggagggagaa atcctccctc tgtgggaggc acttactgca ttccagttct 2226
ccctcctgcg ccctgagact ggaccagggt ttgatggctg gcagcttctc aaggggcagc 2286
ttgtcttact tgttaatttt agaggtatat agccatattt atttataaat aaatatttat 2346
ttatttattt ataagtagat gtttacatat gcccaggatt ttgaagagcc tggtatcttt 2406
gggaagccat gtgtctggtt tgtcgtgctg ggacagtcat gggactgcat cttccgactt 2466
gtccacagca gatgaggaca gtgagaatta agttagatcc gagactgcga agagcttctc 2526
tttcaagcgc cattacagtt gaacgttagt gaatcttgag cctcatttgg gctcagggca 2586
gagcaggtgt ttatctgccc cggcatctgc catggcatca agagggaaga gtggacggtg 2646
```

cttgggaatg gtgtgaaatg gttgccgact caggcatgga tgggcccctc tcgcttctgg 2706
tggtctgtga actgagtccc tgggatgcct tttagggcag agattcctga gctgcgtttt 2766
agggtacaga ttccctgttt gaggagcttg gcccctctgt aagcatctga ctcatctcag 2826
agatatcaat tcttaaacac tgtgacaacg ggatctaaaa tggctgacac atttgtcctt 2886
gtgtcacgtt ccattatttt atttaaaaac ctcagtaatc gttttagctt ctttccagca 2946
aactcttctc cacagtagcc cagtcgtggt aggataaatt acggatatag tcattctagg 3006
ggtttcagtc ttttccatct caaggcattg tgtgttttgt tccgggactg gtttggctgg 3066
gacaaagtta gaactgcctg aagttcgcac attcagattg ttgtgtccat ggagttttag 3126
gaggggatgg cctttccggt cttcgcactt ccatcctctc cccacttccc atctggcgtc 3186
ccacaccttg tcccccctgca cttctggatg accagggtgc tgctgcctcc tagtctttgc 3246
ctttgctggg ccttctgtgc aggagacttg gtctcaaagc tcagagagag ccagtccggt 3306
cccagctcct ttgtcccttc ctcagaggcc ttccttgaag atgcatctag actaccagcc 3366
ttatcagtgt ttaagcttat tcctttaaca taagcttcct gacaacatga aattgttggg 3426
gttttttggc gtttgttgat ttgtttaggt tttgctttat acccgggcca aatagcacat 3486
aacacctggt tatatatgaa atactcatat gtttatgacc aaaataaata tgaaacctca 3546
aaaaaaaaaa aaaaaaaaaa 3566

<210> 2
<211> 386
<212> PRT
<213> Homo sapiens

<400> 2

```
Met Gly Leu Trp Gly Gln Ser Val Pro Thr Ala Ser Ser Ala Arg Ala
-55                 -50                 -45                 -40

Gly Arg Tyr Pro Gly Ala Arg Thr Ala Ser Gly Thr Arg Pro Trp Leu
                -35                 -30                 -25

Leu Asp Pro Lys Ile Leu Lys Phe Val Val Phe Ile Val Ala Val Leu
            -20                 -15                 -10

Leu Pro Val Arg Val Asp Ser Ala Thr Ile Pro Arg Gln Asp Glu Val
        -5                  -1   1              5

Pro Gln Gln Thr Val Ala Pro Gln Gln Gln Arg Arg Ser Leu Lys Glu
 10                  15                  20                  25

Glu Glu Cys Pro Ala Gly Ser His Arg Ser Glu Tyr Thr Gly Ala Cys
                30                  35                  40

Asn Pro Cys Thr Glu Gly Val Asp Tyr Thr Ile Ala Ser Asn Asn Leu
            45                  50                  55

Pro Ser Cys Leu Leu Cys Thr Val Cys Lys Ser Gly Gln Thr Asn Lys
        60                  65                  70
```

```
Ser Ser Cys Thr Thr Thr Arg Asp Thr Val Cys Gln Cys Glu Lys Gly
    75                  80                  85

Ser Phe Gln Asp Lys Asn Ser Pro Glu Met Cys Arg Thr Cys Arg Thr
90                  95                  100                 105

Gly Cys Pro Arg Gly Met Val Lys Val Ser Asn Cys Thr Pro Arg Ser
                110                 115                 120

Asp Ile Lys Cys Lys Asn Glu Ser Ala Ala Ser Ser Thr Gly Lys Thr
            125                 130                 135

Pro Ala Ala Glu Glu Thr Val Thr Thr Ile Leu Gly Met Leu Ala Ser
        140                 145                 150

Pro Tyr His Tyr Leu Ile Ile Ile Val Val Leu Val Ile Ile Leu Ala
    155                 160                 165

Val Val Val Val Gly Phe Ser Cys Arg Lys Lys Phe Ile Ser Tyr Leu
170                 175                 180                 185

Lys Gly Ile Cys Ser Gly Gly Gly Gly Gly Pro Glu Arg Val His Arg
                190                 195                 200

Val Leu Phe Arg Arg Arg Ser Cys Pro Ser Arg Val Pro Gly Ala Glu
            205                 210                 215

Asp Asn Ala Arg Asn Glu Thr Leu Ser Asn Arg Tyr Leu Gln Pro Thr
        220                 225                 230

Gln Val Ser Glu Gln Glu Ile Gln Gly Gln Glu Leu Ala Glu Leu Thr
    235                 240                 245

Gly Val Thr Val Glu Ser Pro Glu Glu Pro Gln Arg Leu Leu Glu Gln
250                 255                 260                 265

Ala Glu Ala Glu Gly Cys Gln Arg Arg Arg Leu Leu Val Pro Val Asn
                270                 275                 280

Asp Ala Asp Ser Ala Asp Ile Ser Thr Leu Leu Asp Ala Ser Ala Thr
            285                 290                 295

Leu Glu Glu Gly His Ala Lys Glu Thr Ile Gln Asp Gln Leu Val Gly
        300                 305                 310

Ser Glu Lys Leu Phe Tyr Glu Glu Asp Glu Ala Gly Ser Ala Thr Ser
    315                 320                 325

Cys Leu
330
```

<210> 3
<211> 331
<212> PRT
<213> Homo sapiens

<400> 3

Met Leu Gly Ile Trp Thr Leu Leu Pro Leu Val Leu Thr Ser Val Ala
1 5 10 15

Arg Leu Ser Ser Lys Ser Val Asn Ala Gln Val Thr Asp Ile Asn Ser

```
            20                      25                      30

Lys Gly Leu Glu Leu Arg Lys Thr Val Thr Val Glu Thr Gln Asn Leu
        35                  40                  45

Glu Gly Leu His His Asp Gly Gln Phe Cys His Pro Cys Pro Pro Gly
    50                  55                  60

Glu Arg Lys Ala Arg Asp Cys Thr Val Asn Gly Asp Glu Pro Asp Cys
65                  70                  75                  80

Val Pro Cys Gln Glu Gly Lys Glu Tyr Thr Asp Lys Ala His Phe Ser
                85                  90                  95

Ser Lys Cys Arg Arg Cys Arg Leu Cys Asp Glu Gly His Gly Leu Glu
            100                 105                 110

Val Glu Ile Asn Cys Thr Arg Thr Gln Asn Thr Lys Cys Arg Cys Lys
        115                 120                 125

Pro Asn Phe Phe Cys Asn Ser Thr Val Cys Glu His Cys Asp Pro Cys
    130                 135                 140

Thr Lys Cys Glu His Gly Ile Ile Lys Glu Cys Thr Leu Thr Ser Asn
145                 150                 155                 160

Thr Lys Cys Lys Glu Glu Gly Ser Arg Ser Asn Gly Trp Leu Cys Leu
                165                 170                 175

Leu Leu Leu Pro Ile Pro Leu Ile Val Trp Val Lys Arg Lys Glu Val
            180                 185                 190

Gln Lys Thr Cys Arg Lys His Arg Lys Glu Asn Gln Gly Ser His Glu
        195                 200                 205

Ser Pro Thr Leu Asn Pro Glu Thr Val Ala Ile Asn Leu Ser Asp Val
    210                 215                 220

Asp Leu Ser Lys Tyr Ile Thr Thr Ile Ala Gly Val Met Thr Leu Ser
225                 230                 235                 240

Gln Val Lys Gly Phe Val Arg Lys Asn Gly Val Asn Glu Ala Lys Ile
                245                 250                 255

Asp Glu Ile Lys Asn Asp Asn Val Gln Asp Thr Ala Glu Gln Lys Val
            260                 265                 270

Gln Leu Leu Arg Asn Trp His Gln Leu His Gly Lys Lys Glu Ala Tyr
        275                 280                 285

Asp Thr Leu Ile Lys Asp Leu Lys Lys Ala Asn Leu Cys Thr Leu Ala
    290                 295                 300

Glu Lys Ile Thr Ile Ile Leu Lys Asp Ile Thr Ser Asp Ser Glu Asn
305                 310                 315                 320

Ser Asn Phe Arg Asn Glu Ile Gln Ser Leu Val
                325                 330
```

<210> 4
<211> 427
<212> PRT
<213> Homo sapiens

<400> 4

```
Met Gly Ala Gly Ala Thr Gly Arg Ala Met Asp Gly Pro Arg Leu Leu
1               5                   10                  15

Leu Leu Leu Leu Leu Gly Val Ser Leu Gly Gly Ala Lys Glu Ala Cys
            20                  25                  30

Pro Thr Gly Leu Tyr Thr His Ser Gly Glu Cys Cys Lys Ala Cys Asn
        35                  40                  45

Leu Gly Glu Gly Val Ala Gln Pro Cys Gly Ala Asn Gln Thr Val Cys
    50                  55                  60

Glu Pro Cys Leu Asp Ser Val Thr Phe Ser Asp Val Val Ser Ala Thr
65                  70                  75                  80

Glu Pro Cys Lys Pro Cys Thr Glu Cys Val Gly Leu Gln Ser Met Ser
                85                  90                  95

Ala Pro Cys Val Glu Ala Asp Asp Ala Val Cys Arg Cys Ala Tyr Gly
            100                 105                 110

Tyr Tyr Gln Asp Glu Thr Thr Gly Arg Cys Glu Ala Cys Arg Val Cys
        115                 120                 125

Glu Ala Gly Ser Gly Leu Val Phe Ser Cys Gln Asp Lys Gln Asn Thr
    130                 135                 140

Val Cys Glu Glu Cys Pro Asp Gly Thr Tyr Ser Asp Glu Ala Asn His
145                 150                 155                 160

Val Asp Pro Cys Leu Pro Cys Thr Val Cys Glu Asp Thr Glu Arg Gln
                165                 170                 175

Leu Arg Glu Cys Thr Arg Trp Ala Asp Ala Glu Cys Glu Glu Ile Pro
            180                 185                 190

Gly Arg Trp Ile Thr Arg Ser Thr Pro Pro Glu Gly Ser Asp Ser Thr
        195                 200                 205

Ala Pro Ser Thr Gln Glu Pro Glu Ala Pro Pro Glu Gln Asp Leu Ile
    210                 215                 220

Ala Ser Thr Val Ala Gly Val Val Thr Thr Val Met Gly Ser Ser Gln
225                 230                 235                 240

Pro Val Val Thr Arg Gly Thr Thr Asp Asn Leu Ile Pro Val Tyr Cys
                245                 250                 255

Ser Ile Leu Ala Ala Val Val Val Gly Leu Val Ala Tyr Ile Ala Phe
            260                 265                 270

Lys Arg Trp Asn Ser Cys Lys Gln Asn Lys Gln Gly Ala Asn Ser Arg
        275                 280                 285

Pro Val Asn Gln Thr Pro Pro Pro Glu Gly Glu Lys Leu His Ser Asp
    290                 295                 300

Ser Gly Ile Ser Val Asp Ser Gln Ser Leu His Asp Gln Gln Pro His
305                 310                 315                 320
```

```
Thr Gln Thr Ala Ser Gly Gln Ala Leu Lys Gly Asp Gly Gly Leu Tyr
                325                 330                 335

Ser Ser Leu Pro Pro Ala Lys Arg Glu Glu Val Glu Lys Leu Leu Asn
            340                 345                 350

Gly Ser Ala Gly Asp Thr Trp Arg His Leu Ala Gly Glu Leu Gly Tyr
        355                 360                 365

Gln Pro Glu His Ile Asp Ser Phe Thr His Glu Ala Cys Pro Val Arg
    370                 375                 380

Ala Leu Leu Ala Ser Trp Ala Thr Gln Asp Ser Ala Thr Leu Asp Ala
385                 390                 395                 400

Leu Leu Ala Ala Leu Arg Arg Ile Gln Arg Ala Asp Leu Val Glu Ser
                405                 410                 415

Leu Cys Ser Glu Ser Thr Ala Thr Ser Pro Val
            420                 425
```

<210> 5
<211> 453
<212> PRT
<213> Homo sapiens

<400> 5

```
Met Gly Leu Ser Thr Val Pro Asp Leu Leu Leu Pro Leu Val Leu Glu
  1               5                  10                  15

Leu Leu Val Gly Ile Tyr Pro Ser Gly Val Ile Gly Leu Val Pro His
             20                  25                  30

Leu Gly Asp Arg Glu Lys Arg Asp Ser Val Cys Pro Gln Gly Lys Tyr
         35                  40                  45

Ile His Pro Asn Asn Ser Ile Cys Cys Thr Lys Cys His Lys Gly Thr
     50                  55                  60

Tyr Leu Tyr Asn Asp Cys Pro Gly Pro Gly Gln Asp Thr Asp Cys Arg
 65                  70                  75                  80

Glu Cys Glu Ser Gly Ser Phe Thr Ala Ser Glu Asn His Leu Arg His
                 85                  90                  95

Cys Leu Ser Cys Ser Lys Cys Arg Lys Glu Met Gly Gln Val Glu Ile
            100                 105                 110

Ser Ser Cys Thr Val Asp Arg Asp Thr Val Cys Gly Cys Arg Lys Asn
        115                 120                 125

Gln Tyr Arg His Tyr Trp Ser Glu Asn Leu Phe Gln Cys Phe Asn Cys
    130                 135                 140

Ser Leu Cys Leu Asn Gly Thr Val His Leu Ser Cys Gln Glu Lys Gln
145                 150                 155                 160

Asn Thr Val Cys Thr Cys His Ala Gly Phe Phe Leu Arg Glu Asn Glu
                165                 170                 175

Cys Val Ser Cys Ser Asn Cys Lys Lys Ser Leu Glu Cys Thr Lys Leu
            180                 185                 190
```

Cys Leu Pro Gln Ile Glu Asn Val Lys Gly Thr Glu Asp Ser Gly Thr
195 200 205

Thr Val Leu Leu Pro Leu Val Ile Phe Phe Gly Leu Cys Leu Leu Ser
210 215 220

Leu Leu Phe Ile Gly Leu Met Tyr Arg Tyr Gln Arg Trp Lys Ser Lys
225 230 235 240

Leu Tyr Ser Ile Val Cys Gly Lys Ser Thr Pro Glu Lys Glu Gly Glu
245 250 255

Leu Glu Gly Thr Thr Thr Lys Pro Leu Ala Pro Asn Pro Ser Phe Ser
260 265 270

Pro Thr Pro Gly Phe Thr Pro Thr Leu Gly Phe Ser Pro Val Pro Ser
275 280 285

Ser Thr Phe Thr Ser Ser Ser Thr Tyr Thr Pro Gly Asp Cys Pro Asn
290 295 300

Phe Ala Ala Pro Arg Arg Glu Val Ala Pro Pro Tyr Gln Gly Ala Asp
305 310 315 320

Pro Ile Leu Ala Thr Ala Leu Ala Ser Asp Pro Ile Pro Asn Pro Leu
325 330 335

Gln Lys Trp Glu Asp Ser Ala His Lys Pro Gln Ser Leu Asp Thr Asp
340 345 350

Asp Pro Ala Thr Leu Tyr Ala Val Val Glu Asn Val Pro Pro Leu Arg
355 360 365

Trp Lys Glu Phe Val Arg Arg Leu Gly Leu Ser Asp His Glu Ile Asp
370 375 380

Arg Leu Glu Leu Gln Asn Gly Arg Cys Leu Arg Glu Ala Gln Tyr Ser
385 390 395 400

Met Leu Ala Thr Trp Arg Arg Arg Thr Pro Arg Arg Glu Ala Thr Leu
405 410 415

Glu Leu Leu Gly Arg Val Leu Arg Asp Met Asp Leu Leu Gly Cys Leu
420 425 430

Glu Asp Ile Glu Glu Ala Leu Cys Gly Pro Ala Ala Leu Pro Pro Ala
435 440 445

Pro Ser Leu Leu Arg
450

<210> 6
<211> 467
<212> PRT
<213> Homo sapiens'

<400> 6

Met Ala Pro Pro Pro Ala Arg Val His Leu Gly Ala Phe Leu Ala Val
1 5 10 15

Thr Pro Asn Pro Gly Ser Ala Ala Ser Gly Thr Glu Ala Ala Ala Ala

```
                    20                  25                  30

Thr Pro Ser Lys Val Trp Gly Ser Ser Ala Gly Arg Ile Glu Pro Arg
        35                  40                  45

Gly Gly Gly Arg Gly Ala Leu Pro Thr Ser Met Gly Gln His Gly Pro
    50                  55                  60

Ser Ala Arg Ala Arg Ala Gly Arg Ala Pro Gly Pro Arg Pro Ala Arg
65                  70                  75                  80

Glu Ala Ser Pro Arg Leu Arg Val His Lys Thr Phe Lys Phe Val Val
                85                  90                  95

Val Gly Val Leu Leu Gln Val Val Pro Ser Ser Ala Ala Thr Ile Lys
            100                 105                 110

Leu His Asp Gln Ser Ile Gly Thr Gln Gln Trp Glu His Ser Pro Leu
        115                 120                 125

Gly Glu Leu Cys Pro Pro Gly Ser His Arg Ser Glu Arg Pro Gly Ala
    130                 135                 140

Cys Asn Arg Cys Thr Glu Gly Val Gly Tyr Thr Asn Ala Ser Asn Asn
145                 150                 155                 160

Leu Phe Ala Cys Leu Pro Cys Thr Ala Cys Lys Ser Asp Glu Glu Glu
                165                 170                 175

Arg Ser Pro Cys Thr Thr Thr Arg Asn Thr Ala Cys Gln Cys Lys Pro
            180                 185                 190

Gly Thr Phe Arg Asn Asp Asn Ser Ala Glu Met Cys Arg Lys Cys Ser
        195                 200                 205

Thr Gly Cys Pro Arg Gly Met Val Lys Val Lys Asp Cys Thr Pro Trp
    210                 215                 220

Ser Asp Ile Glu Cys Val His Lys Glu Ser Gly Asn Gly His Asn Ile
225                 230                 235                 240

Trp Val Ile Leu Val Val Thr Leu Val Val Pro Leu Leu Leu Val Ala
                245                 250                 255

Val Leu Ile Val Cys Cys Cys Ile Gly Ser Gly Cys Gly Gly Asp Pro
            260                 265                 270

Lys Cys Met Asp Arg Val Cys Phe Trp Arg Leu Gly Leu Leu Arg Gly
        275                 280                 285

Pro Gly Ala Glu Asp Asn Ala His Asn Glu Ile Leu Ser Asn Ala Asp
    290                 295                 300

Ser Leu Ser Thr Phe Val Ser Glu Gln Gln Met Glu Ser Gln Glu Pro
305                 310                 315                 320

Ala Asp Leu Thr Gly Val Val Gln Ser Pro Gly Glu Ala Gln Cys Leu
                325                 330                 335

Leu Gly Pro Ala Glu Ala Glu Gly Ser Gln Arg Arg Arg Leu Leu Val
            340                 345                 350

Pro Ala Asn Gly Ala Asp Pro Thr Glu Thr Leu Met Leu Phe Phe Asp
```

355 360 365

Lys Phe Ala Asn Ile Val Pro Phe Asp Ser Trp Asp Gln Leu Met Arg
370 375 380

Gln Leu Asp Leu Thr Lys Asn Glu Ile Asp Val Val Arg Ala Gly Thr
385 390 395 400

Ala Gly Pro Gly Asp Ala Leu Tyr Ala Met Leu Met Lys Trp Val Asn
405 410 415

Lys Thr Gly Arg Asn Ala Ser Ile His Thr Leu Leu Asp Ala Leu Glu
420 425 430

Arg Met Glu Glu Arg His Ala Lys Glu Lys Ile Gln Asp Leu Leu Val
435 440 445

Asp Ser Gly Lys Phe Ile Tyr Leu Glu Asp Gly Thr Gly Ser Ala Val
450 455 460

Ser Leu Glu
465

<210> 7
<211> 344
<212> DNA
<213> Homo sapiens

<400> 7

gtcacgttcc attattttat ttaaaaacct cagtaatcgt tttagcttct ttccagcaaa 60

ctcttctcca cagtagccca gtcgtggtag gataaattac ggatatagtc attctagggg 120

tttcagtctt ttccatctca aggcattgtg tgttttgttc cgggactggt ttggctggga 180

caaagttaga actgcctgaa gttcgcacat tcagattgtt gtgtccatgg agttttagga 240

ggggatggcc tttccggtct tcgcacttcc atcctctccc acttccatct ggcgtnccac 300

aacttgtccc ctgcacttct ggatgacaca gggtgctgct gcct 344

<210> 8
<211> 280
<212> DNA
<213> Homo sapiens

<400> 8

gtggacggtg cttgggaatg gtgtgaaatg gttgccgact caggcatgga tgggcccctc 60
tcgcttctgg tggtctgtga actgagtccc tgggatgcct ttnagggcag agattcctga 120
gctgcgtttt agggtacaga ttccctgttt gaggagcttg gcccctctgt aagcgtctga 180
ctcatctcag agatatcaat tcttaaacac tgtgacaacg ggatctaaaa tggctgacac 240
atttgtcctt gtgtcacgtt ccattatttt atttaaaatt 280

<210> 9
<211> 261
<212> DNA
<213> Homo sapiens

<400> 9

ggccacgtag tgccacgtng ccacaaacta cgggggacga tttctgantt gaatttttgg 60
cgctttcaat ccaccctcct cccttctaat gggactttgg ggacaaagng tnccgaccgc 120
ctcgagcgnt cgancagggc gctatccagg agccaggaca gcgtcgggaa ccagaccatg 180
gctcctggac cccaagatcc ttaagttcgt cgtcttcatc gtcgnggttc tnctgccggt 240
aagtntagnn gaggtccctg g 261

<210> 10
<211> 26
<212> DNA
<213> Homo sapiens

<400> 10

cgcccatggc caccatcccc cggcag 26

<210> 11
<211> 30
<212> DNA .
<213> Homo sapiens

<400> 11

cgcaagcttt tagtagtgat agggagaggc 30

<210> 12
<211> 33
<212> DNA
<213> Homo sapiens

<400> 12

cgcggatccg ccatcatggg actttgggga caa 33

<210> 13
<211> 30
<212> DNA
<213> Homo sapiens

<400> 13

cgcggtacct tagtagtgat agggagaggc 30

<210> 14
<211> 58
<212> DNA
<213> Homo sapiens

<400> 14

cgctctagat caagcgtagt ctgggacgtc gtatgggtag taagtgatag ggagaggc 58

<210> 15
<211> 398
<212> DNA
<213> Homo sapiens

<400> 15

gagtttgacc agagatgcaa ggggtgaagg agcgcttcct accgttagga actctgggga 60

cagagcgccc cggccgcctg atggcgaggc agggtgcgac ccaggaccca ggacggcgtc 120

gggaaccata ccatggcccg gatccccaag accctaaagt tcgtcgtcgt catcgtcgcg 180

gtcctgctgc cagtcctagc ttactctgcc accactgccc ggcagaggga agttccccag 240

cagacagtgg ccccacagca acagaggcac agcttcaagg gggaggagtg tccagcagga 300

tctcatagat cagaacatac tggagcctgt aacccgtgca cagagggtgt ggattacacc 360

aacgcttcca acaatgaacc ttcttgcttc ccatgtac 398

<210> 16
<211> 733
<212> DNA
<213> Homo sapiens

<400> 16

```
gggatccgga gcccaaatct tctgacaaaa ctcacacatg cccaccgtgc ccagcacctg 60
aattcgaggg tgcaccgtca gtcttcctct tccccccaaa acccaaggac accctcatga 120
tctcccggac tcctgaggtc acatgcgtgg tggtggacgt aagccacgaa gaccctgagg 180
tcaagttcaa ctggtacgtg gacggcgtgg aggtgcataa tgccaagaca aagccgcggg 240
aggagcagta caacagcacg taccgtgtgg tcagcgtcct caccgtcctg caccaggact 300
ggctgaatgg caaggagtac aagtgcaagg tctccaacaa agccctccca acccccatcg 360
agaaaaccat ctccaaagcc aaagggcagc cccgagaacc acaggtgtac accctgcccc 420
catcccggga tgagctgacc aagaaccagg tcagcctgac ctgcctggtc aaaggcttct 480
atccaagcga catcgccgtg gagtgggaga gcaatgggca gccggagaac aactacaaga 540
ccacgcctcc cgtgctggac tccgacggct ccttcttcct ctacagcaag ctcaccgtgg 600
acaagagcag gtggcagcag gggaacgtct tctcatgctc cgtgatgcat gaggctctgc 660
acaaccacta cacgcagaag agcctctccc tgtctccggg taaatgagtg cgacggccgc 720
gactctagag gat                                                   733
```

1

13